# EUROPEAN PATENT SPECIFICATION

(11) **EP 3 794 079 B1**
(45) Date of publication and mention of the grant of the patent: **21.08.2024**
(21) Application number: 19803564.4
(22) Date of filing: 17.05.2019
(51) Int. Cl.: A61L 15/24, A61L 15/42, A61L 15/46, A61L 27/34, A61L 27/50, A61L 27/54, A61L 29/08, A61L 29/14, A61L 29/16, A61L 31/10, A61L 31/14, A61L 31/16, B05D 5/08, C09D 5/00, C09D 7/65, B05D 7/02, B05D 7/22

(54) **SURFACES RESISTANT TO BACTERIAL ADHESION**
GEGEN BAKTERIELLE ADHÄSION RESISTENTE OBERFLÄCHEN
SURFACES RÉSISTANTES À L'ADHÉRENCE BACTÉRIENNE

(30) Priority: 18.05.2018 US 201862673490 P
(43) Date of publication of application: 24.03.2021
(73) Proprietor: Evonik Canada Inc., Burlington, Ontario L7N3J5 (CA)
(72) Inventor: PIOTROWICZ, Alexandra, Mississauga, Ontario L5A 2M3 (CA); MACDONALD, Kyle William, Stittsville, Ontario, K2S 0M1 (CA); CILLERO RODRIGO, Antonio, Toronto, Ontario M4Y 3C4 (CA); MULLICK, Sanjoy, Brampton, Ontario L7A 1Y5 (CA); SWENOR, Jamie Robert, Toronto, Ontario M5R 2R3 (CA); SANTERRE, J. Paul, Toronto, Ontario M4Y 1G3 (CA); HO, Jeannette, Toronto, Ontario M4K 1Z1 (CA)
(74) Representative: Evonik Patent Association
(86) International application number: PCT/CA2019/050683
(87) International publication number: WO 2019/218088

(56) References cited:
- WO-A1-2017/195035
- WO-A1-2018/218347
- CA-A1- 2 228 505
- CA-A1- 2 672 593
- CA-A1- 2 971 113
- MCCLOSKEY ET AL.: "Effect of Fluorinated Surface-Modifying Macromolecules on the Molecular Surface Structure of a Polyether Poly(urethane urea", MACROMOLECULES, vol. 35, 2002, pages 924 - 933, XP055653380

## Description

### Background

The invention features surfaces resistant to bacterial adhesion.

Bacteria generally exist in one of two types of populations: planktonic, freely existing in bulk solution, and sessile, as a unit attached to a surface or within the confines of a biofilm. A biofilm is attached to a substrate and consists of many bacteria co-adhered by means of physical appendages and extracellular polymeric substances. Bacterial biofilms are the root cause of biofouling in most biomedical and industrial systems.

Implantable or insertable medical devices frequently occlude due to microbial colonization and adhesion. This problem is particularly prevalent with medical devices that are adapted to remain implanted for a relatively long-term, i.e., from about 30 days to about 12 months or longer. Microbes, such as bacteria, often colonize on and around the medical device and, upon attaching to surfaces of the device, proliferate and form aggregates within a complex matrix consisting of extracellular polymeric substances, typically polysaccharides. The mass of attached microorganisms and the associated extracellular polymeric substances is commonly referred to as a biofilm or slime. Antimicrobial agents have difficulty penetrating biofilms and killing and/or inhibiting the proliferation of the microorganisms within the biofilm. The colonization of the microbes on and around the device and the synthesis of the biofilm barrier eventually result in encrustation, occlusion, failure of the device, and local or systemic infection.

WO 2017/195035 A1 discloses an implantable glucose sensor, which comprises a glucose detector and an enclosure comprising semipermeable biointerface film, wherein the semipermeable biointerface film comprises a base polymer and a biostabilizing additive, wherein the biostable surface exhibits reduced protein and cell deposition as compared to a reference film that differs from the semipermeable biointerface film only by the absence of the biostabilizing additive in the reference film.

CA 2971113 A1 discloses surface-modifying macromolecules used in admixture with base polymers for industrial and medical applications requiring enhanced surface properties such as reducing or preventing biofouling.

One way to enhance the management of biofouling of systems would be to inhibit or compromise bacterial adhesion to surfaces within such systems.

### Summary of the Invention

The invention refers to specific uses of a composition comprising a base polymer admixed with 0.05% (w/w) to 15% (w/w) of a specific compound of any one of formulas (I)-(XXI) as set out in the independent claims.

In one aspect, the invention features a specific use of reducing bacterial adhesion to a polymeric surface. One use includes admixing a base polymer with a compound of any formulas (I)-(XXI) to form the polymeric surface, where bacterial adhesion is reduced by at least 50% relative to the surface formed from the base polymer in the absence of the compound, (e.g., 55%, 60%, 65%, 70%, 75%, 80%, 85%, 90%, 95%, or 99%).

In another aspect, the invention features a use of reducing bacterial adhesion to a polymeric surface under flow conditions. One use includes (i) providing the polymeric surface formed from an admixture comprising a base polymer admixed with a compound of any formulas (I)-(XXI), and (ii) subjecting the polymeric surface to flow conditions, where bacterial adhesion is reduced by at least 50% under flow conditions relative to the surface formed from the base polymer in the absence of the compound (e.g., 55%, 60%, 65%, 70%, 75%, 80%, 85%, 90%, 95%, or 99%). In particular embodiments, the flow conditions include an aqueous solution having a shear rate of from 1 s⁻¹ to 3500 s⁻¹ (e.g., from 1 s⁻¹ to 15 s⁻¹, from 5 s⁻¹ to 100 s⁻¹, from 50 s⁻¹ to 500 s⁻¹, from 500 s⁻¹ to 1000 s⁻¹, or from 950 s⁻¹ to 3500 s⁻¹).

In an aspect, the invention features a use of reducing bacterial adhesion to a polymeric surface under static aqueous conditions. One use includes (i) providing the polymeric surface formed from an admixture comprising a base polymer admixed with a compound of any formulas (I)-(XXI), and (ii) subjecting the polymeric surface to static aqueous conditions, where bacterial adhesion is reduced by at least 50% under static aqueous conditions relative to the surface formed from the base polymer in the absence of the compound (e.g., at least 55%, 60%, 65%, 70%, 75%, 80%, 85%, 90%, 95%, or 99%).

In particular embodiments of the above uses, the use includes reducing bacterial adhesion while contacting the polymeric surface with a protein-containing aqueous mixture.

In another aspect, the invention features a use of reducing bacterial adhesion to a polymeric surface exposed to ambient air. One use includes (i) providing the polymeric surface formed from an admixture comprising a base polymer admixed with a compound of any formulas (I)-(XXI), and (ii) subjecting the polymeric surface to ambient air conditions, where bacterial adhesion is reduced by at least 50% under ambient air conditions relative to the surface formed from the base polymer in the absence of the compound (e.g., at least 55%, 60%, 65%, 70%, 75%, 80%, 85%, 90%, 95%, or 99%).

In one aspect, the invention features a use of reducing bacterial adhesion to a blood dwelling polymeric surface. One use includes (i) providing the polymeric surface formed from an admixture comprising a base polymer admixed with a compound of any formulas (I)-(XXI), and (ii) contacting said polymeric surface with blood, where bacterial adhesion is reduced by at least 50% under blood dwelling conditions relative to the surface formed from the base polymer in the absence of the compound (e.g., at least 55%, 60%, 65%, 70%, 75%, 80%, 85%, 90%, 95%, or 99%).

In another aspect, the invention features a use of reducing bacterial adhesion to a urine dwelling polymeric surface. One use includes (i) providing the polymeric surface formed from an admixture comprising a base polymer admixed with a compound of any formulas (I)-(XXI), and (ii) contacting said polymeric surface with urine, where bacterial adhesion is reduced by at least 50% under urine dwelling conditions relative to the surface formed from the base polymer in the absence of the compound (e.g., at least 55%, 60%, 65%, 70%, 75%, 80%, 85%, 90%, 95%, or 99%).

In an aspect, the invention features a use of reducing bacteria mediated salt formation on a polymeric surface of a urine dwelling device. One use includes (i) providing the polymeric surface formed from an admixture comprising a base polymer admixed with a compound of any formulas (I)-(XXI), and (ii) contacting said polymeric surface with urine, where salt deposition is reduced by at least 20% under urine dwelling conditions relative to the surface formed from the base polymer in the absence of the compound (e.g., at least 25%, 30%, 35%, 40%, 45%, 50%, 55%, 60%, 65%, 70%, 75%, 80%, 85%, 90%, 95%, or 99%).

In another aspect, the invention features a use of reducing bacterial biofilm formation on a polymeric surface. One use includes providing the polymeric surface formed from an admixture comprising a base polymer admixed with a compound of any formulas (I)-(XXI), where bacterial biofilm formation is reduced by at least 20% relative to the surface formed from the base polymer in the absence of the compound (e.g., at least 25%, 30%, 35%, 40%, 45%, 50%, 55%, 60%, 65%, 70%, 75%, 80%, 85%, 90%, 95%, or 99%).

In one aspect, the invention features a use of reducing the bacterial bioburden on a polymeric surface. One use includes admixing a base polymer with a compound of any formulas (I)-(XXI) to form the polymeric surface, where bacterial bioburden is reduced by at least 20% relative to the surface formed from the base polymer in the absence of the compound (e.g., at least 25%, 30%, 35%, 40%, 45%, 50%, 55%, 60%, 65%, 70%, 75%, 80%, 85%, 90%, 95%, or 99%).

In another aspect, the invention features a use of reducing bacterial growth rate on a surface, one use including (i) providing a surface including a base polymer admixed with a compound of any formulas (I)-(XXI), and (ii) contacting the surface with an antimicrobial, antiseptic, or disinfectant, wherein the bacterial growth rate is reduced by at least 50% (e.g., at least 55%, 60%, 65%, 70%, 75%, 80%, 85%, 90%, 95%, or 99%) relative to the surface formed from the base polymer in the absence of the compound. In particular embodiments, at least 6 hours, 12 hours, or 24 hours following step (ii), the bacterial growth rate is reduced by at least 50% (e.g., at least 55%, 60%, 65%, 70%, 75%, 80%, 85%, 90%, 95%, or 99%) relative to the surface formed from the base polymer in the absence of the compound.

In one aspect, the invention features a use of reducing bacterial adhesion on a surface, one use including (i) providing a surface including a base polymer admixed with a compound of any formulas (I)-(XXI), and (ii) contacting the surface with an antimicrobial, antiseptic, or disinfectant, wherein the bacterial adhesion is reduced by at least 50% (e.g., at least 55%, 60%, 65%, 70%, 75%, 80%, 85%, 90%, 95%, or 99%) relative to the surface formed from the base polymer in the absence of the compound. In particular embodiments, at least 6 hours, 12 hours, or 24 hours following step (ii), the bacterial adhesion is reduced by at least 50% (e.g., at least 55%, 60%, 65%, 70%, 75%, 80%, 85%, 90%, 95%, or 99%) relative to the surface formed from the base polymer in the absence of the compound.

In another aspect, the invention features a use for reducing the rate of biofilm formation on a polymeric surface, one use including (i) providing a surface including a base polymer admixed with a compound of any formulas (I)-(XXI); and (ii) contacting the surface with an antimicrobial agent, antiseptic or disinfectant, wherein the rate of biofilm formation is reduced by at least 20% (e.g., at least 25%, 30%, 35%, 40%, 45%, 50%, 55%, 60%, 65%, 70%, 75%, 80%, 85%, 90%, 95%, or 99%) relative to the surface formed from the base polymer in the absence of the compound. In particular embodiments, at least 6 hours, 12 hours, or 24 hours following step (ii), the rate of biofilm formation is reduced by at least 20% (e.g., at least 25%, 30%, 35%, 40%, 45%, 50%, 55%, 60%, 65%, 70%, 75%, 80%, 85%, 90%, 95%, or 99%) relative to the surface formed from the base polymer in the absence of the compound.

In an embodiment of any of the above uses, the polymeric surface can be in an ex vivo environment. For example, the polymeric surface can be a surface on a cooling tower, pump, heat exchanger, pipeline, heating system, fuel tank, pharmaceutical equipment, waste water treatment system, water purification system, cooling system, bioreactor, food processing system, scrubbing system, metal working fluid, papermaking equipment, ship hull, or textile manufacture equipment.

In the above uses, the polymeric surface can be in an in vivo environment.

In the above uses, the polymeric surface can be a surface of a medical device.

In the above uses, the polymeric surface of the medical device can further include a filler, a radiopaque material (e.g., barium sulfate), a colorant, or an antimicrobial agent. For example, the polymeric surface of the medical device can include from 1% to 45% (w/w) (e.g., 1% to 10% (w/w), 10% to 30% (w/w), 20% to 40% (w/w), 25% to 45% (w/w), 20% to 35% (w/w), 25% to 40% (w/w), 30% to 45% (w/w), or 35% to 45% (w/w)) of barium sulfate filler. In other embodiments, the polymeric surface of the medical device does not include barium sulfate filler. When the medical device includes an antimicrobial agent, the antimicrobial agent can be any antimicrobial agent described herein.

In one aspect, the invention features a use for reducing the amount of bacterial adhesion on a polymeric surface of an implanted medical device, one use including inserting into the subject a medical device, wherein the medical device includes a surface including (i) an antimicrobial agent and (ii) a base polymer admixed with a compound of any formulas (I)-(XXI), wherein at least 24 hours following step (ii), the amount of bacterial adhesion is reduced by at least 50% relative to the surface formed from the base polymer in the absence of the compound.

In another aspect, the invention features a use for reducing the amount of bacterial adhesion on a polymeric surface of an implanted medical device, one use including (i) administering to the subject an antimicrobial agent; and (ii) inserting into the subject a medical device, wherein the medical device includes a surface including a base polymer admixed with a compound of any formulas (I)-(XXI), wherein at least 6 hours, 12 hours, or 24 hours (e.g., 36 hours, 48 hours, or 72 hours) following step (ii), the amount of bacterial adhesion is reduced by at least 50% (e.g., at least 55%, 60%, 65%, 70%, 75%, 80%, 85%, 90%, 95%, or 99%) relative to the surface formed from the base polymer in the absence of the compound.

In another aspect, the invention features a use for reducing the amount of bacteria adhesion on a polymeric surface of an implanted medical device, one use including (i) providing a subject that is undergoing treatment with an antimicrobial agent; and (ii) inserting into the subject a medical device, wherein the medical device includes a surface including a base polymer admixed with a compound of any formulas (I)-(XXI), wherein at least 6 hours, 12 hours, or 24 hours (e.g., 36 hours, 48 hours, or 72 hours) hours following step (ii), the amount of bacterial adhesion is reduced by at least 50% (e.g., at least 55%, 60%, 65%, 70%, 75%, 80%, 85%, 90%, 95%, or 99%) relative to the surface formed from the base polymer in the absence of the compound.

In an embodiment of any of the above uses, the polymeric surface is a coating on a substrate. In particular embodiments, the substrate is a medical device. The medical device can be partially or fully implanted, or contacted with the body of a subject for a limited period of time (e.g., fewer than 48 hours, 24 hours, 12 hours, or 4 hours).

In an embodiment of any of the above uses, the polymeric surface is a surface on a medical or biotechnology product such as wound dressings, bandages, gauzes, tapes, pads, sponges, blood oxygenators, ventilators, pumps, tubing, wiring, electrodes, contraceptive devices, feminine hygiene products, endoscopes, dialysis membranes, guide wires, fluid collection bags, drug delivery bags and tubing, feeding tubes, blood bags, and tissue regeneration or cell culture devices.

In the above uses, the polymeric surface can be blood dwelling, urine dwelling, and/or the polymeric surface can be in contact with a proteinaceous environment. When the polymeric surface is the surface of a medical device, the medical device can be selected from medical instruments, dental devices, dental implants, drug delivery devices, grafts, stents, pacemakers, implantable cardioverter-defibrillators, cardiac resynchronization therapy devices, cardiovascular device leads, ventricular assist devices and drivelines, heart valves, vena cava filters, endovascular coils, catheters, catheter connectors, catheter valves, intravenous delivery lines, intravenous delivery manifolds, shunts, wound drains, drainage catheters, infusion ports, cochlear implants, endotracheal tubes, tracheostomy tubes, ventilator breathing tubes and circuits, implantable sensors, ophthalmic devices, orthopedic devices, dental implants, periodontal implants, breast implants, penile implants, maxillofacial implants, cosmetic implants, valves, appliances, scaffolding, suturing material, needles, hernia repair meshes, tension-free vaginal tape and vaginal slings, prosthetic neurological devices, and ear tubes.

In particular embodiments, (i) the polymeric surface is in a subject that is undergoing treatment with less than a standard regimen of the antimicrobial agent, and (ii) the polymeric surface and the antimicrobial are each present in an amount that together is sufficient to reduce bacterial adhesion on the polymeric surface relative to the subject receiving the antimicrobial treatment in the presence of a polymeric surface that does not contain the compound. In particular embodiments, the subject is receiving antimicrobial therapy as prophylaxis against infection in conjunction with a medical procedure (e.g., insertion of a medical device), and the risk of infection is reduced by including the compound in the polymeric surface.

In certain embodiments, (i) the polymeric surface is in a subject that is undergoing treatment with less than a standard regimen of the antimicrobial agent, and (ii) the polymeric surface and the antimicrobial are each present in an amount that together is sufficient to reduce the risk of infection in the subject relative to the subject receiving the antimicrobial treatment in the presence of a polymeric surface that does not contain the compound. In particular embodiments, the subject is receiving antimicrobial therapy as prophylaxis against infection in conjunction with a medical procedure (e.g., insertion of a medical device), and the risk of infection is reduced by including the compound in the polymeric surface.

In particular embodiments of any of the above uses, the polymeric surface is a surface of an article, material or device, medical or not, which is to maintain a human or animal health-safe unfouled state of cleanliness over a period of time. The article, material or device can be any article, material or device described herein.

In some embodiments, the antimicrobial is silver, penicillin G, penicillin V, methicillin, oxacillin, cloxacillin, dicloxacillin, nafcillin, ampicillin, amoxicillin, carbenicillin, ticarcillin, mezlocillin, piperacillin, azlocillin, temocillin, cepalothin, cephapirin, cephradine, cephaloridine, cefazolin, cefamandole, cefuroxime, cephalexin, cefprozil, cefaclor, loracarbef, cefoxitin, cefmatozole, cefotaxime, ceftizoxime, ceftriaxone, cefoperazone, ceftazidime, cefixime, cefpodoxime, ceftibuten, cefdinir, cefpirome, cefepime, BAL5788, BAL9141, imipenem, ertapenem, meropenem, astreonam, clavulanate, sulbactam, tazobactam, streptomycin, neomycin, kanamycin, paromycin, gentamicin, tobramycin, amikacin, netilmicin, spectinomycin, sisomicin, dibekalin, isepamicin, tetracycline, chlortetracycline, demeclocycline, minocycline, oxytetracycline, methacycline, doxycycline, erythromycin, azithromycin, clarithromycin, telithromycin, ABT-773, lincomycin, clindamycin, vancomycin, oritavancin, dalbavancin, teicoplanin, quinupristin and dalfopristin, sulphanilamide, para-aminobenzoic acid, sulfadiazine, sulfisoxazole, sulfamethoxazole, sulfathalidine, linezolid, nalidixic acid, oxolinic acid, norfloxacin, perfloxacin, enoxacin, ofloxacin, ciprofloxacin, temafloxacin, lomefloxacin, fleroxacin, grepafloxacin, sparfloxacin, trovafloxacin, clinafloxacin, gatifloxacin, moxifloxacin, gemifloxacin, sitafloxacin, metronidazole, daptomycin, garenoxacin, ramoplanin, faropenem, polymyxin, triclosan, rifampin, minocycline, tigecycline, AZD2563, trimethoprim, or combinations thereof.

In some embodiments, the disinfectant or antiseptic is formaldehyde, glutaraldehyde, ortho-phthalaldehyde, hydrogen peroxide, peracetic acid, hydrogen peroxide/peracetic acid combination, sodium hypochlorite, iodophors (e.g., povidone iodine), chlorhexidine, isopropyl alcohol, phenols, quaternary ammonium compounds, or combinations thereof.

In any embodiments of the foregoing uses, the compound is any one of compounds 1-57 (as depicted in the Figures), or the compound has the formula of any one of SMM 1 - SMM 16 (as described in Table 1). The polymeric surface includes from 0.05% (w/w) to 15% (w/w) (e.g., from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w)) of the compound of any formulas (I)-(XXI), e.g., any one of compounds 1-57 and/or a compound having the formula of any one of SMM 1 - SMM 16.

In any embodiments of the foregoing uses, the base polymer is selected from the group including silicone, polyolefin, polyester, polycarbonate, polysulfone, polyamide, polyether, polyurea, polyurethane (PU), polyetherimide, polystyrene, cellulosic polymer, polypropylene (PP), polyethylene (PE), polyvinylchloride (PVC), polyvinyl alcohol (PVA), polyvinylpyrrolidone (PVP), polyacrylamide (PAAM), polyethylene oxide (PEO), polyethylene oxide)-b-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyether-b-polyamide, a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), poly(styrene-*block*-isobutylene-*block*-styrene) (SIBS), and ethylene-vinyl acetate (EVA),.

In any embodiments of the foregoing uses, the base polymer is a polyurethane (PU). The PU base polymer can be any PU base polymer described herein. In particular embodiments of the foregoing uses, the base polymer is silicone (SI). The silicone base polymer can be any silicone base polymer described herein.

In a related aspect a medical device, not included in the invention, includes a surface including (i) an antimicrobial agent and (ii) a base polymer admixed with a compound of any formulas (I)-(XXI). In any embodiments of the foregoing medical devices, the compound is any one of compounds 1-57. In any embodiment of the foregoing medical devices, the compound has the formula of any one of SMM 1 - SMM 16. The surface includes from 0.05% (w/w) to 15% (w/w) (e.g., from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w)) of the compound of any formulas (I)-(XXI), e.g., any one of compounds 1-57 and/or a compound having the formula of any one of SMM 1 - SMM 16. In an embodiment of the foregoing medical devices, the base polymer is selected from any base polymer described in the above uses or described herein. In another embodiment of the foregoing medical devices, the antimicrobial agent is selected from any antimicrobial agent described in the above uses or described herein. In particular embodiments, the medical device is an implantable medical device (e.g., a catheter) or a wound dressing. The medical device can contain a polymeric surface of the invention that further includes silver, triclosan, rifampin, minocycline, or a combination thereof.

In some embodiments of any of the preceding aspects, the compound of any formulas (I)-(XXI) has the formula of SMM 1 and the base polymer is PU or SI (e.g., wherein the compound is compound 40, 45, or 54). In other embodiments, the compound of any formulas (I)-(XXI) has the formula of SMM 2 the base polymer is PU or SI (e.g., wherein the compound is compound 56). In other embodiments, the compound of any formulas (I)-(XXI) has the formula of SMM 3 the base polymer is PU or SI (wherein the compound is compound 57). In further embodiments, the compound of any formulas (I)-(XXI) has the formula of SMM 4 the base polymer is PU or SI (e.g., wherein the compound is compound 2).

In other embodiments of any of the preceding aspects, the compound of any formulas (I)-(XXI) has the formula of SMM 5 the base polymer is PU or SI (e.g., wherein the compound is compound 37 or 38). In still other embodiments, the compound of any formulas (I)-(XXI) has the formula of SMM 6 the base polymer is PU or SI (e.g., wherein the compound is compound 11). In further embodiments, the compound of any formulas (I)-(XXI) has the formula of SMM 7 the base polymer is PU or SI (e.g., wherein the compound is compound 1). In still further embodiments, the compound of any formulas (I)-(XXI) has the formula of SMM 8 the base polymer is PU or SI (e.g., wherein the compound is compound 44). In yet other embodiments, the compound of any formulas (I)-(XXI) has the formula of SMM 9 the base polymer is PU or SI (e.g., wherein the compound is compound 21).

In still further embodiments of any of the preceding aspects, the compound of any formulas (I)-(XXI) has the formula of SMM 10 the base polymer is PU or SI (e.g., wherein the compound is compound 22 or 39). In some embodiments, the compound of any formulas (I)-(XXI) has the formula of SMM 11 the base polymer is PU or SI (e.g., wherein the compound is compound 24). In some embodiments, the compound of any formulas (I)-(XXI) has the formula of SMM 12 the base polymer is PU or SI (e.g., wherein the compound is compound 18). In further embodiments, the compound of any formulas (I)-(XXI) has the formula of SMM 13 the base polymer is PU or SI (e.g., wherein the compound is compound 20). In other embodiments, the compound of any formulas (I)-(XXI) has the formula of SMM 14 the base polymer is PU or SI (e.g., wherein the compound is compound 13).

In yet further embodiments of any of the preceding aspects, the compound of any formulas (I)-(XXI) has the formula of SMM 15 the base polymer is PU or SI (e.g., wherein the compound is compound 55). In some embodiments, the compound of any formulas (I)-(XXI) has the formula of SMM 16 the base polymer is PU or SI (e.g., wherein the compound is compound 43).

For any one of the compounds of any formulas (I)-(XXI), e.g., a compound having the formula of any one of SMM 1 - SMM 16, F_{T} can be a polyfluoroorgano group having a theoretical molecular weight of from 100 Da to 1,500 Da. For example, F_{T} is CF₃(CF₂)ᵣ(CH₂CH₂)ₚ- wherein p is 0 or 1, r is 2-20, and CF₃(CF₂)ₛ(CH₂CH₂O)_{X}, where X is from 0 to 10 and s is from 1 to 20. F_{T} may also be CHₘF₍₃₋ₘ₎(CF₂)ᵣCH₂CH₂- or CHₘF₍₃₋ₘ₎(CF₂)ₛ(CH₂CH₂O)_{X}-, where m is 0, 1, 2, or 3; X is an integer from 0 to 10; r is an integer from 2 to 20; and s is an integer from 1 to 20. In other embodiments, F_{T} is 1H,1H,2H,2H-perfluoro-1-decanol; 1H,1H,2H,2H-perfluoro-1-octanol; 1H,1H,5H-perfluoro-1-pentanol; or 1H,1H-perfluoro-1-butanol, or a mixture thereof.

The polymeric surface includes from 0.05% (w/w) to 15% (w/w) (e.g., from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w)) of the compound of any formulas (I)-(XXI), e.g., any one of compounds 1-57 and/or a compound having the formula of any one of SMM 1 - SMM 16.

In another aspect, the invention refers to a specific use of a composition including a base polymer admixed with a compound of any one of formulas (I)-(XXI), wherein the composition is useful (e.g., for reducing bacterial adhesion) in the uses of the invention. In particular embodiments, the composition includes 0.05% (w/w) to 15% (w/w) (e.g., from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w)) of the compound of any formulas (I)-(XXI), e.g., any one of compounds 1-57 and/or a compound having the formula of any one of SMM 1 - SMM 16.

Further described herein is an article, not included in the invention, including a composition of the invention. In some embodiments the article is: (a) a medical device is selected from medical instruments, dental devices, dental implants, drug delivery devices, grafts, stents, pacemakers, implantable cardioverter-defibrillators, cardiac resynchronization therapy devices, cardiovascular device leads, ventricular assist devices and drivelines, heart valves, vena cava filters, endovascular coils, catheters, catheter connectors, catheter valves, intravenous delivery lines, intravenous delivery manifolds, shunts, wound drains, drainage catheters, infusion ports, cochlear implants, endotracheal tubes, tracheostomy tubes, ventilator breathing tubes and circuits, implantable sensors, ophthalmic devices, orthopedic devices, dental implants, periodontal implants, breast implants, penile implants, maxillofacial implants, cosmetic implants, valves, appliances, scaffolding, suturing material, needles, hernia repair meshes, tension-free vaginal tape and vaginal slings, prosthetic neurological devices, and ear tubes, or (b) a wound dressing, a bandage, a gauze, a tape, a pad, a sponge, a blood oxygenator, a ventilator, a pump, tubing, wiring, an electrode, a contraceptive device, a feminine hygiene product, an endoscope, a dialysis membrane, a guide wire, a fluid collection bag, a drug delivery bag and tubing, a feeding tube, a blood bag, or a tissue regeneration or cell culture devices. For example, the article can be a catheter, a drainage catheter, a stent, a shunt, an infusion port, an intravenous delivery line, a dental device, a blood bag, a breast implant, a penile implant, a wound drain, a feeding tube, an endotracheal tube, a breathing tube, an ear tube, an endoscope, or a feminine hygiene product. In particular embodiments, the article is a catheter comprising a base polymer that is PU, SI, or polyvinyl chloride, where the base polymer is admixed with a compound . In certain embodiments, the article is an infusion port comprising a base polymer that is PU or SI, where the base polymer is admixed with a compound . In other embodiments, the article is a stent comprising a base polymer that is PU, SI, or polyethylene, where the base polymer is admixed with a compound . In certain embodiments, the article is a shunt comprising a base polymer that is PU or SI, where the base polymer is admixed with a compound. In some embodiments, the article is a tube (e.g., an ear tube, endotracheal tube, breathing tube, or feeding tube) comprising a base polymer that is PU, SI, polyethylene, or polyvinylchloride, where the base polymer is admixed with a compound . In certain embodiments, the article is an intravenous delivery line comprising a base polymer that is PU, where the base polymer is admixed with a compound. In particular embodiments, the article is a blood bag comprising a base polymer that is polyvinylchloride, polyethylene, or polypropylene, where the base polymer is admixed with a compound. In certain embodiments, the article is a prosthetic implant (e.g., a breast implant or a penile implant) comprising a base polymer that is SI, where the base polymer is admixed with a compound. In some embodiments, the article is an orthodontic aligner or orthodontic appliance comprising a base polymer that is PU or a polyacrylate, where the base polymer is admixed with a compound. In certain embodiments, the article is a wound drain comprising a base polymer that is PU, SI, or polyvinylchloride, where the base polymer is admixed with a compound. In particular embodiments, the article is an endoscope comprising a base polymer that is PU or polyethylene, where the base polymer is admixed with a compound. In certain embodiments, the article is a feminine hygiene product comprising a base polymer that is PU or SI, where the base polymer is admixed with a compound. In some embodiments, the article is a metallic stent coated with a base polymer (e.g., a base polymer that is PU, SI, or polyethylene), where the base polymer is admixed with a compound in the coating on the metallic stent (e.g., a coronary, ureteral, renal, biliary, colorectal, esophageal, pulmonary, urethral, or vascular stent). In any of the above embodiments, the base polymer is admixed with a compound of any one of formulas (I)-(XXI), wherein the composition includes 0.05% (w/w) to 15% (w/w) (e.g., from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w)) of the compound of any formulas (I)-(XXI), e.g., any one of compounds 1-57 and/or a compound having the formula of any one of SMM 1 - SMM 16.

It is disclosed a method of forming an article not according to the invention, wherein the method includes (i) preparing a composition including a base polymer admixed with a compound of any one of formulas (I)-(XXI), and (ii) processing (e.g., extruding, injection molding, calendaring, mixing, spraying, dipping, solution fiber spinning, electrospinning, or casting) the composition to form or to coat the article.

### Definitions

The term "about," as used herein, refers to a value that is ±20% of the recited number.

The term "additive," as used herein, refers to a segmented compound of any one of formulae (I), (II), (III), (IV), (V), (VI), (VII), (VIII), (IX), (X), (XI), (XII), (XIII), (XIV), (XV), (XVI), (XVII), (XVIII), (XIX), (XX), and (XXI). Certain additives can have a theoretical molecular weight of less than or equal to 20 kDa (e.g., less than or equal to 10 kDa). Certain additives can have a theoretical molecular weight of greater than or equal to 200 Da (e.g., greater than or equal to 300 Da). Non-limiting examples of additives include those having a theoretical molecular weight of from 500 to 10,000 Daltons, from 500 to 9,000 Daltons, from 500 to 5,000 Daltons, from 1,000 to 10,000 Daltons, from 1,000 to 6,000 Daltons, or from 1,500 to 8,000 Daltons. One of skill in the art will recognize that these structural formulae represent idealized theoretical structures. Specifically, the segments are reacted in specific stoichiometries to furnish an additive as a distribution of molecules having varying ratios of segments. Accordingly, the variable n in formulae (I)-(XXI) indicates the theoretical stoichiometry of the segments.

The term "ambient air" refers to atmospheric air in its natural state, not contaminated by air-borne pathogens.

The term "bacterial adhesion" refers to a process that allows bacteria to attach or adhere to other cells and surfaces. Adhesion is an important step for colonization of a new host or environment and can contribute to bacterial pathogenesis. The methods can reduce bacterial adhesion on a polymeric surface by at least 50%, e.g., at least 55%, 60%, 65%, 70%, 75%, 80%, 85%, 90%, 95%, or 99%.

The term "base polymer," as used herein, refers to a polymer having a theoretical molecular weight of greater than or equal to 20 kDa (e.g., greater than or equal to 50 kDa, greater than or equal to 75 kDa, greater than or equal to 100 kDa, greater than or equal to 150 kDa, or greater than 200 kDa). Non-limiting examples of base polymers include: silicone, polyolefin, polyester, polycarbonate, polysulfone, polyamide, polyether, polyurea, polyurethane, polyetherimide, cellulosic polymer, and copolymers thereof, and blends thereof. Further non-limiting examples of the base polymers include polypropylene (PP), polyethylene (PE), polyvinylchloride (PVC), polyvinyl alcohol (PVA), polyvinylpyrrolidone (PVP), polyacrylamide (PAAM), polyethylene oxide (PEO), polyethylene oxide)-*b*-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyether-b-polyamide (e.g., PEBAX), a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), poly(styrene-*block*-isobutylene-*block*-styrene) (SIBS), ethylene-vinyl acetate (EVA), and copolymers thereof, and blends thereof.

The term "bioburden" refers to a degree or amount of microbial contamination or microbial load on a surface. The methods can reduce bacterial bioburden on a polymeric surface by at least 20%, e.g., at least 25%, 30%, 35%, 40%, 45%, 50%, 55%, 60%, 65%, 70%, 75%, 80%, 85%, 90%, 95%, or 99%.

The term "biofilm" refers to the mass of microorganisms attached to a surface, such as a surface of a medical device, and the associated extracellular substances produced by one or more of the attached microorganisms. The extracellular substances are typically polymeric substances and commonly include a matrix of complex polysaccharides, proteinaceous substances and glycopeptides. The methods can reduce bacterial biofilm formation on a polymeric surface by at least 20%, e.g., at least 25%, 30%, 35%, 40%, 45%, 50%, 55%, 60%, 65%, 70%, 75%, 80%, 85%, 90%, 95%, or 99%.

The term "blood dwelling polymeric surface" refers to a polymeric surface that is in contact with blood, e.g., surgical, medical, diagnostic, or dental instruments, dental devices or implants, tubing, wiring, feminine hygiene products, grafts (including small diameter<6 mm), stents (including coronary, ureteral, renal, biliary, colorectal, esophageal, pulmonary, urethral, and vascular), stent grafts (including abdominal, thoracic, and peripheral vascular), pacemakers, implantable cardioverter-defibrillators, cardiac resynchronization therapy devices, cardiovascular device leads, ventricular assist devices and drivelines, heart valves, vena cava filters, endovascular coils, catheters, intravenous delivery lines and manifolds, shunts, (internal or external including ventricular, ventriculoatrial, ventriculoperitoneal, and lumboperitoneal), wound drains, drainage catheters, dialysis membranes, infusion ports, cochlear implants, endotracheal tubes, tracheostomy tubes, ventilator breathing tubes and circuits, guide wires, fluid collection bags, drug delivery bags and tubing, implantable sensors (e.g., intravascular, transdermal, intracranial), ophthalmic devices including contact lenses, orthopedic devices (including hip implants, knee implants, shoulder implants, spinal implants (including cervical plates systems, pedicle screw systems, interbody fusion devices, artificial disks, and other motion preservation devices).

As used herein, "C" refers to a chain terminating group. Chain terminating groups include monofunctional groups containing an amine, alcohol, or carboxylic acid functionality.

The term "carbonate linkage," as used herein, refers to an ester of carbonic acid.

The term "flow conditions" refers to conditions under which a polymeric surface is subjected to flow of a liquid, e.g., urine or blood, at a rate of at least 0.1 mL/min (e.g., 0.2 mL/min, 0.3 mL/min, 0.4 mL/min, 0.5 mL/min, 0.6 mL/min, 0.7 mL/min, 0.8 mL/min, 0.9 mL/min, or 1.0 mL/min). Testing and evaluation can be conducted under flow conditions as described in Example 12.

The term "LinkB," as used herein, refers to a coupling segment linking two oligomeric segments and a surface-active group. Typically, LinkB has a molecular weight ranging from 40 to 700. LinkB is selected from the group of functionalized diamines, diisocyanates, disulfonic acids, dicarboxylic acids, diacid chlorides, and dialdehydes, where the functionalized component has secondary functional group, through which a surface-active group is attached. Such secondary functional groups can be esters, carboxylic acid salts, sulfonic acid salts, phosphonic acid salts, thiols, vinyls, and primary or secondary amines. Terminal hydroxyls, amines, or carboxylic acids of an oligomeric segment intermediate can react with a diamine to form an oligo-amide; react with a diisocyanate to form an oligo-urethane, an oligo-urea, or an oligo-amide; react with a disulfonic acid to form an oligo-sulfonate or an oligo-sulfonamide; react with a dicarboxylic acid to form an oligo-ester or an oligo-amide; react with a diacyl dichloride to form an oligo-ester or an oligo-amide; or react with a dicarboxaldehyde to form an oligo-acetal or an oligo-imine.

The term "linker with two terminal carbonyls," as used herein, refers to a divalent group having a molecular weight of between 56 Da and 1,000 Da, in which the first valency belongs to a first carbonyl, and a second valency belongs to a second carbonyl. Within this linker, the first carbonyl is bonded to a first carbon atom, and the second carbonyl is bonded to a second carbon atom. The linker with two terminal carbonyls can be a small molecule dicarbonyl (e.g., norbornene-dicarbonyl, benzene-dicarbonyl, biphenyl-dicarbonyl, alkylene-dicarbonyl (e.g., succinoyl, glutaryl, adipoyl, pimeloyl, suberoyl, etc.)

The term "molecular weight," as used herein, refers to a theoretical weight of an Avogadro number of molecules of identical composition. As preparation of an additive can involve generation of a distribution of compounds, the term "molecular weight" refers to a molar mass of an idealized structure determined by the stoichiometry of the reactive ingredients. Thus, the term "molecular weight," as used herein, refers to a theoretical molecular weight.

The term "oligomeric linker," as used herein, refers to a divalent group containing from two to fifty bonded to each other identical chemical moieties. The chemical moiety can be an alkylene oxide (e.g., ethylene oxide).

The term "oligomeric segment," as used herein, refers to a relatively short length of a repeating unit or units, generally less than about 50 monomeric units and theoretical molecular weights less than 10,000 Daltons, but preferably <7,000 Daltons and in some examples, <5,000 Daltons. In certain embodiments, oligo is selected from the group consisting of polyurethane, polyurea, polyamide, polyalkylene oxide, polycarbonate, polyester, polylactone, polysilicone, polyethersulfone, polyolefin, polyvinyl, polypeptide, polysaccharide, and ether and amine linked segments thereof.

The term "oxycarbonyl bond," as used herein, refers to a bond connecting an oxygen atom to a carbonyl group. Exemplary oxycarbonyl bonds can be found in esters and urethanes. Preferably, the oxycarbonyl bond is a bond in an ester.

The term "polysulfone," as used herein, refers to a class of polymers that include as a repeating subunit the moiety -aryl-SO₂-aryl-. Polysulfones include, without limitation, polyethersulfones and poly(oxy-1,4-phenylene sulfonyl-1,4-phenyleneoxy-1,4-phenyleneisopropylidene-1,4-phenylene).

The term "polyalkylene," when used herein in reference to a base polymer, refers to a base polymer composed of linear or branched alkylene repeating units having from 2 to 4 carbon atoms and/or optionally a cyclic olefin of 3 to 10 carbon atoms (e.g., norbornene or tetracyclododecene). Each alkylene repeating unit is optionally substituted with one substituent selected from the group consisting of chloro, methoxycarbonyl, ethoxycarbonyl, hydroxyethoxycarbonyl, pyrrolidone, hydroxy, acetoxy, cyano, and phenyl. Non-limiting examples of polyalkylene base polymers include polystyrene, a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), and polyacrylate (e.g., PMMA).

The term "polyfluoroorgano group," as used herein, refers to a hydrocarbon group that may be optionally interrupted by one, two, or three non-contiguous oxygen atoms, in which from two to fifty-nine hydrogen atoms were replaced with fluorine atoms. The polyfluoroorgano group contains one to thirty carbon atoms. The polyfluoroorgano group can contain linear alkyl, branched alkyl, or aryl groups, or any combination thereof. The polyfluoroorgano group (e.g., polyfluoroalkyl) can be a "polyfluoroacyl," in which the carbon atom, through which the polyfluoroorgano group (e.g., polyfluoroalkyl) is attached to the rest of the molecule, is substituted with oxo. The alkyl chain within polyfluoroorgano group (e.g., polyfluoroalkyl) can be interrupted by up to nine oxygen atoms, provided that two closest oxygen atoms within polyfluoroorgano are separated by at least two carbon atoms. When the polyfluoroorgano consists of a linear or branched alkyl optionally substituted with oxo and/or optionally interrupted with oxygen atoms, as defined herein, such group can be called a polyfluoroalkyl group. Some polyfluoroorgano groups (e.g., polyfluoroalkyl) can have a theoretical molecular weight of from 100 Da to 1,500 Da. A polyfluoroalkyl can be CF₃(CF₂)ᵣ(CH₂CH₂)ₚ-, where p is 0 or 1, r is from 2 to 20, or CF₃(CF₂)ₛ(CH₂CH₂O)_{X}-, where X is from 0 to 10, and s is from 1 to 20. Alternatively, polyfluoroalkyl can be CHₘF₍₃₋ₘ₎(CF₂)ᵣCH₂CH₂- or CHₘF₍₃₋ₘ₎(CF₂)ₛ(CH₂CH₂O)_{X}-, where m is 0, 1, 2, or 3; X is from 0 to 10; r is an integer from 2 to 20; and s is an integer from 1 to 20. In particular embodiments, X is 0. In certain embodiments, polyfluoroalkyl is formed from 1H,1H,2H,2H-perfluoro-1-decanol; 1H,1H,2H,2H-perfluoro-1-octanol; 1H,1H,5H-perfluoro-1-pentanol; or 1H,1H, perfluoro-1-butanol, and mixtures thereof. In other embodiments, polyfluoroalkyl is perfluoroheptanoyl. In still other embodiments, polyfluoroalkyl is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

The term "rate of biofilm formation" refers to a growth rate of a mass of microorganisms attached to a surface. The rate of biofilm formation can be reduced by at least 20% relative to the surface formed from the base polymer in the absence of the compound, e.g., at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99%.

The term "salt formation" refers to salt formation and deposition on a surface that is in contact with urine, e.g., sodium chloride, calcium phosphate, magnesium ammonium phosphate, etc.

As used herein, the term "less than a standard regimen" refers to the use of the surfaces and methods of the invention in subjects receiving antimicrobial therapy, where the amount of the antimicrobial administered to the subject is less than 80%, 70%, 60%, 50%, 40%, or 30% of the amount otherwise indicated on the package insert (e.g., either by reducing the dosing level, reducing the dosing frequency, and/or reducing the period of time during which a subject undergoes antimicrobial therapy) for the use of the antimicrobial in the treatment and/or prophylaxis of an infection.

The term "static aqueous conditions" refers to conditions under which a polymeric surface is subjected to an aqueous environment, e.g., urine or blood, in which no flow is present.

The term "surface-active group," as used herein, refers to a hydrophobic group bonded to a segment of an additive. For example, the surface-active group can be positioned to cap two, three, or four termini of the central, segmented polymeric portion of the additive and/or can be attached to one or more side chains present in the central polymeric portion of the surface modifier. Examples of surface-active groups include, without limitation, polydimethylsiloxanes, hydrocarbons, polyfluoroalkyl, fluorinated polyethers, and combinations thereof.

The term "urine dwelling polymeric surface" refers to a polymeric surface that is in contact with urine, e.g., catheters (including dialysis access, urinary, diagnostic, drug delivery, etc.), and catheter connectors and valves (including needleless connectors).

Other features and advantages of the invention will be apparent from the Drawings, Detailed Description, and the claims.

### Brief Description of the Drawings

FIGS. 1A-1G show structures of: FIG. 1A compound 1; FIG. 1B compound 2; FIG. 1C compound 3; FIG. 1D compound 4; FIG. 1E compound 5; FIG. 1F compound 6; and FIG. 1G compound 7.
FIGS. 2A-2E show structures of: FIG. 2A compound 8; FIGS. 2B compound 9; FIG. 2C compound 10; FIG. 2D compound 11; and FIG. 2E compound 12.
FIGS. 3A-3C show structures of: FIG. 3A compound 13; FIG. 3B compound 14; and FIG. 3C compound 15.
FIGS. 4A-4C show structures of: FIG. 4A compound 16; FIG. 4B compound 17; and FIG. 4C compound 18.
FIGS. 5A-5C show structures of: FIG. 5A compound 19; FIG. 5B compound 20; and FIG. 5C compound 21.
FIGS. 6A-6C show structures of: FIG. 6A compound 22; FIG. 6B compound 23; and FIG. 6C compound 24.
FIGS. 7A-7C show structures of: FIG. 7A compound 25; FIG. 7B compound 26; and FIG. 7C compound 27.
FIGS. 8A-8D show structures of: FIG. 8A compound 28; FIG. 8B compound 29; FIG. 8C compound 30; and FIG. 8D compound 31.
FIGS. 9A-9D show structures of: FIG. 9A compound 32; FIG. 9B compound 33; FIG. 9C compound 34; and FIG. 9D compound 35.
FIGS. 10A-10D show structures of: FIG. 10A compound 36; FIG. 10B compound 37; FIG. 10C compound 38; and FIG. 10D compound 39.
FIGS. 11A-11D show structures of: FIG. 11A compound 40; FIG. 11B compound 42; FIG. 11C compound 43; and FIG. 11D compound 44.
FIGS. 12A-12E show structures of: FIG. 12A compound 45; FIG. 12B compound 46; FIG. 12C compound 47; FIG. 12D compound 48; and FIG. 12E compound 49.
FIGS. 13A-13H show structures of: FIG. 13A compound 50; FIG. 13B compound 51; FIG. 13C compound 52; FIG. 13D compound 53; FIG. 13E compound 54; FIG. 13F compound 55; FIG. 13G compound 56; and FIG. 13H compound 57.
FIGS. 14A-14D are a series of graphs showing bacterial adhesion on polyurethane (PU) rods after 2 h incubation in PBS.
FIGS. 15A-15D are a series of graphs showing bacterial and fungi adhesion on PU rods after 2 h incubation in PBS.
FIG. 16 is a graph showing a summary of microbial adhesion on PU rods after 2 h of incubation in PBS.
FIG. 17 is a graph showing a summary of microbial adhesion on PU rods after 2 h of incubation in PBS.
FIG. 18 is a series of SEM Images showing S. *aureus* adhesion on PU rods with compound 41 after 2 h incubation in PBS.
FIGS. 19A-19E are a series of graphs showing bacterial adhesion on PU rods after 2 h incubation in PBS.
FIG. 20 is a summary graph showing bacterial adhesion on PU rods with compound 22 after 2 h incubation in PBS.
FIGS. 21A-21B are a series of graphs showing bacterial adhesion on PU rods with compound 22 after 2 h incubation in artificial urine or human pooled urine.
FIG. 22 is a graph showing *E.coli* adhesion on PU rods after 12 h incubation in human pooled urine.
FIG. 23 is a graph showing extended *E.coli* adhesion (up to 7 days) on PU rods with compound 22 in human pooled urine.
FIG. 24 is a graph showing extended *E.coli* adhesion (8 days) on PU rods with compound 22 in human pooled urine and Mueller Hinton Broth.
FIG. 25 is a series of SEM mages showing *E. coli* adhesion on PU rods with compound 22 after 24 h incubation in human pooled urine.
FIG. 26 is a graph showing bacterial adhesion on PU catheter tubing with compound 22 after 24 h incubation in clinical urine.
FIG. 27 is a graph showing bacterial adhesion on PU catheter tubing with compound 22 after 24 h incubation in artificial urine and human pooled urine.
FIG. 28 is a diagram depicting the re-circulating flow system for investigating bacterial adhesion under flow conditions.
FIG. 29 is a graph showing bacterial adhesion on PU catheter tubing with compound 22 after 24 h in artificial urine under static and flow conditions.
FIG. 30 is an image depicting ureteral stent prototypes tested in in-vivo rabbit study.
FIG. 31 is a graph showing *E. coli* adhesion on PU rods with compound 22 after 7 day incubation in Mueller Hinton Broth followed by 24 h ciprofloxacin antibiotic exposure.
FIGS. 32A-32B are a series of graphs showing *S. epidermidis* and *S. aureus* adhesion on Si rods after 2 h incubation in PBS.
FIGS. 33A-33B are a series of graphs showing uropathogen adhesion on Si rods after 2 h incubation in human pooled urine.
FIGS. 34A-34B are a series of graphs showing *P. aeruginosa* and *E. faecalis* adhesion on Si rods after up to 24 h incubation in human pooled urine
FIG. 35 is a graph showing *E. coli* adhesion on Si rods after up to 3 days incubation in human pooled urine.
FIG. 36 is a summary graph showing bacterial adhesion on Si rods with compound 43.
FIG. 37 is a graph showing *S. aureus* adhesion on PU rods in diluted plasma or serum.
FIG. 38 is a graph showing *E. coli* adhesion on PU catheter tubing with compound 22 at different flow / shear rates.
FIG. 39 is a diagram depicting the non-circulating flow system for investigating bacterial adhesion under flow conditions.
FIG. 40 is a graph showing *E. coli* adhesion on PU catheter tubing with compound 22 over 7 days of AU flow.
FIG. 41 are photographs showing biofouling and *E. coli* biofilm formation on PU catheter tubing over 7 days of AU flow as seen visually and through Crystal Violet staining.
FIG. 42 is a series of SEM images showing *E. coli* biofilm on PU catheter tubing over 7 days of AU flow.
FIG. 43 is a graph showing adhesion of various bacterial species on PU catheter tubing with compound 22 after 24h of flow.
FIG. 44 is a series of graphs showing uropathogen adhesion on PU tubing compared to commerical ureteral stent tubing after 24h incubation in human pooled urine or artificial urine.
FIG. 45 is a graph showing *E. coli* adhesion on PU tubing under flow conditions in comparison to commerical ureteral stent tubing.
FIG. 46 is a graph showing mass of encrustation deposits on PU tubing with compound 22 after 2 week incubation in artificial urine with *P. mirabilis* bacteria.
FIG. 47 is a series of graphs showing *S. aureus* and *S. epidermidis* adhesion on PU rods after 2h incubation in PBS.
FIG. 48 is a graph showing *E. coli* adhesion on PU rods after 24h incubation in human pooled urine.
FIG. 49 is a series of graphs showing bacterial adhesion on PU rods with and without radiopaque BaSO₄ filler.
FIG. 50 is a series of graphs showing bacterial adhesion on PU rods with compounds 54 and 55 after 2h incubation in PBS or human pooled urine.

### Detailed Description

Described are surfaces, not included in the invention, formed from a base polymer admixed with an additive of the invention. The surfaces can be resistant to bacterial adhesion. The surfaces can be applied to: (a) any medical device or material that is implanted in or juxtaposed to a human or animal tissue or body fluid, in vivo or ex vivo, permanently or for a short period, and (b) any useful article, material or device, medical or not, which is to maintain a human or animal health-safe unfouled state of cleanliness over a period of time. In these medical and non-medical applications it is known that undesirable fouling phenomenon can take place which might harm a patient, user or consumer of the compromised device, equipment or material processed by, transported or stored in contamination prone articles, equipment or environments. Such fouling phenomena include biofilm development and bacterial colony development. Such fouling can cause irritation and swelling, allergic reactions, toxic reactions, poisoning or infections, as well as deterioration or destruction of the function of the article.

Articles, not included in the invention, can be prepared, at least in part, from a base polymer using a process requiring a high temperature processing, melt processing, or solution processing (e.g., extruding, injection molding, calendaring, mixing, spraying, dipping, solution fiber spinning, electrospinning, or casting). Solution spinning can be performed, e.g., at 100 °C. In some embodiments, PU and PVC are processed at 180 °C. In another example, COC and COP often require processing temperatures of greater than 200 °C (e.g., greater than or equal to 250 °C or greater than or equal to 300 °C). The additives described herein can be thermally stable (e.g., can have a thermal degradation temperature of greater than or equal to 200 °C (e.g., greater than or equal to 250 °C or greater than or equal to 300 °C). Accordingly, articles can be formed from an admixture of a base polymer and an additive at a temperature of greater than 200 °C (e.g., greater than or equal to 250 °C or greater than or equal to 300 °C).

Articles can be manufactured (e.g., through high temperature processing, melt processing, or solution processing) from an admixture of a base polymer and an additive. The additive can be added prior to melt processing of the base polymer to produce an article described. To form an admixture by melt processing, the additive can be, for example, mixed with pelletized or powdered polymer and then melt processed by known methods such as, for example, molding or melt extrusion. The additive can be mixed directly with the polymer in the melt condition or can first be pre-mixed with the polymer in the form of a concentrate of the additive/polymer admixture in a brabender mixer. If desired, an organic solution of the additive can be mixed with powdered or pelletized base polymer, followed by evaporation of the solvent and then by melt processing. Alternatively, the additive can be injected into a molten polymer stream to form an admixture immediately prior to extrusion into the desired shape.

After melt processing, an annealing step can be carried out to enhance the development of advantageous properties described herein in the base polymer. In addition to, or in lieu of, an annealing step, the melt processed combination can also be embossed between two heated rolls, one or both of which can be patterned. An annealing step typically is conducted below the melt temperature of the polymer (e.g., at from about 50 °C to about 220 °C).

An admixture can also be formed by solution processing, such as coating, micro printing, emulsion processing, dot printing, micropatterning, fiber spinning, solvent blow molding, electrospraying, and electrospinning. Electrospraying can be performed, e.g., by dissolving the admixture in a solvent and electrospraying to form micro- and nanobeads, or loading the solution into a syringe and injecting onto a stationary collection plate. Between the needle and collecting surface, a potential difference can be maintained. Electrospinning can be performed, e.g., by dissolving the admixture in a solvent and injecting the solution from a syringe at a particular rate onto a cylindrical mandrel rotating at a particular rotational speed to obtain aligned fibers, or onto a stationary collector surface to obtain unaligned fibers. A potential difference can be maintained between the needle and collecting surface for aligned and random fibers.

The additive is added to a base polymer in amounts sufficient to achieve the desired surface properties for a particular application. The amount of additive used is in the range of 0.05-15% (w/w) of the admixture. The amounts can be determined empirically and can be adjusted, as necessary or desired, to achieve the desired surface properties without compromising other physical properties of the base polymer.

### Base Polymers

The base polymer utilized in the uses of the invention and articles, not included in the invention, can be a silicone, polyolefin, polyester, polycarbonate, polysulfone, polyamide, polyether, polyurea, polyurethane (PU, e.g., Carbothane 85A (CB)), polyetherimide, or cellulosic polymer, or a copolymer thereof or a blend thereof (e.g., polypropylene (PP), polyethylene (PE), polyvinylchloride (PVC), polyvinyl alcohol (PVA), polyvinylpyrrolidone (PVP), polyacrylamide (PAAM), polyethylene oxide (PEO), polyethylene oxide)-b-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), poly(styrene-*block*-isobutylene-*block*-styrene) (SIBS), ethylene-vinyl acetate (EVA), or a copolymer thereof or a blend thereof). A base polymer used in the devices and coatings may be a thermoplastic polymer (e.g., a thermoplastic polyurethane). The base polymers of the devices and coatings may also be cross-linked.

For example, the base polymer utilized in the uses of the invention and articles, not included in the invention, can be a thermoplastic polyurethane. Thermoplastic polyurethanes encompass many different types of materials as well as materials of different durometers. Initial selection of a polyurethane may be based on the performance of the material. Thermoplastic polyurethanes are available with Shore Durometers from 60A to 85D. Thermoplastic polyurethanes come in a variety of different chemical structures, which are selected based upon how the base polymer is being used, and for how long.

Polyurethanes useful for the uses of the invention and articles, not included in the invention, but are not limited to, polyether-based polyurethanes (e.g., TECOFLEX^{®}, QUADRA-FLEX^{®}), polycarbonate-based polyurethanes (e.g., CARBOTHANE^{®}, BIONATE^{®}, QUADRATHANE^{™}), aromatic polyester or polyether polyurethanes (e.g., PELLETHANE^{®}, TECOTHANE^{®}), aliphatic or aromatic polyester or polyether polyurethanes (e.g., ELASTOLLAN^{®}), hydrophilic polyurethanes (e.g., TECOPHILIC^{®}, HYDROTHANE^{®}), and silicone-polyurethane copolymers (e.g., CARBOSIL^{®}, ELASTEON^{™}). The polyurethane may have a durometer of 80A, 85A, 88A, 90A, 95A, 55D, or 75D.

TECOFLEX^{®} medical grade thermoplastic polyurethanes (Grades EG-80A, EG-93A and EG-60D) are a group of aliphatic, polyether based resins that have established credentials for implants including having passed the following standard screening tests: MEM Elution, Hemolysis, USP Class VI, 30 Day Implant, and Ames Mutagenicity.

TECOFLEX^{®} EG-80A is a medical-grade, aliphatic, polyether-based thermoplastic polyurethane elastomer with a durometer value of 72A. TECOFLEX^{®} EG-85A is a medical-grade, aliphatic, polyether-based thermoplastic polyurethane elastomer with a durometer value of 77A. CARBOTHANE^{®} PC-3575A is a medical-grade, aliphatic, polycarbonate-based thermoplastic polyurethane elastomer with a durometer value of 73A. CARBOTHANE^{®} PC-3585A is a medical-grade, aliphatic, polycarbonate-based thermoplastic polyurethane elastomer with a durometer value of 84A. PELLETHANE^{®} polyurethanes are polyether-based thermoplastic polyurethane elastomers. BIONATE^{®} thermoplastic polycarbonate polyurethanes are a family of thermoplastic elastomers formed as a reaction product of a hydroxyl terminated polycarbonate, an aromatic diisocyanate and a low molecular weight glycol to form the soft segment.

Additional exemplary polycarbonate urethanes) that may be included in the admixtures include, without limitation, CHRONOFLEX^{®} AL (aliphatic), CHRONOFLEX^{®} AR (aromatic), and CHRONOFLEX^{®} C (aromatic), in a variety of durometers 80A, 85A, 88A, 90A, 95A, 55D, and 75D.

Other base polymers that can be useful in the admixtures of the invention include silicones, such as medical grade silicone elastomers. Some examples of silicone polymers that can be used are MED-4780, MED-4765, MED3-6320, MED-6340, MED-6345, MED-6350 from Nusil^{™}. Another example is Silastic^{®} family of products such as Q7-4780 or Dow Corning^{®} family of products such as C6-235.

In another example, the admixtures can be used with a polyethylene base polymer, e.g., for biliary or ureteral stents. The admixtures may also be used with a PVC base polymer, e.g., for urinary catheters or medical tubing. In further example, the admixtures may be used with poly(styrene-*block*-isobutylene-*block*-styrene) (SIBS) base polymers, e.g., for ureteral stents. Polyamide base polymers (e.g., Nylon 6, Nylon 6-6, Nylon 11, Nylon 12, polyether block amide (PEBA), VESTAMID^{®}, PEBAX^{®}) may be used with the admixtures of the invention, e.g., for catheters. Polypropylene base polymers may be used with the admixtures, e.g., for platelet bags and mesh implants. The admixtures may also be used with ethylene-vinyl acetate (EVA) base polymers, e.g., for ureteral stents.

### Additives

The additives used in the articles, devices, and surfaces is described by the structure of any one of formulae (I), (II), (III), (IV), (V), (VI), (VII), (VIII), (IX), (X), (XI), (XII), (XIII), (XIV), (XV), (XVI), (XVII), (XVIII), (XIX), (XX), and (XXI) shown below.
(1) Formula (I):

   F_{T}-[B-A]ₙ-B-F_{T} (I)

   where
   (i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
   (ii) B is a segment including a urethane; and
   (iii) F_{T} is a polyfluoroorgano group, and
   (iv) n is an integer from 1 to 10.
(2) Formula (II):

   F_{T}-[B-A]ₙ-B-F_{T} (II)

   where
   (i) B includes a urethane;
   (ii) A includes polypropylene oxide, polyethylene oxide, or polytetramethylene oxide;
   (iii) F_{T} is a polyfluoroorgano group; and
   (iv) n is an integer from 1 to 10.
(3) 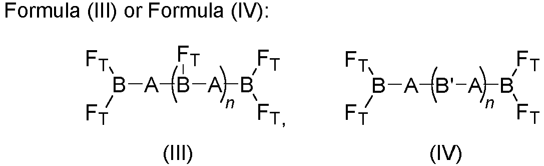 where
   (i) A is an oligomeric segment containing an ether linkage, an ester linkage, a carbonate linkage, or a polyalkylene and having a theoretical molecular weight of from 500 to 3,500 Daltons (e.g., from 500 to 2,000 Daltons, from 1,000 to 2,000 Daltons, or from 1,000 to 3,000 Daltons);
   (ii) B is a segment including a isocyanurate trimer or biuret trimer; B', when present, is a segment including a urethane;
   (iii) each F_{T} is a polyfluoroorgano group; and
   (iv) n is an integer between 0 to 10.
(4) Formula (V):

   F_{T}-[B-A]ₙ-B-F_{T} (V)

   where
   (i) A is an oligomeric segment including polypropylene oxide, polyethylene oxide, or polytetramethylene oxide and having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment formed from a diisocyanate;
   (iii) F_{T} is a polyfluoroorgano group; and
   (iv) n is an integer from 1 to 10.
(5) 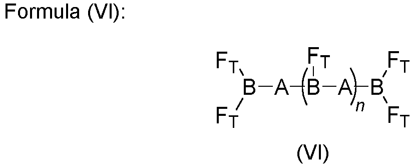 where
   (i) A is an oligomeric segment including polyethylene oxide, polypropylene oxide, polytetramethylene oxide, or a mixture thereof, and having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(6) Formula (VII):

   F_{T}-[B-A]ₙ-B-F_{T} (VII)

   where
   (i) A is a polycarbonate polyol having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment formed from a diisocyanate;
   (iii) F_{T} is a polyfluoroorgano group; and
   (iv) n is an integer from 1 to 10.
(7) 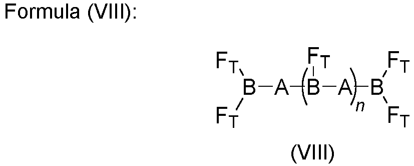 where
   (i) A is an oligomeric segment including a polycarbonate polyol having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(8) 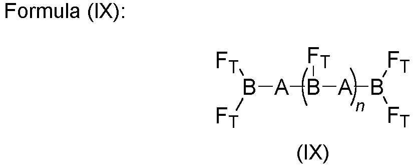 where
   (i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Daltons (e.g., from 1,000 to 3,000 Daltons, from 2,000 to 5,000 Daltons, or from 2,500 to 5,000 Daltons);
   (ii) B is a segment including an isocyanurate trimer or biuret trimer;
   (iii) F_{T} is a polyfluoroorgano group; and
   (iv) n is an integer from 0 to 10.
(9) Formula (X):

   F_{T}-[B-A]ₙ-B-F_{T} (X)

   where
   (i) A is a segment selected from the group consisting of hydrogenated polybutadiene (e.g., HLBH), polybutadiene (e.g., LBHP), hydrogenated polyisoprene (e.g., HHTPI), polysiloxane-polyethylene glycol block copolymer, and polystyrene and has a theoretical molecular weight of from 750 to 3,500 Daltons (e.g., from 750 to 2,000 Daltons, from 1,000 to 2,500 Daltons, or from 1,000 to 3,500 Daltons);
   (ii) B is a segment formed from a diisocyanate;
   (iii) F_{T} is a polyfluoroorgano group; and
   (iv) n is an integer from 1 to 10.
(10) 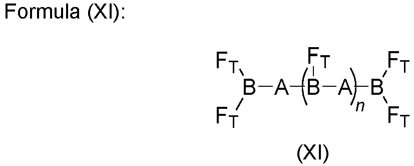 where
   (i) A is hydrogenated polybutadiene (e.g., HLBH), polybutadiene (e.g., LBHP), hydrogenated polyisoprene (e.g., HHTPI), or polystyrene and has a theoretical molecular weight of from 750 to 3,500 Daltons (e.g., from 750 to 2,000 Daltons, from 1,000 to 2,500 Daltons, or from 1,000 to 3,500 Daltons);
   (ii) B is a segment including an isocyanurate trimer or biuret trimer;
   (iii) F_{T} is a polyfluoroorgano group; and
   (iv) n is an integer from 0 to 10.
(11) 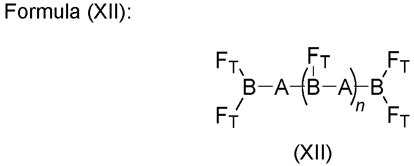 where
   (i) A is a polyester having a theoretical molecular weight of from 500 to 3,500 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(12) Formula (XIII):

   F_{T}-A-F_{T} (XIII)

   where F_{T} is a polyfluoroorgano group and A is an oligomeric segment.
(13) 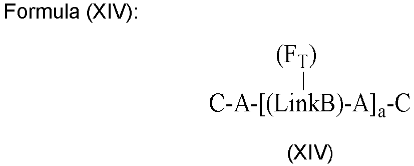 where
   (i) F_{T} is a polyfluoroorgano group covalently attached to LinkB;
   (ii) C is a chain terminating group;
   (iii) A is an oligomeric segment;
   (iv) LinkB is a coupling segment; and
   (v) a is an integer greater than 0.
(14) 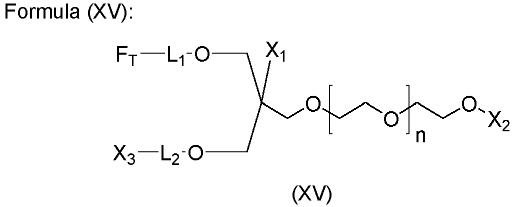 where
   (i) each F_{T} is a polyfluoroorgano group;
   (ii) X₁ is H, CH₃, or CH₂CH₃;
   (iii) each of X₂ and X₃ is independently H, CH₃, CH₂CH₃, or F_{T};
   (iv) each of L₁ and L₂ is independently a bond, an oligomeric linker, or a linker with two terminal carbonyls; and
   (v) n is an integer from 5 to 50.
(15) 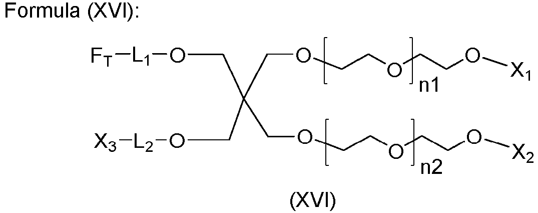 where
   (i) each F_{T} is a polyfluoroorgano group;
   (ii) each of X₁, X₂, and X₃ is independently H, CH₃, CH₂CH₃, or F_{T};
   (iii) each of L₁ and L₂ is independently a bond, an oligomeric linker, a linker with two terminal carbonyls, or is formed from a diisocyanate; and
   (iv) each of n1 and n2 is independently an integer from 5 to 50.
(16) Formula (XVII):

   G-Aₘ-[B-A]ₙ-B-G (XVII)

   where
   (i) each A comprises hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
   (ii) each B is independently a bond, an oligomeric linker, or a linker with two terminal carbonyls;
   (iii) each G is H or a polyfluoroorgano group, provided that at least one G is a polyfluoroorgano group;
   (iv) n is an integer from 1 to 10; and
   (v) m is 0 or 1.
(17) Formula (XVIII):

   F_{T}-OC(O)O-B-OC(O)O-[A-OC(O)O-B]ₙ-OC(O)O-F_{T} (XVIII)

   where
   A comprises a soft segment and is covalently bound to B via a carbonate linkage;
   (ii) B comprises a polyalkylene oxide or a moiety described by the formula: 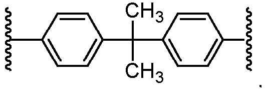 and is covalently bound to A via a carbonate linkage; and
   (iii) F_{T} is a surface active group comprising a polyfluoroorgano group, wherein F_{T} is covalently bound to B via a carbonate linkage; and
   (iv) n is an integer from 1 to 10.
(18) Formula (XIX):

   F_{T}-OC(O)O-(CH₂CH₂O)ₘ-OC(O)O-[A-OC(O)O-(CH₂CH₂O)ₘ]ₙ-OC(O)O-F_{T} (XIX)

   where
   (i) A comprises a soft segment;
   (ii) F_{T} is a surface active group comprising a polyfluoroorgano group;
   (iii) m is an integer from 2 to 4; and
   (iv) n is an integer from 1 to 10.
(19) 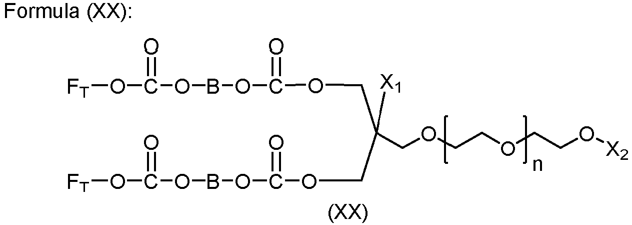 where
   (i) F_{T} is a polyfluoroorgano group;
   (ii) each of X₁ and X₂ is, independently, is H, CH₃, or CH₂CH₃;
   (iii) B comprises a polyalkylene oxide; and
   (v) n is an integer from 5 to 100.
(20) 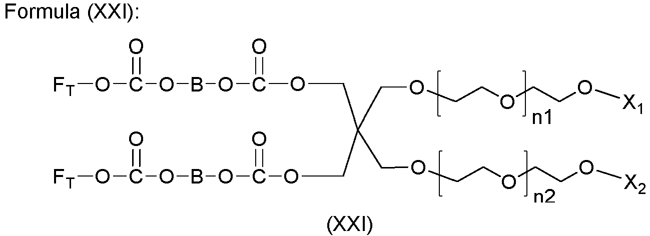 where
   (i) each F_{T} is a polyfluoroorgano group;
   (ii) each of X₁ and X₂ is, independently, H, CH₃, or CH₂CH₃;
   (iii) B comprises a polyalkylene oxide; and
   (iv) each of n1 and n2 is independently an integer from 5 to 50.

The additive of formula (I) includes B formed from a diisocyanate (e.g., 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; or hexamethylene diisocyanate). The variable n may be 1 or 2. The articles, devices, and surfaces, which are not included in the invention, may include a surfacecontaining a base polymer and the additive of formula (I).

The additive of formulae (III) and (IV) include A that is an oligomeric segment containing hydrogenated polybutadiene (HLBH), poly((2,2-dimethyl)-1,3-propylene carbonate) (PCN), polybutadiene (LBHP), polytetramethylene oxide (PTMO), polypropylene oxide (PPO), (diethyleneglycol-orthophthalic anhydride) polyester (PDP), hydrogenated polyisoprene (HHTPI), poly(hexamethylene carbonate), poly((2-butyl-2-ethyl)-1,3-propylene carbonate), or hydroxylterminated polydimethylsiloxane (C22). In the additive of formulae (III) and (IV), B is formed by reacting a triisocyanate (e.g., hexamethylene diisocyanate (HDI) biuret trimer, isophorone diisocyanate (IPDI) trimer, or hexamethylene diisocyanate (HDI) trimer) with a diol including the oligomeric segment A. The articles, devices, and surfaces, which are not included in the invention, may include a surface containing a base polymer and the additive of formula (III). The articles, devices, and surfaces, which are not included in the invention, may include a surface containing a base polymer and the additive of formula (IV).

In the additive of formula (V), B may is a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. In the additive of formula (V), segment A can be polyethylene oxide)-*b*-poly(propylene oxide)-b-poly(ethylene oxide). The variable n may be an integer from 1 to 3. The articles, devices, and surfaces, which are not included in the invention, may include a surface containing a base polymer and the additive of formula (V).

In the additive of formula (VI), B is a segment formed by reacting a triisocyanate with a diol of A. The triisocyanate may be hexamethylene diisocyanate (HDI) biuret trimer, isophorone diisocyanate (IPDI) trimer, or hexamethylene diisocyanate (HDI) trimer. In the additive of formula (VI), segment A can be polyethylene oxide)-*b*-poly(propylene oxide)-b-poly(ethylene oxide). The variable n may be 0, 1, 2, or 3. The articles, devices, and surfaces, which are not included in the invention, may include a surface containing a base polymer and the additive of formula (VI).

In the additive of formula (VII), A can include poly((2,2-dimethyl)-1 ,3-propylene carbonate) (PCN). B may be a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The articles, devices, and surfaces, which are not included in the invention may include a surface containing a base polymer and the additive of formula (VII).

In the additive of formula (VIII), B is a segment formed by reacting a triisocyanate with a diol of A (e.g., the oligomeric segment). The triisocyanate may be hexamethylene diisocyanate (HDI) biuret trimer, isophorone diisocyanate (IPDI) trimer, or hexamethylene diisocyanate (HDI) trimer. The segment A can include poly((2,2-dimethyl)-1,3-propylene carbonate) (PCN) or poly(hexamethylene carbonate) (PHCN). The variable n may be 0, 1, 2, or 3. The articles, devices, and surfaces, which are not included in the invention, may include a surface containing a base polymer and the additive of formula (VIII).

In the additive of formula (IX), B is a segment formed by reacting a triisocyanate with a diol of A. In segment A, the number of first block segments and second block segments can be any integer or non-integer to provide the approximate theoretical molecule weight of the segment. The segment A can include polypropylene oxide and polydimethylsiloxane. The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The articles, devices, and surfaces, which are not included in the invention, may include a surface containing a base polymer and the additive of formula (IX).

In additive of formula (X), B is a segment formed from a diisocyanate. The segment A includes hydrogenated polybutadiene. Alternatively, the segment A includes polysiloxane-polyethylene glycol block copolymer (e.g., PEG-PDMS-PEG). The segment B may be formed from 3-isocyanatomethyl-3,5,5-trimethy-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The articles, devices, and surfaces, which are not included in the invention, may include a surface containing a base polymer and the additive of formula (X).

In the additive of formula (XI), B is a segment formed by reacting a triisocyanate with a diol of A. The segment A is hydrogenated polybutadiene (HLBH) or hydrogenated polyisoprene (HHTPI). The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The articles, devices, and surfaces, which are not included in the invention, may include a surface containing a base polymer and the additive of formula (XI).

In the additive of formula (XII), B is a segment formed by reacting a triisocyanate with a diol of A (e.g., polyester). The segment A may be poly(diethylene glycol)adipate, (neopentyl glycol-ortho phthalic anhydride) polyester, (diethylene glycol-ortho phthalic) anhydride polyester, or (1,6-hexanediol-ortho phthalic anhydride) polyester. The triisocyanate may be hexamethylene diisocyanate (HDI) biuret trimer, isophorone diisocyanate (IPDI) trimer, and hexamethylene diisocyanate (HDI) trimer. The variable n may be 0, 1, 2, or 3. The articles, devices, and surfaces, which are not included in the invention, may include a surface containing a base polymer and the additive of formula (XII).

The additive of formula (XIII) includes a segment A that is a branched or non-branched oligomeric segment of fewer than 20 repeating units (e.g., from 2 to 15 units, from 2 to 10 units, from 3 to 15 units, and from 3 to 10 units). In certain embodiments, the additive of formula (XIII) include an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments. The articles, devices, and surfaces, which are not included in the invention, may include a surface containing a base polymer and the additive of formula (XIII).

The additive of formula (XIV) includes a segment A that is a branched or non-branched oligomeric segment of fewer than 20 repeating units (e.g., from 2 to 15 units, from 2 to 10 units, from 3 to 15 units, and from 3 to 10 units). In certain embodiments, the additive of formula (XIV) include an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide. The articles, devices, and surfaces, which are not included in the invention, may include a surface containing a base polymer and the additive of formula (XIV).

The additive of formula (XV) includes a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XV), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XV), each of L₁ and L₂ is a bond. In certain embodiments of formula (XV), the additive includes an oligomeric segment (e.g., in any one of L₁ and L₂) selected from the group consisting of polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, and polybutadiene. In some embodiments of formula (XV), the additive is a compound of formula (XV-A): 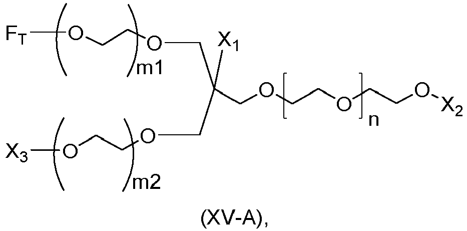 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XV) or (XV-A), X₂ is F_{T}. In other embodiments, X₂ is CH₃ or CH₂CH₃. In particular embodiments of formula (XV) or (XV-A), X₃ is F_{T}. In other embodiments, each F_{T} is independently a polyfluoroorgano group (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In certain embodiments of formula (XV) or (XV-A), n is an integer from 5 to 40 (e.g., from 5 to 20, such as from 5, 6, 7, 8, 9, or 10). In some embodiments of formula (XV) or (XV-A), each F_{T} includes (CF₂)₅CF₃. The articles, devices, and surfaces, which are not included in the invention, may include a surface containing a base polymer and the additive of formula (XV). The articles, devices, and surfaces, which are not included in the invention, may include a surface containing a base polymer and the additive of formula (XV-A).

The additive of formula (XVI) includes a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XVI), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XVI), each of L₁ and L₂ is a bond. In certain embodiments of formula (XVI), the additive includes an oligomeric segment (e.g., in any one of L₁ and L₂) selected from polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, or polybutadiene. In some embodiments of formula (XVI), the additive is a compound of formula (XVI-A): 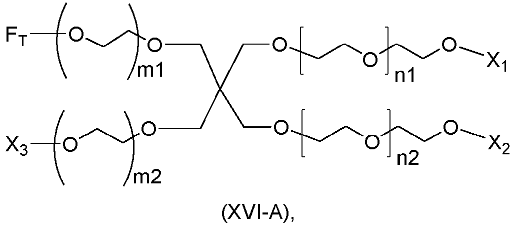 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XVI) or (XVI-A), X₂ is F_{T}. In other embodiments of formula (XVI) or (XVI-A), X₂ is CH₃ or CH₂CH₃. In particular embodiments of formula (XVI) or (XVI-A), X₃ is F_{T}. In other embodiments of formula (XVI) or (XVI-A), each F_{T} is independently a polyfluoroorgano group (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVI) or (XVI-A), each F_{T} includes (CF₂)₅CF₃. The articles, devices, and surfaces of the invention may include a surface containing a base polymer and the additive of formula (XVI). The articles, devices, and surfaces, which are not included in the invention, may include a surface containing a base polymer and the additive of formula (XVI-A).

In some embodiments of formula (XVII), m is 1. The additive of formula (XVII) can be a compound of formula (XVII-A):

G-A-[B-A]ₙ-G (XVII-A).

In other embodiments of formula (XVII), m is 0. The additive of formula (XVII) can be a compound of formula (XVII-B):

G-[B-A]ₙ-B-G (XVII-B).

In particular embodiments of formula (XVII), (XVII-A), or (XVII-B), each B is a linker with two terminal carbonyls. In certain embodiments of formula (XVII), (XVII-A), or (XVII-B), each B is a bond. In some embodiments of Formula (XVII), (XVII-A), or (XVII-B), the bond connecting G and B is an oxycarbonyl bond (e.g., an oxycarbonyl bond in an ester). In other embodiments of formula (XVII), (XVII-A), or (XVII-B), n is 1 or 2.

The additive of formula (XVII) can be a compound of formula (XVII-C):

G-A-G (XVII-C).

In formula (XVII), (XVII-A), (XVII-B), or (XVII-C), G can be a polyfluoroorgano group (e.g., a polyfluoroalkyl). In some embodiments of formula (XVII), (XVII-A), (XVII-B), or (XVII-C), G is F_{T} (e.g., each F_{T} is independently a polyfluoroorgano group (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVII), (XVII-A), (XVII-B), or (XVII-C), each F_{T} includes (CF₂)₅CF₃. The articles, devices, and surfaces of the invention may include a surface containing a base polymer and the additive of formula (XVII). The articles, devices, and surfaces, which are not included in the invention, may include a surface containing a base polymer and the additive of formula (XVII-A). The articles, devices, and surfaces of the invention may include a surface containing a base polymer and the additive of formula (XVII-B). The articles, devices, and surfaces, which are not included in the invention, may include a surface containing a base polymer and the additive of formula (XVII-C).

In formula (XVIII), B contains polypropylene oxide, polyethylene oxide, or polytetramethylene oxide. In formula (XVIII), B can be formed from triethylene glycol, tetraethylene glycol, or bisphenol A.

In formula (XVIII) or (XIX), A contains hydrogenated polybutadiene (HLBH), hydrogenated polyisoprene (HHTPI), poly((2,2-dimethyl)-1,3-propylene carbonate), polybutadiene, poly(diethylene glycol)adipate (PEGA), poly(hexamethylene carbonate) (PHCN), poly(ethylene-co-butylene), (diethylene glycol-ortho phthalic anhydride) polyester, (1,6-hexanediol-ortho phthalic anhydride) polyester, (neopentyl glycol-ortho phthalic anhydride) polyester (PDP), a polysiloxane, bisphenol A ethoxylate, polyethylene oxide)-b-poly(propylene oxide)-b-poly(ethylene oxide) (PLN), polyethylene oxide (PEO), polypropylene oxide (PPO), or polytetramethylene oxide (PTMO).

In some embodiments of formula (XVIII) or (XIX), A includes no ester linkages. For example, A includes hydrogenated polybutadiene (HLBH), hydrogenated polyisoprene (HHTPI), poly((2,2-dimethyl)-1,3-propylene carbonate), polybutadiene, poly(ethylene-co-butylene), a polysiloxane, bisphenol A ethoxylate, polyethylene oxide)-b-poly(propylene oxide)-b-poly(ethylene oxide) (PLN), polyethylene oxide (PEO), polypropylene oxide (PPO), or polytetramethylene oxide (PTMO).

In formula (XIX), A can contain a triblock copolymer PPO-b-PEO-b-(polysiloxane)-b-PEO-b-PPO (PLNSi). In formula (XIX), A can contain hydrogenated polyisoprene (HHTPI) or hydrogenated polybutadiene (HLBH). In formula (XIX), A can contain polypropylene oxide (PPO) or polytetramethylene oxide (PTMO). In formula (XIX), A can contain polyethylene oxide-polydimethylsiloxane-polyethylene oxide (C10 MW _{PEO} = 2,500 Da), polyethylene oxide-polydimethylsiloxane-polyethylene oxide (C15 MW _{PEO} = 1,000 Da), or polyethylene oxide-polydimethylsiloxane-polyethylene oxide (C22 MW _{PEO} = 2,500 Da). In formula (XIX), A can contain propylene oxide-polydimethylsiloxane-propylene oxide block copolymer (C22 MW _{PPO} = 2,500 Da). In formula (XIX), A can contain polyethylene oxide (PEO). In formula (XIX), A can contain diethylene glycol-ortho phthalic anhydride. In formula (XIX), A can contain polyethylene oxide)-b-poly(propylene oxide)-b-poly(ethylene oxide) (PLN).

In formula (XX) or (XXI), B can contain polypropylene oxide, polyethylene oxide, or polytetramethylene oxide. In formula (XX) or (XXI), B can be formed from triethylene glycol or tetraethylene glycol. In formula (XX), B can be polyethylene oxide, X₁ is ethyl, and X₂ is H (YmerOH-1226-PCT-PC). In formula (XX), B can be polyethylene oxide, X₁ is ethyl, and X₂ is methyl (Ymer-1226-PCT-PC). In formula (XXI), B can be polyethylene oxide, X₁ is H, and X₂ is H (Xmer-1226-PCT-PC).

In formula (XVIII), (XIX), (XX), or (XXI), the compound can have a theoretical molecular weight of less than 10,000 Da.

For any of the additives formed from a diisocyanate, the diisocyanate may be 3-isocyanatomethyl-3,5,5-trimethyl-cyclohexylisocyanate; 4,4'-methylene bis(cyclohexyl isocyanate) (HMDI); 2,2'-, 2,4'-, and 4,4'-methylene bis(phenyl isocyanate) (MDI); toluene-2,4-diisocyanate; aromatic aliphatic isocyanate, such 1,2-, 1,3-, and 1,4-xylene diisocyanate; meta-tetramethylxylene diisocyanate (m-TMXDI); para-tetramethylxylene diisocyanate (p-TMXDI); hexamethylene diisocyanate (HDI); ethylene diisocyanate; propylene-1,2-diisocyanate; tetramethylene diisocyanate; tetramethylene-1,4-diisocyanate; octamethylene diisocyanate; decamethylene diisocyanate; 2,2,4-trimethylhexamethylene diisocyanate; 2,4,4-trimethylhexamethylene diisocyanate; dodecane-1,12-diisocyanate; dicyclohexylmethane diisocyanate; cyclobutane-1,3-diisocyanate; cyclohexane-1,2-diisocyanate; cyclohexane-1,3-diisocyanate; cyclohexane-1,4-diisocyanate; methyl-cyclohexylene diisocyanate (HTDI); 2,4-dimethylcyclohexane diisocyanate; 2,6-dimethylcyclohexane diisocyanate; 4,4'-dicyclohexyl diisocyanate; 2,4'-dicyclohexyl diisocyanate; 1,3,5-cyclohexane triisocyanate; isocyanatomethylcyclohexane isocyanate; 1-isocyanato-3,3,5-trimethyl-5-isocyanatomethylcyclohexane; isocyanatoethylcyclohexane isocyanate; bis(isocyanatomethyl)-cyclohexane; 4,4'-bis(isocyanatomethyl) dicyclohexane; 2,4'-bis(isocyanatomethyl) dicyclohexane; isophoronediisocyanate (IPDI); 2,4-hexahydrotoluene diisocyanate; 2,6-hexahydrotoluene diisocyanate; 3,3'-dimethyl-4,4'-biphenylene diisocyanate (TODI); polymeric MDI; carbodiimide-modified liquid 4,4'-diphenylmethane diisocyanate; para-phenylene diisocyanate (PPDI); meta-phenylene diisocyanate (MPDI); naphthylene-1,5-diisocyanate; 2,4'-, 4,4'-, or 2,2'-biphenyl diisocyanate; polyphenyl polymethylene polyisocyanate (PMDI); mixtures of MDI and PMDI; mixtures of PMDI and TDI; dimerized uretdione of any isocyanate described herein, such as uretdione of toluene diisocyanate, uretdione of hexamethylene diisocyanate, or a mixture thereof; or a substituted or isomeric mixture thereof.

For any of the additives formed from an isocyanate trimer, the isocyanate trimer can be hexamethylene diisocyanate (HDI) biuret or trimer, isophorone diisocyanate (IPDI) trimer, hexamethylene diisocyanate (HDI) trimer; 2,2,4-trimethyl-1,6-hexane diisocyanate (TMDI) trimer; a trimerized isocyanurate of any isocyanates described herein, such as isocyanurate of toluene diisocyanate, trimer of diphenylmethane diisocyanate, trimer of tetramethylxylene diisocyanate, or a mixture thereof; a trimerized biuret of any isocyanates described herein; modified isocyanates derived from the above diisocyanates; or a substituted or isomeric mixture thereof.

The additive includes the group F_{T} that is a polyfluoroorgano group having a theoretical molecular weight of from 100 Da to 1,500 Da. For example, F_{T} may be CF₃(CF₂)ᵣ(CH₂CH₂)ₚ-wherein p is 0 or 1, r is 2-20, and CF₃(CF₂)ₛ(CH₂CH₂O)_{X}, where X is from 0 to 10 and s is from 1 to 20. Alternatively, F_{T} may be CHₘF₍₃₋ₘ₎(CF₂)ᵣCH₂CH₂- or CHₘF₍₃₋ₘ₎(CF₂)ₛ(CH₂CH₂O)_{X}-, where m is 0, 1, 2, or 3; X is an integer from 0 to 10; r is an integer from 2 to 20; and s is an integer from 1 to 20. In certain embodiments, F_{T} is 1H,1H,2H,2H-perfluoro-1-decanol; 1H,1H,2H,2H-perfluoro-1-octanol; 1H,1H,5H-perfluoro-1-pentanol; or 1H,1H-perfluoro-1-butanol, or a mixture thereof. In particular embodiments, F_{T} is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

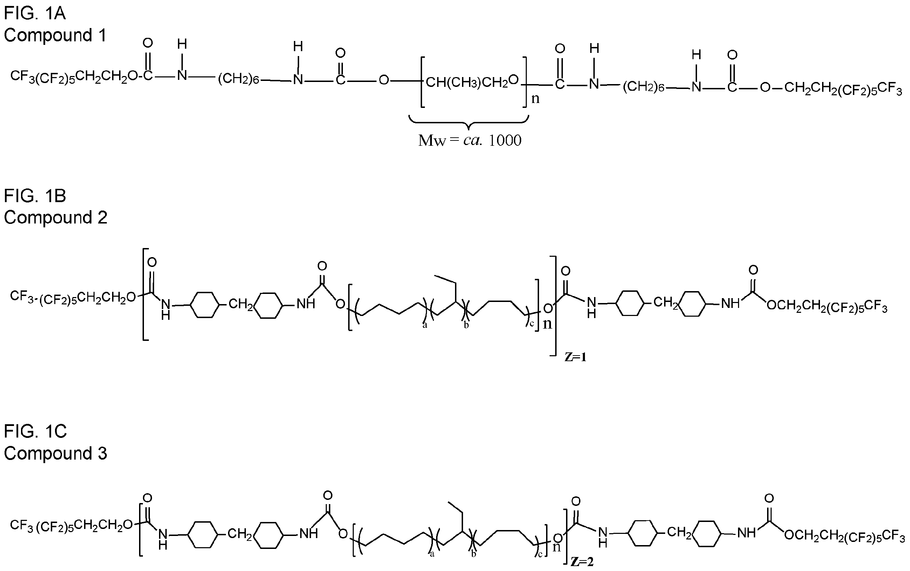

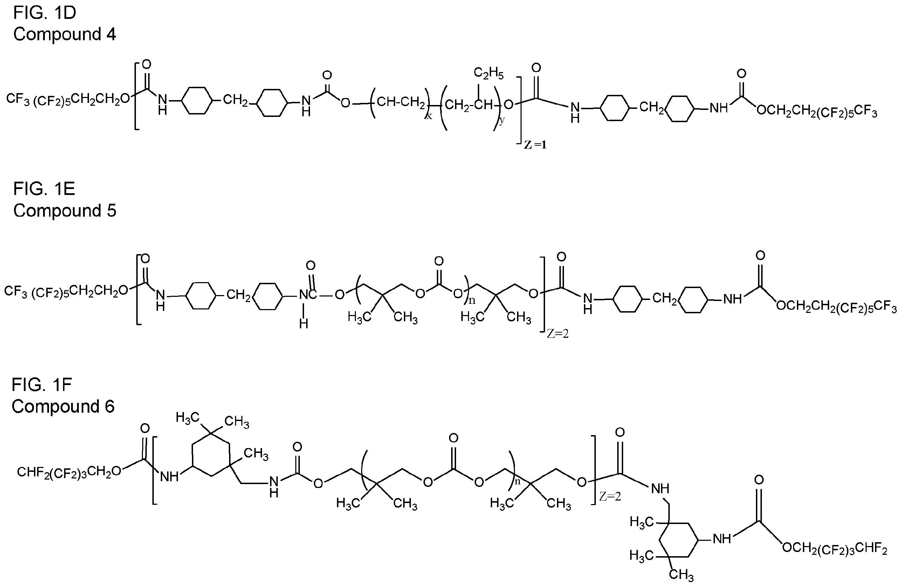

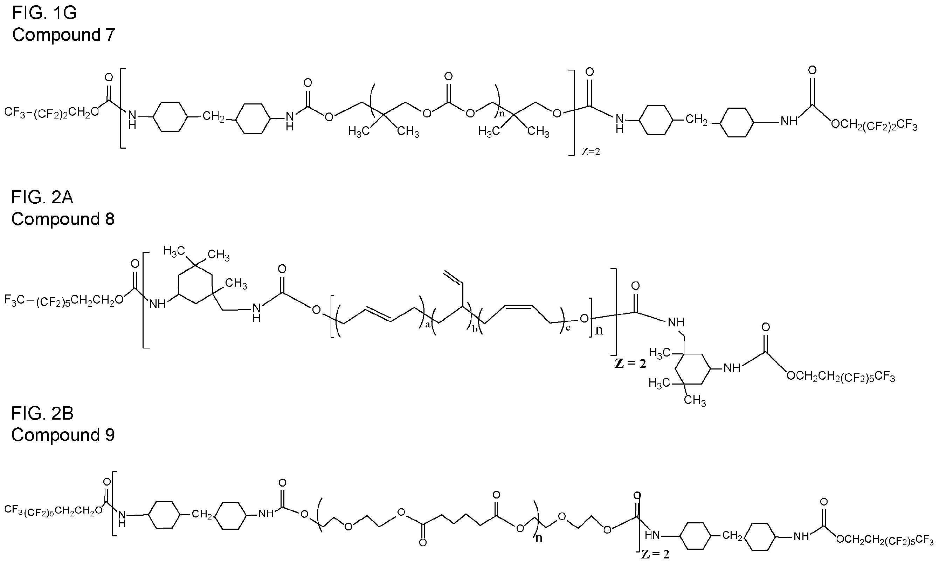

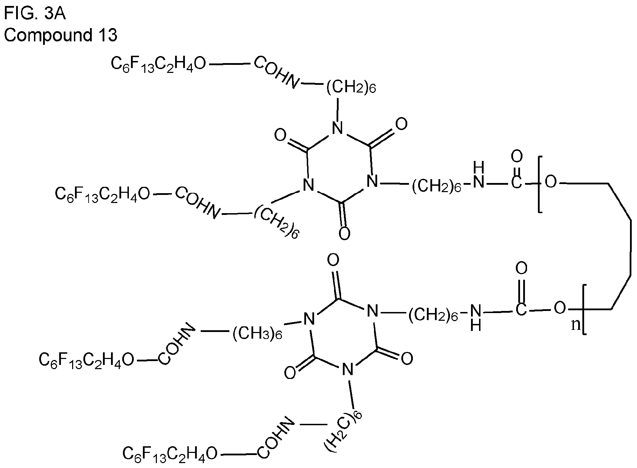

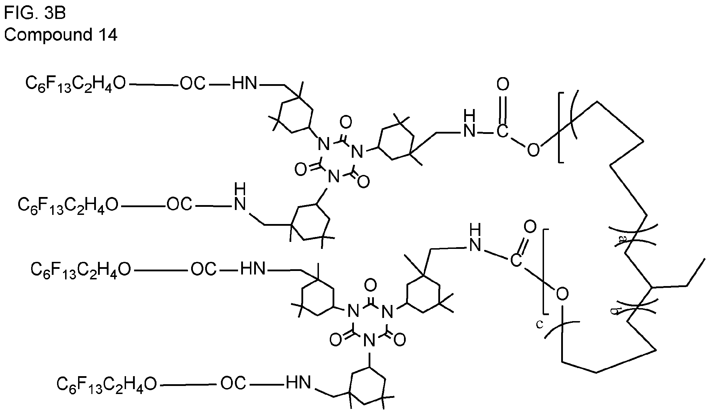

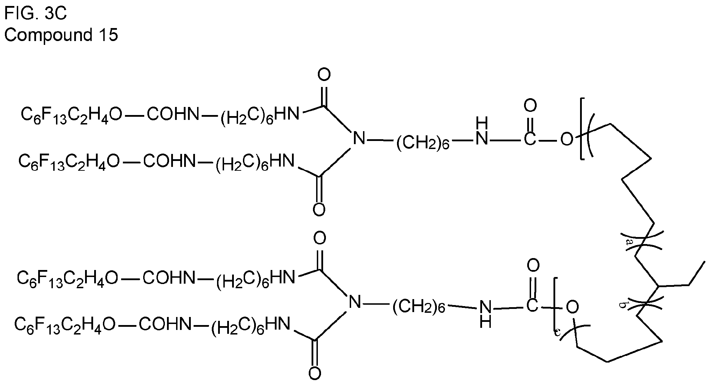

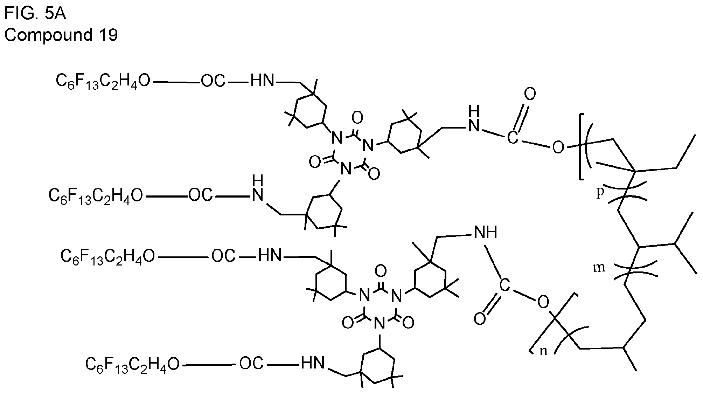

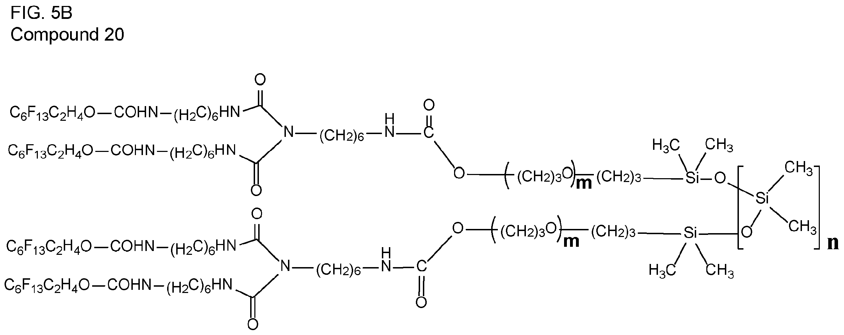

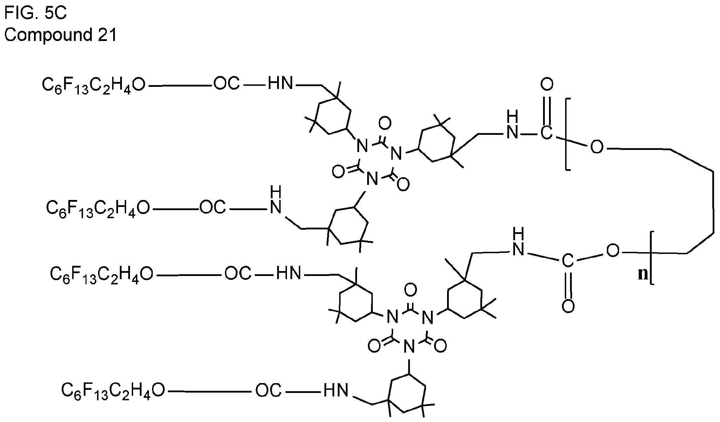

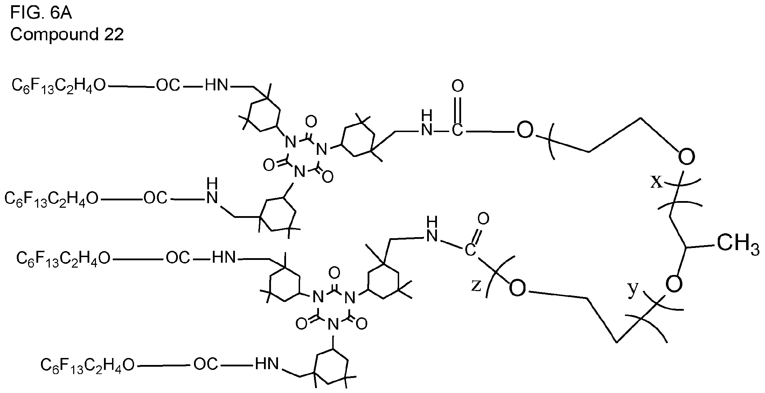

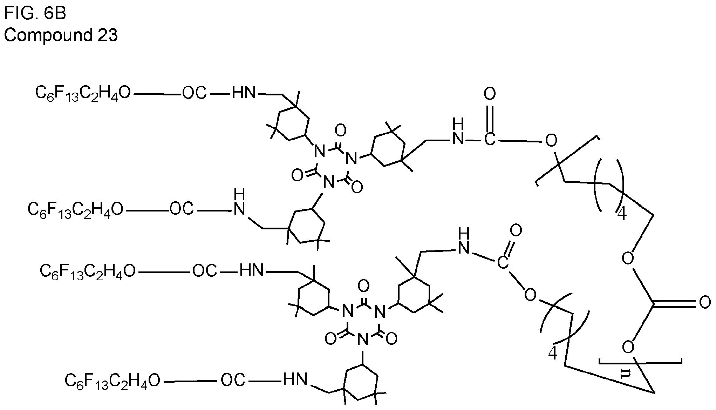

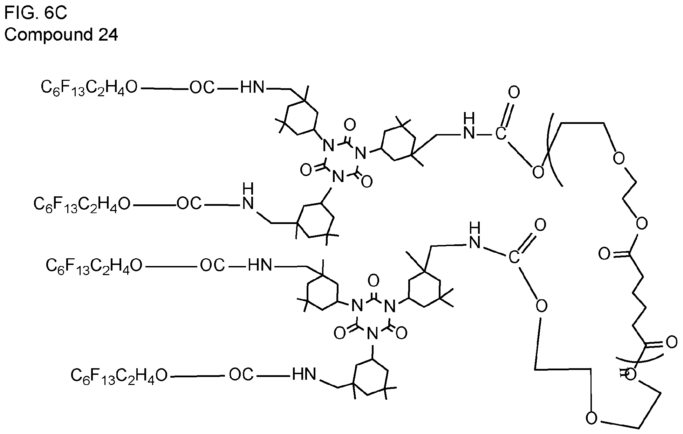

In some embodiments, the additive is a structure described by any one of formulae (I)-(XXI). In certain embodiments, the additive is any one of compounds 1-57. The theoretical structures of compounds 1-57 are illustrated in FIGS. 1-13.

In some embodiments, the additive of any one of formulae (I)-(XXI) is a linear surface modifying molecule (SMM) of formula (A) or a branched SMM of formula (B1 or B2):

F_{T}-[B-A]ₙ-B-F_{T} (A)

where B is a hard segment; A is a soft segment; F_{T} is a polyfluoroorgano group; and n is an integer from 1 to 10, or 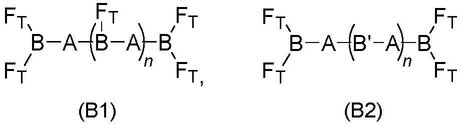 where A is a soft segment; B is a branched hard segment; B', when present, is linear hard segment; each F_{T} is a polyfluoroorgano group; and n is an integer between 0 to 10, and includes the soft and hard segments of any one of the exemplary additives provided in Table 1 below, where the hard and soft segments are defined in the Examples;
or wherein the additive is an SMM of formula (C1 or C2): 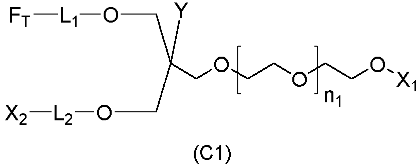 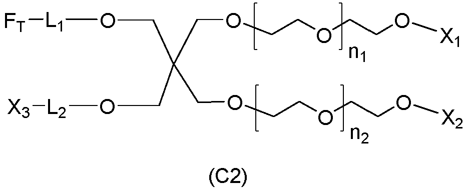 where F_{T} is a polyfluoroorgano group, each of X₁, X₂, and X₃ is, independently, H, CH₃, CH₂CH₃, or F_{T}, Y is H, CH₃, or CH₂CH₃, each of L₁ and L₂ is, independently, a bond, an oligomeric linker, a linker with two terminal carbonyls, or is formed from a diisocyanate, and each of n₁ and n₂ is, independently, an integer from 5 to 50.

**Table 1**

| **Additive** | **Soft segment** | **Hard segment** |
|---|---|---|
| **Additives of Formulas (A), (B1), or (B2)** | | |
| SMM 1 | PLN | HMDI |
| SMM 2 | PEGA | HMDI |
| SMM 3 | PDP | HMDI |
| SMM 4 | HLBH | HMDI |
| SMM 5 | C10 (diol) | HMDI |
| SMM 6 | PTMO | HDI |
| SMM 7 | PPO | HDI |
| SMM 8 | PLN | HDI |
| SMM 9 | PTMO | IPDI trimer isocyanurate |
| SMM 10 | PLN | IPDI trimer isocyanurate |
| SMM 11 | PEGA | IPDI trimer isocyanurate |
| SMM 12 | PDP | IPDI trimer isocyanurate |
| SMM 13 | C22 | HDI biuret |
| SMM14 | PTMO | HDI trimer isocyanurate |
| SMM 15 | Polyethylene glycol) | HMDI |

| **Additives of Formula (C1) or (C2)** | | |
|---|---|---|
| SMM 16 | Trimethylolpropane ethoxylate | |

In particular embodiments of formulations SMM 1 - SMM 16, PLN has a theoretical molecular weight of about 1,000 Da - 15,000 Da (e.g., about 1,900 Da, about 3,000 Da); PEGA has a theoretical molecular weight of about 1,000 Da - 10,000 Da (e.g., about 2,500 Da); PDP has a theoretical molecular weight of about 1,000 Da - 10,000 Da (e.g., about 2,000 Da); HLBH has a theoretical molecular weight of about 1,000 Da - 10,000 Da (e.g., about 2,000 Da); C10 (diol) has a theoretical molecular weight of about 1,000 Da - 10,000 Da; PPO has theoretical molecular weight of about 1,000 Da - 10,000 Da (e.g., about 1,000 Da); and polyethylene glycol) has a theoretical molecular weight of about 1,000 Da - 10,000 Da (e.g., about 2,000 Da).

In particular embodiments of formulations SMM 1 - SMM 16, F_{T} is a polyfluoroorgano group having a theoretical molecular weight of from 100 Da to 1,500 Da. For example, F_{T} may be CF₃(CF₂)ᵣ(CH₂CH₂)ₚ- wherein p is 0 or 1, r is 2-20, and CF₃(CF₂)ₛ(CH₂CH₂O)_{X}, where X is from 0 to 10 and s is from 1 to 20. Alternatively, F_{T} may be CHₘF₍₃₋ₘ₎(CF₂)ᵣCH₂CH₂- or CHₘF₍₃₋ₘ₎(CF₂)ₛ(CH₂CH₂O)_{X}-, where m is 0, 1, 2, or 3; X is an integer from 0 to 10; r is an integer from 2 to 20; and s is an integer from 1 to 20. In certain embodiments, F_{T} is 1H,1H,2H,2H-perfluoro-1-decanol; 1H,1H,2H,2H-perfluoro-1-octanol; 1H,1H,5H-perfluoro-1-pentanol; or 1H,1H-perfluoro-1-butanol, or a mixture thereof. In particular embodiments, F_{T} is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10. The F_{T} moieties can be formed from C6-FOH, C8-FOH, C6-C8 FOH, C10-FOH, C8-C10 FOH, C5-FOH, C4-FOH, and/or C3-FOH (as defined in the Examples).

In certain embodiments, any of formulations SMM 1 - SMM 16 is admixed with a base polymer to produce a surface resistant to bacterial adhesion. In particular embodiments, the base polymer is selected from the group including silicone, polyolefin, polyester, polycarbonate, polysulfone, polyamide, polyether, polyurea, polyurethane (PU), polyetherimide, polystyrene, cellulosic polymer, polypropylene (PP), polyethylene (PE), polyvinylchloride (PVC), polyvinyl alcohol (PVA), polyvinylpyrrolidone (PVP), polyacrylamide (PAAM), polyethylene oxide (PEO), polyethylene oxide)-b-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyether-b-polyamide, a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), poly(styrene-*block-*isobutylene-*block*-styrene) (SIBS), and ethylene-vinyl acetate (EVA),. In certain embodiments, any of SMM 1 - SMM 16 is admixed with a polyurethane (PU) base polymer. The PU base polymer can be any PU base polymer described herein, e.g., a polyether-based polyurethane (e.g., TECOFLEX^{®}, QUADRA-FLEX^{®}), a polycarbonate-based polyurethane (e.g., CARBOTHANE^{®}, BIONATE^{®}, QUADRATHANE^{™}), an aromatic polyester or polyether polyurethanes (e.g., PELLETHANE^{®}, TECOTHANE^{®}), an aliphatic or aromatic polyester or polyether polyurethanes (e.g., ELASTOLLAN^{®}), a hydrophilic polyurethanes (e.g., TECOPHILIC^{®}, HYDROTHANE^{®}), or a silicone-polyurethane copolymers (e.g., CARBOSIL^{®}, ELASTEON^{™}). For example, any of SMM 1 - SMM 16 can be admixed with CARBOTHANE^{®}, CHRONOFLEX^{®}AL (aliphatic), CHRONOFLEX^{®} AR (aromatic), CHRONOFLEX^{®} C (aromatic), CARBOSIL^{®} and BIONATE^{®} (aromatic), TECOFLEX^{®}, PELLETHANE^{®}, and ELASTOLLAN^{®}, in a variety of durometers 80A, 85A, 88A, 90A, 95A, 55D, and 75D. In some embodiments, any of SMM 1 - SMM 16 is admixed with a silicone base polymer. The silicone base polymer can be any silicone base polymer described herein. For example, any of SMM 1 - SMM 16 can be admixed with medical grade silicone elastomers, such as MED-4780, MED-4765, MED3-6320, MED-6340, MED-6345, MED-6350 from Nusil^{™}, or Silastic^{®} Q7-4780 or Dow Corning^{®} C6-235. In further embodiments, any of SMM 1 - SMM 16 is admixed with a polyethylene base polymer, a PVC base polymer, a SIBS base polymer, a polyamide base polymer, a polypropylene base polymer, or an EVA base polymer.

### Use in combination with antimicrobial agents

The invention features uses and described are articles, devices, and surfaces, which are not included in the invention, that may increase the susceptibility of bacteria present on the polymeric surface to an antimicrobial that may be present in the surrounding environment. Antimicrobial agents that can be applicable in the methods and articles, devices, and surfaces of the invention include, without limitation, silver, penicillin G, penicillin V, methicillin, oxacillin, cloxacillin, dicloxacillin, nafcillin, ampicillin, amoxicillin, carbenicillin, ticarcillin, mezlocillin, piperacillin, azlocillin, temocillin, cepalothin, cephapirin, cephradine, cephaloridine, cefazolin, cefamandole, cefuroxime, cephalexin, cefprozil, cefaclor, loracarbef, cefoxitin, cefmatozole, cefotaxime, ceftizoxime, ceftriaxone, cefoperazone, ceftazidime, cefixime, cefpodoxime, ceftibuten, cefdinir, cefpirome, cefepime, BAL5788, BAL9141, imipenem, ertapenem, meropenem, astreonam, clavulanate, sulbactam, tazobactam, streptomycin, neomycin, kanamycin, paromycin, gentamicin, tobramycin, amikacin, netilmicin, spectinomycin, sisomicin, dibekalin, isepamicin, tetracycline, chlortetracycline, demeclocycline, minocycline, oxytetracycline, methacycline, doxycycline, erythromycin, azithromycin, clarithromycin, telithromycin, ABT-773, lincomycin, clindamycin, vancomycin, oritavancin, dalbavancin, teicoplanin, quinupristin and dalfopristin, sulphanilamide, para-aminobenzoic acid, sulfadiazine, sulfisoxazole, sulfamethoxazole, sulfathalidine, linezolid, nalidixic acid, oxolinic acid, norfloxacin, perfloxacin, enoxacin, ofloxacin, ciprofloxacin, temafloxacin, lomefloxacin, fleroxacin, grepafloxacin, sparfloxacin, trovafloxacin, clinafloxacin, gatifloxacin, moxifloxacin, gemifloxacin, sitafloxacin, metronidazole, daptomycin, garenoxacin, ramoplanin, faropenem, polymyxin, triclosan, rifampin, minocycline, tigecycline, AZD2563, and trimethoprim. In some embodiments, the antimicrobial agents are in a disinfectant form. Non-limiting examples of disinfectants are aldehydes (e.g., formaldehyde, or glutaraldehyde, ortho-phthalaldehyde), hydrogen peroxide, peracetic acid, hydrogen peroxide/peracetic acid combination, chlorine-based agents (e.g., sodium hypochlorite), iodophors (e.g., iodine, or povidone iodine), chlorhexidine, alcohols (e.g., isopropyl alcohol, or phenols), or quaternary ammonium compounds.

### Use in combination with radiopaque materials

The polymeric surfaces can include other materials, such as radiopaque materials (e.g., as powders or other particulates). Suitable radiopaque additives include bismuth subcarbonate, bismuth oxychloride, bismuth trioxide, tungsten, and preferably barium sulfate (e.g., from 1% to 45% (w/w) (e.g., 1% to 10% (w/w), 10% to 30% (w/w), 20% to 40% (w/w), 25% to 45% (w/w), 20% to 35% (w/w), 25% to 40% (w/w), 30% to 45% (w/w), or 35% to 45% (w/w)) radiopaque material). Other additives include colorants such as pigments, dyes, or other suitable colorant materials.

### Applications

Articles, which are not included in the invention, that can formed from, or coated with, the admixtures include films, packaging materials, particles, fibers (wound dressings, bandages, gauze, tape, pads, sponges, including woven and non-woven sponges and those designed specifically for dental or ophthalmic surgeries), surgical, medical or dental instruments, dental devices or implants, blood oxygenators, ventilators, pumps, drug delivery devices, tubing, wiring, electrodes, contraceptive devices, feminine hygiene products, endoscopes, grafts (including small diameter<6 mm), stents (including coronary, ureteral, renal, biliary, colorectal, esophageal, pulmonary, urethral, and vascular), stent grafts (including abdominal, thoracic, and peripheral vascular), pacemakers, implantable cardioverter-defibrillators, cardiac resynchronization therapy devices, cardiovascular device leads, ventricular assist devices and drivelines, heart valves, vena cava filters, endovascular coils, catheters (including central venous, peripheral central, midline, peripheral, tunneled, dialysis access, urinary, neurological, peritoneal, intra-aortic balloon pump, angioplasty balloon, diagnostic, interventional, drug delivery, etc.), catheter connectors and valves (including needleless connectors), intravenous delivery lines and manifolds, shunts, (internal or external including ventricular, ventriculoatrial, ventriculoperitoneal, and lumboperitoneal), wound drains, drainage catheters, dialysis membranes, infusion ports, cochlear implants, endotracheal tubes, tracheostomy tubes, ventilator breathing tubes and circuits, guide wires, fluid collection bags, drug delivery bags and tubing, implantable sensors (e.g., intravascular, transdermal, intracranial), ophthalmic devices including contact lenses, punctal plugs, intra-ocular devices or implants, orthopedic devices (including hip implants, knee implants, shoulder implants, spinal implants (including cervical plates systems, pedicle screw systems, interbody fusion devices, artificial disks, and other motion preservation devices), screws, plates, rivets, rods, intramedullary nails, bone cements, artificial tendons, and other prosthetics or fracture repair devices), dental implants, periodontal implants, breast implants, penile implants, maxillofacial implants, cosmetic implants, valves, appliances, scaffolding, suturing material, needles, hernia repair meshes, tension-free vaginal tape and vaginal slings, prosthetic neurological devices, ear tubes, feeding tubes, blood bags, tissue regeneration or cell culture devices, or other medical devices used within or in contact with the body or any portion of any of these.

In one embodiment, the article, which is not included in the invention, is a vascularly inserted device such as a peripherally inserted central catheter (PICC), central venous catheter (CVC), hemodialysis catheter, or venous valve. In another embodiment, the substrate is a vascularly inserted catheter formed from a medical grade silicone or polyurethane, such as CARBOTHANE^{®}, or formed from a material coated with a medical grade silicone or polyurethane.

The admixtures can also be added to coatings and filters to prevent mildew, bacterial contamination, and in other applications where it is desirable to prevent bacteria mediated fouling, such as marine applications (e.g., exterior surfaces of marine vessels including ships and associated bilge tanks, gray water tanks, and water inlet/outlet pipes), airline industry, furniture industry (e.g., children's cribs, handles on exercise equipment, or exercise equipment), transportation industry (e.g., ambulances, buses, or public transit), swimming pools, fuel tanks, oil pipelines, industrial piping, pharmaceutical equipment, drug delivery devices such as inhalers, contact lenses, dental implants, coatings for in vivo sensors, textiles such as hospital drapes, gowns, or bedding, ventilation conduits, doorknobs, devices for waste water treatment, water purification, bioreactors, and food processing.

Articles, which are not included in the invention, that can be formed from, or coated with, the admixtures can also include consumer and/or consumable products, e.g., clothing/protective personal wear, personal care product containers, food containers, food and preparation surfaces, general storage containers, contraceptive devices, feminine hygiene products, electronic devices, adult toys (e.g., silicone adult toys), and office supplies (e.g., paper, pens, file folders, notes, notebooks, and toner or ink cartridges).

The following examples, as set forth below, are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

### Examples

### Example 1: Preparation of additives

The additives used in the articles of the invention can be prepared using methods known in the art from the appropriately selected reagents, such as diisocyanates, triisocyanates, dicarboxylic acids, diols, perfluorinated acid chlorides, bischloroformates, and fluorinated alcohols to form a wide range of additives. The reagents include but are not limited to the component reagents mentioned below.

### Diisocyanates

HMDI = 4,4'-methylene bis(cyclohexyl isocyanate)
IPDI = isophorone diisocyanate
TMXDI = m-tetramethylenexylene diisocyanate
HDI = hexamethylene diisocyanate

### Triisocyanates

Desmodur N3200 or Desmodur N-3200 = hexamethylene diisocyanate (HDI) biuret trimer
Desmodur Z4470A or Desmodur Z-4470A = isophorone diisocyanate (IPDI) trimer
Desmodur N3300 = hexamethylene diisocyanate (HDI) trimer

### Diols/Polyols

HLBH = hydrogenated-hydroxyl terminated polybutadiene,
PCN = poly(2,2-dimethyl-1-3-propylenecarbonate) diol
PHCN = poly(hexamethylene carbonate)diol
PEB = poly(ethylene-co-butylene)diol
LBHP = hydroxyl-terminated polybutadiene polyol
PEGA = poly(diethylene glycol)adipate
PTMO = poly(tetramethylene oxide) diol
PDP = diethylene glycol-ortho phthalic anhydride polyester polyol
HHTPI = hydrogenated hydroxyl-terminated polyisoprene
C22 = hydroxyl-terminated polydimethylsiloxanes block copolymer
C25 (diol) = hydroxy-terminated polydimethylsiloxane (ethylene oxide-PDMS-ethylene oxide) block copolymer
C10 (diol) = hydroxy-terminated polydimethylsiloxane (ethylene oxide-PDMS-ethylene oxide) block copolymer
PLN = polyethylene glycol)-*block*-poly(propylene glycol))-*block*-poly(ethylene glycol) polymer (PEO-PPO-PEO pluronic polymers)
PLN-Si = hydroxyl-terminated (PPO-PEO-PDMS-PEO-PPO) pluronic-siloxane copolymers
PLN3K = polyethylene glycol)-*block*-poly(propylene glycol))-*block*-poly(ethylene glycol) polymer (PEO-PPO-PEO pluronic polymers) (MW ≈ 3000 Da)
PLN8K = poly(ethylene glycol)-*block*-poly(propylene glycol))-*block*-poly(ethylene glycol) polymer (PEO-PPO-PEO pluronic polymers) (MW ≈ 8000 Da)
PEG2K (diol) = poly(ethylene glycol) diol (MW ≈ 2000 Da)
DDD = 1,12-dodecanediol
SPH = 1,6-hexanediol-ortho phthalic anhydride polyester polyol
SPN = neopentyl glycol-ortho phthalic anhydride polyester polyol
BPAE = bisphenol A ethoxylate diol
Ymer (diol) = hydroxy-terminated polyethylene glycol monomethyl ether
YmerOH(triol) = trimethylolpropane ethoxylate
Xmer (tetraol) = pentaerythritol ethoxylate

### Fluorinated End-Capping Groups

C6-FOH = (CF₃)(CF₂)₅CH₂CH₂OH (1H,1H,2H,2H-perfluorohexanol)
C8-FOH = 1H,1H,2H,2H-perfluorooctanol
C6-C8 FOH = (CF₃)(CF₂)₇CH₂CH₂OH and (CF₃)(CF₂)₅CH₂CH₂OH (mixtures of C6-FOH and C8-FOH; also designated as BAL-D)
C10-FOH = 1H,1H,2H,2H-perfluorodecanol
C8-C10 FOH = mixtures of C8-FOH and C10-FOH
C5-FOH = 1 H,1H,5H-perfluoro-1-pentanol
C4-FOH = 1H,1H-perfluorobutanol
C3-FOH = (CF₃)(CF₂)₂CH₂OH (1H,1H-perfluorobutanol)

### Non-Tin Based Catalyst

Bi348 = bismuth carboxylate type 1
Bi221 = bismuth carboxylate type 2
Bi601 = bismuth carboxylate type 3

The bismuth catalysts listed above can be purchased from King Industries (Norwalk CT). Any bismuth catalyst known in the art can be used to synthesize the additives described herein. Also, tin-based catalysts useful in the synthesis of polyurethanes may be used instead of the bismuth-based catalysts for the synthesis of the additives described herein.

### Compound 1

Compound 1 was synthesized with PPO diol of molecular weight 1000 Da, 1,6-hexamethylene diisocyanate (HDI), and the low boiling fraction of the fluoroalcohol (BA-L). The conditions of the synthesis were as follows: 10 g of PPO were reacted with 3.36 g of HDI for 2 h, and then 5 g of BA-L (low boiling fraction) were added to the reaction. The mixture was reacted with 42.5 mg of the catalyst, dibutyltin dilaurate, in 130 mL of dimethylacetamide, and the reaction temperature for the prepolymer step was maintained within 60-70 °C. The polystyrene equivalent weight average molecular weight is 1.6+/-0.2×10⁴ and its total fluorine content is 18.87+/-2.38% by weight. Thermal transitions for compound 1 are detectable by differential scanning calorimetry. Two higher order thermal transitions at approximately 14 °C and 85 °C were observed. The theoretical chemical structure of the compound 1 is shown FIG. 1A.

### Compound 2

All glassware used for the synthesis was dried in an oven at 110 °C overnight. To a 3-neck 1000 mL oven-dried flask equipped with a stir bar was added 175 g (72 mmol) of hydrogenated-hydroxyl terminated polybutadiene (HLBH polyol, MW = 2000 Da). The flask with the polyol was degassed overnight and then purged with dry N₂. A 1000 mL graduated cylinder was filled with 525 mL anhydrous toluene, sealed by a rubber septum and purged with dry N₂. The toluene was transferred to the 3-neck flask via a double-edged needle and the polyol stirred vigorously to dissolve in the solvent. The flask was placed in an oil bath at 65-70 °C. 39.70 g (151 mmol) of 4,4'-methylene bis(cyclohexyl isocyanate) (HMDI) was added to a degassed 250 mL flask equipped with a stir bar. To this flask was added 150 mL of anhydrous toluene from a degassed, N₂ purged 250 mL septa-sealed cylinder also using a double-edged needle and the mixture was stirred to dissolve the HMDI in the solvent. To a degassed 50 mL round bottom flask was added 8.75 g (5.00% w/w based on diol) of the bismuth carboxylate catalyst followed by 26 mL of toluene to dissolve the catalyst. The HMDI solution was transferred to the 1000 mL flask containing the polyol. The bismuth catalyst solution was added (20 mL) immediately following the addition of the HMDI. The reaction mixture was allowed to stir for 5 h at 70 °C to produce a HMDI-HLBH prepolymer.

In another 50 mL round bottom flask 74.95 g (180 mmol) of C8-C10 FOH (mixture of C8-FOH and C10-FOH) was added, capped with a septum, degassed and then purged with N₂. This was added to the 1000 mL flask containing prepolymer. All additions and transfers were conducted carefully in an atmosphere of dry N₂ to avoid any contact with air. The resulting mixture was heated to 45 °C for 18 h to produce the SMM with the end-capped C8-C10 FOH. The SMM solution was allowed to cool to ambient temperature and formed a milky solution. The milky solution was precipitated in MeOH (methanol) and the resulting precipitate was washed repeatedly with MeOH to form a white viscous material with dough-like consistency. This viscous, semi-solid material was washed twice in THF/EDTA (ethylene diamine tetraacetic acid) to remove residual catalyst followed by two more successive washes in THF/MeOH to remove unreacted monomers, low molecular weight byproducts, and catalyst residues. The SMM was first dried in a flow oven from at 40-120 °C in a period of 10 h gradually raising the temperature and finally dried under vacuum at 120 °C (24 h) and stored in a desiccator as a colorless rubbery semi-solid. The theoretical chemical structure of compound 2 is shown FIG. 1B.

### Compound 3

The reaction was carried out as described for compound 2 using 180 g (74 mmol) hydrogenated-hydroxyl terminated polybutadiene (HLBH polyol, MW = 2000 Da) and 30.14 g (115 mmol) of 4,4'-methylene-bis(cyclohexyl isocyanate) (HMDI) to form the prepolymer. The prepolymer was end-capped with 40.48 g (111.18 mmol) of 1H,1H,2H,2H-perfluoro-1-octanol (C8-FOH) to form compound 3 as a colorless rubbery semi-solid. As described above, the couplings were carried out in the presence of bismuth carboxylate catalyst, and compound 3 was washed similarly to compound 2 and dried prior to use. The theoretical chemical structure of compound 3 is shown in FIG. 1C.

### Compound 4

The reaction was carried out as described for compound 3 using 10 g (4 mmol) poly(ethylene-*co*-butylene (PEB polyol, MW= 2500 Da) and 2.20 g (8.4 mmol) of 4,4'-methylene-bis(cyclohexyl isocyanate) (HMDI) to form the prepolymer. The prepolymer was capped with 3.64 g (10 mmol) of 1H, 1H, 2H, 2H-perfluoro-1-octanol (C8-FOH) to form compound 4. As described above, the couplings were carried out in the presence of bismuth carboxylate catalyst, and the compound 4 was washed similarly to compound 2 and dried prior to use. The theoretical chemical structure of compound 4 is shown in FIG. 1D.

### Compound 5

The reaction was carried out as described for compound 4, except the solvent was changed from toluene to DMAc. Here, 100 g (100 mmol) poly(2,2-dimethyl-1,3-propylenecarbonate) diol (PCN, MW= 1000 Da) and 40.7 g (155 mmol) of 4,4'-methylene-bis(cyclohexyl isocyanate) (HMDI) to form a prepolymer. The prepolymer was end-capped with 45.5 g (125 mmol) of 1H,1H,2H,2H-perfluoro-1-octanol (C8-FOH) to form compound 5. The work-up after the reaction and the subsequent washing procedures are modified from the compound 4 synthesis as follows. Compound 5 from the reaction mixture in DMAc was precipitated in distilled water and washed successively in IPA/EDTA (isopropanol/ethylene diamine tetraacetic acid) solution followed by another wash in IPA/hexanes to remove unreacted monomers, low molecular weight byproducts, and catalyst residues to yield compound 5 as a white amorphous powder. As described above, the couplings were carried out in the presence of bismuth carboxylate catalyst and dried under vacuum prior to use. The theoretical chemical structure of compound 5 is shown in FIG. 1E.

### Compound 6

The reaction was carried out as described for compound 5 using 6.0 g (6.0 mmol) poly(2,2 dimethyl-1,3-propylenecarbonate) diol (MW= 1000 Da) and 1.90 g (8.5 mmol) of isophorone diisocyanate (IPDI) to form the prepolymer. The prepolymer was end-capped with 1.4 g (6.0 mmol) of 1H,1H,5H-perfluoro-1-pentanol (C5-FOH) to form compound 6 as a white amorphous solid. As described above, the couplings were carried out in the presence of bismuth carboxylate catalyst, and compound 6 was washed similarly to compound 5 and dried prior to use. The theoretical chemical structure of compound 6 is shown in FIG. 1F.

### Compound 7

The reaction was carried out as described for compound 5 using 10.0 g (10.0 mmol) poly(2,2-dimethyl-1 ,3-propylenecarbonate) diol (MW= 1000 Da) and 4.07 g (15.5 mmol) of 4,4'-methylene-bis(cyclohexyl isocyanate) (HMDI) to form the prepolymer. The prepolymer was capped with 2.5 g (12.5 mmol) of 1H, 1H-Perfluoro-1-butanol (C4-FOH) to form compound 8 as a white amorphous solid. As described above, the couplings were carried out in the presence of bismuth carboxylate catalyst, and compound 7 was washed similar to compound 5 and dried prior to use. The theoretical chemical structure of compound 7 is shown in FIG. 1G.

### Compound 8

The reaction was carried out as described for compound 5 using 180 g (84.8 mmol) hydroxyl-terminated polybutadiene (LBHP polyol, MW = 2000 Da) and 29.21 g (131.42 mmol) of isophorone diisocyanate (IPDI) to form the prepolymer. The prepolymer was capped with 46.31 g (127.18 mmol) of 1H,1H,2H,2H-perfluoro-1-octanol (C8-FOH) to form compound 8 as an off-white opaque viscous liquid. As described above, the couplings were carried out in the presence of bismuth carboxylate catalyst, and compound 8 was washed similarly to compound 5 and dried prior to use. The theoretical chemical structure of compound 8 is shown in FIG. 2A.

### Compound 9

The reaction was carried out as described for compound 5 using 10 g (3.92 mmol) poly(diethyhlene glycol adipate) (PEGA polyol, MW= 2500 Da) and 1.59 g (6.08 mmol) of 4,4'-methylene-bis(cyclohexyl isocyanate) (HMDI) to form a prepolymer. The prepolymer was capped with 2.14 g (5.88 mmol) of 1H,1H,2H,2H-perfluoro-1-octanol (C8-FOH) to form compound 9 as an off-white opaque viscous liquid. As described above, the couplings were carried out in the presence of bismuth carboxylate catalyst, and compound 9 was washed similarly to compound 5 and dried prior to use. The theoretical chemical structure of compound 9 is shown in FIG. 2B.

### Compound 10

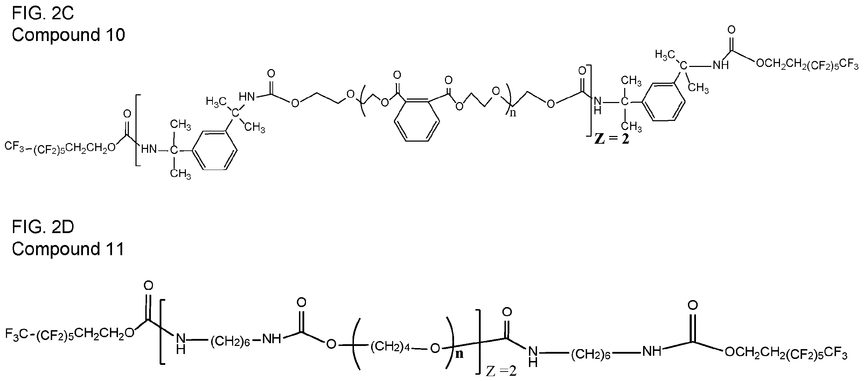

The reaction was carried out as described for compound 5 using 10 g (5.06 mmol), ortho phthalate-diethylene glycol-based polyester polyol (PDP polyol, MW = 2000 Da) and 1.92 g (7.85 mmol) of m-tetramethylenexylene diisocyanate (TMXDI) to form a prepolymer. The prepolymer was capped with 2.76 g (7.59 mmol) of 1H,1H,2H,2H-perfluoro-1-octanol (C8-FOH) to form compound 10 as a colorless solid. As described above, the couplings were carried out in the presence of bismuth carboxylate catalyst, and compound 10 was washed similarly to compound 5 and dried prior to use. The theoretical chemical structure of compound 10 is shown in FIG. 2C.

### Compound 11

Compound 11 was synthesized with PTMO diol (MW = 1000 Da), 1,6-hexamethylene diisocyanate (HDI), and the low boiling fraction of the fluoroalcohol (BA-L). The conditions of the synthesis were as follows: 10 g of PTMO were reacted with 3.36 g of HDI for two h and then 9 g of BA-L (low boiling fraction) were added to the reaction. The mixture was reacted with 60 mL of the catalyst, dibutyltin dilaurate, in 70 mL of dimethyl-acetamide (DMAc), and the reaction temperature for the prepolymer step was maintained within 60-70 °C. The polystyrene equivalent weight average molecular weight is 3.0×10⁴ and its total fluorine content is 7.98% by weight. The theoretical chemical structure of compound 11 is shown in FIG. 2D.

### Compounds 12-26

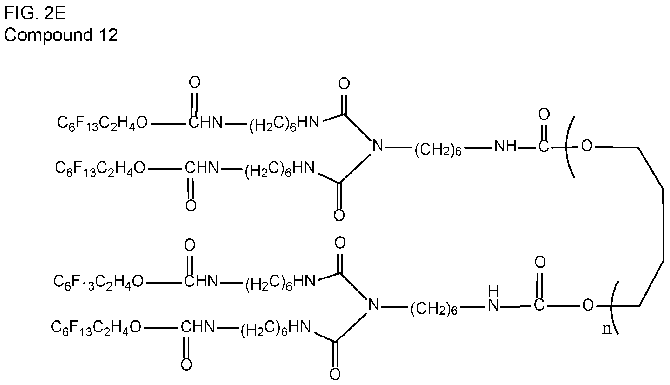

Surface modifiers of the invention such as compound 15 and compound 17 may be synthesized by a 2-step convergent method according to the schemes depicted in schemes 1 and 2. Briefly, the polyisocyanate such as Desmodur N3200 or Desmodur 4470 is reacted drop-wise with the surface-active group (e.g., a fluoroalcohol) in an organic solvent (e.g., anhydrous THF or dimethylacetamide (DMAc)) in the presence of a catalyst at 25 °C for 2 h. After addition of the fluoroalcohol, stirring is continued for 1 h at 50 °C and for a further 1 h at 70 °C. These steps lead to the formation of a partially fluorinated intermediate that is then coupled with the polyol (e.g., hydrogenated-hydroxyl terminated polybutadiene, or poly(2,2-dimethyl-1,3-propylenecarbonate)diol) at 70 °C over a period of 14 h to provide the SMM. Because the reactions are moisture sensitive, they are carried out under an inert N₂ atmosphere and anhydrous conditions. The temperature profile is also maintained carefully, especially during the partial fluorination, to avoid unwanted side reactions. The reaction product is precipitated in MeOH and washed several times with additional MeOH. The catalyst residues are eliminated by first dissolving the additive in hot THF or in hot IPA followed by reacting the additive with EDTA solution, followed by precipitation in MeOH. Finally, the additive is dried in a rotary evaporator at 120-140 °C prior to use. The theoretical chemical structures of compounds 12 to 26 are shown in FIGS. 2E to 7B.

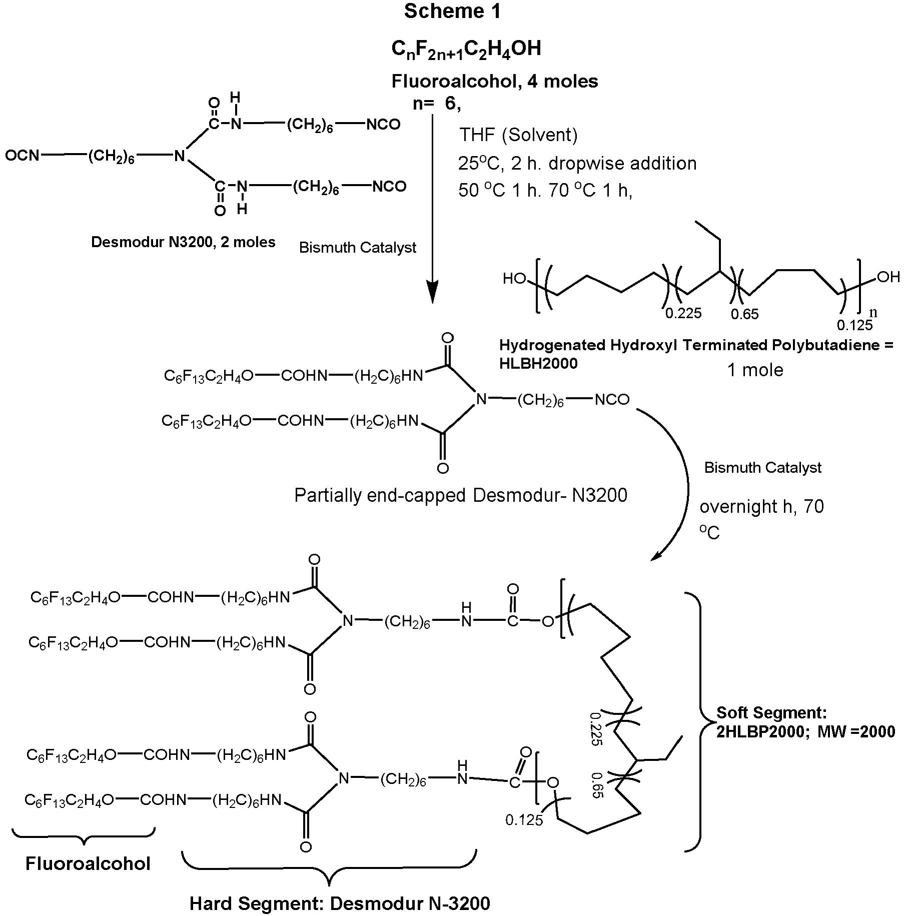

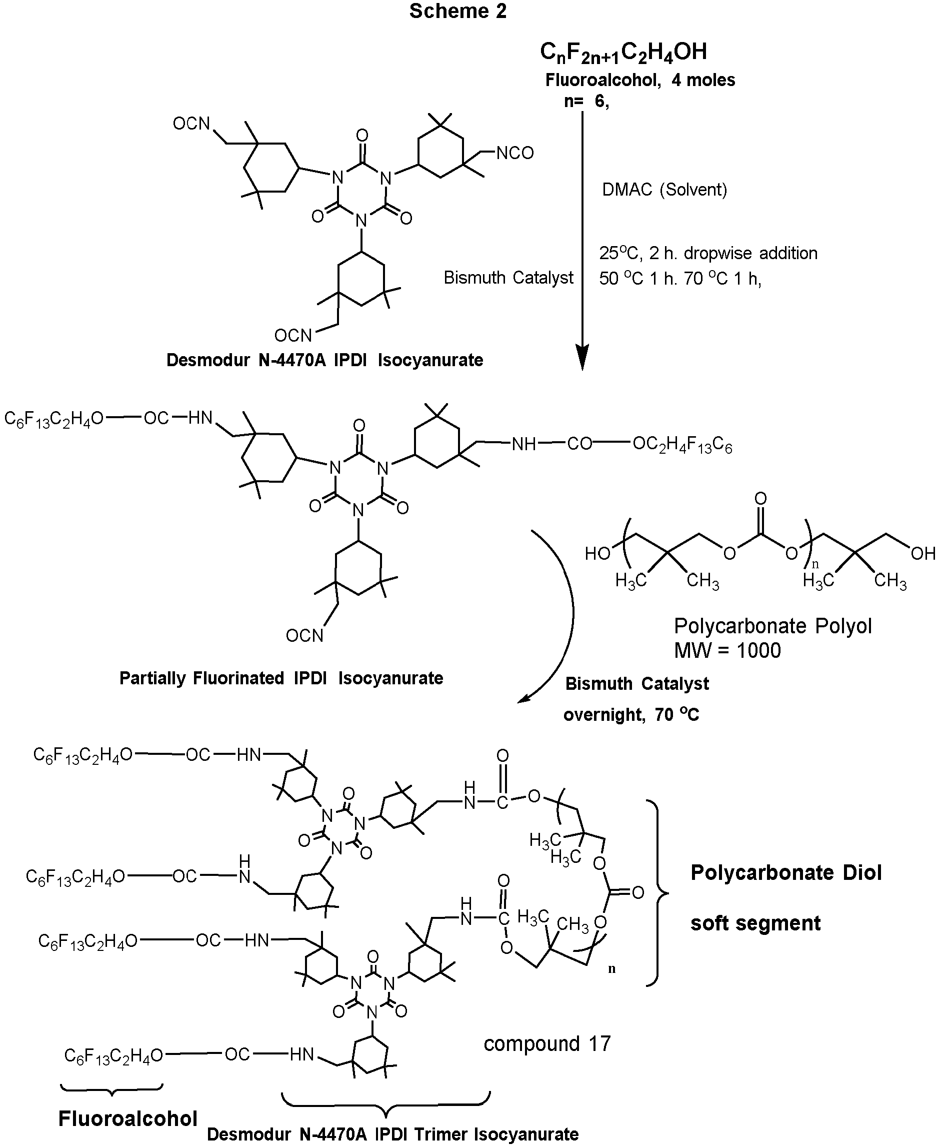

All glassware was dried in the oven overnight at 110 °C. To a 3-neck 5000 mL reactor equipped with a stir bar and a reflux condenser was added 300 g (583 mmol) of Desmodur N3300. The mixture was degassed overnight at ambient temperature. Hydrogenated-hydroxyl terminated polybutadiene (HLBH polyol MW = 2000 Da) was measured into a 2000 mL flask and degassed at 60 °C overnight. The bismuth catalyst K-Kat 348 (a bismuth carboxylate; available from King Industries) was measured out into a 250 mL flask and degassed overnight at ambient temperature. The perfluorinated alcohol was measured into a 1000 mL flask and degassed for 30 min at ambient temperature. After degassing, all the vessels were purged with N₂.

300 mL of THF (or DMAc) was then added to the Desmodur N3300 contaning vessel, and the mixture was stirred to dissolve the polyisocyanate. Similarly, 622 mL of THF was added to the HLBH polyol, and the mixture was stirred to dissolve the polyol. Likewise, 428 mL of THF (or DMAC) was added to the perfluorinated alcohol and the mixture was stirred to dissolve. Similarly, for K-Kat 348 which was dissolved in 77 mL of THF or DMAC. Stirring was continued to ensure all the reagents were dissolved in their respective vessels.

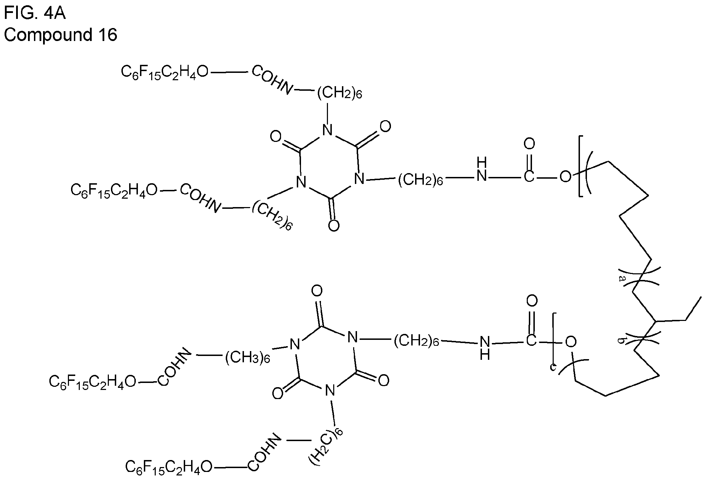

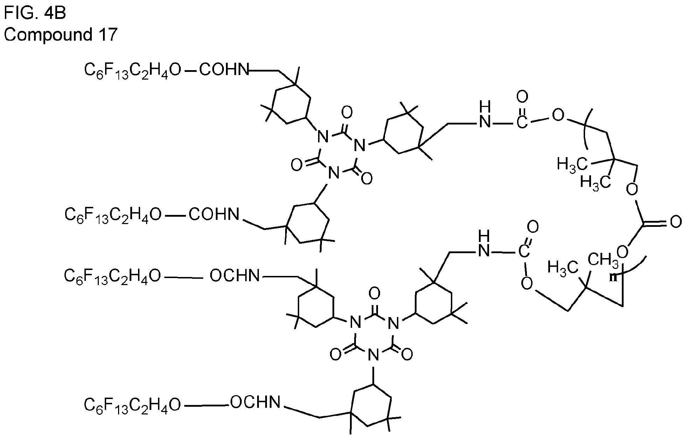

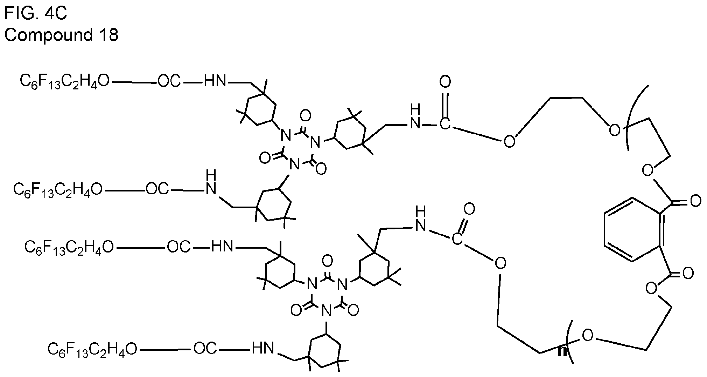

Half the K-Kat solution was transferred to the perfluorinated solution which was stirred for 5 min. This solution was added to the reaction vessel containing the Desmodur N3300A solution drop-wise over a period of 2 h at ambient (25 °C) temperature through a cannula (double ended needle) under positive N₂ pressure. After addition, the temperature was raised to 50 °C for 1 h and 70 °C for another 1 h. Proper stirring was maintained throughout. The remaining K-Kat 348 catalyst was transferred to the HLBH-2000 flask; after stirring to dissolve, this was added to the reactor containing the N3300. The reaction mixture was allowed to react overnight for 14 h at 70 °C to produce compound 16 with four fluorinated end groups. The theoretical chemical structure of compound 16 is shown in FIG. 4A.

### General Synthesis Description for Ester-based Additives

A diol such as Ymer diol, hydroxyl terminated polydimethylsiloxane, or polyols such as trimethylolpropane ethoxylate or pentaerythritol ethoxylate are reacted in a one-step reaction with a surface-active group precursor (e.g., perfluoroheptanoyl chloride) at 40 °C in a chlorinated organic solvent e.g., chloroform or methylene chloride in the presence of an acid scavenger like pyridine or triethylamine for 24 h. This reaction end-caps the hydroxyl groups with polyfluoroorgano groups. Because the reactions are moisture sensitive, the reactions are carried out under a N₂ atmosphere using anhydrous solvents. After the reaction, the solvent is rotary evaporated and the product is dissolved in tetrahydrofuran (THF) which dissolves the product and precipitates the pyridine salts which are filtered off, and the filtrate rotary evaporated further to dryness. The product is then purified by dissolving in minimum THF and precipitating in hexanes. This is performed three times and after which the final product is again rotary evaporated and finally dried in a vacuum oven at 60 °C overnight.

### Compound 27

Glassware used for the synthesis was dried in an oven at 110 °C overnight. To a 2-neck 1000 mL oven-dried round bottom flask equipped with a stir bar was added 85 g (24 mmol) of C25-diol (MW = 3500 Da). The flask with the diol was degassed overnight at 60 °C with gentle stirring and then purged with dry N₂ the following day. The heating was turned off. A 1000 mL graduated cylinder was charged with 320 mL anhydrous CHCl₃, sealed by a rubber septum and purged with dry N₂. The CHCl₃ was transferred to the 2-neck flask via a cannula and the diol stirred vigorously to dissolve in the solvent. Anhydrous pyridine (11.53 g, 146 mmol) was added to the C25-diol solution using a plastic syringe, and the resulting mixture was stirred to dissolve all materials. Another oven-dried 2-neck 1000 mL flask was charged with 32.51 g (85 mmol) of perfluoroheptanoyl chloride. The flask was sealed with rubber septa and degassed for 5 min, then purge with N₂. At this time, 235 mL of anhydrous CHCl₃ were added via cannula to the 1000 mL 2-neck flask containing the perfluoroheptanoyl chloride, and stirred at room temperature to dissolve the acid chloride. This flask was fitted with an addition funnel and the C25-diol-pyridine solution in CHCl₃ was transferred via a cannula into the addition funnel. N₂ flow through the reactor was adjusted to a slow and steady rate. Continuous drop-wise addition of C25-diol-pyridine solution to the acid chloride solution was started at room temperature, and was continued over a period of ~4 h. Stirring was maintained at a sufficient speed to achieve good mixing of reagents. After completing addition of the C25-diol-pyridine solution, the addition funnel was replaced with an air condenser, and the 2-neck flask was immersed in an oil bath placed on a heater fitted with a thermocouple unit. The temperature was raised to 40 °C, and the reaction continued at this temperature under N₂ for 24 h.

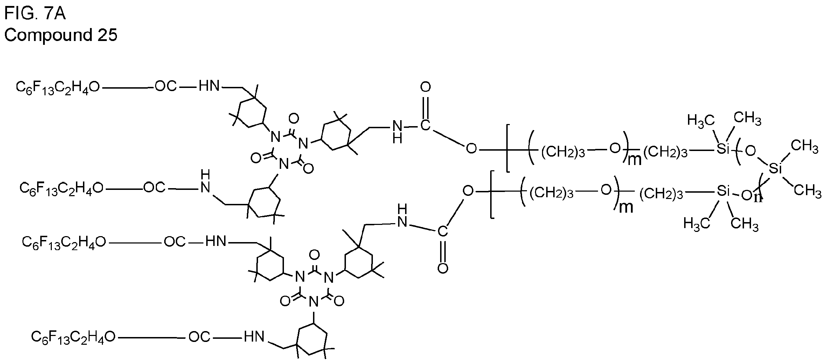

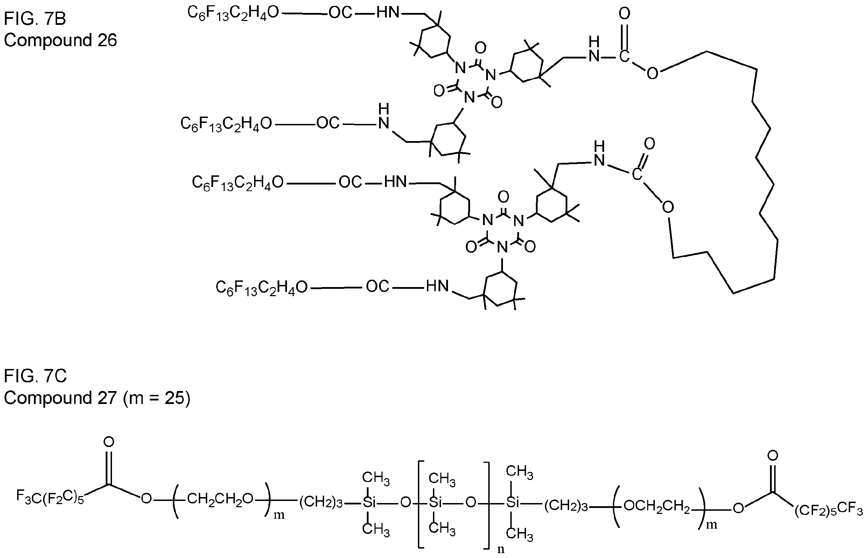

The product was purified by evaporating CHCl₃ in a rotary evaporator, and by filtering the pyridine salts after addition of THF. The crude product was then precipitated in isopropanol/hexanes mixture twice. The oil from the IPA/hexanes that precipitated was subjected to further washing with hot hexanes as follows. About 500 mL of hexanes was added to the oil in a 1 L beaker with a stir bar. The mixture was stirred while the hexanes was heated to boiling. The heating was turned off, and the mixture was allowed to cool for 5 min. The oil settled at the bottom, at which point the hexane top layer was decanted. The isolated oil was further dissolved in THF, transferred to a round bottom flask, and then the solvents were rotary evaporated. The oil was finally dried in a vacuum oven at 40 °C for 24 h. The purified product (a mixture of di- and mono-substituted products) was characterized by GPC (molecular weight based on polystyrene standards), Elemental Analysis for fluorine, ¹⁹F NMR, ¹H NMR and FTIR. Appearance: viscous oil. Weight average molecular weight (polystyrene equivalent) = 5791 g/mol. PD: 2.85. Elemental Analysis: F: 7.15%. ¹⁹F NMR (CDCl₃, 400 MHz, ppm): δ -80.78 (m, CF₃), -118.43 (m, CF₂), -121.85 (m, CF₂), -122.62 (m, CF₂), -126.14 (m, CF₂). ¹H NMR (CDCl₃, 400 MHz): δ ppm = 0.0 (m, CH₃Si), 0.3 (br m, CH₂Si), 1.4 (br m, CH₂), 3.30 (m, CH₂'s), 4.30 (m, CH₂COO-). FTIR, neat (cm⁻¹): 3392 (OH), 2868 (CH₂), 1781 (O-C=O, ester), 1241, 1212, 1141, 1087 (CF₃, CF₂,). The theoretical chemical structure of compound 27 is shown in FIG. 7C.

### Compound 28

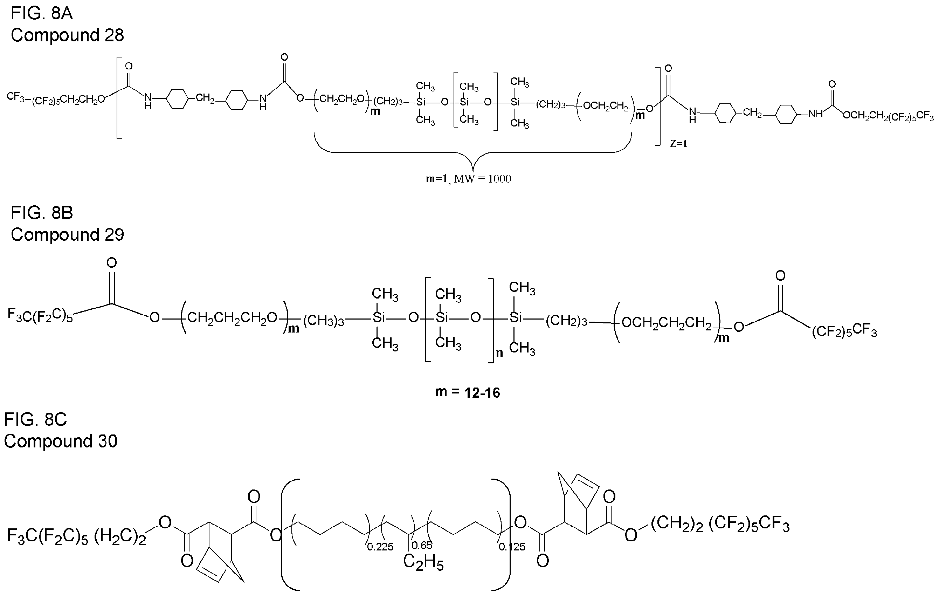

To a dried 5 L reactor, 394.4 g (1.5 mol) of HMDI followed by 350.6 g of THF was added, the reactor was placed in a water batch at 20 °C, and degassed by bubbling argon for 1 h. To a 2 L flask, 900 g (0.75 mol) of polysiloxane diol DMS-15 was added followed by 800.1 g of THF, and the solution was degassed at room temperature by bubbling argon for 30 min. To a 250 mL graduated cylinder was added 4.5 g of bismuth min. Half of the catalyst solution was transferred via cannula to the DMS-15 solution, and the mixture was stirred well. The DSM-15 solution was added to the main reactor containing HMDI slowly over a period of 1 h at constant rate. The solution was maintained at 20 °C with stirring. After addition, the temperature was raised to 45 °C, held for 1 h, and then increased to 65 °C for 1 h. To a dried 2 L addition flask was added 684.2 g (1.88 mol) of Capstone 62-AL, followed by 608.2 g of THF, and this was attached onto the reactor. It was degassed at room temperature by bubbling argon through it for 15 min. The remaining bismuth carboxylate catalyst was transferred to the Capstone 62-Al solutions, and the whole mixture was stirred well. The Capstone 62-Al fluoroalcohol was added to the main reactor, the temperature was raised to 70 °C, and the reaction allowed to proceed overnight with stirring under argon. After completion of reaction, the solution was allowed to cool to room temperature and 50% of the THF solvent was removed on a rotary evaporator. The remaining solution was added to 10 L of DI water in a 22 L flask equipped with mechanical stirrer to form a precipitate. Stirring was maintained for 10 min, and then stopped to allow for the precipitate to settle. The supernatant was decanted off, and the polymer was dissolved in 1500 g of isopropanol at 64 °C. The crude polymer was treated with 100 mL of EDTA solution (pH ~9) at 65 °C with stirring for 30 min, after which an additional 100 g of EDTA was added, and the stirring continued for 30 min at room temperature. Stirring was stopped and the solution was precipitated in 10 L of DI water in a 22 L flask with a mechanical stirrer. After 5 min, stirring was stopped and the polymer was allowed to settle, and the water was decanted off. The polymer was washed twice with 10 L of water, and then dried on a rotary evaporator to a maximum temperature of 115 °C to yield a yellow waxy solid (79%). Final drying was performed in a vacuum oven at 60 °C for 24 h. The purified product was characterized by GPC (molecular weight based on polystyrene standards), and Elemental Analysis for fluorine. The average molecular weight (polystyrene equivalent) was 9060 g/mol. Polydispersity = 1.47. Elemental Analysis shows %F = 19%. Thermal decomposition temperature (TGA, under N₂), at first onset: 266 °C (at 5 % wt. loss). The chemical structure of compound 28 is shown in FIG. 8A.

### Compound 29

Glassware used for the synthesis was dried in an oven at 110 °C overnight. To a 2-neck 100 mL oven-dried round bottom flask equipped with a stir bar was added 10 g (5 mmol) of PDMS C22-diol (C22 diol, MW = 3000 Da). The flask with the diol was degassed overnight at 60 °C with gentle stirring and then purged with dry N₂ the following day. Heating was turned off. A 100 mL graduated cylinder was filled with 50 mL anhydrous CHCl₃, sealed with a rubber septum, and purged with dry N₂. The CHCl₃ was transferred to the 2-neck flask via a cannula, and the diol was stirred vigorously to dissolve in the solvent. Anhydrous pyridine (0.53 g, 7 mmol) was then added to the C22-diol solution using a plastic syringe, and the resulting mixture was stirred to dissolve all materials. Another oven-dried 2-neck 250 mL flask was charged with 3.19 g (8 mmol) perfluoroheptanoyl chloride. The flask was then sealed with a rubber septum, and the mixture in the flask was degassed for 5 min, and purged with N₂. Then, 22 mL of anhydrous CHCl₃ were added using a graduated cylinder and a cannula to transfer the solvent to the 250 mL 2-neck flask containing the perfluoroheptanoyl chloride. The resulting mixture was stirred at room temperature to dissolve the acid chloride. The flask was then equipped with an addition funnel, and the C22-diol-pyridine solution in CHCl₃ was transferred to the addition funnel using a cannula. N₂ flow through the reactor was adjusted to a slow and steady rate. C22-diol-pyridine solution was then added continuously drop-wise to the acid chloride solution at room temperature over a period of ~4 h. Stirring was maintained at a sufficient speed to achieve good mixing of reagents. After completing the addition of the C22-diol, the addition funnel was replaced with an air condenser, and the 2-neck flask was immersed in an oil bath placed on a heater fitted with a thermocouple unit. The temperature was raised to 50 °C, and the reaction mixture was left at this temperature under N₂ for 24 h.

Then, heating and stirring were turned off. The flask was removed and its contents were poured into a round bottom flask. Volatiles were removed by rotary evaporation. Upon concentration, a dense precipitate (pyridine salts) formed. THF was added to dissolve the product, and the precipitated pyridine salts were removed by filtration using a coarse Whatman Filter paper (No 4), as the pyridine salts were insoluble in THF. Volatiles were removed by rotary evaporation. The crude product was then dissolved in 100 mL of CHCl₃ and poured into a separatory funnel. 150 mL of water and 5 mL of 5 N HCl were added to neutralize any remaining pyridine. The funnel was shaken, and the product was extracted into CHCl₃. The bottom CHCl₃ layer containing product was then washed in a separatory funnel sequentially with water, 5 mL of 5% (w/v) NaHCO₃ solution to neutralize any remaining HCl, and with distilled water. The CHCl₃ layer was separated and concentrated by rotary evaporation to obtain crude product, which was then dissolved in 10 mL of isopropanol. The resulting solution was added drop-wise to a 1 L beaker containing 200 mL of DI water with 1% (v/v) MeOH with continuous stirring. The product separated out as oil, at which time the solution was kept in an ice bath for 20 min, and the top aqueous layer was decanted. The oil was dissolved in THF and transferred into a 200 mL round bottom flask. The volatiles were removed by rotary evaporation at a maximum of 80 °C and 4 mbar to remove residual solvents. The resulting product was dried in a vacuum oven at 60 °C for 24 h to give a purified product as a light yellow, clear oil (-64% yield). The purified product was characterized by GPC (molecular weight based on polystyrene standards), and Elemental Analysis (for fluorine). Appearance: light yellow clear oil. Weight average molecular weight (polystyrene equivalent) Mw = 5589 Da. PD = 1.15. Elemental Analysis F: 12.86%. The theoretical chemical structure of compound 29 is shown in FIG. 8B.

### Compound 30

Glassware used for the synthesis was dried in an oven at 110 °C overnight. To a 2-neck 250 mL oven-dried round bottom flask equipped with a stir bar was added 20 g (8.0 mmol) of hydrogenated-hydroxyl terminated polybutadiene (HLBH diol, MW = 2000 Da). The flask with the diol was degassed overnight at 60 °C with gentle stirring, and then purged with dry N₂ the following day. At this time, the heating was turned off. A 200 mL graduated cylinder was charged with 104 mL anhydrous CHCl₃, sealed by a rubber septum, and purged with dry N₂. The CHCl₃ was transferred to the 2-neck flask via a cannula, and the diol was stirred vigorously to dissolve in the solvent. At this time, anhydrous pyridine (3.82 g, 48 mmol) was added to the HLBH diol solution using a plastic syringe, and the resulting mixture was stirred to dissolve all materials. Another oven-dried 2-neck 100 mL flask was charged with trans-5-norbornene-2,3-dicarbonyl chloride ("NCI;" 3.70 g, 17 mmol), sealed with rubber septa, degassed for 5 min, and then purged with N₂. At this time, 52 mL of anhydrous CHCl₃ were added using a graduated cylinder and a cannula to transfer the solvent to the 100 mL 2-neck flask containing NCI. The resulting mixture was stirred to dissolve NCI. The 250 mL 2-neck flask was then fitted with an addition funnel, and the solution of NCI in CHCl₃ was transferred to the addition funnel using a cannula. N₂ flow was adjusted through the reactor to a slow and steady rate. The solution of NCI was added continuously drop-wise to the HLBH-pyridine solution at room temperature over a period of ~1 h to form a pre-polymer. Stirring was maintained at a sufficient speed to achieve good mixing of reagents.

In parallel, another oven-dried 50 mL flask was charged with Capstone^{™} AI-62 perfluorinated reagent (5.45 g, 15 mmol). The flask was sealed with a rubber septum, degassed for 15 min, and purged with N₂. Anhydrous CHCl₃ (17 mL) and anhydrous pyridine (1.9 g, 24 mmol) were added. The mixture was stirred to dissolve all reagents. After the addition of the NCI solution to the 250 mL 2-neck flask was complete, the Capstone^{™} AI-62 perfluorinated reagent solution was added to this flask using a cannula with stirring. The addition funnel was replaced with an air condenser, and the 250-mL 2-neck flask was immersed in an oil bath placed on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts were insoluble in THF. THF was removed by rotary evaporation. The crude product was dissolved in 100 mL of CHCl₃ and was poured into a separatory funnel. 100 mL of water were added, followed by the addition of 5 mL of 5 N HCl to neutralize any remaining pyridine. The funnel was shaken, and the product was extracted into CHCl₃. The bottom CHCl₃ layer containing product was isolated and washed in a separatory funnel with water (5 mL of 5% NaHCO₃ solution were added to neutralize any remaining HCl). The organic layer was then washed once more with plain distilled water. Isolated CHCl₃ layer was concentrated by rotary evaporation to obtain crude product. The crude product was dissolved in 10 mL of isopropanol (IPA) and was then added drop-wise to a beaker containing 200 mL of DI water containing 1% (v/v) MeOH with continuous stirring. Product separated out as an oil. The mixture was kept in ice bath for 20 min, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~55% yield). The purified product (a mixture of di- and mono-substituted products) was characterized by GPC and Elemental Analysis for fluorine. Appearance: light yellow viscous liquid. Weight average molecular weight (polystyrene equivalent) = 12389 g/mol. PD: 1.43. Elemental Analysis: F: 10.6%. The theoretical chemical structure of compound 30 is shown in FIG. 8C.

### Compound 31

Compound 31 was prepared according to a procedure similar to compound 30. Glassware used for the synthesis was dried in an oven at 110 °C overnight. To a 2-neck 250 mL oven-dried round bottom flask equipped with a stir bar was added 15 g (6.0 mmol) of hydrogenated-hydroxyl terminated polybutadiene (HLBH diol, MW = 2000 Da). The flask with the diol was degassed overnight at 60 °C with gentle stirring, and then purged with dry N₂ the following day. At this time, the heating was turned off. A 100 mL graduated cylinder was charged with 12 mL anhydrous CHCl₃, sealed with a rubber septum, and purged with dry N₂. The CHCl₃ was transferred to the 2-neck flask via a cannula, and the diol was stirred vigorously to dissolve in the solvent. At this time, anhydrous pyridine (0.95 g, 12 mmol) was added to the HLBH diol solution using a plastic syringe, and the resulting mixture was stirred to dissolve all materials. Another oven-dried 2-neck 100 mL flask was charged with terephthaloyl chloride (2.57 g, 13 mmol), sealed with rubber septa, degassed for 5 min, and then purged with N₂. At this time, 85 mL of anhydrous CHCl₃ were added using a graduated cylinder and a cannula to transfer the solvent to the 100 mL 2-neck flask. The resulting mixture was stirred to dissolve terephthaloyl chloride. The 250 mL 2-neck flask was then fitted with an addition funnel, and the solution of terephthaloyl chloride in CHCl₃ was transferred to the addition funnel using a cannula. N₂ flow was adjusted through the reactor to a slow and steady rate. The solution of terephthaloyl chloride was added continuously drop-wise to the HLBH-pyridine solution at room temperature over a period of ~1 h to form a pre-polymer. Stirring was maintained at a sufficient speed to achieve good mixing of reagents.

In parallel, another oven-dried 50 mL flask was charged with Capstone^{™} AI-62 perfluorinated reagent (5.45 g, 15 mmol). The flask was sealed with a rubber septum, degassed for 15 min, and purged with N₂. Anhydrous CHCl₃ (12 mL) and anhydrous pyridine (0.95 g, 12 mmol) were added. The mixture was stirred to dissolve all reagents. After the addition of the terephthaloyl chloride solution to the 250 mL 2-neck flask was complete, the Capstone^{™} AI-62 perfluorinated reagent solution was added to this flask with stirring. The addition funnel was replaced with an air condenser, and the 250 mL 2-neck flask was immersed in an oil bath placed on a heater fitted with a thermocouple unit. The temperature was raised to 50 °C, and the reaction continued at this temperature under N₂ for 24 h.

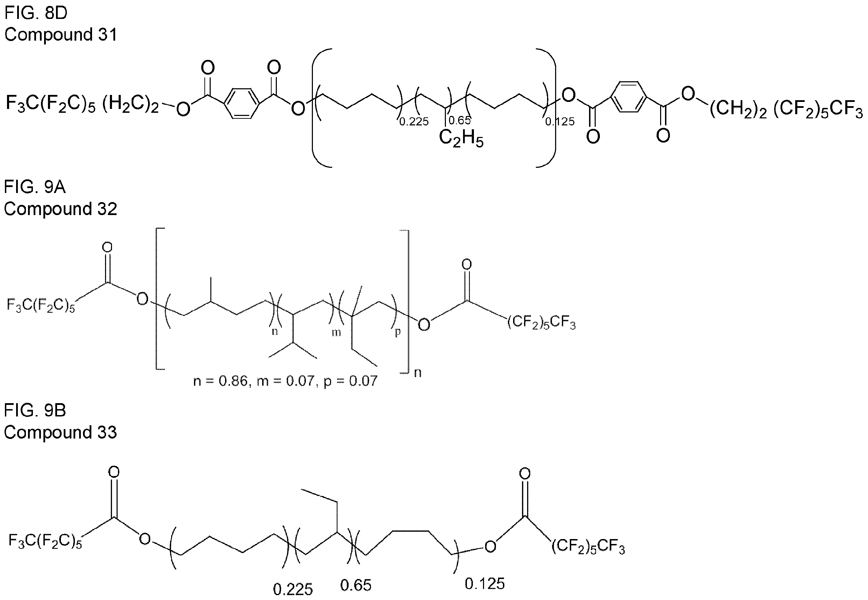

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts were insoluble in THF. THF was removed by rotary evaporation. The crude product was dissolved in 100 mL of CHCl₃ and was poured into a separatory funnel. 100 mL of water were added, followed by the addition of 5 mL of 5 N HCl to neutralize any remaining pyridine. The funnel was shaken, and the product was extracted into CHCl₃. The bottom CHCl₃ layer containing product was isolated and washed in a separatory funnel with water (5 mL of 5% NaHCO₃ solution were added to neutralize any remaining HCl). The organic layer was then washed once more with plain distilled water. Isolated CHCl₃ layer was concentrated by rotary evaporation to obtain crude product. The crude product was dissolved in 10 mL of isopropanol (IPA), and was then added drop-wise to a beaker containing 200 mL of DI water containing 1% (v/v) MeOH with continuous stirring. The product separated out as an oil. The mixture was kept in ice bath for 20 min, and the top water layer was decanted. The oil was dissolved in THF, and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~87% yield). The purified product (a mixture of di- and mono-substituted products) was characterized by GPC and Elemental Analysis for fluorine. Appearance: off-white viscous liquid. Weight average molecular weight (polystyrene equivalent) = 10757 g/mol. PD: 1.33. Elemental Analysis: F: 11.29%. The theoretical chemical structure of compound 31 is shown in FIG. 8D.

### Compound 32

Glassware used for the synthesis was dried in an oven at 110 °C overnight. To a 2-neck 100 mL oven-dried round bottom flask equipped with a stir bar was added 10 g (5 mmol) of hydrogenated-hydroxyl terminated polyisoprene (HHTPI diol, MW = 2000 Da). The flask with the diol was degassed overnight at 60 °C with gentle stirring, and then purged with dry N₂ the following day. At this time, the heating was turned off. A 100 mL graduated cylinder was charged with 50 mL anhydrous CHCl₃, sealed by a rubber septum, and purged with dry N₂. The CHCl₃ was transferred to the 2-neck flask via a cannula, and the diol was stirred vigorously to dissolve in the solvent. At this time, excess anhydrous pyridine (0.75 g, 9 mmol) was added to the HHTPI diol solution using a plastic syringe, and the resulting mixture was stirred to dissolve all materials. Another oven-dried 2-neck 250 mL flask was charged with perfluoroheptanoyl chloride (4.51 g, 12 mmol), sealed with rubber septa, degassed for 5 min, and then purged with N₂. At this time, 22 mL of anhydrous CHCl₃ was added using a graduated cylinder and a cannula to transfer the solvent to the 250 mL 2-neck flask containing the perfluoroheptanoyl chloride. The resulting mixture was stirred at room temperature to dissolve the acid chloride. An addition funnel was fitted to this flask, and the HHTPI-pyridine solution in CHCl₃ was added into the addition funnel. N₂ flow was adjusted through the reactor to a slow and steady rate. HHTPI-pyridine solution was added continuously drop-wise to the acid chloride solution at room temperature over a period of ~4 h. Stirring was maintained at a sufficient speed to achieve good mixing of reagents. After completing addition of the HHTPI diol, the addition funnel was replaced with an air condenser, and the 2-neck flask was immersed in an oil bath on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts were insoluble in THF. THF was removed by rotary evaporation. The crude product was dissolved in 100 mL of CHCl₃ and was poured into a separatory funnel. 150 mL of water were added, followed by the addition of 5 mL of 5 N HCl to neutralize any remaining pyridine. The funnel was shaken, and the product was extracted into CHCl₃. The bottom CHCl₃ layer containing product was isolated and washed in separatory funnel with water (5 mL of 5% NaHCO₃ solution were added to neutralize any remaining HCl). The organic layer was then washed once more with plain distilled water. Isolated CHCl₃ layer was concentrated by rotary evaporation to obtain crude product. The crude product was dissolved in 10 mL of isopropanol (IPA) and was added drop-wise to a 1 L beaker containing 200 mL of DI water containing 1% (v/v) MeOH with continuous stirring. Product separated out as an oil. The mixture was kept in ice bath for 20 min, and the top water layer was decanted. The oil was dissolved in THF, and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a colorless viscous oil (-99.9% yield). The purified product (a mixture of di- and mono-substituted products) was characterized by GPC and Elemental Analysis for fluorine. Appearance: colorless viscous liquid. Weight average molecular weight (polystyrene equivalent) = 12622 g/mol. PD: 1.53. Elemental Analysis: F: 13.50%. The theoretical chemical structure of compound 32 is shown in FIG. 9A.

### Compound 33

Glassware used for the synthesis was dried in an oven at 110 °C overnight. To a 2-neck 1000 mL oven-dried round bottom flask equipped with a stir bar was added 100 g (40 mmol) of hydrogenated-hydroxyl terminated polybutadiene (HLBH diol, MW= 2000 Da). The flask with the diol was degassed overnight at 60 °C with gentle stirring, and then purged with dry N₂ the following day. At this time, the heating was turned off. A 1000 mL graduated cylinder was charged with 415 mL anhydrous CHCl₃, sealed by a rubber septum, and purged with dry N₂. The CHCl₃ was transferred to the 2-neck flask via a cannula, and the diol was stirred vigorously to dissolve in the solvent. Excess anhydrous pyridine (19.08 g, 241 mmol) was added to the HLBH diol solution using a plastic syringe, and the resulting mixture was stirred to dissolve all materials. Another oven-dried 2-neck 1000 mL flask was charged with 38.45 g, (101 mmol) perfluoroheptanoyl chloride, sealed with rubber septa, degassed for 5 min, and then purged with N₂. At this time, 277 mL of anhydrous CHCl₃ was added using a graduated cylinder and a cannula to transfer the solvent to the 1000 mL 2-neck flask containing the perfluoroheptanoyl chloride. The resulting mixture was stirred at room temperature to dissolve the acid chloride. An addition funnel was fitted to this flask, and the HLBH-pyridine solution in CHCl₃ was added into the addition funnel using a cannula. N₂ flow was adjusted through the reactor to a slow and steady rate. Continuous drop-wise addition of HLBH-pyridine solution to the acid chloride solution was started at room temperature over a period of ~4 h. Stirring was maintained at a sufficient speed to achieve good mixing of reagents. After completing addition of the HLBH, the addition funnel was replaced with an air condenser, and the 2-neck flask was immersed in an oil bath on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts were insoluble in THF. THF was removed by rotary evaporation. The crude product was dissolved in 400 mL of CHCl₃ and was poured into a separatory funnel. 500 mL of water were added, followed by the addition of 20 mL of 5 N HCl to neutralize any remaining pyridine. The funnel was shaken, and the product was extracted into CHCl₃. The bottom CHCl₃ layer containing product was isolated, and washed in a separatory funnel with water (20 mL of 5% NaHCO₃ solution were added to neutralize any remaining HCl). The organic layer was then washed once more with plain distilled water. Isolated CHCl₃ layer was concentrated by rotary evaporation to obtain crude product. The crude product was dissolved in 20 mL of THF and was then added drop-wise to a 4 L beaker containing 1200 mL of DI water containing 1% (v/v) MeOH with continuous stirring. The product separated out as an oil. The mixture was kept in ice bath for 20 min, and the top hexane layer was decanted. The oil was dissolved in THF and transferred into 500 mL round bottom flask. THF was removed by rotary evaporation at a maximum temperature of 80 °C and 4 mbar to remove all residual solvents. The resulting product was dried in a vacuum oven at 60 °C for 24 h to give a purified product as a yellow viscous oil (~80% yield). The purified product (a mixture of di- and mono-substituted products) was characterized by GPC and Elemental Analysis for fluorine. Appearance: light yellow viscous liquid. Weight average molecular weight (polystyrene equivalent) = 6099 g/mol. PD: 1.08. Elemental Analysis: F: 12.84%. The theoretical chemical structure of compound 33 is shown in FIG. 9B.

### Compound 34

Glassware used for the synthesis was dried in an oven at 110 °C overnight. To a 2-neck 1000 mL oven-dried round bottom flask equipped with a stir bar was added 65 g (63 mmol) of Ymer-diol (MW = 1000 Da). The flask with the diol was degassed overnight at 60 °C with gentle stirring and then purged with dry N₂ the following day. At this time, heating was turned off. A 1000 mL graduated cylinder was charged with 374 mL anhydrous CHCl₃, sealed by a rubber septum, and purged with dry N₂. The CHCl₃ was transferred to the 2-neck flask via a cannula, and the diol was stirred vigorously to dissolve in the solvent. Excess anhydrous pyridine (30 g, 375 mmol) was added to the Ymer-diol solution using a plastic syringe, the resulting stir to dissolve all materials. Another oven-dried 2-neck 1000 mL flask was charged with 59.82 g (156 mmol) of perfluoroheptanoyl chloride, sealed with rubber septa, degassed for 5 min, then purged with N₂. At this time 250 mL of anhydrous CHCl₃ were added using a graduated cylinder and cannula to transfer the solvent to the 1000 mL 2-neck flask containing the perfluoroheptanoyl chloride. The resulting mixture was stirred at room temperature to dissolve the acid chloride. An addition funnel was fitted to this flask and using a cannula transfer the Ymer-diol-pyridine solution in CHCl₃ into the addition funnel. N₂ flow through the reactor was adjusted to a slow and steady rate. Ymer-diol-pyridine solution was added drop-wise, continuously to the acid chloride solution at room temperature over a period of ~4 h. Stirring was maintained at a sufficient speed to achieve good mixing of reagents. After completing the addition of the Ymer-diol-pyridine solution, the addition funnel was replaced with an air condenser, and the 2-neck flask was immersed in an oil bath placed on a heater fitted with a thermocouple unit. The temperature was raised to 40 °C, and the reaction continued at this temperature under N₂ for 24 h.

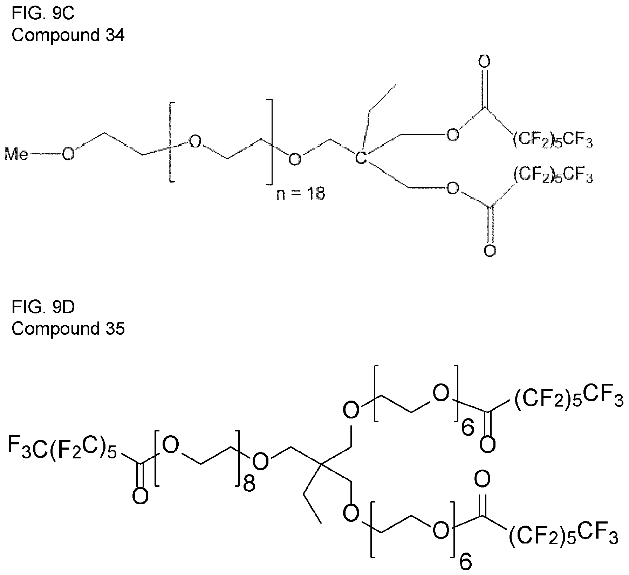

After the reaction, heating and stirring were turned off. The reaction flask was removed, and the contents were poured into a round bottom flask. CHCl₃ was removed by rotary evaporation. Upon concentration, a dense precipitate (pyridine salts) formed. THF was added to dissolve the product. The flask was cooled in an ice bath for 20 min, at which time, the precipitated pyridine salts were removed by gravity filtration using a coarse Whatman filter paper (No 4). Pyridine salts were insoluble in THF. THF was removed by rotary evaporation. The resulting crude product was dissolved in a minimum quantity of Isopropanol (IPA), and this solution was added to 700 mL of hexanes in a beaker with a stir bar. An oil separated out. The top layer was decanted and washed once with 200 mL of hexanes. The residue was then dissolved in 200 mL of THF and transferred to a 500 mL round bottom flask. Rotary evaporation of the solvents at a maximum temperature of 75 °C and 4 mbar vacuum furnished an oil, which was then transferred to a wide mouth jar. It was further dried for 24 h at 60 °C under vacuum to yield the pure product, which solidifies upon cooling at room temperature to an off white waxy semi-solid (82% yield). The purified product was characterized by GPC (molecular weight based on polystyrene standards), Elemental Analysis for fluorine, ¹⁹F NMR, ¹H NMR and FTIR. Appearance: waxy semi-solid. Weight average molecular weight (polystyrene equivalent) = 2498 g/mol. PD: 1.04. Elemental Analysis: F: 27.79%. ¹⁹F NMR (CDCl₃, 400 MHz): δ ppm -81.3 (m, CF₃), -118.88 (m, CF₂), -122.37 (m, CF₂), -123.28 (m, CF₂), -126 (m, CF₂). ¹H NMR (CDCl₃, 400 MHz): δ ppm 0.83 (t, CH₃CH₂), 1.44 (q, CH₂CH₃), 3.34 (m, CH₂), 3.51 (m, CH₂), 3.54 (m, CH₂), 4.30 (m, CH₂COO-). FTIR, neat (cm⁻¹): 2882 (CH2), 1783 (O-C=O, ester), 1235, 1203, 1143, 1104 (CF₃, CF₂). The theoretical chemical structure of compound 34 is shown in FIG. 9C.

### Compound 35

Compound 35 was prepared according to a procedure similar to that used for the preparation of compound 34.

Glassware used for the synthesis was dried in an oven at 110 °C overnight. To a 2-neck 1000 mL oven-dried round bottom flask equipped with a stir bar was added 60 g (59 mmol) of YmerOH-triol (MW = 1014 Da). The flask with the triol was degassed overnight at 60 °C with gentle stirring, and then purged with dry N₂ the following day. Heating was turned off. A 1000 mL graduated cylinder was charged with 435 mL anhydrous CHCl₃, sealed with a rubber septum, and purged with dry N₂. The CHCl₃ liquid was transferred to the 2-neck flask via a cannula, and the triol was stirred vigorously to dissolve in the solvent. Excess anhydrous pyridine (37 g, 473 mmol) was added to the YmerOH-triol solution using a plastic syringe, the resulting mixture was stirred to dissolve all materials. Another oven-dried 2-neck 1000 mL flask was charged with 84.88 g (222 mmol) of perfluoroheptanoyl chloride, sealed with rubber septa, degassed for 5 min, and then purged with N₂. 290 mL of anhydrous CHCl₃ were added using a graduated cylinder and cannula to transfer the solvent to the 1000 mL 2-neck flask containing the perfluoroheptanoyl chloride. The mixture was stirred at room temperature to dissolve the acid chloride. An addition funnel was fitted to this flask, and the YmerOH-triol-pyridine solution in CHCl₃ was transferred to the addition funnel using a cannula. N₂ flow through the reactor was adjusted to a slow and steady rate. YmerOH-triol-pyridine solution was added continuously drop-wise to the acid chloride solution at room temperature over a period of ~4 h. Stirring was maintained at a sufficient speed to achieve good mixing of reagents. After completing the addition of the YmerOH-triol-pyridine solution, the addition funnel was replaced with an air condenser, and the 2-neck flask was immersed in an oil bath placed on a heater fitted with a thermocouple unit. The temperature was raised to 40 °C, and the reaction was continued at this temperature under N₂ for 24 h.

The resulting product was purified in a similar manner to compound 7 described above. The purification involved rotary evaporation of CHCl₃, addition of THF, and separation of the pyridine salts by filtration. The product was then precipated in isopropanol (IPA)/hexanes, washed as described above for compound 7, and dried at 75 °C and 4 mbar. Final drying was also done under vacuum at 60 °C for 24 h to yield an oil (78.2% yield). The purified product was characterized by GPC (molecular weight based on polystyrene standards), Elemental Analysis for fluorine, ¹⁹F NMR, ¹H NMR, and FTIR. Appearance: light yellow, viscous oil. Weight average molecular weight (polystyrene equivalent) = 2321g/mol. PD: 1.06. Elemental Analysis: F: 35.13%. ¹⁹F NMR (CDCl₃, 400 MHz, ppm): δ ppm - 81.30 (m, CF₃), -118.90 (m, CF₂), -122.27 (m, CF₂), - 123.07 (m, CF₂), -126.62 (m, CF₂). ¹H NMR (CDCl₃, 400 MHz): δ ppm 0.83 (t, CH₃CH₂), 1.44 (q, CH₂CH₃), 3.34 (m, CH₂O), 3.41 (m, CH₂'s), 3.74 (m, CH₂), 4.30 (m, CH₂COO-). FTIR, neat (cm⁻¹): 2870 (CH₂), 1780 (O-C=O, ester), 1235, 1202, 1141, 1103 (CF₃, CF₂). The theoretical chemical structure of compound 35 is shown in FIG. 9D.

### Compound 36

Compound 36 was prepared according to a procedure similar to that used for the preparation of compound 34.

Glassware used for the synthesis was dried in an oven at 110 °C overnight. To a 2-neck 1000 mL oven-dried round bottom flask equipped with a stir bar was added 50 g (65 mmol) of Xmer-tetraol (MW = 771 Da). The flask with the tetraol was degassed overnight at 60 °C with gentle stirring, and then purged with dry N₂ the following day. Heating was turned off. A 1000 mL graduated cylinder was charged with 400 mL anhydrous CHCl₃, sealed with a rubber septum, and purged with dry N₂. CHCl₃ was transferred to the 2-neck flask via a cannula, and the tetraol was stirred vigorously to dissolve in the solvent. Excess anhydrous pyridine (51.30 g, 649 mmol) was added to the Xmer-tetraol solution using a plastic syringe, and the resulting mixture was stirred to dissolve all materials. Another oven-dried 2-neck 1000 mL flask was charged with 111.63 g (292 mmol) of perfluoroheptanoyl chloride, sealed with rubber septa, degassed for 5 min, and then purged with N₂. 300 mL of anhydrous CHCl₃ were added using a graduated cylinder and cannula to transfer the solvent to the 1000 mL 2-neck flask containing perfluoroheptanoyl chloride. The resulting mixture was stirred at room temperature to dissolve the acid chloride. An addition funnel was attached to this flask, and the Xmer-tetraol-pyridine solution in CHCl₃ was transferred into the addition funnel via a cannula. N₂ flow through the reactor was adjusted to a slow and steady rate. Xmer-tetraol-pyridine solution was added continuously drop-wise to the acid chloride solution at room temperature over a period of ~4 h. Stirring was maintained at a sufficient speed to achieve good mixing of reagents. After completing addition of the Xmer-tetraol-pyridine solution, the addition funnel was replaced with an air condenser, and the 2-neck flask was immersed in an oil bath placed on a heater fitted with a thermocouple unit. The temperature was raised to 40 °C, and the reaction continued at this temperature under N₂ for 24 h.

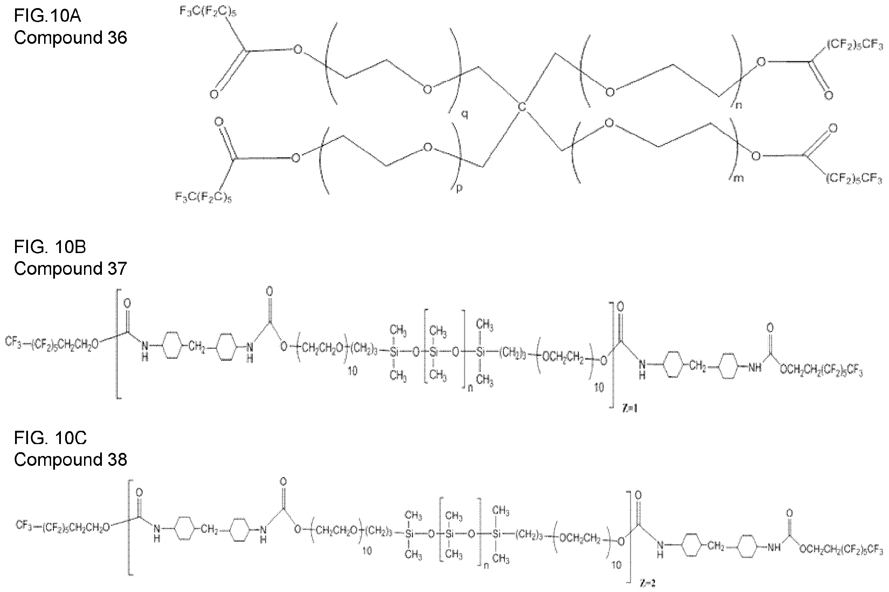

The resulting product was purified in a similar manner to compound 7 described above, where the CHCl₃ was removed by rotary evaporation, addition of THF, and the separation of pyridine salts by filtration after adding THF. The product was then precipitated in isopropanol (IPA)/hexanes, washed as described for compound 7, and dried at 75 °C and 4 mbar. Final drying was also done under vacuum at 60 °C for 24 h to yield an oil (80.9% yield). The purified product was characterized by GPC (molecular weight based on polystyrene standards), Elemental Analysis for fluorine, ¹⁹F NMR, ¹H NMR, FTIR, and TGA. Appearance: light yellow, viscous oil. Weight average molecular weight (polystyrene equivalent) = 2410 g/mol. PD: 1.04. Elemental Analysis: F: 44.07%. ¹⁹F NMR (CDCl₃, 400 MHz, ppm): δ ppm -81.37 (m, CF₃), -118.89 (m, CF₂), -122.27 (m, CF₂), -123.06 (m, CF₂), -26.64 (m, CF₂). ¹H NMR (CDCl₃, 400 MHz): δ ppm 3.36 (m, CH₂'s), 3.75 (m, CH₂O), 4.39 (m, CH₂O), 4.49 (m, CH₂COO-). FTIR, neat (cm⁻¹): 2870 (CH₂), 1780 (O-C=O, ester), 1235, 1202, 1141, 1103 (CF₃, CF₂). Thermal decomposition temperature (TGA), N₂, at *ca.* 10% (w/w) loss = 327 °C. The theoretical chemical structure of compound 36 is shown in FIG. 10A.

### Compounds 37

Glassware used for the synthesis was dried in an oven at 110 °C overnight. 25.04 g (9.7 mmol) of pegylated polydimethylsiloxane diol (C10-diol) was weighed out in a 250 mL 2-neck flask, heated to 50 °C, and degassed overnight with stirring. The diol was then purged with N₂ and dissolved in 25 mL of anhydrous THF. To the resulting mixture was added 36 mg of bismuth carboxylate catalyst in THF (concentration of 0.02 g/mL) followed by a solution of HMDI diisocyanate in THF (5.34 g, 20.4 mmol) which was previously degassed for 30 min followed by N₂ purge. The addition was performed using a syringe. The reaction vessel was fitted with an air condenser, and the mixture was allowed to react at 60 °C with stirring for 4 h. While the prepolymer reaction was under way, capstone C6-FOH (fluoroalcohol) (8,82 g, 24.2 mmol) was degassed for 15 min in a separate flask, and then purged with N₂. The fluoroalcohol was dissolved in THF, and a further 24 mg of bismuth carboxylate catalyst in THF was added. This mixture was then added to the prepolymer reaction vessel via syringe. After the addition was completed, the reaction mixture was allowed to react overnight at 45 °C under a N₂ atmosphere. After the reaction, the THF solvent was removed on a rotary evaporator, and the crude residue was dissolved in isopropanol (IPA). The bismuth catalyst residues were extracted using EDTA solution (pH ~9). The solution containing EDTA was precipitated in 1% methanol solution in distilled water mixture to form a viscous oil. The water layer was decanted and the residue dried in a rotary evaporator to give the product as an amber viscous liquid. Final drying was done under vacuum at 60 °C for 24 h to yield a viscous oil (74 % yield). The purified product was characterized by GPC (molecular weight based on polystyrene standards), Elemental Analysis for fluorine, and TGA. Appearance: amber, viscous oil. Weight average molecular weight (polystyrene equivalent) = 13583 g/mol. PD: 1.73. Elemental Analysis: F: 12.20%. Thermal decomposition temperature (TGA), N₂, at *ca.* <5% (w/w) loss = 231 °C. The theoretical chemical structure of compound 37 is shown in FIG. 10B.

### Compound 38

Compound 38 is synthesized flowing a procedure similar to that used in the preparation of compound 37 with some modifications in the purification process. 25.01 g (10.2 mmol) of C10-diol was reacted with 4.29 g (16.3 mmol) of HMDI in THF, in the presence of bismuth carboxylate catalyst to form a prepolymer. The prepolymer was then endcapped with 6.50 g (17.9 mmol) Capstone C6-FOH (fluoroalcohol) to yield the product as a viscous oil. This oil was dissolved in isopropanol at 85 °C, and treated with 2x15 mL of EDTA (ethylenediaminetetraacetic acid solution, pH ~9). The EDTA solution of the polymer was then precipitated in1% methanol solution in distilled water to form an oil which was washed once with boiling hexanes, and then further washed 2x with hexanes at room temperature to obtain an oil (66% yield). The purified product was characterized by GPC (molecular weight based on polystyrene standards), Elemental Analysis for Fluorine, and TGA. Appearance: amber, viscous oil. Weight average molecular weight (polystyrene equivalent) = 16,278 g/mol. PD: 1.82. Elemental Analysis: F: 8.8%. Thermal decomposition temperature (TGA), N₂, at ca. <5% (w/w) loss = 299 °C. The theoretical chemical structure of compound 38 is shown in FIG. 10C.

### Compound 39

Compound 39 was synthesized by a 2-step convergent method according to scheme 2. Briefly, the polyisocyanate desmodur 4470 (11.45 g, 11 mmol) was reacted with capstone C6-FOH (7.65 g, 21 mmol) in anhydrous THF in the presence of bismuth carboxylate catalyst at 25 °C for 10 min. After the drop-wise addition of the fluoroalcohol to the polyisocyanate, stirring was continued for 4 h at 40 °C. These steps lead to the formation of a partially fluorinated intermediate that is then coupled with the PLN8K diol (40 g, 5 mmol) at 70 °C over a period of 14 h to provide compound 39. Because the reactions are moisture sensitive, they are carried out under an inert atmosphere (N₂) and anhydrous conditions. The temperature profile is also maintained carefully, especially during the partial fluorination, to avoid unwanted side reactions. Over the course of the reaction, the reaction mixture becomes very viscous, and continuous stirring must be maintained to prevent localized heating.

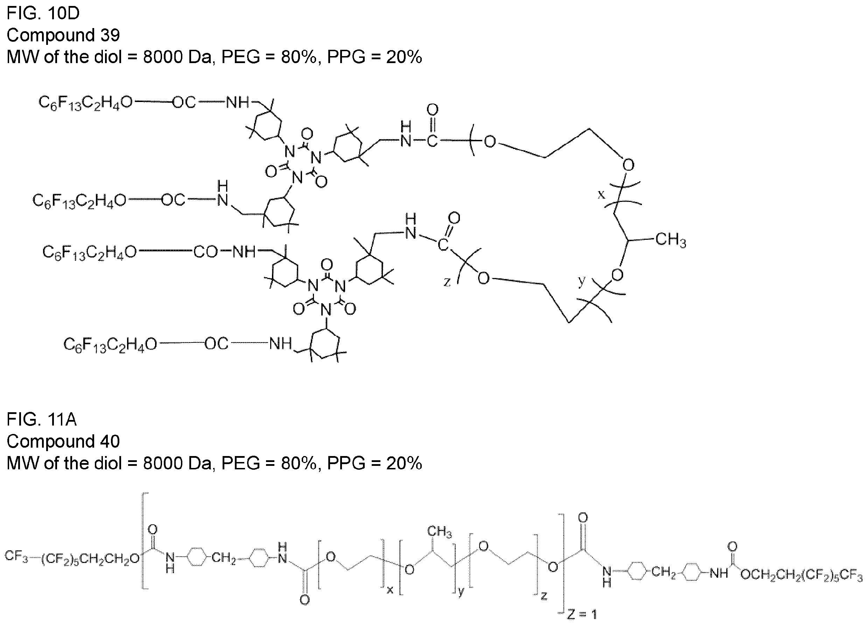

After the reaction, the THF solvent was evaporated on a rotary evaporator to yield the crude product. The product was purified by dissolving in chloroform and adding the EDTA solution (pH ~ 9.0). The mixture was then transferred to a separatory funnel, and the catalyst residues were separated with the aqueous layer. The organic layer was concentrated, and the product was dissolved in isopropanol and precipitated in hexanes to yield a white chunky solid which was dried under vacuum (66% yield). The purified product was characterized by GPC (molecular weight based on polystyrene standards), Elemental Analysis for fluorine, and TGA. Appearance: white chunky solid. Weight average molecular weight (polystyrene equivalent) = 31806 g/mol. PD: 1.32. Elemental Analysis: F: 3.6%. Thermal decomposition temperature (TGA), N₂, at ca. <5% (w/w) loss = 295 °C. The theoretical chemical structure of compound 39 is shown in FIG. 10D.

### Compound 40

Compound 40 was synthesized following a procedure similar to that which was used in the preparation of compound 37. Thus, 50.0 g (5.7 mmol) of PLN8K diol were reacted with 4.5 g (17.1 mmol) of HMDI in THF in the presence of bismuth carboxylate catalyst to form the prepolymer. The prepolymer was then endcapped with 7.28 g (20 mmol) capstone C6-FOH (fluoroalcohol) to yield the crude product. The EDTA washes to eliminate the catalyst residues were similar. Final purification was performed by dissolving in isopropanol and precipitating with hexanes to yield a white solid (86% yield). The purified product was characterized by GPC (molecular weight based on polystyrene standards), Elemental Analysis for fluorine, and TGA. Appearance: While solid. Weight average molecular weight (polystyrene equivalent) = 9253 g/mol. PD: 1.28. Elemental Analysis: F: 3.14%. Thermal decomposition temperature (TGA), N₂, at *ca.* <5% (w/w) loss = 303 °C. The theoretical chemical structure of compound 40 is shown in FIG. 11A.

### Compound 41

Compound 41 was synthesized following a procedure similar to that which was used in the preparation of compound 27. The theoretical chemical structure of compound 41 is shown in FIG. 7C, with the exception that the middle triblock copolymer is formed from a C10-diol.

The purified product was characterized by GPC (molecular weight based on polystyrene standards), Elemental Analysis for fluorine, and TGA. Appearance: colorless viscous liquid. Weight average molecular weight (polystyrene equivalent) = 5858 g/mol. PD: 1.21. Elemental Analysis: F: 18.39%. Thermal decomposition temperature (TGA), N₂, at *ca.* <10% (w/w) loss = 310 °C.

### Compound 42

All glassware used for the synthesis was dried in an oven at 110 °C overnight. To a 2-neck 1000 mL oven-dried round bottom flask equipped with a stir bar was added 60 g (59 mmol) of YmerOH-diol (MW = 1014 Da). The flask with was degassed overnight at 60 °C with gentle stirring and then purged with dry N₂ the following day. The heating was turned off. A 1000 mL graduated cylinder was filled with 435 mL anhydrous CHCl₃, sealed by a rubber septum and purged with dry N₂. The CHCl₃ was transferred to the 2-neck flask via a cannula and the diol stirred vigorously to dissolve in the solvent. Excess anhydrous pyridine (37 g, 473 mmol) was added to the solution using a syringe, stirred to dissolve all materials. To an oven-dried 2-neck 1000 mL flask, 84.88 g (222 mmol) perfluoroheptanoyl chloride were added, sealed with rubber septa, degassed for 5 min, and then purged with N₂. 290 mL of anhydrous CHCl₃ were added using a graduated cylinder and cannula, and transferred to 1000 mL 2-neck flask containing the perfluoroheptanoyl chloride. The reaction mixture was stirred at room temperature to dissolve the acid chloride. Using a cannula, CHCl₃ was transferred into the addition funnel. The continuous drop-wise addition to the acid chloride solution at room temperature was performed over a period of ~4 h. After completing of addition of the pyridine solution, the reaction mixture was heated to 40 °C for 24 h.

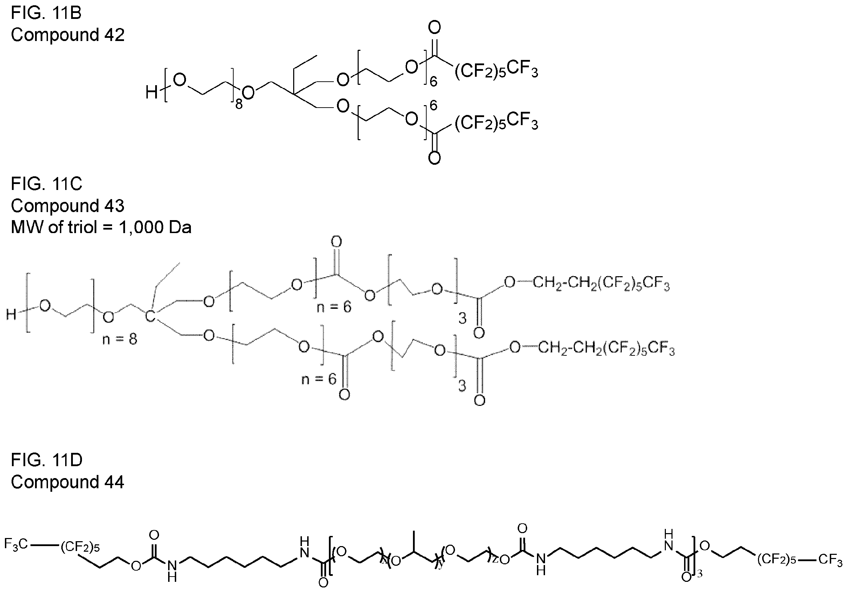

The product was purified in a similar manner to compound 33. The product was precipitated in IPA/hexanes, and then dried at 75 °C and 4 mbar (78.2% yield). Mw (Polystyrene Equivalent) g/mol = 2321. PD = 1.06. Elemental Analysis (atomic % F) = 35.13. ¹⁹F NMR CDCl₃ 400 MHz (ppm): δ - 81.30 (m, CF₃), -118.90 (m, CF₂), -122.27 (m, CF₂), -123.07 (m, CF₂), -126.62 (m, CF₂). ¹H NMR, CDCl₃, 400 MHz (ppm): δ 0.83 (t, CH₃CH₂), 1.44 (q, CH₂CH₃), 3.34 (m, CH₂O), 3.41 (m, CH₂'s), 3.74 (m, CH₂), 4.30 (m, CH₂COO-). FT-IR Neat (cm⁻¹): 2870 (CH₂), 1780 (O-C=O, ester), 1235, 1202, 1141, 1103 (CF₃, CF₂). The theoretical chemical structure of compound 42 is shown in FIG. 11B.

### Compound 43

All glassware used for the synthesis was dried in an oven at 110 °C overnight. 20 g (0.020 mol) of YmerOH triol (MW = 1000 Da) was added to a 200 mL 2-neck flask which was degassed overnight then purged with N₂. To a 500 mL 2-neck oven-dried flask equipped with a stir bar was added 21.70 g (0.079 mol) triethylene glycol (TEG) bis chloroformate. The flask was degassed for 15 min and then purged with dry N₂. After purging, 62 mL of anhydrous toluene were transferred into the flask by means of a cannula. The TEG bis chloroformate was stirred to dissolve in the solvent. It was then cooled under an ice bath for 15 min. To a 50 mL addition funnel was added 28.73 g (0.079 mol) of Capstone 62-AL fluoroalcohol (1H,1H,2H,2H-perfluoro-1-octanol), then degassed for 15 min and purged with dry N₂. To this addition funnel were added 21 mL of anhydrous toluene, followed by 7 g of anhydrous pyridine, and the funnel was shaken to dissolve all reagents. The addition funnel was attached to the reaction flask and a drop-wise addition of Capstone 62-AL to the cooled solution of TEG bis chloroformate was started until completion in ~2 h. During addition, the stirring was kept to a minimum. During addition, pyridine salts precipitated out. The temperature was maintained at ice bath conditions, and the reaction was allowed to proceed for additional 10 min under N₂ to form a partially fluorinated TEG bis chloroformate-Capstone 62-AL intermediate. While the partial fluorination was in progress, anhydrous toluene (125 mL) was added to the flask containing the YmerOH triol via a cannula, followed by 6 g of anhydrous pyridine, and the mixture was stirred to dissolve the YmerOH triol. A 250 mL addition funnel was attached to the 500 mL 2-neck flask containing the partially fluorinated TEG bis chloroformate-Capstone 62-AL intermediate and the YmerOH-triol solution was transferred via cannula to the funnel. The YmerOH-triol solution was added to the 500 mL vessel at ice bath conditions in a slow continuous stream until all the YmerOH-triol was added. The mixture was allowed to stir at room temperature under N₂ for 1 h, then the temperature was raised to 50 °C, and the reaction allowed to proceed for 24 h with stirring. The reaction generated a large quantity of white pyridine salts, which precipitated during the reaction. All additions and transfers were conducted under a dry N₂ atmosphere to avoid any contact with air.

The purification involved vacuum filtration of the pyridine salts using a Whatman filter paper (No 4), followed by rotary evaporation of the toluene. The product was treated with 1 N HCl, and was extracted in dichloromethane-water mixture to remove excess pyridine, then neutralized with 5 % NaHCO₃ solution. The bottom organic layer was collected, washed twice with distilled water, and then rotary evaporated. The crude product (viscous oil) was incubated in a 250 mL round bottom flask with 20 mL distilled water for 48 h at 37 °C with gentle shaking to remove unreacted YmerOH-triol. The oil is extracted in dichloromethane-water mixture, and after isolation, further purified by dissolving in ethyl acetate, and precipitating in hexanes to yield a clear viscous oil. The purified product was dried at 75 °C and 4 mbar to yield a viscous clear oil (42% yield). The purified product was characterized by GPC and Elemental Analysis. The average molecular weight (polystyrene equivalent) = 5083 g/mol. Polydispersity = 1.44. Elemental Analysis shows %F = 11.24%. Thermal decomposition temperature: 308 °C at 5% (w/w) loss. The chemical structure of compound 43 is shown in FIG. 11C.

### Compound 44

Glassware used for the synthesis was dried in an oven at 110 °C overnight. 84.72g (44.6 mmol) of polyethylene glycol)-block-poly(propylene glycol)-block-poly(ethylene glycol) (PLN diol) was weighed in a 250 mL 3-neck round bottom flask, and degassed overnight under vacuum with stirring. The diol was then purged with N₂ and dissolved in 85 mL of anhydrous THF. 10g (59.5 mmol) HDI was degassed for 1 h at room temperature, and purged with N₂. The HDI was dissolved in 10 mL THF, and then added to PLN diol solution. The reaction vessel was fitted with an air condenser, and the mixture was heated to 50 °C. 20 mg of DBTDL was dissolved in 1mL THF, and half of the solution was transferred to the reaction mixture when the temperature reached 50 °C. The reaction mixture was allowed to proceed at 65 °C for 4 h. FOH (fluoroalcohol) (11.91 g, 32.7 mmol) was degassed for 30 min and purged with N₂. After 4 h, the reaction temperature was cooled to 45 °C. The FOH was dissolved in 12 mL THF, and added to the reaction mixture. The other half of the DBTDL solution was also added to the reaction mixture. The reaction was allowed to react at 45 °C overnight. After reaction, the solvent was removed on a rotary evaporator, and the crude product was dissolved in 120 mL of hot isopropanol. Polymer was then precipitated using 2.5 L hexanes. The supernatant was decanted, and the polymer was rinsed twice with 100 mL of hexane. The polymer was dissolved in THF, and transferred to a round bottom flask for rotary evaporation. The polymer was dried in a vacuum oven at room temperature overnight. The polymer yield was 82%. The purified product was characterized by GPC and Elemental Analysis. Appearance: tacky solid. Weight average molecular weight (polystyrene equivalent) = 35, 848 g/mol. PDI = 1.66. Elemental Analysis: F: 2.3%. Thermal decomposition temperature at first onset = 289 °C at 5% (w/w) loss. The theoretical chemical structure of compound 44 is shown in FIG. 11D.

### Compound 45

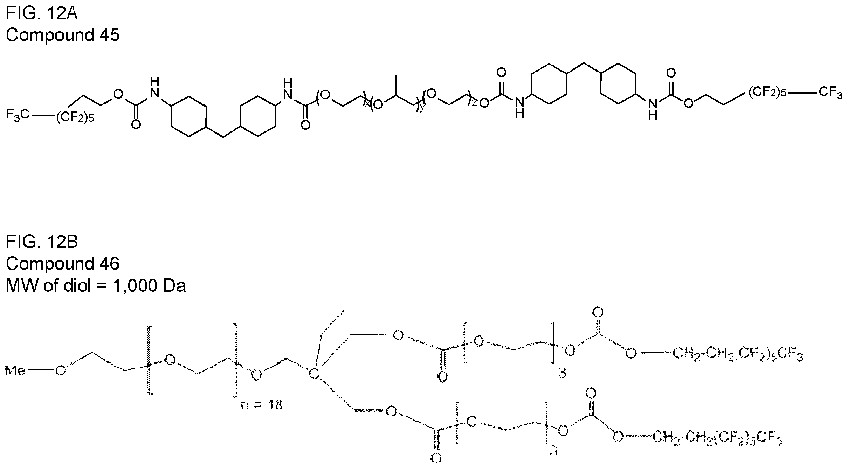

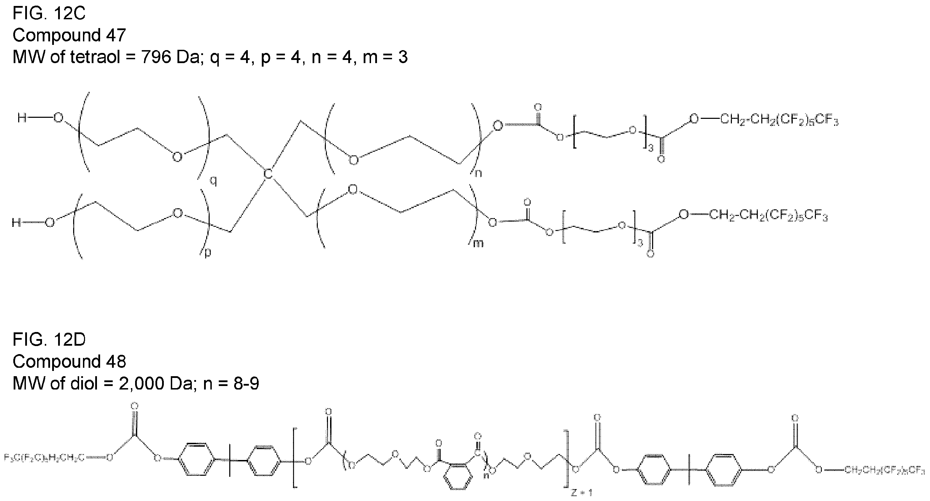

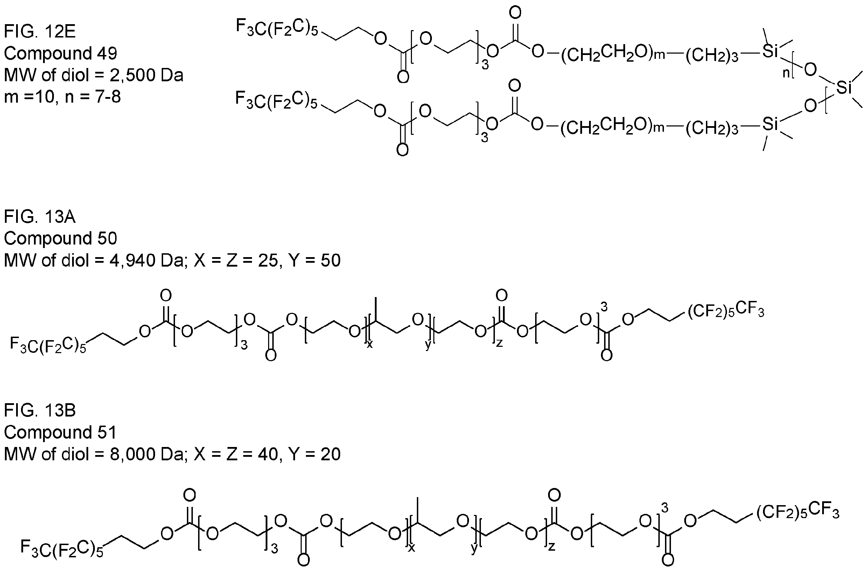

Glassware used for the synthesis was dried in an oven at 110 °C overnight. 36.21 g (19.1 mmol) of polyethylene glycol)-block-poly(propylene glycol)-block-poly(ethylene glycol) (PLN diol) was weighed in a 250 mL 3-neck round bottom flask, and degassed overnight under vacuum with stirring. The diol was then purged with N₂ and dissolved in 36 mL of anhydrous THF. 10 g (38.1 mmol) HMDI was degassed for 1 h at room temperature, and purged with N₂. The HMDI was dissolved in 10 mL THF, and then added to PLN diol solution. The reaction vessel was fitted with an air condenser, and the mixture was heated to 50 °C. 20 mg of DBTDL was dissolved in 1 mL THF, half of the solution was transferred to the reaction mixture when the temperature reached 50 °C. The reaction mixture was allowed to proceed at 65 °C for 4 h. FOH (fluoroalcohol) (15.27 g, 41.9 mmol) was degassed for 30 min and purged with N₂. After 4 h reaction, the reaction temperature was cooled at 45 °C. The FOH was dissolved in THF, and added to the reaction mixture. The other half of the DBTDL solution was also added to the reaction mixture. The reaction was allowed to react at 45 °C overnight. After reaction, the solvent was removed on a rotary evaporator, and the crude product was dissolved in 100 mL hot isopropanol. Polymer was then precipitated using 1.5 L hexanes. The supernatant was decanted, and the polymer was rinsed twice with 80 mL hexanes. The polymer was dissolved in THF, and transferred to a round bottom flask for rotary evaporation. The polymer was final dried in a vacuum oven at room temperature overnight. The polymer yield was 73%. The purified product was characterized by GPC and Elemental Analysis. Appearance: viscous liquid. Weight average molecular weight (polystyrene equivalent) = 12,793 g/mol. PDI: 1.36. Elemental Analysis: F: 8.1%. Thermal decomposition temperature = 282 °C at 5% (w/w) loss. The theoretical chemical structure of compound 45 is shown in FIG. 12A.

### Compound 46

All glassware used for the synthesis was dried in an oven at 110 °C overnight. To a 200 mL 2-neck oven-dried flask equipped with a stir bar was added 50 g (0.048 mol) Ymer diol (MW = 1000 Da), and degassed overnight with gentle stirring at 60 °C. Then, the Ymer diol was purged with dry N₂, 53 mL of anhydrous chloroform (CHCl₃) were added to the flask using a cannula, followed by 15 g of anhydrous pyridine. The reaction mixture was stirred to dissolve the reagents and obtain a homogeneous solution. To a 1 L 2-neck oven-dried flask equipped with a stir bar was added 60.9 g (0.221 mol) triethylene glycol (TEG) bis chloroformate. The flask was degassed for 15 min, and then purged with dry N₂. After purging, 157 mL of anhydrous CHCl₃ were transferred into the flask by means of a cannula. The TEG bis chloroformate was stirred to dissolve in the solvent. To a 500 mL 2-neck flask was added 73.59 g (0.202 mol) of Capstone 62-AL fluoroalcohol (1H,1H,2H,2H-perfluoro-1-octanol), then degassed for 15 min and purged with dry N₂. To this was added 314 mL of anhydrous CHCl₃ followed by 28 g of pyridine. The flask was stirred to dissolve all the reagents. The Capstone 62-AL fluoroalcohol solution was transferred to a 500 mL addition funnel that was previously degassed and purged with N₂ using a cannula. The addition funnel was attached to the 1 L reaction vessel containing the TEG bis chloroformate solution that was cooled in an ice bath. The addition of the fluoroalcohol was done dropwise under N₂ for 1 h. Stirring was kept to a minimum during the reaction to form a partially fluorinated TEG bis chloroformate-Capstone 62-AL fluoroalcohol intermediate. Next, the Ymer diol solution was transferred to the 1 L reaction vessel using a 20 gauge cannula in a slow continuous stream while the reaction vessel was cooled under an ice bath until all of the Ymer diol solution had been added. The ice bath was removed and the reaction was allowed to proceed at room temperature for additional 10 min. The temperature was then raised to 50 °C and the reaction was allowed to run overnight. All additions and transfers were conducted under a dry N₂ atmosphere to avoid any contact with air.

The crude product was purified by first removing the CHCl₃ solvent on a rotary evaporator, dissolving the crude product in minimum THF, and cooling with ice bath for 20 min to precipitate the pyridine salts. The solution was vacuum filtered and the THF was evaporated using a rotary evaporator. The product was treated with 1 N HCl and extracted in dichloromethane-water mixture to remove excess pyridine, then neutralized with 1 N NaOH solution. The bottom organic layer was collected, washed twice with distilled water, and then rotary evaporated. Finally, the product was dissolved in minimum isopropyl alcohol (IPA), precipitated out in hexanes, washed 2× with hexanes, and dried under vacuum. The product was dried overnight at 60 °C in a vacuum oven to yield the product as a viscous liquid (59% yield). The purified product was characterized by GPC (molecular weight based on polystyrene standards), and elemental analysis for fluorine. The average molecular weight (polystyrene equivalent) was 3422 g/mol. Polydispersity = 1.15. Elemental analysis: F = 19%. Thermal decomposition temperature (TGA, under N₂), at first onset: 203 °C (at 5% wt loss). The chemical structure of compound 46 is shown in FIG. 12B.

### Compound 47

All glassware used for the synthesis was dried in an oven at 110 °C overnight. To a 200 mL 2-neck flask equipped with a stir bar was added 12 g (0.016 mol) of Xmer tetraol (MW = 771 Da), degassed overnight with gentle stirring at 60 °C, and then purged with N2. To a 500 mL 2-neck oven-dried flask equipped with a stir bar was added 9 g (0.033 mol) triethylene glycol (TEG) bis chloroformate. The flask was degassed for 15 min and then purged with dry N₂. After purging, 65 mL of anhydrous CHCl₃ was transferred into the flask by means of a syringe. The TEG bis chloroformate was stirred to dissolve in the solvent. To a 50 mL addition funnel was added 15 g (0.033 mol) of Capstone 62-AL fluoroalcohol (1H,1H,2H,2H-perfluoro-1-octanol), and degassed for 15 min and purged with dry N₂. To this addition funnel was added 20 mL of anhydrous CHCl₃ followed by 3 g of anhydrous pyridine, and the flask shaken to dissolve all reagents. The addition funnel was attached to the 500 mL reaction flask which was cooled in ice and dropwise addition of Capstone 62-AL fluoroalcohol to the TEG bis chloroformate was performed. The addition took 1 h to complete, and the reaction was allowed to proceed for additional 10 min at room temperature (25 °C) under N₂ atmosphere to form a partially fluorinated TEG bis chloroformate-Capstone 62-AL intermediate. While the partial fluorination was in progress, anhydrous CHCl₃ (122 mL) was added to the flask containing the Xmer tetraol via a cannula, followed by 2.5 g of anhydrous pyridine, and the mixture was stirred to dissolve the reagents. Next, the Xmer tetraol solution was transferred to the 500 mL reaction vessel using a 20 gauge cannula in a slow continuous stream, while the reaction vessel was cooled in an ice bath until all of the Xmer tetraol solution had been added. The ice bath was removed and the reaction was allowed to proceed at room temperature for additional 10 min. The temperature was then raised to 50 °C and the reaction was allowed to run overnight. All additions and transfers were conducted under a dry N₂ atmosphere to avoid any contact with air.

The purification involved rotary evaporation of CHCl₃ from the reaction mixture, addition of THF, and separation of the pyridine salts by vacuum filtration. The product was treated with 1 N HCl and extracted in dichloromethane-water mixture to remove excess pyridine, then neutralized with 1 N NaOH solution. The bottom organic layer was collected, further washed twice with distilled water and then rotary evaporated. Finally, the product was dissolved in minimum isopropyl alcohol (IPA), precipitated out in hexanes, washed 2× with hexanes, and dried under vacuum. The product was dried overnight at 60 °C in a vacuum oven to yield the product as a viscous liquid (59% yield).

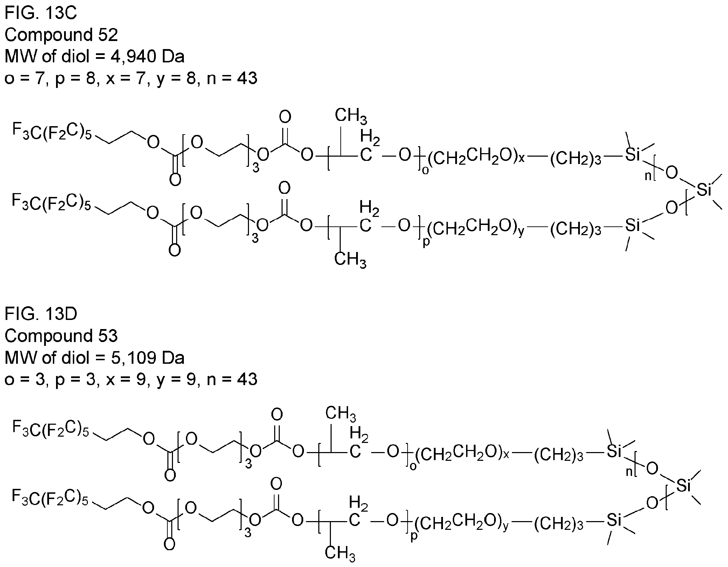

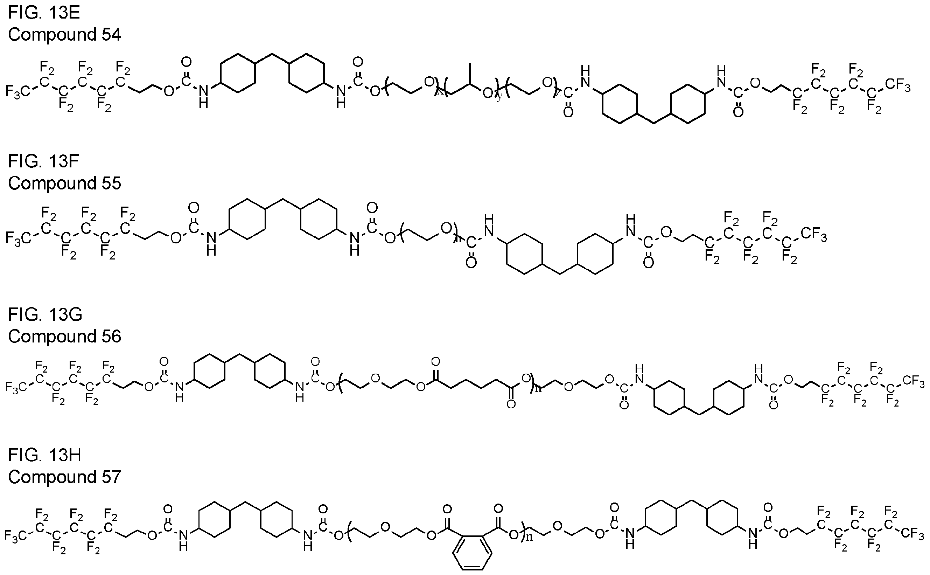

The purified product was characterized by GPC (molecular weight based on polystyrene standards), and elemental analysis for fluorine. The average molecular weight (polystyrene equivalent) was 2322 g/mol. Polydispersity = 1.12. Elemental analysis shows F = 25.8%. Thermal decomposition temperature (TGA, under N₂), at first onset: 221 °C (at 10 wt% loss). The chemical structure of compound 47 is shown in FIG. 13C.

### Compound 48

All glassware used for the synthesis was dried in an oven at 110 °C overnight. To a 100 mL 2-neck oven-dried flask equipped with a stir bar was added 15 g (0.008 mol) of PDP diol (MW = 2000 Da), and degassed overnight with gentle stirring at 60 °C. Then the PDP diol was purged with dry N₂, 90 mL of anhydrous CHCl₃ were transferred into the flask using a cannula, followed by 2 g of anhydrous pyridine. The reaction mixture was stirred to dissolve and obtain a homogeneous solution. To a 50 mL oven-dried addition funnel was added 5.63 g (0.016 mol) of bisphenol A chloroformate, the funnel was degassed for 15 min and then purged with dry N₂. After purging, 45 mL of anhydrous CHCl₃ were transferred into the funnel by means of a cannula. The funnel was shaken to dissolve the chloroformate. The addition funnel was attached to the flask containing the PDP diol and dropwise addition of bisphenol A chloroformate was performed over a period of 1 h at room temperature. The reaction was allowed to proceed for 3 h, allowing the formation of a PDP-bisphenol A prepolymer intermediate. While the prepolymer intermediate reaction was run, 7 g (0.019 mol) of Capstone 62-AL fluoroalcohol was added to a 50 mL 2-neck flask. The flask was degassed for 15 min and then purged under dry N₂. After purging with N₂, 15 mL of anhydrous CHCl₃ was added to the flask, followed by 2 g of pyridine. The flask was shaken to dissolve the fluoroalcohol, which was then added to the 200 mL flask containing the prepolymer intermediate using a cannula in a slow continuous stream. The temperature was raised to 60 °C and the final end-capping reaction was allowed to proceed overnight. The product was purified by pouring the CHCl₃ reaction mixture into a separatory funnel containing deionized water, and the aqueous layer was acidified with 5 N HCl to neutralize any residual pyridine.

The product was extracted into the organic layer neutralized with 1 N NaOH solution and washed 2x with DI water. The organic layer was dried over anhydrous Na₂SO₄. The solvent was removed on a rotary evaporator, the solid residue was dissolved in THF, and precipitated in a 3:1 water/methanol mixture. The product was dried in a vacuum oven (30 mbar) for 2 days to yield a solid. The purified product was characterized by GPC (molecular weight based on polystyrene standards). The average molecular weight (polystyrene equivalent) was 20704g/mol. Polydispersity = 1.52. Elemental analysis showed 4.20 wt% F, Thermal decomposition temperature (TGA, under N₂), at first onset: 314 °C (at 5 wt% loss). The chemical structure of compound 48 is shown in FIG. 12D

### Compounds 49-57

Compounds 49-57 can be synthesized using methodologies analogous to those described for compounds 1-48.

### Example 2: Bacterial adhesion on polyurethane (PU) rods after 2 h incubation in PBS

Polyurethane rods containing 1-2 wt% compounds of the invention for bacterial adhesion assays were prepared using a laboratory microcompounder. Specifically, Carbothane 3585A (CB 85A) polyurethane resin from Lubrizol was dried in a vacuum oven at 60 °C for 4 h. The resin was blended with different compounds of the invention using a 15 mL DSM twin-screw microcompounder in batch mode, with a cycle time of 3-5 min (after resin load) and melt temperatures between 215-225 °C. The blend was extruded into rods approximately 3.5 mm in diameter by opening the mixing chamber valve to release the molten polymer. The resulting rods were quenched in a water bath and air dried.

Rod samples were cut into 1.5 cm segments, ethylene oxide sterilized, and placed in sterile microcentrifuge tubes. Bacterial strains were grown overnight in Brain Heart Infusion (BHI) broth at 37 °C with shaking. To prepare bacterial inoculum solution the bacterial cultures were centrifuged at 4,000 rpm for 10 min, washed with PBS, and re-suspended in PBS at a concentration of 10⁸ colony forming units/mL (CFU/mL). The following bacterial strains were used for testing: *S. epidermidis* 3399, *S. aureus* Newman, *E. faecalis* 33186, *E. coli* GR-12, *K. pneumoniae* 280, *P. aeruginosa* AK-1, and *P. mirabilis* 296. Testing was also conducted with a fungal strain, *C. albicans ATCC 28367*, which was grown overnight in BHI at 30 °C with shaking and washed/re-suspended in PBS as described above. All microbial strains were obtained from Lawson Health Research Institute (London, Ontario), and all strains except *S. epidermidis* and *E. faecalis* were clinical isolates from human infections (blood, urinary tract or other).

One millilitre (1 mL) of inoculum solution was pipetted into the microcentrifuge tubes containing the samples and incubated for 2 h at 37 °C with minimal agitation. After 2 h, the samples were washed 3x with 500 µL PBS to remove loosely adhered bacteria and transferred to new microcentrifuge tubes with 1 mL of PBS. The samples were sonicated for 30 min, followed by vortexing for 30 sec, to detach adhered bacteria. The sonicated solutions were serially diluted and drop plated on agar plates. The plates were incubated at 37 °C overnight after which bacterial colonies were counted and bacterial adhesion was calculated in CFU/cm².

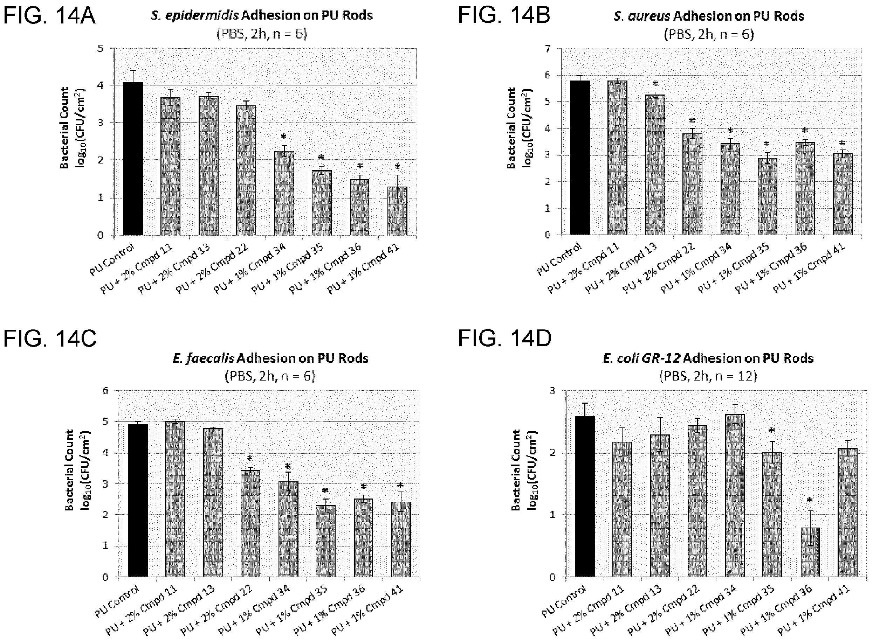

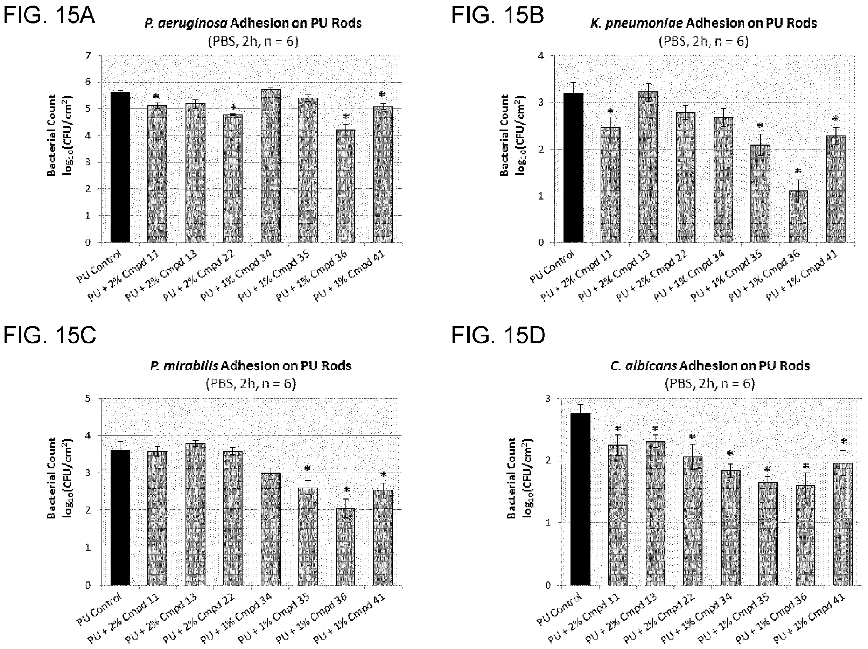

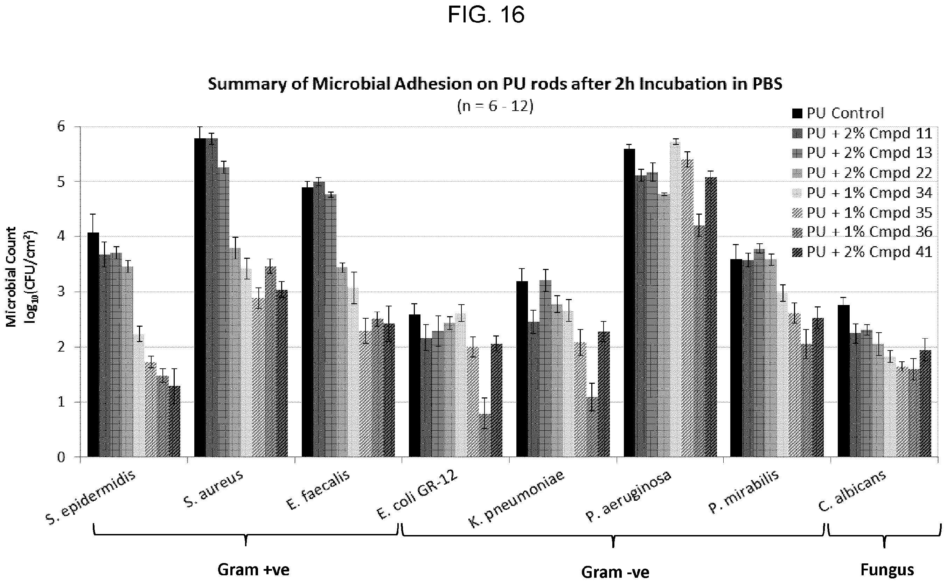

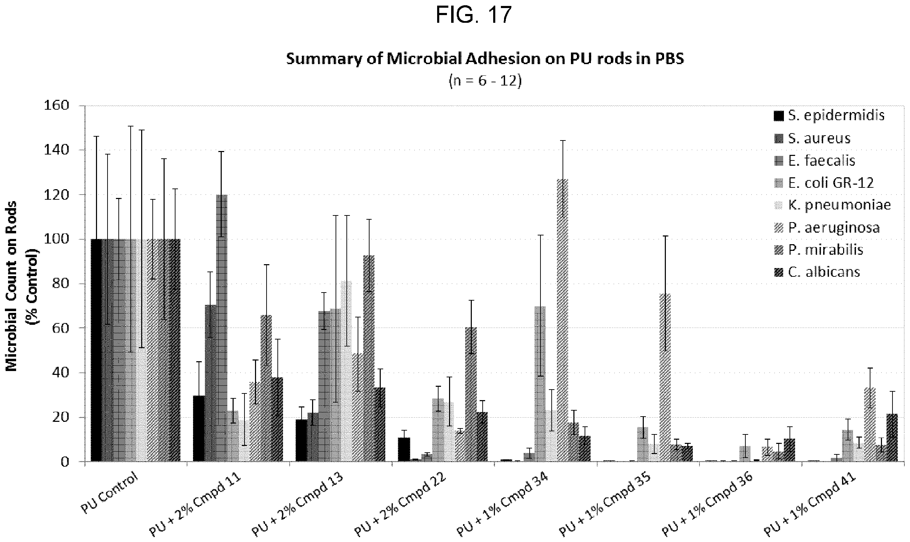

At least two duplicate experiments with n = 3 rod samples were conducted for each microbe and the data is shown in FIGS. 14-15. Bacterial counts are presented in log format and error bars represent standard error. Additive-modified samples having statistically significant differences (t-test with p<0.05) in adhesion relative to unmodified controls are denoted with a (*) symbol. FIG. 16 is a summary graph showing the cumulative data from FIGS. 14 and 15. FIG. 17 is a summary graph showing the cumulative data from FIGS. 14 and 15 but with average adhesion on additive-modified samples normalized to average adhesion on the controls. This graph allows for easy viewing of percent reductions in adhesion on additive-modified samples.

Reductions in bacterial adhesion on additive modified samples relative to unmodified controls ranged from 0 to approximately 3 log (99.9%), depending on the additive formulation and bacterial species tested.

### Example 3: S. aureus adhesion on PU rods with compound 41 after 2 h incubation in PBS

Rods were prepared and incubated with *S. aureus* Newman inoculum solution for 2 h at 37 °C as described in Example 2. After 2 h, the samples were washed 3x with 500 µL PBS to remove loosely adhered bacteria. The samples were then transferred to microcentrifuge tubes containing 2% glutaraldehyde in cacodylate buffer and fixed at 4 °C overnight. After fixing, the samples were dehydrated in graded ethanol solutions (e.g., 50%, 70%, 90%, and 100%) for 10 min, dried by immersion in hexamethyldisilazane for 10 min, followed by air drying overnight.

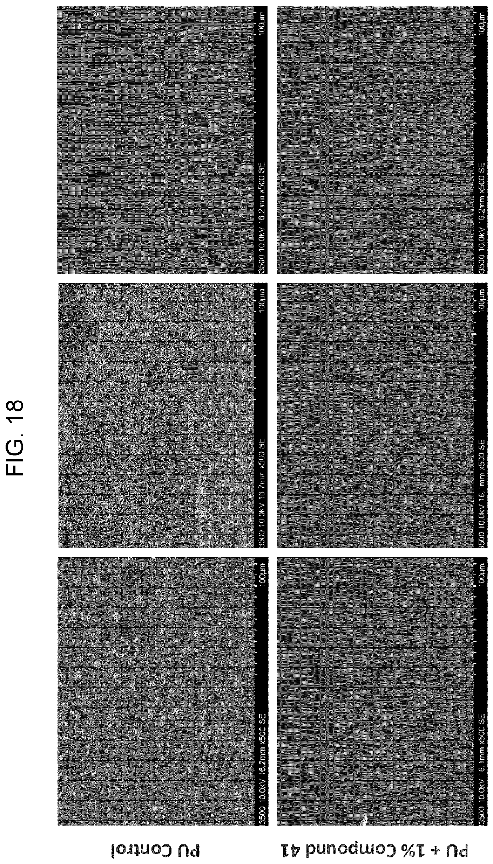

Dried samples were mounted in triplicate on aluminum SEM stubs using double sided adhesive carbon tabs, sputter-coated with gold, and imaged on a Hitachi SU3500 SEM with a secondary electron detector. An accelerating voltage of 10 kV, a spot intensity of 30, and a working distance of 15 mm were used. Representative images at 500x magnification of three replicate rods of unmodified PU control and PU modified with 1% of Compound 41 are shown in FIG. 18. High concentrations of adhered *S. aureus* (light gray spherical-shaped structures) were visible on the unmodified PU controls samples, present either as independent bacteria or aggregates. In contrast, very few *S. aureus* bacteria were observed on the additive-modified surfaces.

### Example 4: Bacterial adhesion on PU rods after 2 h incubation in PBS

Polyurethane rods with additional compounds to those presented in Example 2 were prepared using the method described in Example 2.

Rod samples were cut into 1.5 cm segments, ethylene oxide sterilized, and placed in sterile microcentrifuge tubes. Bacterial strains were grown overnight in Tryptic Soy Broth (TSB) at 37 °C with shaking. To prepare the bacterial inoculum solution the bacterial culture was centrifuged at 4,500 rpm for 10 min, washed with PBS, and re-suspended in PBS at a concentration of 10⁸ CFU/mL. The following bacterial strains were used for testing: *S. epidermidis* 3399, *S. aureus* Newman, *E. coli* 67, *P. aeruginosa* AK-1, and *P. mirabilis* 296. All microbial strains were obtained from Lawson Health Research Institute (London, Ontario), and all strains except *S. epidermidis* were clinical isolates from human infections (blood, urinary tract or other).

One millilitre (1 mL) of inoculum solution was pipetted into the microcentrifuge tubes containing the samples and incubated for 2 h at 37 °C with minimal agitation. After 2 h, the samples were washed 3x with 750 µL PBS to remove loosely adhered bacteria and transferred to new microcentrifuge tubes with 1 mL of PBS. The samples were sonicated for 30 min, followed by vortexing for 30 sec, to detach adhered bacteria. The sonicated solutions were serially diluted and drop plated in quadruplicate on Tryptic Soy Broth (TSB) agar plates. The plates were incubated at 37 °C overnight after which bacterial colonies were counted and bacterial adhesion was calculated in CFU/cm².

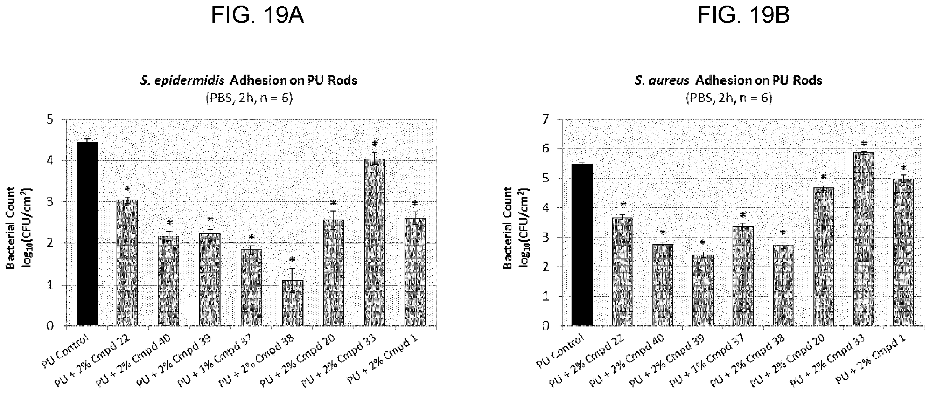

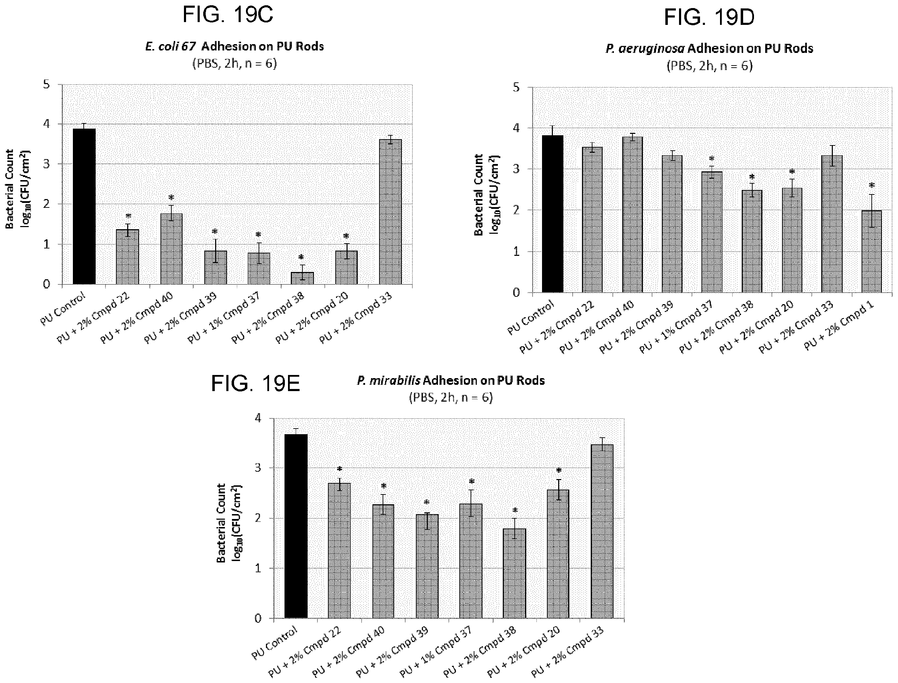

Two duplicate experiments with n = 3 rod samples were conducted for each bacterial strain and the data is shown in FIG. 19. Bacterial counts are presented in log format and error bars represent standard error. Additive-modified samples having statistically significant differences (t-test with p<0.05) in adhesion relative to unmodified controls are denoted with a (*) symbol. Reductions in bacterial adhesion on additive modified samples relative to unmodified controls ranged from 0 to approximately 3.5 log (>99.9%), depending on the additive formulation and bacterial species tested.

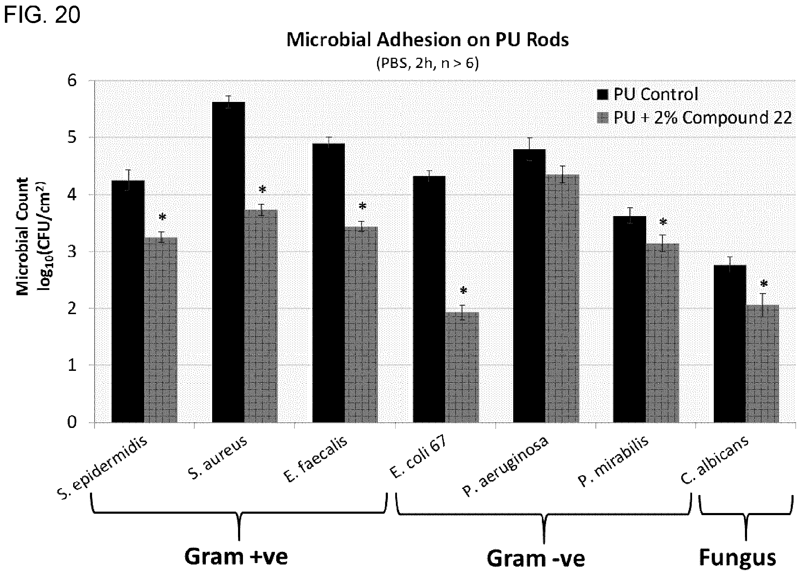

The adhesion data for PU rods with compound 22 from FIGS. 14, 15 and 19 is cumulated in a summary graph in FIG. 20, with error bars representing standard error. This graph shows the broad spectrum efficacy of additive compound 22 in reducing adhesion of both gram positive and gram negative bacterial species as well as fungal microbes.

### Example 5: Bacterial adhesion on PU rods with compound 22 after 2 h incubation in artificial urine and human pooled urine

Polyurethane rods with 2 wt% of additive compound 22 were prepared as described in Example 2.

Artificial urine (AU) was prepared according to a recipe described in literature (McLean RJ et al., An in vitro ultrastructural study of infectious kidney stone genesis, Infection and Immunity, 1985, vol 49(3):p805-811). Human urine (HU) was collected and pooled from three healthy volunteers. Both AU and HU were filter-sterilized, stored at 4 °C, and used within a week of preparation or collection. Bacterial adhesion testing was conducted using the methods described in Example 4, with bacterial inoculum solutions prepared at 10⁸ CFU/mL in AU or HU. Bacterial strains representative of pathogens prevalent in urinary tract infections (*E*. *coli 67, E. faecalis* 33186, and *P. mirabilis* 296) were employed for testing.

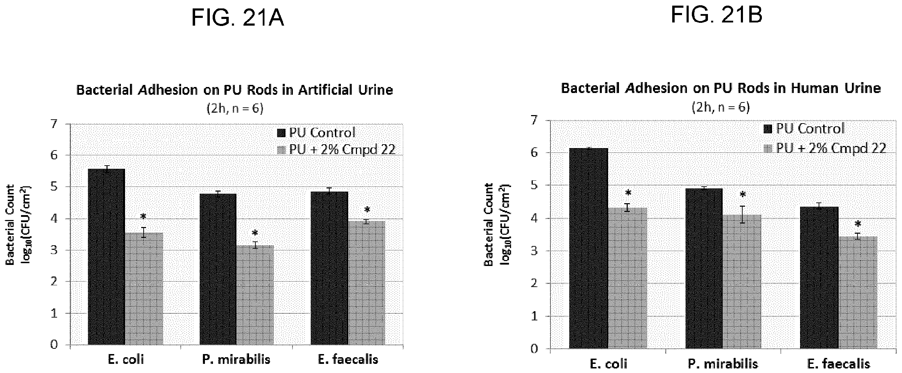

Two duplicate experiments with n = 3 rod samples were conducted for each bacterial strain and the data is shown in FIG. 21. Bacterial counts are presented in log format and error bars represent standard error. Additive-modified samples having statistically significant differences (t-test with p<0.05) in adhesion relative to unmodified controls are denoted with a (*) symbol. Reductions in uropathogen adhesion of up to approximately 2 log (99%) were observed on additive-modified samples relative to unmodified controls in both AU and HU.

### Example 6: E. coli adhesion on PU rods after 12 h incubation in human pooled urine

Polyurethane rods with 2 wt% of additive compounds 22, 38 and 39 were prepared as described in Example 2.

Human urine (HU) for bacterial adhesion experiments was collected and pooled from three healthy volunteers. HU was filter-sterilized, stored at 4 °C, and used within a week of collection. Testing was conducted using the methods described in Example 4, with bacterial inoculum solutions prepared using a clinical uropathogenic *E. coli* strain (*E. coli* 67) at 10⁶ CFU/mL in HU.

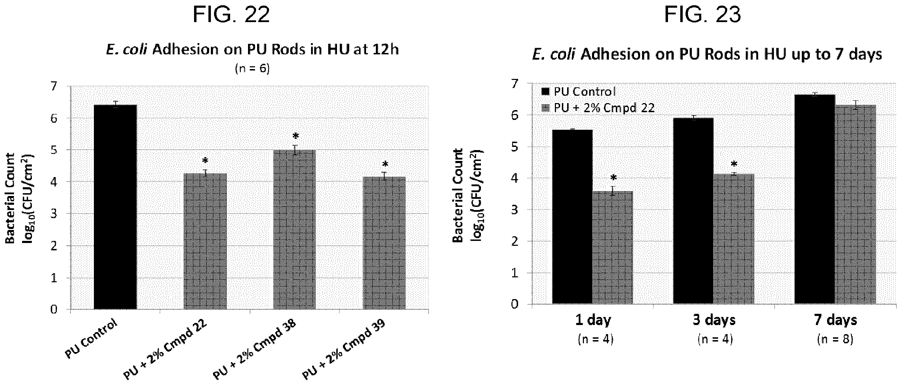

Two duplicate experiments with n = 3 rod samples were conducted and the data is shown in FIG. 22. Bacterial counts are presented in log format and error bars represent standard error. Additive-modified samples having statistically significant differences (t-test with p<0.05) in adhesion relative to unmodified controls are denoted with a (*) symbol. Reductions in *E.coli* adhesion of approximately 1-2 log (90-99%) were observed on the additive modified surfaces relative to unmodified controls.

### Example 7: Extended E. coli adhesion studies (up to 7 days) on PU rods with compound 22 in human pooled urine

Polyurethane rods with 2 wt% of additive compound 22 were prepared as described in Example 2. Rod samples for testing were cut into 1.5 cm segments, ethylene oxide sterilized, and placed in sterile microcentrifuge tubes.

Human urine (HU) for bacterial adhesion experiments was collected and pooled from three healthy volunteers. The HU was filter-sterilized, stored at 4 °C, and used within a week of collection. An inoculum of a clinical uropathogenic *E.coli* strain (*E.coli* 67) was prepared at 10⁶ CFU/mL in HU using the methods described in Example 4. One millilitre (1 mL) of inoculum solution was added to the microcentrifuge tubes containing the samples and the samples were incubated for 24 h at 37 °C with minimal agitation. After 24 h, the samples were washed 3x with 750 µL PBS and transferred to new microcentrifuge tubes with 1 mL sterile HU. The incubation was continued up to 7 days with daily sample washes and HU exchanges. Bacterial adhesion to the samples was determined after 1, 3 and 7 days using the methods described in Example 4.

Two duplicate experiments were conducted with n = 4 rod samples analyzed at days 1, 3, and 7 in experiment #1, and day 7 only in experiment #2. The data is shown in FIG. 23. Bacterial counts are presented in log format and error bars represent standard error. Additive-modified samples having statistically significant differences (t-test with p<0.05) in adhesion relative to unmodified controls are denoted with a (*) symbol. Reductions in *E. coli* adhesion of approximately 2 log (99%) were observed on additive modified surfaces relative to unmodified controls at days 1 and 3, but the reductions diminished by day 7. The diminished efficacy could be the result of in-vitro experimental conditions, such as limited media exchange and flow, and may not reflect in-vivo or clinical performance.

### Example 8: Extended E. coli adhesion studies (8 days) on PU rods with compound 22 in human pooled urine versus Meuller Hinton Broth

Polyurethane rods with 2 wt% of additive compound 22 were prepared as described in Example 2. Rod samples for testing were cut into 1.5 cm segments, ethylene oxide sterilized, and placed in sterile microcentrifuge tubes.

Human urine (HU) for bacterial adhesion experiments was collected and pooled from three healthy volunteers. The HU was filter-sterilized, stored at 4 °C, and used within a week of collection. An inoculum of a clinical uropathogenic *E.coli* strain (*E.coli* 67) was prepared at 10⁶ CFU/mL in either HU or Meuller Hinton Broth (MHB) bacterial growth media using the methods described in Example 4. One millilitre (1 mL) of inoculum solution was added to the microcentrifuge tubes containing the samples and the samples were incubated for 24 h at 37 °C with minimal agitation. After 24 h, the samples were washed 3x with 750 µL PBS and transferred to new microcentrifuge tubes with 1 mL of sterile HU or MHB. The incubation was continued up to 7 days with daily sample washes and HU or media exchanges. Bacterial adhesion to the samples was determined after 7 days using the methods described in Example 4.

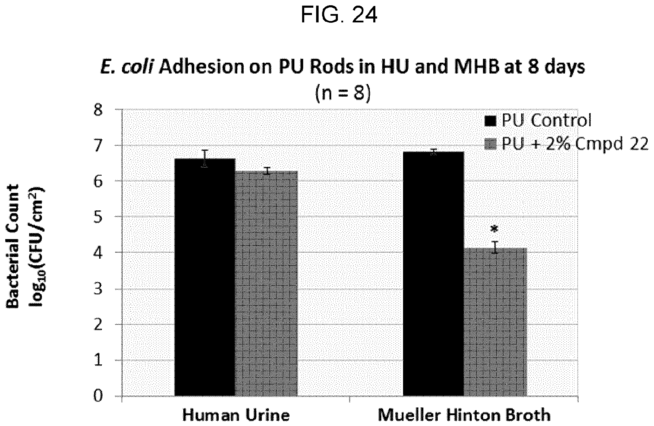

Two duplicate experiments were conducted with n = 4 rod samples and the data is shown in FIG. 24. Bacterial counts are presented in log format and error bars represent standard error. Additive-modified samples having statistically significant differences (t-test with p<0.05) in adhesion relative to unmodified controls are denoted with a (*) symbol. Approximately 3 log (99.9%) reductions in *E.coli* adhesion were observed on the additive modified samples relative to unmodified controls after 8 day incubation in MHB, while no reductions were observed after 8 day incubation in human pooled urine under the conditions of this experiment. This demonstrates the differential impact of experimental conditions on in-vitro bacterial-surface interactions.

### Example 9: SEM imaging of E. coli adhesion PU rods with compound 22 after 24 h incubation in human pooled urine

Polyurethane rods with 2 wt% of additive compound 22 were prepared as described in Example 2. Rod samples for testing were cut into 1.5 cm segments, ethylene oxide sterilized, and placed in sterile microcentrifuge tubes.

Human urine (HU) for bacterial adhesion experiments was collected and pooled from three healthy volunteers. The HU was filter-sterilized, stored at 4 °C, and used within a week of collection. An inoculum of a clinical uropathogenic *E.coli* strain (*E.coli* 67) was prepared of 10⁶ CFU/mL in HU using the methods described in Example 4. One millilitre (1 mL) of inoculum solution was added to the microcentrifuge tubes containing the samples and the samples were incubated for 12 h at 37 °C with minimal agitation. After 12 h, the samples were washed 3x with 750 µL PBS and transferred to new microcentrifuge tubes with 1 mL of sterile HU. The samples were further incubated for another 12 h at 37 °C. Afterwards, the samples were washed 3x with 750 µL PBS and transferred to microcentrifuge tubes containing 2% glutaraldehyde in cacodylate buffer for overnight fixation at 4 °C. After fixing, the samples were dehydrated in graded ethanol solutions (e.g., 50%, 70%, 90%, and 100%) for 10 min, then dried by immersion in hexamethyldisilazane for 10 min, followed by air drying overnight.

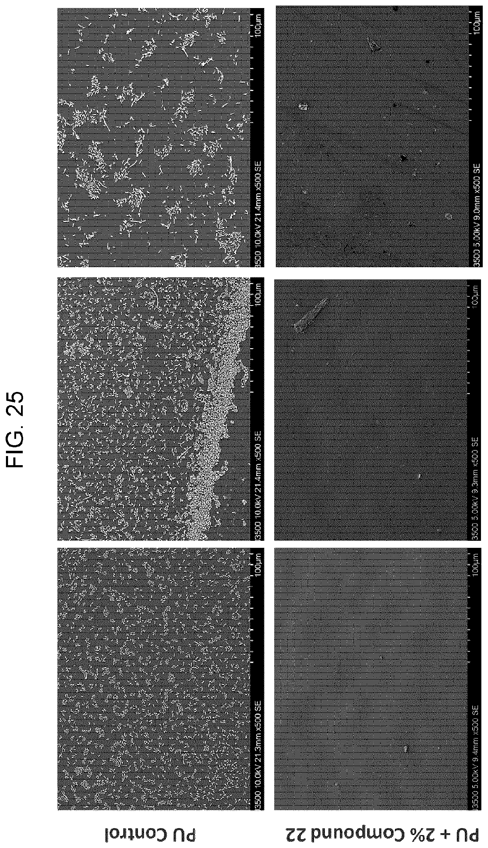

Dried samples were mounted in triplicate on aluminum SEM stubs using double sided adhesive carbon tabs, sputter coated with gold, and imaged on a Hitachi SU3500 SEM with a secondary electron detector. An accelerating voltage of 10 kV and a working distance of 5 mm were used. Representative images at 500x magnification of three replicate rods of unmodified PU control and PU modified with 2% of Compound 22 are shown in FIG. 25. High concentrations of adhered *E. coli* (light gray rod-shaped structures) were visible on the unmodified PU controls samples, present either as independent bacteria or aggregates. In contrast, very few *E. coli* were observed on the additive-modified surfaces.

### Example 10: Bacterial adhesion on PU catheter tubing with compound 22 after 24 h incubation in clinical urine

Polyurethane catheter tubing for testing was prepared by first compounding additive compound 22 into Carbothane 3595A (CB 95A) polyurethane resin at 2% loading using commercial processes. Both virgin CB 95A resin and compounded CB 95A resin with compound 22 were then extruded into 7F tubing (outer diameter = 2.4 mm; inner diameter = 1.5 mm) using commercial processes.

Clinical urine samples were collected from patients with indwelling ureteral stents and were stored at 4 °C until use. Five hundred (500 µL) of each sample was incubated at 37 °C for 24 h with shaking and then culture plated to enumerate total planktonic bacteria. Urine samples showing bacterial growth (> 2.0 × 10³ CFU/mL) were used for bacterial adhesion testing with polyurethane tubing samples. Several of the urine samples contained multiple bacterial strains as observed from the culture plates.

Tubing samples were cut into 1.0 cm segments, ethylene oxide sterilized, and placed in sterile microcentrifuge tubes. Five hundred (500 µL) of clinical urine was added to the microcentrifuge tubes containing the samples and the samples were incubated for 24 h at 37 °C with minimal agitation. After 24 h, bacterial adhesion to the samples was quantified using the methods described in Example 4.

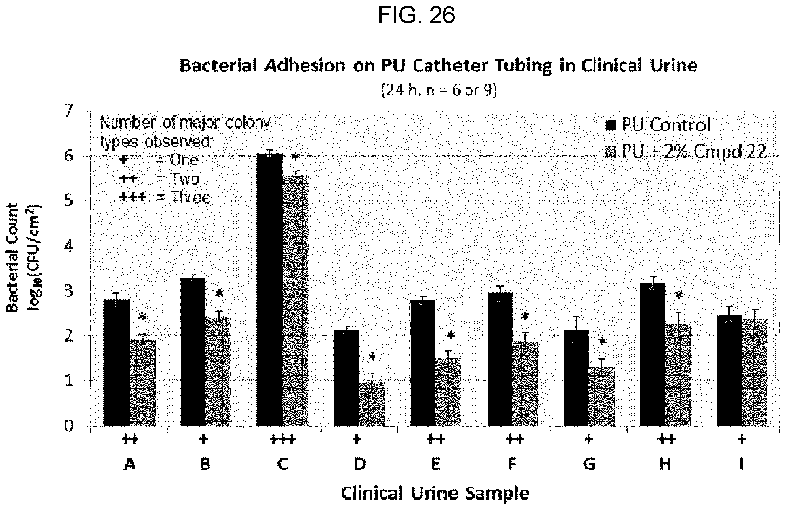

Two or three replicate experiments with n = 3 rod samples were conducted for each clinical urine sample and the data is shown in FIG. 26. Bacterial counts are presented in log format and error bars represent standard error. Additive-modified samples having statistically significant differences (t-test with p<0.05) in adhesion relative to unmodified controls are denoted with a (*) symbol. The number of major bacterial colony types observed in each clinical urine sample are denoted with the (+) symbol. Statistical significant reductions in bacterial adhesion were observed on additive modified samples in 8/9 urine clinical urine samples tested.

### Example 11: E. coli adhesion on PU catheter tubing with compound 22 after 24 h incubation in artificial urine and human pooled urine

Polyurethane catheter tubing for testing was prepared by first compounding additive compound 22 into Carbothane 3595A (CB 95A) polyurethane resin at 2% loading using commercial processes. Both virgin CB 95A resin and compounded CB 95A resin with compound 22 were then extruded into 7F tubing (outer diameter = 2.4 mm; inner diameter = 1.5 mm) using commercial processes.

Artificial urine (AU) was prepared according to a recipe described in literature (McLean RJ et al., An in vitro ultrastructural study of infectious kidney stone genesis, Infection and Immunity, 1985, vol 49(3):p805-811). Human urine (HU) was collected and pooled from three healthy volunteers. Both AU and HU were filter-sterilized, stored at 4 °C, and used within a week of preparation or collection. Bacterial adhesion testing was conducted using the methods described in Example 4, with tubing samples 1.5 cm in length and bacterial inoculum solutions prepared at 10⁶ CFU/mL in AU or HU.

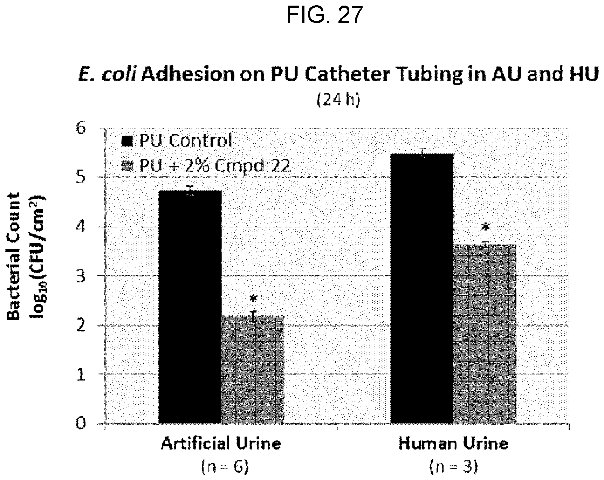

One experiment with n = 3 tubing samples was conducted in HU and two duplicate experiments with n = 3 samples were conducted in AU. The results are shown in FIG. 27. Bacterial counts are presented in log format and error bars represent standard error. Additive-modified samples having statistically significant differences (t-test with p<0.05) in adhesion relative to unmodified controls are denoted with a (*) symbol. Reductions in *E. coli* adhesion of approximately 2-3 log (99-99.9%) were observed on additive-modified catheter tubing relative to unmodified control tubing in both AU and HU after 24 h of incubation.

### Example 12: E. coli adhesion on PU catheter tubing with compound 22 under flow conditions

Polyurethane catheter tubing for testing was prepared by first compounding additive compound 22 into Carbothane 3595A (CB 95A) polyurethane resin at 2% loading using commercial processes. Both virgin CB 95A resin and compounded CB 95A resin with compound 22 were then extruded into 7F tubing (outer diameter = 2.4 mm; inner diameter = 1.5 mm) using commercial processes.

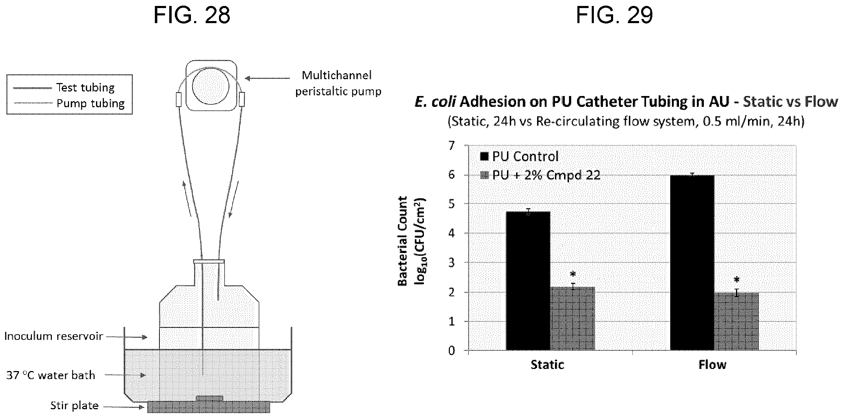

A re-circulating system was set-up for bacterial adhesion testing under flow as depicted in FIG. 28. The system consisted of a reservoir containing the inoculum solution which was circulated through test circuits using a peristaltic pump. Each test circuit consisted of two test tubing pieces 20" long connected via a silicone pump tubing piece (length = 18", inner diameter = 1.65 mm). The inlet tubing tip of each circuit was submerged in the inoculum solution, while the outlet tip was suspended above the inoculum solution. The reservoir was maintained in a 37 °C water bath and kept sealed to the environment. One unmodified control tubing circuit and one additive-modified tubing circuit was tested per experiment and both circuits were fed from the same inoculum reservoir.

All components of the re-circulating flow system were sterilized either by autoclave or ethylene oxide. Artificial urine (AU) was prepared according to a recipe described in literature (McLean RJ et al., An in vitro ultrastructural study of infectious kidney stone genesis, Infection and Immunity, 1985, vol 49(3):p805-811). The AU was filter sterilized and kept at 4 °C until use. A clinical uropathogenic *E. coli* strain (*E. coli* 67) was grown overnight in Tryptic Soy Broth (TSB) at 37 °C with shaking. To prepare the bacterial inoculum solution the bacterial culture was centrifuged at 4,500 rpm for 10 min, washed, and re-suspended in AU at a concentration of 10⁶ CFU/mL

At the start of the experiment, a reservoir with sterile AU was connected to the flow system and sterile AU was circulated through the test circuits at 0.5 mL/min for 10 min to pre-condition the tubing and confirm the flow rate. After 10 min, the clean AU reservoir was replaced with a reservoir containing 300 mL of *E. coli* inoculum solution that was continuously stirred using a magnetic stir bar. The inoculum solution was circulated through the test circuits at 0.5 mL/min for 24 h.

After 24 h, the pump was stopped and the inoculum solution was drained from the circuits. Four samples of the test tubing, 1.5 cm in length, were cut from the distal portion of each test circuit (downstream of the pump tubing). Bacterial adhesion on the samples was determined using the methods described in Example 4. Duplicate experiments were conducted and the data for bacterial adhesion under flow is presented in FIG. 29 along with adhesion under static conditions for the same type of samples and same media / timeframe (data taken from Example 11). Bacterial counts are presented in log format and error bars represent standard error. Additive-modified samples having statistically significant differences (t-test with p<0.05) in adhesion relative to unmodified controls are denoted with a (*) symbol.

In the presence of flow, bacterial adhesion reductions on additive-modified samples versus unmodified controls were observed to be further magnified compared to the static condition. Specifically, reductions of approximately 4 log (99.99%) were observed on additive-modified tubing under flow conditions. The quantitative reductions in bacterial adhesion were further confirmed by SEM imaging.

### Example 13: In vivo rabbit study on PU ureteral stent prototypes with compound 22

A preliminary in vivo study was conducted to evaluate the potential of medical device surface modification with an example additive of the invention to prevent establishment of an infection after bacterial exposure. A rabbit urinary tract infection model was used for the study with ureteral stents (tubing inserted into the ureter to prevent or treat obstruction of urine flow from the kidneys) as model devices. Outcome measures included urine bacterial counts throughout the study and bacterial adhesion to the device surfaces upon explantation.

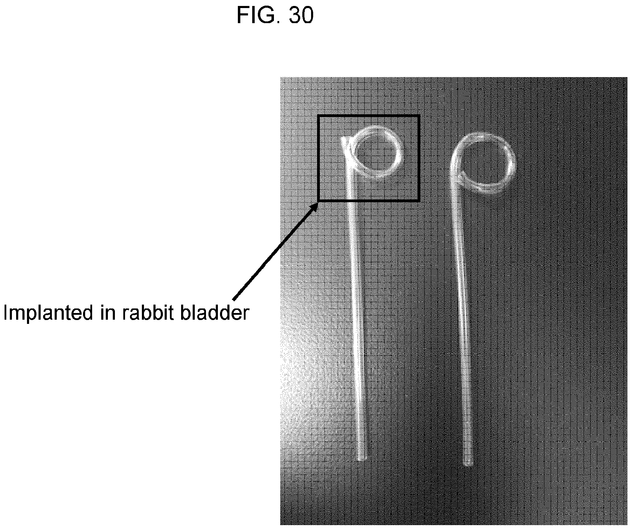

To prepare prototypes, additive compound 22 was first compounded into Carbothane 3595A (CB 95A) polyurethane resin at 2% loading using commercial processes. Both virgin CB 95A resin and compounded CB 95A resin with compound 22 were then extruded into 7F tubing (outer diameter = 2.4 mm; inner diameter = 1.5 mm) using commercial processes. Extruded tubing was sectioned into pieces approximately 12 cm long and one end of each piece was heat formed into pig-tail curls with a diameter of 1.0 cm to yield ureteral stent prototypes. The stent-curl portions of the prototypes consisting of approximately 5 cm length of tubing, shown in FIG. 30, were used in the study.

All devices for testing were EtO sterilized. A rabbit uropathogenic *E-coli* strain (WE 6933) was used to initiate infection in the rabbits. To prepare bacterial inoculum, the strain was grown overnight in Tryptic Soy Broth (TSB) at 37 °C with shaking. The bacterial culture was then centrifuged at 4,500 rpm for 10 min, washed, and re-suspended in saline at a concentration of 10⁷ CFU/mL.

Stent-curls were inserted transurethrally into bladders of New Zealand White rabbits under general anaesthesia. Specifically, a 6Fr catheter was guided into the bladder via ultrasound, with a flexible tip guidewire tunneled through. The catheter was withdrawn and the stent-curl segment was placed over the guidewire and inserted into the bladder using the catheter as a pusher. The guidewire was removed, the bladder was drained, and 1.5 mL of the bacterial inoculum was injected in the bladder through the catheter followed by a 0.5 mL sterile saline flush. The catheter was withdrawn and ultrasound was used to confirm location of the stent-curl in the bladder. The rabbits were recovered from anaesthesia and housed in individual cages for the duration of the study (7 days) with standard diet and water provided *ad libitum.*

Immediately prior to device placement, and under sedation on days 1, 3, 5 and 7 thereafter, urine was collected from the bladder of each rabbit via cystocenthesis. Samples of rabbit urine were drop and spread plated on LB agar plates and incubated for 24 h at 37 °C, after which colonies were counted to determine urine *E.coli* concentration in CFUs/mL. On day 7, the rabbits were euthanized and stent-curls were retrieved for bacterial adhesion analysis. Stent-curls removed from bladders were found to be covered in encrustation deposits, which is common in the rabbit model as rabbit urine is known to be prone to formation of precipitates. In order to separate bacteria adhered to the device surface from that trapped in the surface deposits the samples were processed in two parts: (1) each stent-curl was sectioned into 1 cm pieces and the encrustation deposits from each piece were gently flaked / scraped off into microcentrifuge tubes; and (2) remaining tubing pieces were placed into separate microcentrifuge tubes. One mL of PBS was added to each microcentrifuge tube and the samples were sonicated to break up encrustation deposits and detach adhered/aggregated bacteria. The solutions were further processed for CFU counts using the methods described in Example 4.

A trend towards decreased *E.coli* urine concentrations was observed in rabbits implanted with additive-modified stent-curls relative to rabbits with unmodified controls (~2 log or 99% reduction on days 5 and 7). Overall *E. coli* adhesion to the stent-curl surfaces, including the bacteria trapped in encrustation deposits, was reduced by ~1 log (90%) on additive-modified stent-curls compared to unmodified controls. *E.coli* adhesion to the stent-curl surfaces alone, excluding bacteria trapped in the encrustation deposits, was reduced by ~3.5 log (99.9%) on additive-modified stent-curls relative to unmodified controls. Statistically analysis could not be done on the data due to a low number of devices analyzed.

### Example 14: Bacterial adhesion on PU rods with compound 22 after external antibiotic exposure (in vitro testing of antibiotic susceptibility)

Implanted medical devices are often used in combination with locally or systemically delivered antibiotics in clinical practice to prevent the onset or persistence of bacterial infection. It was of interest to evaluate if additive-modified surfaces could impact the levels of externally applied antibiotics required to control the growth and adhesion of bacteria and biofilm, and therefore play a role in helping to limit clinical infection rates.

Polyurethane rods with 2% of additive formulation 22 were prepared using the method described in Example 2. Rod samples were cut into 1.5 cm segments, ethylene oxide sterilized, and placed in sterile microcentrifuge tubes.

A clinical uropathogenic *E. coli* strain (*E. coli* 67) was grown overnight in Meuller Hinton Broth (MHB) at 37 °C with shaking. To prepare the bacterial inoculum solution the bacterial culture was centrifuged at 4,500 rpm for 10 min, washed, and re-suspended in fresh MHB at a concentration of 10⁶ CFU/mL. One mL of inoculum solution was added to the microcentrifuge tubes with the samples and the samples were incubated for 24 h at 37 °C with minimal agitation. After 24 h, the samples were washed 3x with 750 µL PBS and transferred to new microcentrifuge tubes with 1 mL of sterile MHB. The incubation was continued for 7 days with daily sample washes and MHB exchanges. After 7 days, the rod samples were washed and transferred to microcentrifuge tubes with 1 mL of ciprofloxacin antibiotic solution in MHB at various concentrations (0-0.5 µg/mL). Rods were incubated in ciprofloxacin solution for 24 h, after which they were washed and bacterial adhesion to the rods was quantified as per the methods described in Example 4.

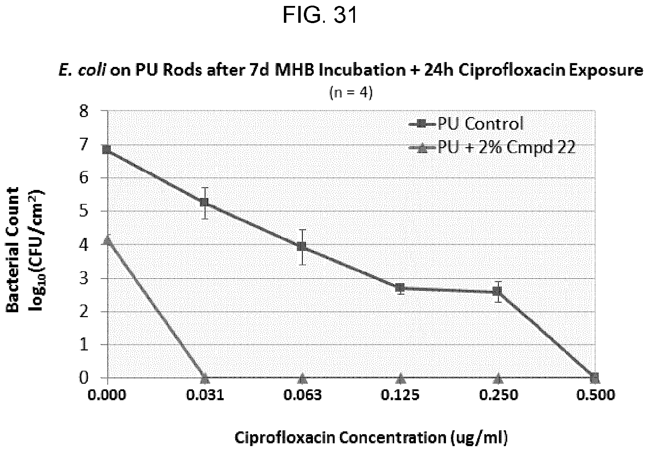

Two experiments were conducted with n = 2 two rods tested at each ciprofloxacin concentration and the results are shown in FIG. 31. Lower concentrations of ciprofloxacin antibiotic were required to achieve complete bacterial kill on additive-modified samples compared to unmodified controls, suggesting that modified surfaces could impact the susceptibility of the bacteria colonizing a device to an externally applied antibiotic. However, it was not clear if this effect was a result of the surface affecting the bacteria's virulence factors directly or if it was simply a result of the surface's ability to limit bacterial adhesion, thereby necessitating less antibiotic for efficacy.

### Example 15: Bacterial adhesion on Silicone rods

Silicone rod prototypes for bacteria adhesion testing were prepared in the laboratory using a method that simulates commercial silicone extrusion or molding processes. Medical grade 2-part curing silicone elastomers MED-4780 and MED-4765 were obtained from Nusil. Equal amounts of supplied Part A and Part B compounds were independently softened using either a Teflon-coated rolling pin on a glass surface or a two-roll mill. The same equipment was then used to blend additive compounds into Part A by repeated kneading motions. The Part A blend was then further blended with equal mass of Part B until a thoroughly mixed resin was produced containing a loading of 2 or 4 wt% additive (depending on formulation used).

The blends were filled into a plastic syringe and mock extruded into a cylindrical rod structure using a syringe pump at a speed of 0.2-0.3 mL/min. The extruded rods were cured in an air flow oven at 116 °C for 20 min.

Rod samples were cut into 1.5 cm segments and tested for bacterial adhesion in either PBS or human pooled urine (HU) using the methods described in Example 4. The HU for experiments was collected and pooled from three healthy volunteers, filter-sterilized, stored at 4 °C, and used within a week of collection. The following bacterial strains were used for testing: *S. epidermidis* ATCC 35984, *S. aureus* Newman, *E. faecalis* 33186, *E. coli* 67, *P. aeruginosa* AK-1, *K. pneumoniae* 280, and *P. mirabilis* 296. All microbial strains were obtained from Lawson Health Research Institute (London, Ontario), and all strains except *E. faecalis* were clinical isolates from human infections (blood, urinary tract or other). Inoculum solutions were prepared at concentration of either 10⁸ CFU/mL (2 h experiments) or 10⁶ CFU/mL (>24 h experiments). For experiments extending past 24 h, sample washes and media exchanges were conducted every 24 h as described in Example 7.

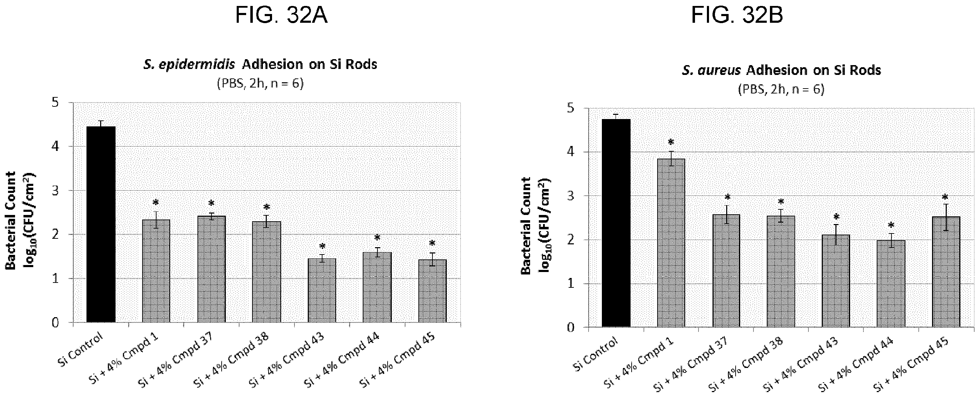

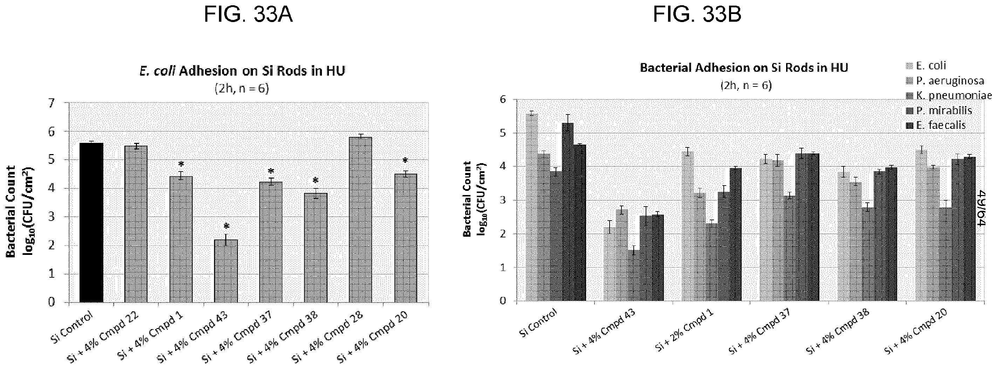

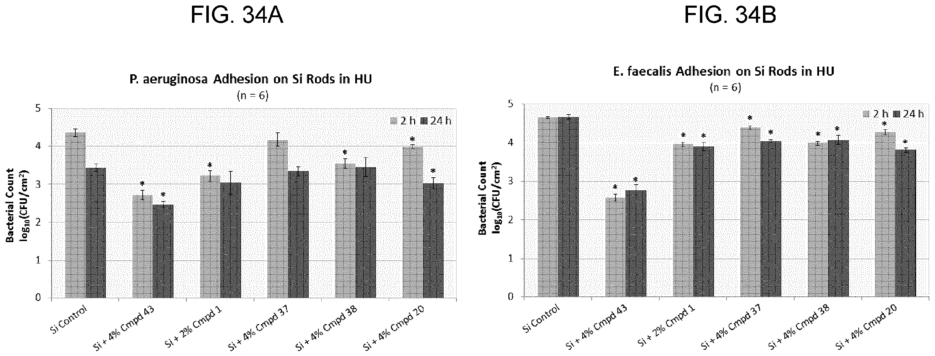

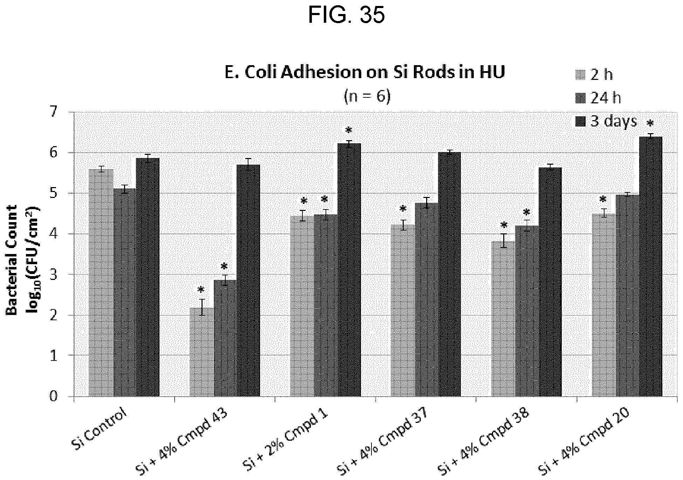

Two duplicate experiments with n = 3 rod samples were conducted for each bacterial strain and condition evaluated and data is shown in FIGS. 32-36. Bacterial counts are presented in log format and error bars represent standard error. Additive-modified samples having statistically significant differences (t-test with p<0.05) in adhesion relative to unmodified controls are denoted with a (*) symbol. Reductions of up to 3 log (99.9%) in bacterial adhesion were observed on additive modified samples relative to unmodified controls at 2 h in PBS and HU, depending on the formulation and bacteria tested. Up to 2 log (99%) reductions were maintained at 24 h in HU with the three bacteria tested (*E*. *coli, P. aeruginosa, E. faecalis*). Extended studies were only conducted with *E.coli* and reductions were diminished at 3 days under the conditions of the in-vitro experiment.

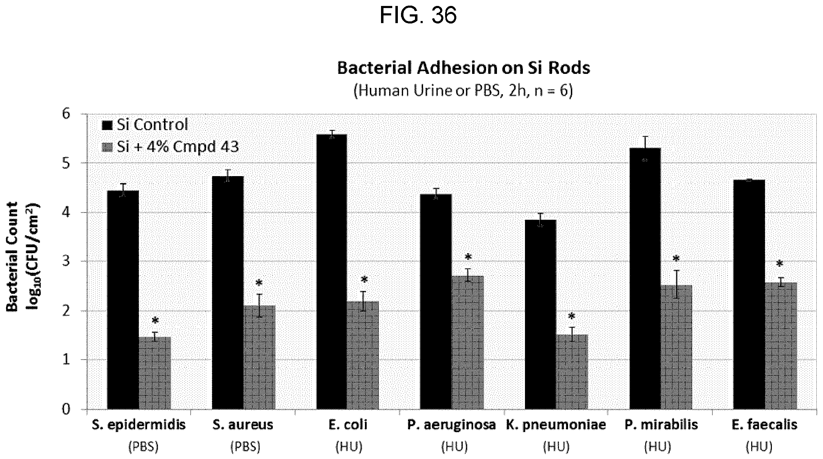

FIG. 36 depicts a cumulative graph showing bacterial adhesion at 2 h for compound 43. This graph demonstrates the broad spectrum efficacy of additive compound 43 in reducing adhesion of both gram positive and gram negative bacterial species on silicone surfaces.

### Example 16: S. aureus adhesion on PU rods with compound 22 after 24h incubation in diluted plasma or serum

Certain medical devices are in contact with biological fluids containing various amount of proteins (for example intravascular catheters in contact with blood), that can adhere to the device surface and influence bacterial adhesion. Therefore, it was of interest to evaluate if additive-modified surfaces could resist bacterial adhesion in the presence of protein-containing fluids. For *in vitro* testing, plasma or serum diluted in PBS and supplemented with TSB growth media was used to represent blood as it contains the same proteins.

Polyurethane rods with 2% of additive compound 22 were prepared using the method described in Example 2. Rod samples were cut into 1.5 cm segments, ethylene oxide sterilized, and tested in a 24h bacterial adhesion assay using the methods described in Example 4. *S. aureus Newman* strain was obtained from Lawson Health Research Institute (London, Ontario). Fresh human plasma (pooled from at least 3 donors and anticoagulated with ACD or Na Citrate) and fresh pooled serum were obtained from Biochemed Services (Winchester, VA). Inoculum solutions were prepared to contain 10⁶ cfu/ml bacteria in a mixture of 50% PBS, 25% TSB growth media and 25% plasma or serum. Rod samples were pre-conditioned by incubating in 100% plasma for 2h to allow protein adhesion prior to transferring to the inoculum solutions for 24h incubation.

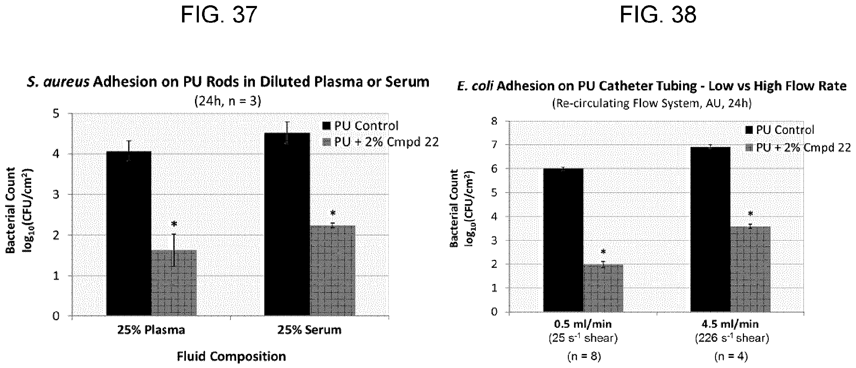

Results of the experiments conducted with n = 3 rod samples are shown in FIG. 37. Bacterial counts are presented in log format and error bars represent standard error. Additive-modified samples having statistically significant differences (t-test with p<0.05) in adhesion relative to unmodified controls are denoted with a (*) symbol. Reductions in *S. aureus* adhesion of ~2 log (99%) were observed on additive modified samples compared to unmodified controls after 24h incubation in diluted plasma or serum. These experiments demonstrate the ability of the modified surfaces to resist bacterial adhesion in the presence of protein-containing solutions.

### Example 17: E.coli adhesion on PU catheter tubing with compound 22 under flow conditions at different flow / shear rates

Medical devices experience a variety of flow / shear rates and biofilm formation is known to be impacted by shear force, with higher shear rates in some cases promoting biofilm development as the bacterial community upregulates growth in response to the aggressive conditions. It was of interest to evaluate if additive-modified surfaces could resist bacterial adhesion at different flow rates.

The methods described in Example 12 were used evaluate *E. coli* 67 adhesion to unmodified and additive-modified 7F polyurethane catheter tubing containing 2% of compound 22 over 24h of artificial urine flow, but using a higher flow rate of 4.5 ml/min (corresponding to a luminal wall shear rate of 226 s⁻¹). One experiment was conducted, and four 1.5 cm long samples from the flow circuits were analyzed for bacterial adhesion. The data is presented in FIG. 38 alongside the analogous data from Example 12 utilizing a 0.5 ml/min flow rate (corresponding to a luminal wall shear rate of 25 s⁻¹). Bacterial counts are presented in log format and error bars represent standard error. Additive-modified samples having statistically significant differences (t-test with p<0.05) in adhesion relative to unmodified controls are denoted with a (*) symbol.

In this experimental set-up, the higher shear rate induced higher overall *E.coli* adhesion and likely biofilm formation on all test samples, but reductions of ~3 log (99.9%) were still observed on tubing modified with 2% compound 22 compared to unmodified control tubing. This data suggests that the modified surfaces have the ability to resist bacterial adhesion and biofilm formation even under aggressive conditions.

### Example 18: E. coli adhesion and biofilm formation on PU catheter tubing with compound 22 under flow conditions over extended timeframe (7 days)

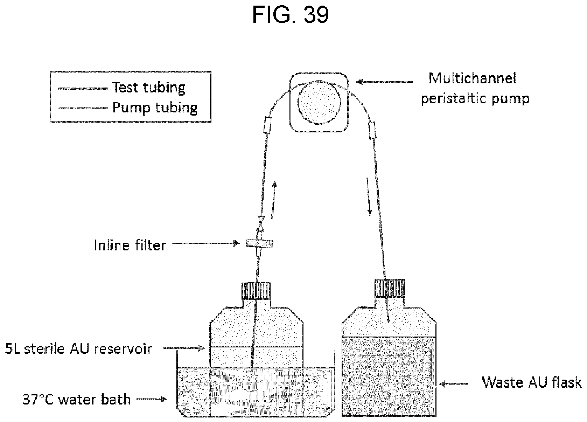

In order to evaluate bacterial adhesion and biofilm formation on polyurethane tubing samples over extended timeframes, a non-circulating flow system was design to allow supply of fresh media and removal of bacterial waste, thereby enabling continuous biofilm growth and more closely simulating in-vivo conditions. The system is depicted in FIG. 39, and consisted of a flow circuit composed of test tubing connected to silicone peristaltic pump tubing and feeding from a 5L sterile artificial urine (AU) reservoir. The lengths of the test tubing segments totaled 59" (proximal and distal of pump) while the silicone pump tubing segment was 18" long.

Unmodified and additive modified 7F polyurethane tubing containing 2% of Compound 22 were manufactured as described in Example 12. Two circuits were set-up simultaneously, one with unmodified control test tubing and one with additive-modified test tubing. All components of the system were sterilized either by autoclave or ethylene oxide. Artificial urine and bacterial inoculum solution containing 10⁸ cfu/ml *E.coli* 67 were also prepared as described in Example 12.

At the start of the experiment, the circuits were primed with sterile AU flow for 10 min. The circuit feed tubing (without inline filter) was then inserted into a flask with inoculum solution maintained under stirring in a 37°C water bath. The inoculum was pumped through the circuits for 2h to seed bacteria on the intraluminal surfaces of the tubing. After 2h, the pump was stopped and fluid was locked in the main portion of the circuits using an in-line stopcock and tubing clamps. The inoculum flask was replaced with a 5L sterile AU feed reservoir, and the feed tubing segment previously immersed in the inoculum solution was discarded and replaced with a new tubing segment connected to a 0.45 µm filter assembly to prevent backflow or migration of bacteria from the seeded circuit into the sterile AU feed reservoir. The new tubing segment and filter were primed with sterile AU through the stopcock to prevent introduction of air bubbles into the circuit. The pump was re-started and stopcock / clamps opened to resume flow at 0.5 ml/min.

At 24h, the pump was stopped and the distal tubing was clamped ~15 cm upstream from the waste flask stopper. The fluid downstream of the clamp was drained and three 1.5 cm tubing samples were cut for bacterial counts, along with samples for SEM and crystal violet (CV) staining to image biofilm formation. The distal tip of the remaining circuit was reinserted into the waste flask and flow was restarted and continued until day 3 and 7, when additional samples were taken. Samples for bacterial counts were rinsed and processed as described in Example 4. Samples for SEM were rinsed, processed and imaged as described in Example 9, using a FEI XL30 ESEM operating with a secondary electron detector. An accelerating voltage of 20 kV, a spot size of 3 and a working distance of 6.5 mm were used. Tubing samples for CV staining were longitudinally sliced and soaked in 0.1% CV solution in water for 15 min. Samples were removed, gently rinsed by dipping 3x in clean water, and photographed. Additionally, the tubing was visually inspected throughout the duration of the experiment for evidence of biofouling.

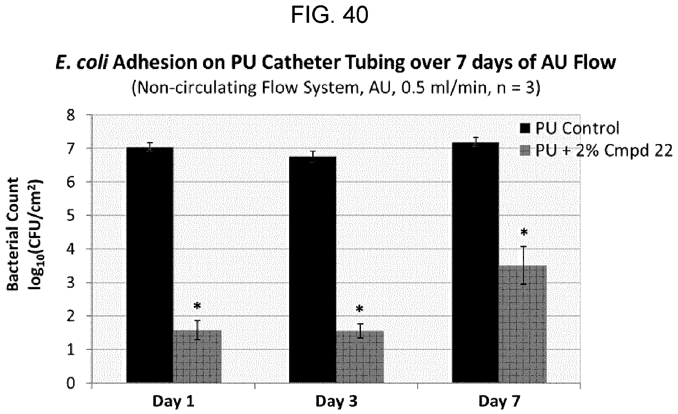

Intraluminal *E.coli* adhesion on the test tubing over 7 days is shown in FIG. 40. Bacterial counts are presented in log format and error bars represent standard error. Additive-modified samples having statistically significant differences (t-test with p<0.05) in adhesion relative to unmodified controls are denoted with a (*) symbol. Significant reductions in *E. coli* adhesion were observed on modified tubing compared to controls at all time points: 5 log (99.999%) up to day 3 and 3 log (99.9%) at day 7.

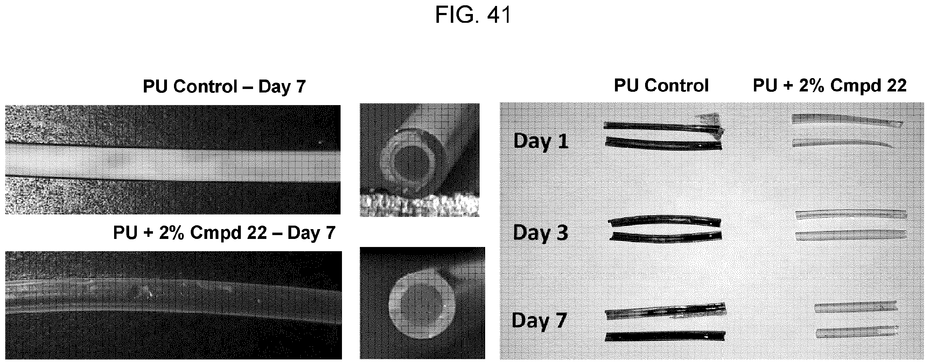

Differences in biofouling on tubing were visually visible starting at day 3. Specifically, white slurry presumed to be biofilm was observed in the lumen of the unmodified tubing, while the tubing modified with Compound 22 remained clean. Crystal violet staining confirmed significant buildup of biofilm on the unmodified tubing, while no biofilm formation was observed on the additive-modified tubing. Photographs of the biofilm formation are shown in FIG. 41.

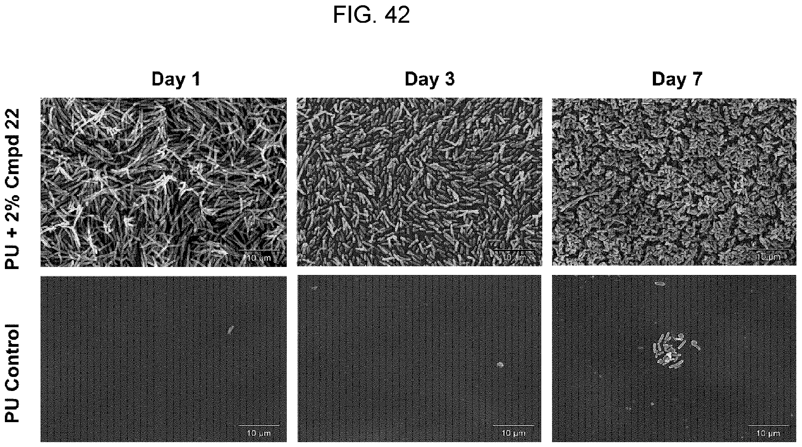

Additionally, differences in biofilm formation on test tubing were confirmed through SEM imaging. For each time point, two 1 cm long samples were imaged for both unmodified control and additive-modified tubing, and four random images were taken of each sample. Representative images at 5000x are shown in FIG. 42. Based on image analysis of the random images, 100% of the unmodified control surfaces were covered with biofilm, while < 5% of the surfaces modified with Compound 22 had visible bacteria.

### Example 19: Bacterial adhesion on PU catheter tubing with compound 22 under flow conditions with different bacterial species

Adhesion of various bacterial species to unmodified control and additive modified 7F polyurethane catheter tubing with 2% of compound 22 was investigated with the non-circulating flow system using the methods described in Example 18.

The following bacterial strains were used for testing: *P. mirabilis* 296, *S. aureus Newman, S. epidermidis* 35984 and *E. faecalis* 33186. All microbial strains were obtained from Lawson Health Research Institute (London, Ontario). Artificial urine was used for experiments with the *P. mirabilis* 296 uropathogen, while a mixture of 75% PBS / 25% TSB (Tryptic Soy Broth) was used for experiments with all other bacteria. Bacterial strains were grown overnight in TSB at 37 °C with shaking. To prepare bacterial inoculum solution the bacterial culture was centrifuged at 4,500 rpm for 10 min, washed, and re-suspended at 10⁸ cfu/ml in the experimental media. A flow rate of 0.5 ml/min was used and four 1.5 cm long samples from the distal portion of each circuit were analyzed for bacterial adhesion after 24h as described in Example 18.

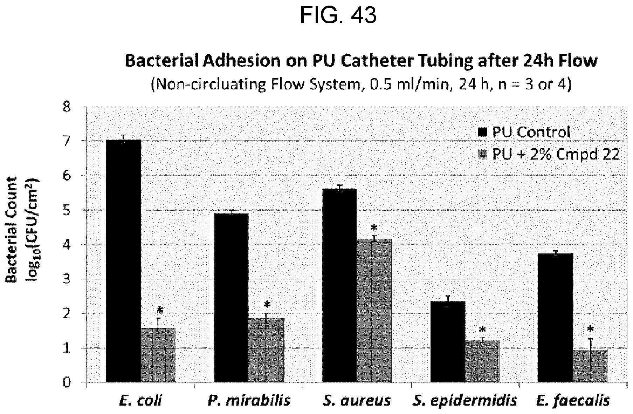

Bacterial adhesion to the tubing after 24h of flow is shown in FIG. 43 along with *E. coli* data from Example 18. Bacterial counts are presented in log format and error bars represent standard error. Additive-modified samples having statistically significant differences (t-test with p<0.05) in adhesion relative to unmodified controls are denoted with a (*) symbol. Significant reductions in bacterial adhesion on tubing modified with compound 22 versus unmodified control tubing were seen at 24h for all species tested, suggesting an overall anti-adhesive property of the modified surfaces.

### Example 20: Uropathogen adhesion on PU tubing with compound 22 in comparison to commercial ureteral stent products

Adhesion of uropathogens to unmodified control and additive modified 7F polyurethane tubing with 2% of compound 22 was investigated in comparison to commercially available 7F ureteral stent tubing from leading manufacturers. The methods described in Example 11 were employed, with the exception of the artificial urine recipe, where a recipe based on Brooks T. et al, "A simple artificial urine for the growth of urinary pathogens." Letters in Applied Microbiology 1997, Vol. 24(3):203-6, was used.

Commercial ureteral stents for testing were obtained from a medical supplies distributor and are listed in Table 2. Stents were sectioned into 1.5 cm pieces, trying to avoid the presence of drainage holes whenever possible, and were sterilized by ethylene oxide. Bacterial strains for testing (*E. coli 67, E. faecalis* 33186 and *P. mirabilis* 296) were obtained from Lawson Health Research Institute (London, Ontario). *E. coli* and *E. faecalis* adhesion was evaluated after 24h incubation in human pooled urine (HU), while *P. mirabilis* adhesion was evaluated in artificial urine (AU) because this species can increase the pH or urine and requires a buffered media to maintain viability in a static in-vitro system.

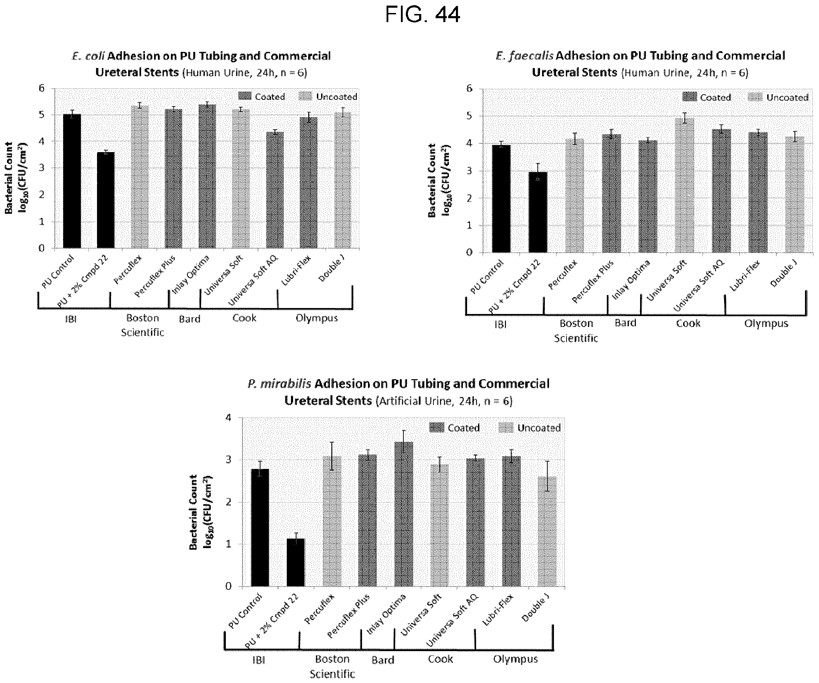

Bacterial adhesion to the tubing samples after 24h is shown in FIG. 44. Bacterial counts are presented in log format and error bars represent standard error. Tubing modified with 2% Compound 22 showed significant reductions in adhesion (t-test with p<0.05) for all 3 bacteria tested compared to unmodified controls and all commercial products. Hydrophilic or lubricious coatings present on certain commercial products did not appear to impact bacterial adhesion in these experiments.

**Table 2. Commercial ureteral stent products used for comparative testing.**

| **Product** | **Manufacturer** | **Material** | **Hydrophilic / Lubricious Coating** | **Inner Diameter (mm)** |
|---|---|---|---|---|
| Percuflex | Boston Scientific | EVA - Firm | -- | 1.50 |
| Percuflex Plus | Boston Scientific | EVA - Firm | Yes | 1.50 |
| Inlay Optima | Bard | PU (Polycarbonate-based) | Yes | 1.35 |
| Universa Soft | Cook | PU | -- | 1.40 |
| Universa Soft - AQ | Cook | PU | Yes | 1.40 |
| Lubri-Flex | Olympus | PU (Tecoflex) - Firm | Yes | 1.30 |
| Double J | Olympus | Silicone | -- | 0.94 |

### Example 21: E. coli adhesion on PU tubing with compound 22 under flow conditions in comparison to a commercial ureteral stent product

*E. coli* adhesion on unmodified control and additive modified 7F polyurethane tubing with 2% of compound 22 under flow was investigated in comparison to a commercial ureteral stent product - the Inlay Optima manufactured by C. R. Bard Inc. The methods described in Example 12 were used, with some modification to the circuit design to accommodate the ureteral stent product. Specifically, because the Bard Inlay Optima has drainage holes spaced ~2 cm apart along the length of the catheter shaft, all test circuits were composed of 7F polyurethane control tubing with four 2 cm segments of the test tubing connected in series downstream of the pump using polypropylene connectors. The same set-up was used for all test groups evaluated. Experiments were conducted with the *E.coli* 67 strain in artificial urine using a flow rate of 0.5 ml/min. At the end of the experiment, the 2 cm sections were removed from the polypropylene connectors and 0.5 cm from each edge was trimmed to remove edge effects. The remaining 1 cm of tubing was processed for bacterial counts as described in Example 12.

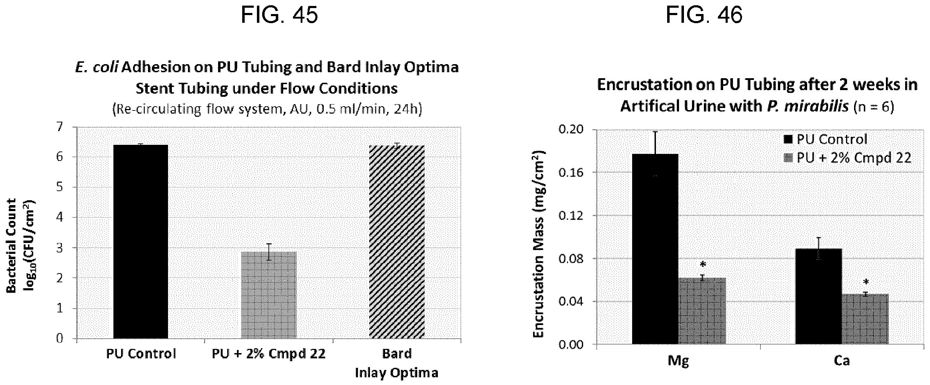

*E. coli* adhesion to the tubing samples after 24h of flow is shown in FIG. 45. Bacterial counts are presented in log format and error bars represent standard error. Tubing modified with 2% Compound 22 showed ~3 log (99.9%) reduction in adhesion (t-test with p<0.05) compared to the unmodified control tubing or the Bard Inlay Optima stent tubing.

### Example 22: Bacteria-mediated encrustation of PU tubing with compound 22 after 2 weeks' incubation in artificial urine

Certain bacterial species can promote encrustation or precipitation of salts from biological fluids on device surfaces. This can affect device function leading to device failure (for example blockage of ureteral stents affecting drainage), as well as having a negative impact on patient comfort and leading to other complications and co-morbidities. *P. mirabilis* is a bacterial species found in human urine that is frequently associated with encrustation on urological devices due to its ability to cleave urea, thereby releasing ammonia which raises the pH of urine. This in turn initiates the precipitation of polyvalent ions present in urine, leading to the formation of struvite (MgNH₃PO₄) or apatite (CaPO₄) crystals on stent or catheter surfaces.

The ability of surfaces modified with compound 22 of the invention to resist bacteria-mediated encrustation was investigated with a 2 week in-vitro model designed by Innovotech, a medical device testing laboratory in Edmonton, Alberta. Unmodified Control and additive-modified 7F polyurethane tubing containing 2% of compound 22 was manufactured as described in Example 12. Tubing was cut into 3.8 cm long samples which were mounted in a "U-shape" on the lid of a 12-well culture plate (Innovotech's BEST^{™} plate) with n = 6 samples per test group. The mounted samples and plates were sterilized by Ethylene Oxide.

Artificial urine (AU) was prepared based on a recipe from Brooks T. et al, "A simple artificial urine for the growth of urinary pathogens." Letters in Applied Microbiology 1997, Vol. 24(3):203-6. The AU was filter-sterilized and stored at 4°C until use. A clinical strain of *P. mirabilis* (Mil# 06-0116-3) stocked by Innovotech was streaked out on trypic soy agar (TSA) from cryogenic stock and cultured for ~20h at 37°C and then similarly sub-cultured. A colony from the sub-culture was grown in TSB at 37 °C for ~20 h with shaking and then used to prepare *P. mirabilis* inoculum in artificial urine at 10⁶ cfu/ml. Four (4) ml of the inoculum solution was added to each well of the culture plate containing the samples, resulting in ~2.8 cm length of tubing submerged in inoculum. The plate was placed on a rotary shaker and incubated at 37°C for 14 days. Every 48h, the inoculum was replaced by transferring the lid to a new plate with fresh inoculum. After 14 days, the samples were rinsed 3x by dipping in 5 ml of sterile water. The samples were then removed from the lid and sonicated in 4% nitric acid for 30 min to dislodge the encrustation deposits. The deposits were allowed to dissolve in nitric acid solution for 24h and then analyzed for Calcium and Magnesium content by atomic adsorption spectroscopy (AGILENT 220 FS Atomic Absorption Spectrophotometer, Chemical and Materials Engineering Department, University of Alberta) using standard methods.

The mass of calcium and magnesium deposited on the tubing test samples, normalized to surface area, is shown in FIG. 46, and correlates to the total mass of encrustation deposits. Error bars represent standard error. Polyurethane tubing modified with 2% compound 22 had 59% less overall Calcium and Magnesium-based deposits on the surface than unmodified polyurethane tubing, suggesting potential of the surface modification to reduce bacteria-mediated encrustation.

### Example 23: Bacterial adhesion on PU rods with compounds 43, 44, 45, and 38 after 2h incubation in PBS

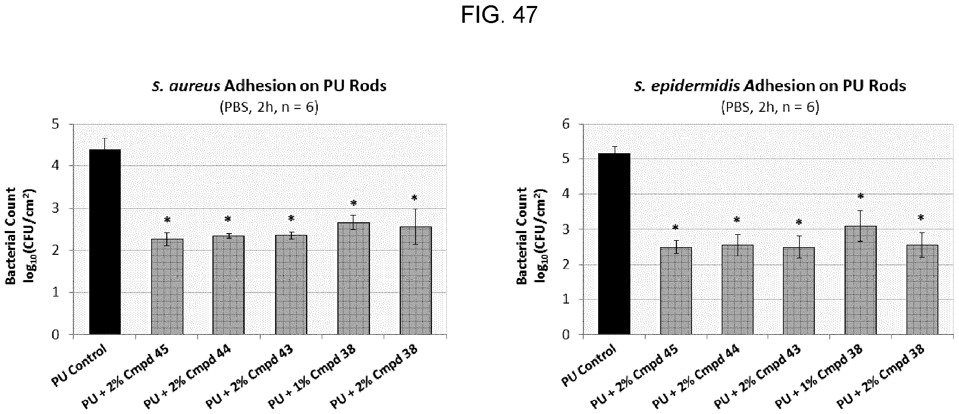

Polyurethane rods with additional compounds to those presented in Example 2 or 4 were prepared using the method described in Example 2. *S. aureus Newman* and *S. epidermidis 35984* adhesion on unmodified control and additive-modified rods was assessed using the methods described in Example 4 and the results are shown in FIG. 47. Bacterial counts are presented in log format and error bars represent standard error. Additive-modified samples having statistically significant differences (t-test with p<0.05) in adhesion relative to unmodified controls are denoted with a (*) symbol. Reductions in *S. aureus* and *S. epidermidis* adhesion on additive modified samples relative to unmodified controls ranged from ~1.5 to 2.5 log (>99%), depending on the additive formulation and bacterial species tested.

### Example 24: E.coli adhesion on PU rods with compounds 43, 44, 45, 38, and 11 after 24h incubation in Human Pooled Urine

Polyurethane rods with additional compounds to those presented in Example 1 or 4 were prepared using the method described in Example 2. Human urine (HU) was collected and pooled from three healthy volunteers. The HU was filter-sterilized, stored at 4 °C, and used within a week of preparation or collection. *E. coli* 67 adhesion testing was conducted using the methods described in Example 4, with rod samples 1.5 cm in length, bacterial inoculum solutions prepared at 10⁶ CFU/mL in HU, and incubation times of 24h.

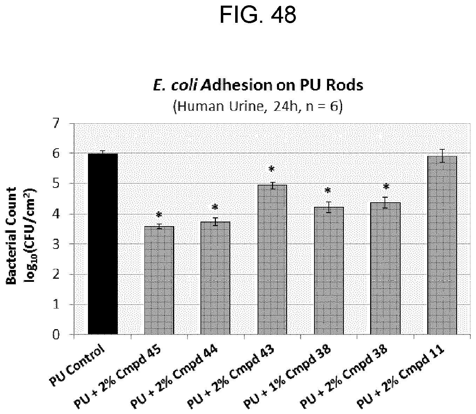

The results are shown in FIG. 48. Bacterial counts are presented in log format and error bars represent standard error. Additive-modified samples having statistically significant differences (t-test with p<0.05) in adhesion relative to unmodified controls are denoted with a (*) symbol. Reductions in *E. coli* adhesion on additive modified samples relative to unmodified controls ranged from 0 to ~2.5 log (> 99%), depending on the additive formulation tested.

### Example 25: Bacterial adhesion on PU rods with compounds 22, 45, and 38 and radiopaque filler

Polyurethane rods with 2% compounds 22, 45 and 38 were prepared using the methods described in Example 2 and Carbothane PC3585A resin with and without 20% BaSO₄ radiopaque filler. Both resins were obtained from a medical polyurethane supplier (LUBRIZOL^{®}). Bacterial adhesion testing was conducted using the methods described in Example 4. *S. aureus Newman* and *S. epidermidis* 35984 adhesion was assessed after 2h incubation in PBS. *E. coli* 67 adhesion was assessed after 2h incubation in human pooled urine (HU). The HU was collected and pooled from three healthy volunteers, filter-sterilized, stored at 4 °C, and used within a week of collection.

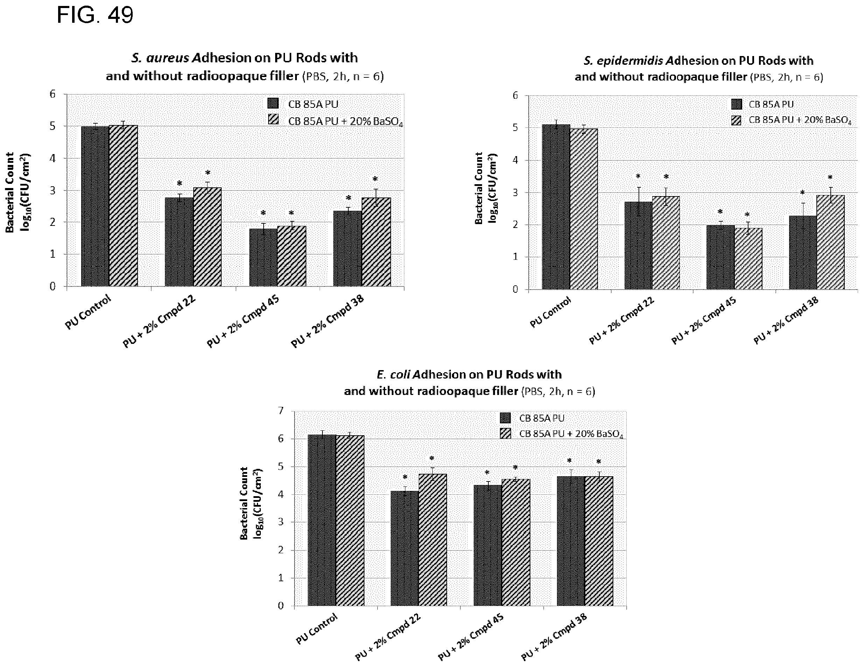

The results are shown in FIG. 49. Bacterial counts are presented in log format and error bars represent standard error. Additive-modified samples having statistically significant differences (t-test with p<0.05) in adhesion relative to the respective unmodified controls are denoted with a (*) symbol. Reductions in bacterial adhesion on additive modified samples relative to the respective controls ranged from ~1.5 to 3 log (99.9%), depending on the additive formulation and bacterial species tested. Reductions in bacterial adhesion were similar between modified samples with and without BaSO₄, suggesting that the presence of inorganic fillers should not affect the efficacy of the surface modification.

### Example 26: Bacterial adhesion on PU rods with compounds 54 and 55 after 2h incubation in PBS or Human Pooled Urine

Polyurethane rods with additional compounds 54 and 55 were prepared using the method described in Example 2. Bacterial adhesion testing was conducted using the methods described in Example 4, but only one experiment was conducted with n = 3 samples for each bacterial species. *S. aureus Newman* and *S. epidermidis* 359841 adhesion was assessed after 2h incubation in PBS. *E. coli* 67 adhesion was assessed after 2h incubation in human pooled urine (HU). The HU was collected and pooled from three healthy volunteers, filter-sterilized, stored at 4 °C, and used within a week of collection.

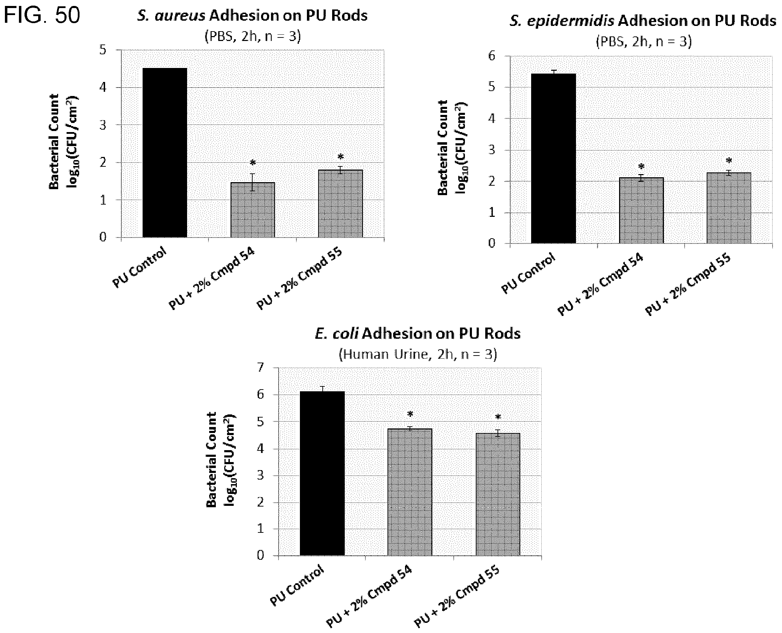

The results are shown in FIG. 50. Bacterial counts are presented in log format and error bars represent standard error. Additive-modified samples having statistically significant differences (t-test with p<0.05) in adhesion relative to the unmodified controls are denoted with a (*) symbol. Reductions in bacterial adhesion on additive modified samples compared to unmodified controls ranged from ~1 to 3 log (90 - 99.9%), depending on the additive formulation and bacterial species tested.

### Example 27: Other polymers

Polyvinyl chloride (PVC) rods containing 0.05-15% (w/w) of one of compounds 1-57 or a compound having the formula of one of SMM 1 - SMM 16 are prepared according to the method of Example 2. The PVC rods are tested for bacterial adhesion according to the method of Example 4. The formulation including any one of compounds 1-57 or a compound having the formula of any one of SMM 1 - SMM 16 exhibits resistance to bacterial adhesion compared to the control.

Similarly, polyethylene rods containing 0.05-15% (w/w) of one of compounds 1-57 or a compound having the formula of one of SMM 1 - SMM 16 are prepared and tested for bacterial adhesion. The formulation including any one of compounds 1-57 or a compound having the formula of any one of SMM 1 - SMM 16 exhibits resistance to bacterial adhesion compared to the control.

In another example, Nylon rods (e.g., Nylon 6, Nylon 6-6, Nylon 11, or Nylon 12) containing 0.05-15% (w/w) of one of compounds 1-57 or a compound having the formula of one of SMM 1 - SMM 16 are prepared and tested for bacterial adhesion. The formulation including any one of compounds 1-57 or a compound having the formula of any one of SMM 1 - SMM 16 exhibits resistance to bacterial adhesion compared to the control.

Ethylene-vinyl acetate (EVA) rods containing 0.05-15% (w/w) of one of compounds 1-57 or a compound having the formula of one of SMM 1 - SMM 16 are similarly prepared and tested for bacterial adhesion. The formulation including any one of compounds 1-57 or a compound having the formula of any one of SMM 1 - SMM 16 exhibits resistance to bacterial adhesion compared to the control.

In a further example, polypropylene rods containing 0.05-15% (w/w) of one of compounds 1-57 or a compound having the formula of one of SMM 1 - SMM 16 are prepared and tested for bacterial adhesion. The formulation including any one of compounds 1-57 or a compound having the formula of any one of SMM 1 - SMM 16 exhibits resistance to bacterial adhesion compared to the control.

Lastly, poly(styrene-*block*-isobutylene-*block*-styrene) (SIBS) rods containing 0.05-15% (w/w) of one of compounds 1-57 or a compound having the formula of one of SMM 1 - SMM 16 are prepared and tested for bacterial adhesion. The formulation including any one of compounds 1-57 or a compound having the formula of any one of SMM 1 - SMM 16 exhibits resistance to bacterial adhesion compared to the control.

### Other Embodiments

Various modifications and variations of the described invention will be apparent to those skilled in the art without departing from the scope of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention as embodied by the claims.

## Claims

1. Use of a composition comprising a base polymer admixed with 0.05% (w/w) to 15% (w/w) of a compound of any one of formulas (I)-(XXI)
Formula (I):
F_{T}-[B-A]ₙ-B-F_{T} (I)
where
(i) A includes hydrogenated polybutadiene, poly((2,2-dimethyl)-1,3-propylene carbonate), polybutadiene, poly(diethylene glycol)adipate, poly(hexamethylene carpbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
(ii) B is a segment including a urethane; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, and
(iv) n is an integer from 1 to 10;
Formula (II):
F_{T}-[B-A]ₙ-B-F_{T} (II)
where
(i) B includes a urethane;
(ii) A includes polypropylene oxide, polyethylene oxide, or polytetramethylene oxide;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 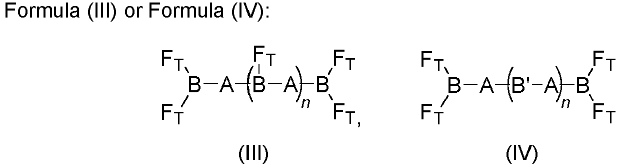
where
(i) A is an oligomeric segment containing an ether linkage, an ester linkage, a carbonate linkage, or a polyalkylene and having a theoretical molecular weight of from 500 to 3,500 g/mol;
(ii) B is a segment including an isocyanurate trimer or biuret trimer; B', when present, is a segment including an urethane;
(iii) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer between 0 to 10;
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 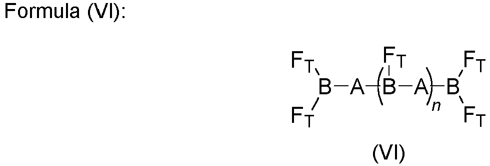
where
(i) A is an oligomeric segment including polyethylene oxide, polypropylene oxide, polytetramethylene oxide, or a mixture thereof, and having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (VII):
F_{T}-[B-A]ₙ-B-F_{T} (VII)
where
(i) A is a polycarbonate polyol having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 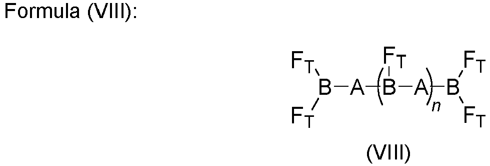
where
(i) A is an oligomeric segment including a polycarbonate polyol having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 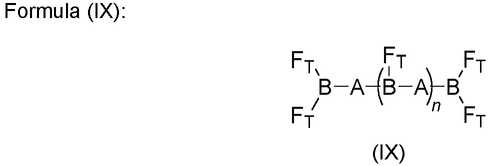
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (X):
F_{T}-[B-A]ₙ-B-F_{T} (X)
where
(i) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 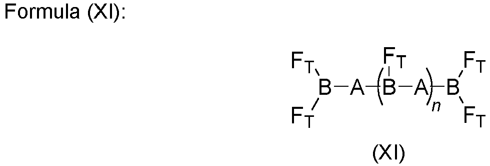
where
(i) A is hydrogenated polybutadiene, polybutadiene, hydrogenated polyisoprene, or polystyrene and has a theoretical molecular weight of from 750 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 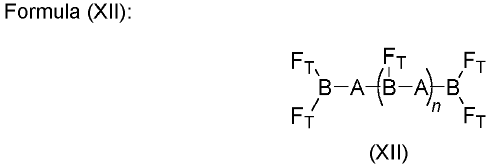
where
(i) A is a polyester having a theoretical molecular weight of from 500 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (XIII):
F_{T}-A-F_{T} (XIII)
where F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms and A is a branched or non-branched oligomeric segment of fewer than 20 repeating units and is selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments; 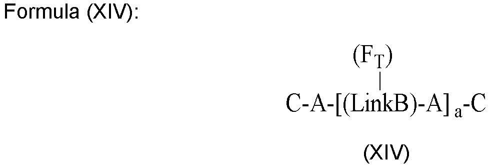 where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms covalently attached to LinkB;
(ii) C is a chain terminating group containing an amine, alcohol, or carboxylic acid functionality ;
(iii) A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide;
(iv) LinkB is a coupling segment having a molecular weight ranging from 40 to 700 selected from the group of functionalized diamines diisocyanates, disulfonic acids, dicarboxylic acids, diacid chlorides, and dialdehydes, where the functionalized component has secondary functional group; and
(v) a is an integer greater than 0; 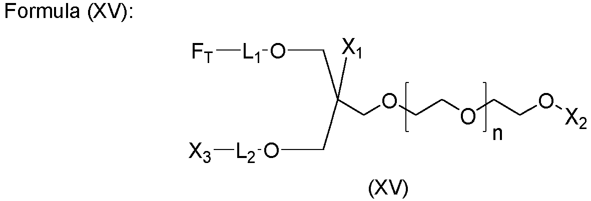
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; 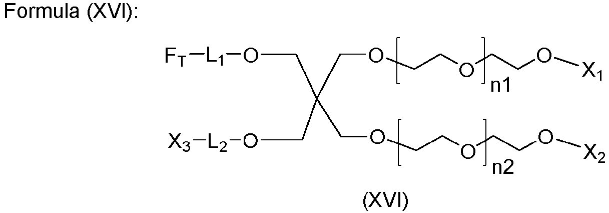
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50;
Formula (XVII):
G-Aₘ-[B-A]ₙ-B-G (XVII)
where
(i) each A comprises hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, provided that at least one G is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iv) n is an integer from 1 to 10; and
(v) m is 0 or 1;
Formula (XVIII):
F_{T}-OC(O)O-B-OC(O)O-[A-OC(O)O-B]ₙ-OC(O)O-F_{T} (XVIII)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide and is covalently bound to
B via a carbonate linkage;
(ii) B comprises a polyalkylene oxide or a moiety described by the formula: 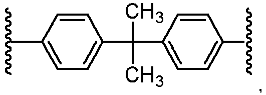 and is covalently bound to A via a carbonate linkage; and
(iii) F_{T} is a surface active group comprising a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, wherein F_{T} is covalently bound to B via a carbonate linkage; and
(iv) n is an integer from 1 to 10;
Formula (XIX):
F_{T}-OC(O)O-(CH₂CH₂O)ₘ-OC(O)O-[A-OC(O)O-(CH₂CH₂O)ₘ]ₙ-OC(O)O-F_{T} (XIX)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide;
(ii) F_{T} is a surface active group comprising a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iii) m is an integer from 2 to 4; and
(iv) n is an integer from 1 to 10; 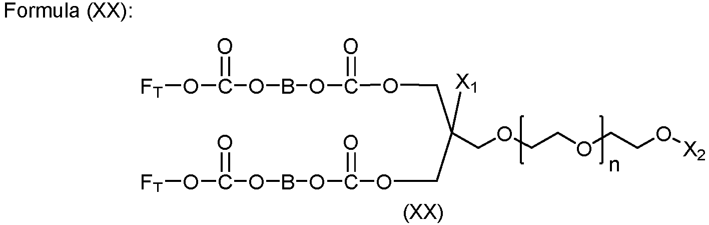
where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, is H, CH₃, or CH₂CH₃;
(iii) B comprises a polyalkylene oxide; and
(iv) n is an integer from 5 to 100; 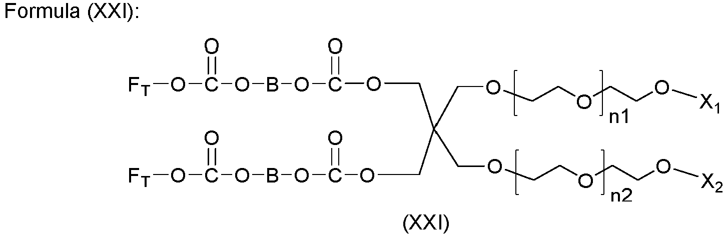
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, H, CH₃, or CH₂CH₃;
(iii) B comprises a polyalkylene oxide; and
(iv) each of n1 and n2 is independently an integer from 5 to 50;
for reducing bacterial adhesion to a polymeric surface formed from a base polymer, wherein bacterial adhesion is reduced by at least 50% relative to the polymeric surface formed from the base polymer in the absence of the compound, and wherein bacterial adhesion is measured by exposing the test surfaces to bacterial inoculum in aqueous media for a specified amount of time, afterwards rinsing and sonicating the surfaces in buffer solution to detach adhered bacteria, and then quantifying the removed bacteria by plating the solutions on bacterial growth substrate, incubating overnight at 37°C, and performing colony counts as disclosed in example 4.

2. Use of a composition comprising a base polymer admixed with 0.05% (w/w) to 15% (w/w) of a compound of any one of formulas (I)-(XXI)
Formula (I):
F_{T}-[B-A]ₙ-B-F_{T} (I)
where
(i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
(ii) B is a segment including a urethane; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, and
(iv) n is an integer from 1 to 10;
Formula (II):
F_{T}-[B-A]ₙ-B-F_{T} (II)
where
(i) B includes an urethane;
(ii) A includes polypropylene oxide, polyethylene oxide, or polytetramethylene oxide;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 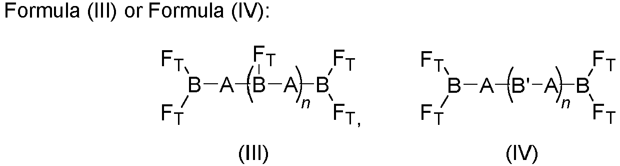
where
(i) A is an oligomeric segment containing an ether linkage, an ester linkage, a carbonate linkage, or a polyalkylene and having a theoretical molecular weight of from 500 to 3,500 g/mol;
(ii) B is a segment including an isocyanurate trimer or biuret trimer; B', when present, is a segment including an urethane;
(iii) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer between 0 to 10;
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 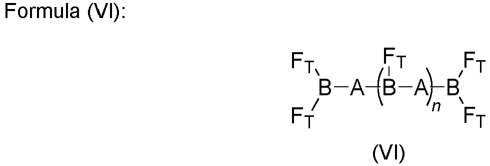
where
(i) A is an oligomeric segment including polyethylene oxide, polypropylene oxide, polytetramethylene oxide, or a mixture thereof, and having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (VII):
F_{T}-[B-A]ₙ-B-F_{T} (VII)
where
(i) A is a polycarbonate polyol having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 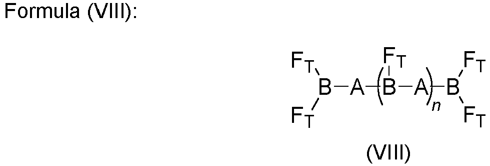
where
(i) A is an oligomeric segment including a polycarbonate polyol having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 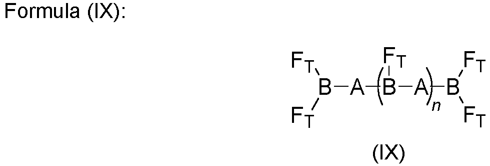
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (X):
F_{T}-[B-A]ₙ-B-F_{T} (X)
where
(i) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 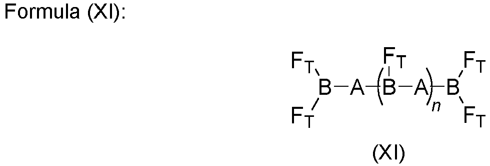
where
(i) A is hydrogenated polybutadiene, polybutadiene, hydrogenated polyisoprene, or polystyrene and has a theoretical molecular weight of from 750 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 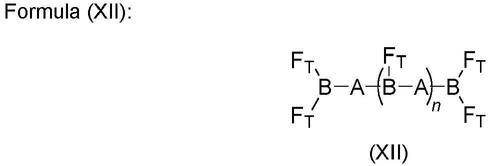
where
(i) A is a polyester having a theoretical molecular weight of from 500 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (XIII):
F_{T}-A-F_{T} (XIII)
where F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms and A is a branched or non-branched oligomeric segment of fewer than 20 repeating units and selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments; 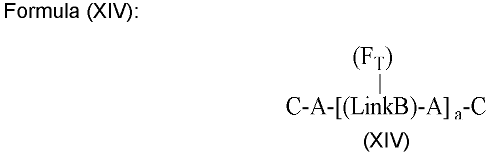 where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms covalently attached to LinkB;
(ii) C is a chain terminating group containing an amine, alcohol, or carboxylic acid functionality;
(vi) A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide;
(vii) LinkB is a coupling segment having a molecular weight ranging from 40 to 700 selected from the group of functionalized diamines diisocyanates, disulfonic acids, dicarboxylic acids, diacid chlorides, and dialdehydes, where the functionalized component has secondary functional group; and
(viii) a is an integer greater than 0; 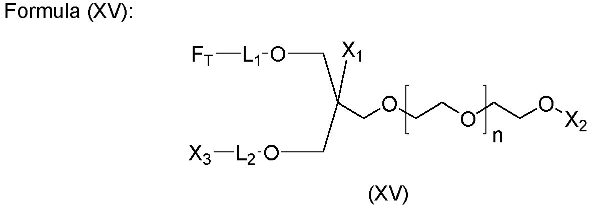
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; 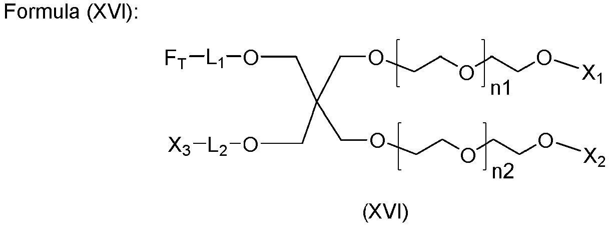
where
(i) each F_{T} isa polyfluoroorganic group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50;
Formula (XVII):
G-Aₘ-[B-A]ₙ-B-G (XVII)
where
(i) each A comprises hydrogenated polybutadiene, poly ((2,2-dimethyl)-1 ,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, provided that at least one G is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iv) n is an integer from 1 to 10; and
(v) m is 0 or 1;
Formula (XVIII):
F_{T}-OC(O)O-B-OC(O)O-[A-OC(O)O-B]ₙ-OC(O)O-F_{T} (XVIII)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide and is covalently bound to B via a carbonate linkage;
(ii) B comprises a polyalkylene oxide or a moiety described by the formula: 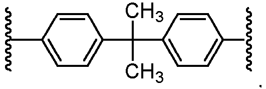 and is covalently bound to A via a carbonate linkage; and
(iii) F_{T} isa polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, wherein F_{T} is covalently bound to B via a carbonate linkage; and
(iv) n is an integer from 1 to 10;
Formula (XIX):
F_{T}-OC(O)O-(CH₂CH₂O)ₘ-OC(O)O-[A-OC(O)O-(CH₂CH₂O)ₘ]ₙ-OC(O)O-F_{T} (XIX)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide;
(ii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iii) m is an integer from 2 to 4; and
(iv) n is an integer from 1 to 10; 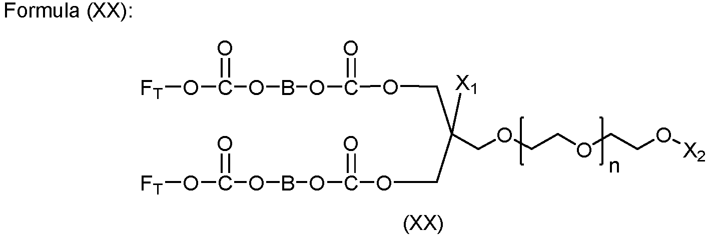
where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, is H, CH₃, or CH₂CH₃;
(iii) B comprises a polyalkylene oxide; and
(iv) n is an integer from 5 to 100; 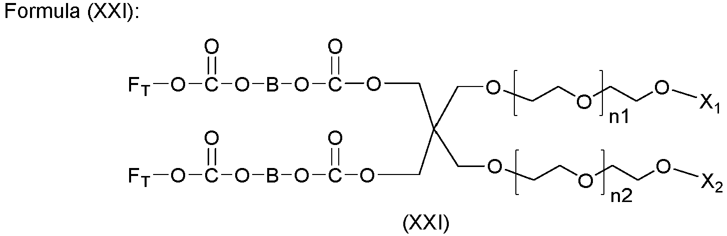
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, H, CH₃, or CH₂CH₃;
(iii) B comprises a polyalkylene oxide; and
(iv) each of n1 and n2 is independently an integer from 5 to 50;
for reducing bacteria mediated salt formation on a polymeric surface of a urine dwelling device by providing polymeric surface formed from the composition and (ii) contacting said polymeric surface with urine, wherein salt deposition is reduced by at least 20% under urine dwelling conditions relative to the polymeric surface formed from the base polymer in the absence of the compound, wherein salt deposition is measured by incubating test surfaces in artificial urine inoculated with *P. mirabilis* bacteria for 14 days, followed by dissolution of the resulting salt deposits in dilute nitric acid and quantification of calcium and magnesium content using atomic absorption spectroscopy or inductively-coupled plasma mass spectrometry as disclosed in example 22.

3. Use of a composition comprising a base polymer admixed with 0.05% (w/w) to 15% (w/w) of a compound of any one of formulas (I)-(XXI)
Formula (I):
F_{T}-[B-A]ₙ-B-F_{T} (I)
where
(i) A includes hydrogenated polybutadiene, poly((2,2-dimethyl)-1 ,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1 ,6-hexanediol-ortho phthalic anhydride) polyester, or bisphenol A ethoxylate;
(ii) B is a segment including a urethane; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, and
(iv) n is an integer from 1 to 10;
Formula (II):
F_{T}-[B-A]ₙ-B-F_{T} (II)
where
(i) B includes an urethane;
(ii) A includes polypropylene oxide, polyethylene oxide, or polytetramethylene oxide;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 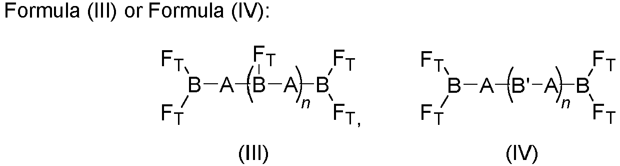
where
(i) A is an oligomeric segment containing an ether linkage, an ester linkage, a carbonate linkage, or a polyalkylene and having a theoretical molecular weight of from 500 to 3,500 g/mol;
(ii) B is a segment including an isocyanurate trimer or biuret trimer; B', when present, is a segment including an urethane;
(iii) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer between 0 to 10;
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 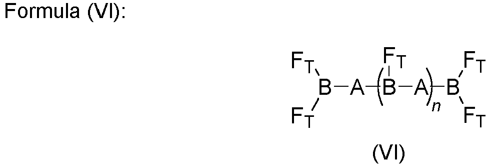
where
(i) A is an oligomeric segment including polyethylene oxide, polypropylene oxide, polytetramethylene oxide, or a mixture thereof, and having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (VII):
F_{T}-[B-A]ₙ-B-F_{T} (VII)
where
(i) A is a polycarbonate polyol having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 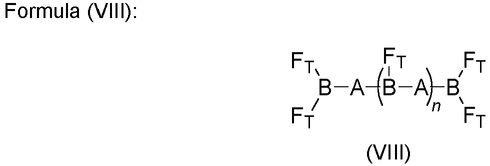
where
(i) A is an oligomeric segment including a polycarbonate polyol having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 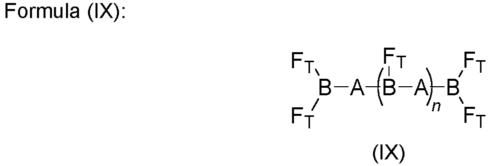
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (X):
F_{T}-[B-A]ₙ-B-F_{T} (X)
where
(i) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 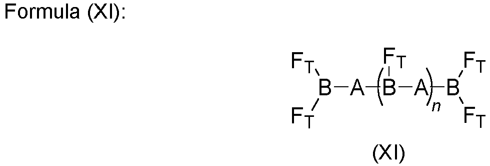
where
(i) A is hydrogenated polybutadiene, polybutadiene, hydrogenated polyisoprene, or polystyrene and has a theoretical molecular weight of from 750 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 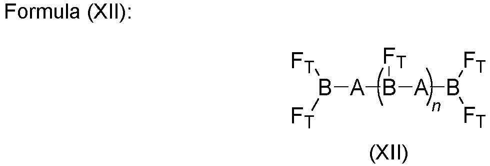
where
(i) A is a polyester having a theoretical molecular weight of from 500 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (XIII):
F_{T}-A-F_{T} (XIII)
where F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms and A is a branched or non-branched oligomeric segment of fewer than 20 repeating units and selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments; 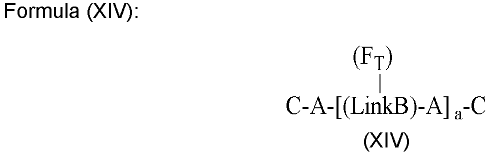 where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms covalently attached to LinkB;
(ii) C is a chain terminating group containing an amine, alcohol, or carboxylic acid functionality;
(iii) A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide;
(iv) LinkB is a coupling segment having a molecular weight ranging from 40 to 700 selected from the group of functionalized diamines diisocyanates, disulfonic acids, dicarboxylic acids, diacid chlorides, and dialdehydes, where the functionalized component has secondary functional group; and
(v) a is an integer greater than 0; 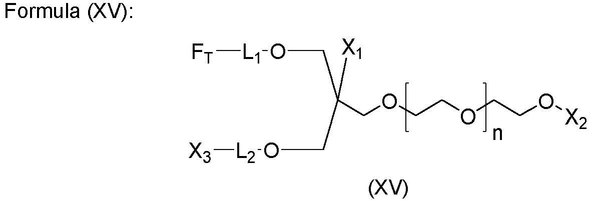
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; 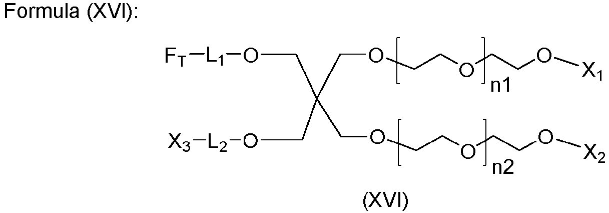
where
(i) each F_{T} is a polyfluoroorganic group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50;
Formula (XVII):
G - Aₘ - [B - A]ₙ - B - G (XVII)
where
(i) each A comprises hydrogenated polybutadiene, poly ((2,2-dimethyl)-1 ,3-propylene carbonate), polybutadiene, poly (diethylene glycol)adipate, poly (hexamethylene carbonate), poly (ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, provided that at least one G is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iv) n is an integer from 1 to 10; and
(v) m is 0 or 1;
Formula (XVIII):
F_{T}-OC(O)O-B-OC(O)O-[A-OC(O)O-B]ₙ-OC(O)O-F_{T} (XVIII)
where
A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide and is covalently bound to B via a carbonate linkage;
(ii) B comprises a polyalkylene oxide or a moiety described by the formula: 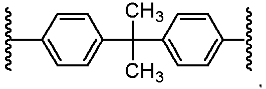 and is covalently bound to A via a carbonate linkage; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, wherein F_{T} is covalently bound to B via a carbonate linkage; and
(iv) n is an integer from 1 to 10;
Formula (XIX):
F_{T}-OC(O)O-(CH₂CH₂O)ₘ-OC(O)O-[A-OC(O)O-(CH₂CH₂O)ₘ]ₙ-OC(O)O-F_{T} (XIX)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide;
(ii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iii) m is an integer from 2 to 4; and
(iv) n is an integer from 1 to 10; 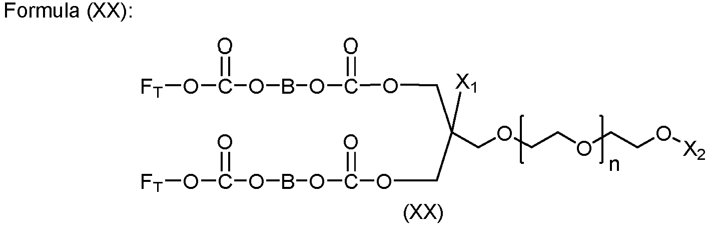
where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, is H, CH₃, or CH₂CH₃;
(iii) B comprises a polyalkylene oxide; and
(iv) n is an integer from 5 to 100; 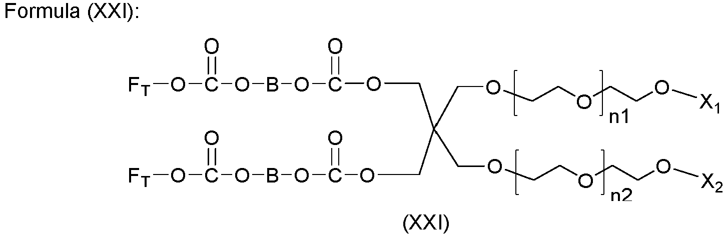
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, H, CH₃, or CH₂CH₃;
(iii) B comprises a polyalkylene oxide; and
(iv) each of n1 and n2 is independently an integer from 5 to 50;
for reducing bacterial biofilm formation on a polymeric surface by providing the polymeric surface formed from the composition, wherein bacterial biofilm formation is reduced by at least 20% relative to the polymeric surface formed from the base polymer in the absence of the compound, and wherein biofilm formation is measured by exposing the test surfaces to bacterial inoculum in aqueous media under conditions that allow biofilm formation, afterwards rinsing the surfaces, and then measuring biofilm surface coverage using optical microscopy or scanning electron microscopy, or staining the biofilm and measuring UV absorbance of extracted stain as disclosed in example 18.

4. Use of a composition comprising a base polymer admixed with 0.05% (w/w) to 15% (w/w) of a compound of any one of formulas (I)-(XXI)
Formula (I):
F_{T}-[B-A]ₙ-B-F_{T} (I)
where
(i) A includes hydrogenated polybutadiene, poly((2,2-dimethyl)-1 ,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1 ,6-hexanediol-ortho phthalic anhydride) polyester, or bisphenol A ethoxylate;
(ii) B is a segment including a urethane; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, and
(iv) n is an integer from 1 to 10;
Formula (II):
F_{T}-[B-A]ₙ-B-F_{T} (II)
where
(i) B includes an urethane;
(ii) A includes polypropylene oxide, polyethylene oxide, or polytetramethylene oxide;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 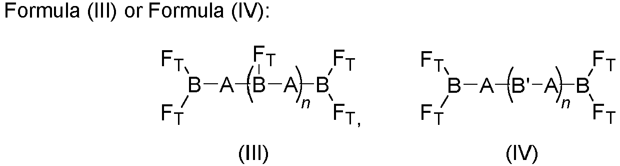
where
(i) A is an oligomeric segment containing an ether linkage, an ester linkage, a carbonate linkage, or a polyalkylene and having a theoretical molecular weight of from 500 to 3,500 g/mol;
(ii) B is a segment including an isocyanurate trimer or biuret trimer; B', when present, is a segment including an urethane;
(iii) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer between 0 to 10;
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 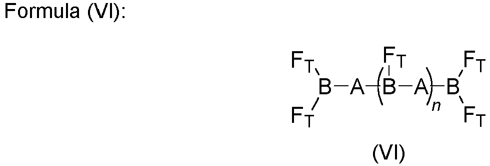
where
(i) A is an oligomeric segment including polyethylene oxide, polypropylene oxide, polytetramethylene oxide, or a mixture thereof, and having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (VII):
F_{T}-[B-A]ₙ-B-F_{T} (VII)
where
(i) A is a polycarbonate polyol having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 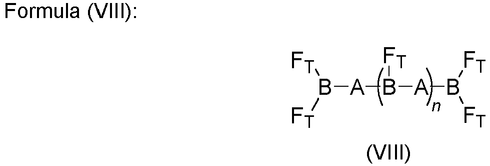
where
(i) A is an oligomeric segment including a polycarbonate polyol having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 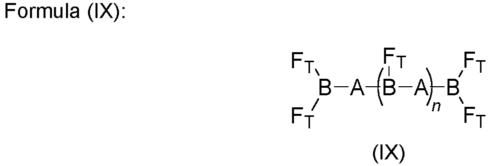
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (X):
F_{T}-[B-A]ₙ-B-F_{T} (X)
where
(i) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 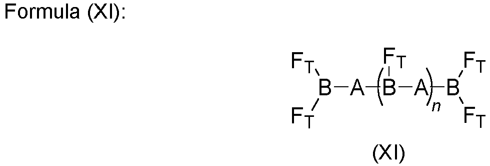
where
(i) A is hydrogenated polybutadiene, polybutadiene, hydrogenated polyisoprene, or polystyrene and has a theoretical molecular weight of from 750 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 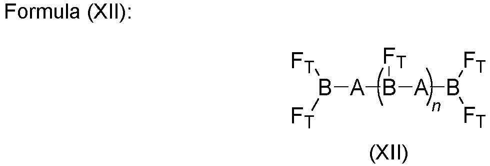
where
(i) A is a polyester having a theoretical molecular weight of from 500 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (XIII):
F_{T}-A-F_{T} (XIII)
where F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms and A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments; 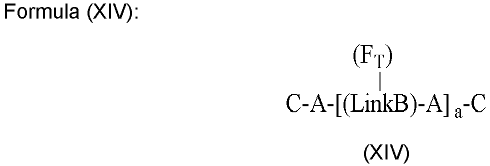 where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms covalently attached to LinkB;
(ii) C is a chain terminating group containing an amine, alcohol, or carboxylic acid functionality;
(ix) A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide;
(x) LinkB is a coupling segment having a molecular weight ranging from 40 to 700 selected from the group of functionalized diamines diisocyanates, disulfonic acids, dicarboxylic acids, diacid chlorides, and dialdehydes, where the functionalized component has secondary functional group; and
(xi) a is an integer greater than 0; 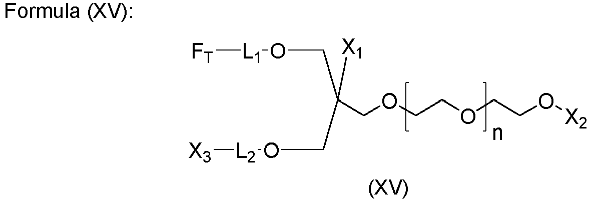 where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; 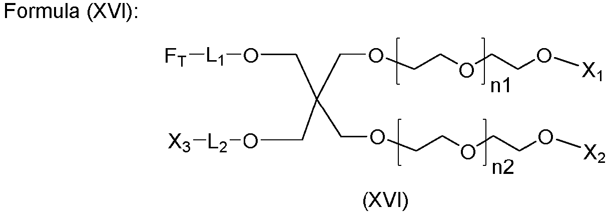
where
(i) each F_{T} is a polyfluoroorganic group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50;
Formula (XVII):
G - Aₘ - [B - A]ₙ - B - G (XVII)
where
(i) each A comprises hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, provided that at least one G is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iv) n is an integer from 1 to 10; and
(v) m is 0 or 1;
Formula (XVIII):
F_{T}-OC(O)O-B-OC(O)O-[A-OC(O)O-B]ₙ-OC(O)O-F_{T} (XVIII)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide and is covalently bound to B via a carbonate linkage;
(ii) B comprises a polyalkylene oxide or a moiety described by the formula: 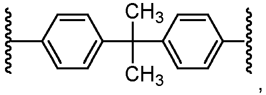 and is covalently bound to A via a carbonate linkage; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, wherein F_{T} is covalently bound to B via a carbonate linkage; and
(iv) n is an integer from 1 to 10;
Formula (XIX):
F_{T}-OC(O)O-(CH₂CH₂O)ₘ-OC(O)O-[A-OC(O)O-(CH₂CH₂O)ₘ]ₙ-OC(O)O-F_{T} (XIX)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide;
(ii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iii) m is an integer from 2 to 4; and
(iv) n is an integer from 1 to 10; 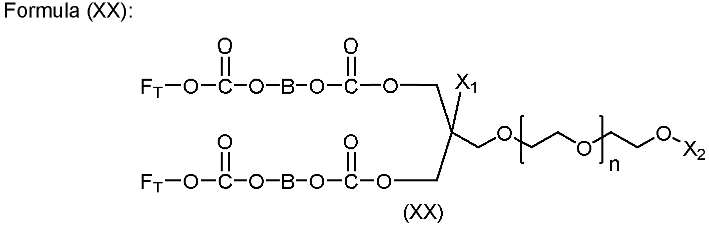
where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, is H, CH₃, or CH₂CH₃;
(iii) B comprises a polyalkylene oxide; and
(iv) n is an integer from 5 to 100; 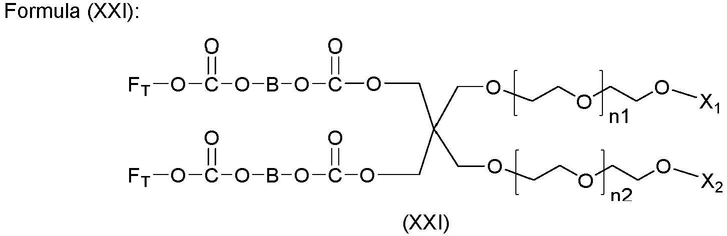
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, H, CH₃, or CH₂CH₃;
(iii) B comprises a polyalkylene oxide; and
(iv) each of n1 and n2 is independently an integer from 5 to 50;
for reducing the bacterial bioburden on a polymeric surface forming the polymeric surface from the composition, wherein bioburden is reduced by at least 20% relative to the polymeric surface formed from the base polymer in the absence of the compound, and wherein bacterial bioburden is measured by exposing the test surfaces to bacterial inoculum in aqueous media for a specified amount of time, afterwards rinsing and sonicating the surfaces in buffer solution to detach adhered bacteria, and then quantifying the removed bacteria by plating the solutions on bacterial growth substrate, incubating overnight at 37°C, and performing colony counts as disclosed in example 4.

5. Use of a composition comprising a base polymer admixed with 0.05% (w/w) to 15% (w/w) of a compound of any one of formulas (I)-(XXI)
Formula (I):
F_{T}-[B-A]ₙ-B-F_{T} (I)
where
(i) A includes hydrogenated polybutadiene, poly((2,2-dimethyl)-1 ,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1 ,6-hexanediol-ortho phthalic anhydride) polyester, or bisphenol A ethoxylate;
(ii) B is a segment including a urethane; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, and
(iv) n is an integer from 1 to 10;
Formula (II):
F_{T}-[B-A]ₙ-B-F_{T} (II)
where
(i) B includes an urethane;
(ii) A includes polypropylene oxide, polyethylene oxide, or polytetramethylene oxide;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 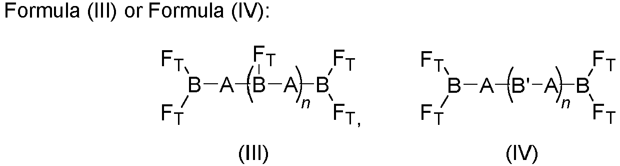
where
(i) A is an oligomeric segment containing an ether linkage, an ester linkage, a carbonate linkage, or a polyalkylene and having a theoretical molecular weight of from 500 to 3,500 g/mol;
(ii) B is a segment including an isocyanurate trimer or biuret trimer; B', when present, is a segment including an urethane;
(iii) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer between 0 to 10;
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 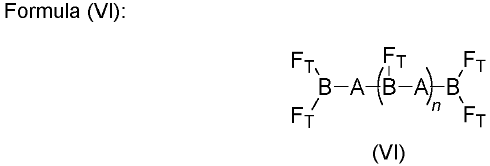
where
(i) A is an oligomeric segment including polyethylene oxide, polypropylene oxide, polytetramethylene oxide, or a mixture thereof, and having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (VII):
F_{T}-[B-A]ₙ-B-F_{T} (VII)
where
(i) A is a polycarbonate polyol having a theoretical molecular weight of from 500 to 3,000 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 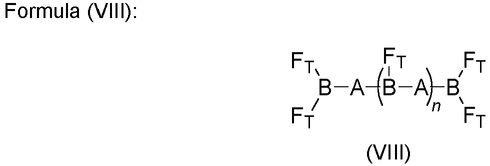
where
(i) A is an oligomeric segment including a polycarbonate polyol having a theoretical molecular weight of from 500 to 3,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 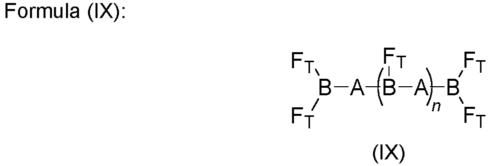
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (X):
F_{T}-[B-A]ₙ-B-F_{T} (X)
where
(i) A is a segment selected from the group consisting of hydrogenated, polybutadiene, hydrogenated polyisoprene, polysiloxane-polyethylene glycol block copolymer, and polystyrene and has a theoretical molecular weight of from 750 to 3,500 g/mol;
(ii) B is a segment formed from a diisocyanate;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 1 to 10; 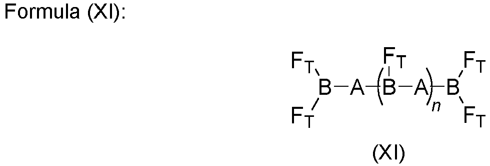
where
(i) A is hydrogenated polybutadiene, polybutadiene, hydrogenated polyisoprene, or polystyrene and has a theoretical molecular weight of from 750 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10; 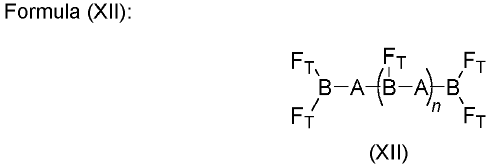
where
(i) A is a polyester having a theoretical molecular weight of from 500 to 3,500 g/mol;
(ii) B is a segment including an isocyanurate trimer or biuret trimer;
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms; and
(iv) n is an integer from 0 to 10;
Formula (XIII):
F_{T}-A-F_{T} (XIII)
where F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms and A is a branched or non-branched oligomeric segment of fewer than 20 repeating units and selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments; 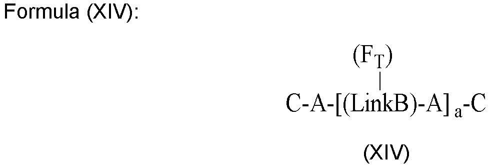 where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms covalently attached to LinkB;
(ii) C is a chain terminating group containing an amine, alcohol, or carboxylic acid functionality;
(iii) A is a branched or non-branched oligomeric segment of fewer than 20 repeating units selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide;
(iv) LinkB is a coupling segment having a molecular weight ranging from 40 to 700 selected from the group of functionalized diamines diisocyanates, disulfonic acids, dicarboxylic acids, diacid chlorides, and dialdehydes, where the functionalized component has secondary functional group; and
(v) a is an integer greater than 0; 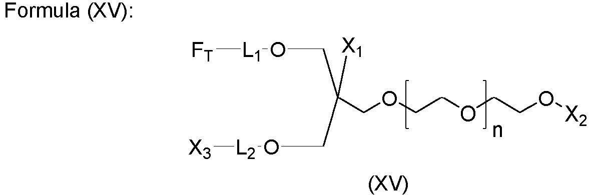
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; 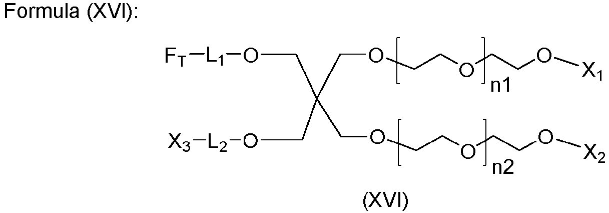
where
(i) each F_{T} is a polyfluoroorganic group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50;
Formula (XVII):
G - Aₘ - [B - A]ₙ - B - G (XVII)
where
(i) each A comprises hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, provided that at least one G is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iv) n is an integer from 1 to 10; and
(v) m is 0 or 1;
Formula (XVIII):
F_{T}-OC(O)O-B-OC(O)O-[A-OC(O)O-B]ₙ-OC(O)O-F_{T} (XVIII)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide and is covalently bound to B via a carbonate linkage;
(ii) B comprises a polyalkylene oxide or a moiety described by the formula: 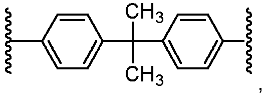 and is covalently bound to A via a carbonate linkage; and
(iii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms, wherein F_{T} is covalently bound to B via a carbonate linkage; and
(iv) n is an integer from 1 to 10;
Formula (XIX):
F_{T}-OC(O)O-(CH₂CH₂O)ₘ-OC(O)O-[A-OC(O)O-(CH₂CH₂O)ₘ]ₙ-OC(O)O-F_{T} (XIX)
where
(i) A comprises a soft segment selected from hydrogenated polybutadiene, hydrogenated polyisoprene, poly((2,2-dimethyl)-1 ,3-propylene carbonate), polybutadiene, poly(diethylene glycol)adipate, poly(hexamethylene carbonate), poly(ethylene-co-butylene), (diethylene glycol-ortho phthalic anhydride) polyester, (1 ,6-hexanediol-ortho phthalic anhydride) polyester, (neopentyl glycol-ortho phthalic anhydride) polyester, a polysiloxane, bisphenol A ethoxylate, polyethylene oxide)-b-poly(propylene oxide)-b-poly(ethylene oxide), polyethylene oxide, polypropylene oxide, or polytetramethylene oxide;
(ii) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(iii) m is an integer from 2 to 4; and
(iv) n is an integer from 1 to 10; 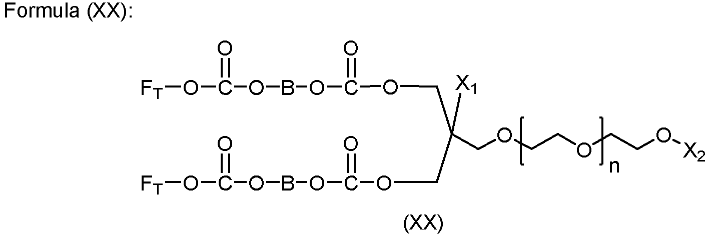
where
(i) F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, is H, CH₃, or CH₂CH₃;
(iii) B comprises a polyalkylene oxide; and
(iv) n is an integer from 5 to 100; 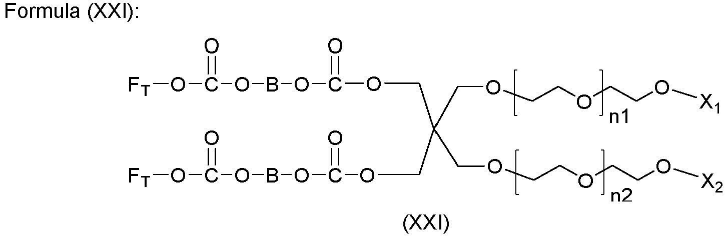
where
(i) each F_{T} is a polyfluoroorgano group, which is a hydrocarbon group that may be optionally interrupted by one, two, or three non-contiguous oxygen atoms, in which from two to fifty-nine hydrogen atoms are replaced with fluorine atoms and contains one to thirty carbon atoms;
(ii) each of X₁ and X₂ is, independently, H, CH₃, or CH₂CH₃;
(iii) B comprises a polyalkylene oxide; and
(iv) each of n1 and n2 is independently an integer from 5 to 50;
for reducing bacterial growth rate on a polymeric surface by providing a polymeric surface comprising the composition, and (ii) contacting the polymeric surface with an antimicrobial, antiseptic, or disinfectant, wherein the bacterial growth rate is reduced by at least 50% relative to the polymeric surface formed from the base polymer in the absence of the compound, wherein bacterial growth rate is measured by sampling the surface at two different points in time, rinsing and sonicating the surface samples in buffer solution to detach adhered bacteria, then quantifying the removed bacteria by plating the solutions on bacterial growth substrate, incubating overnight at 37°C, and performing colony counts, and then calculating the change in bacterial count over time as disclosed in example 14.

6. The use of any one of claims 1-5, wherein the compound is any one of compounds 1-57

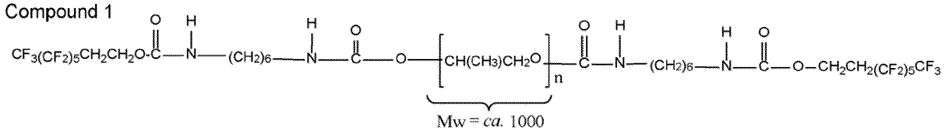

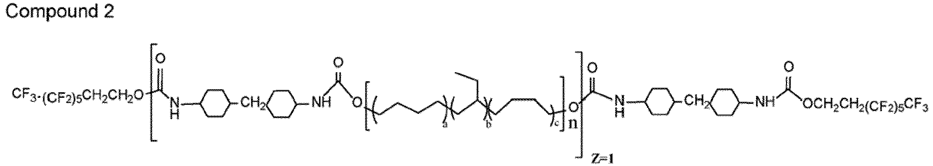

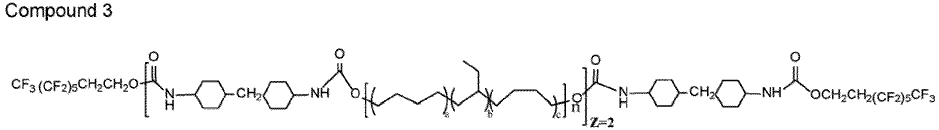

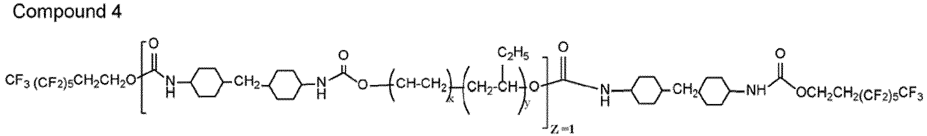

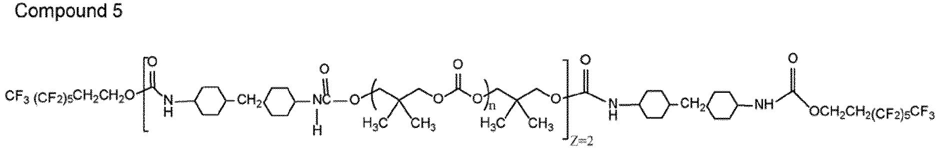

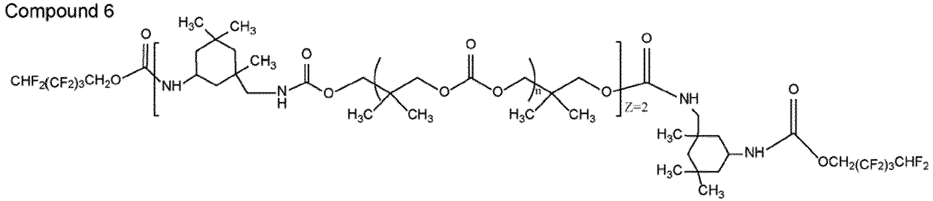

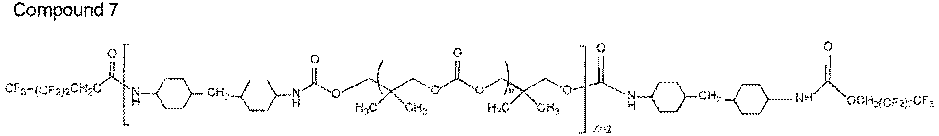

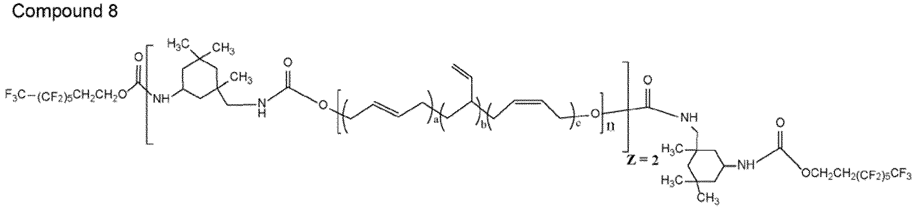

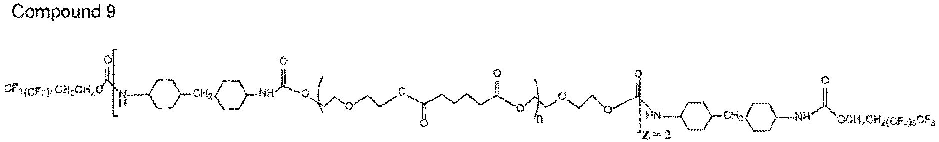

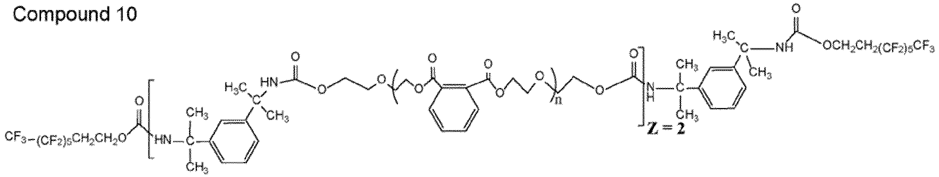

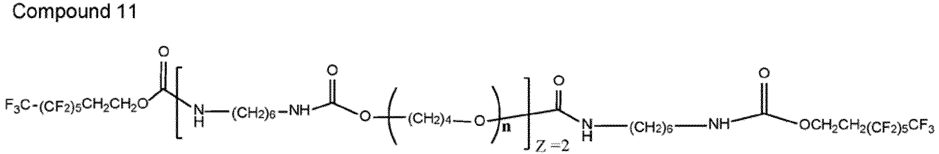

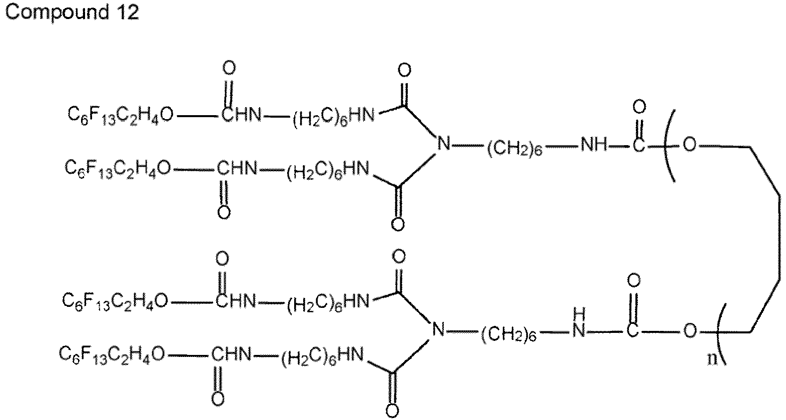

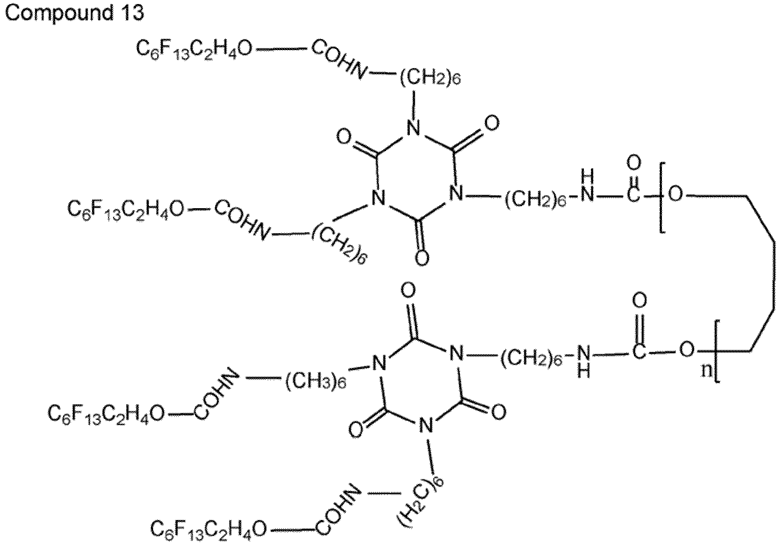

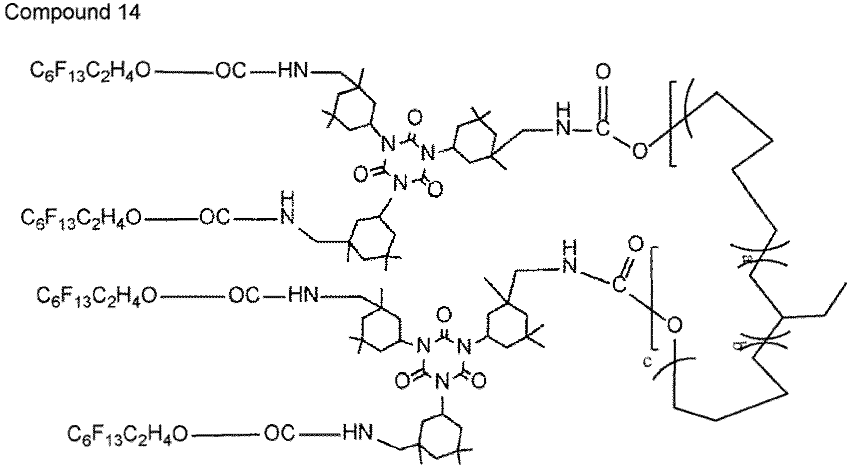

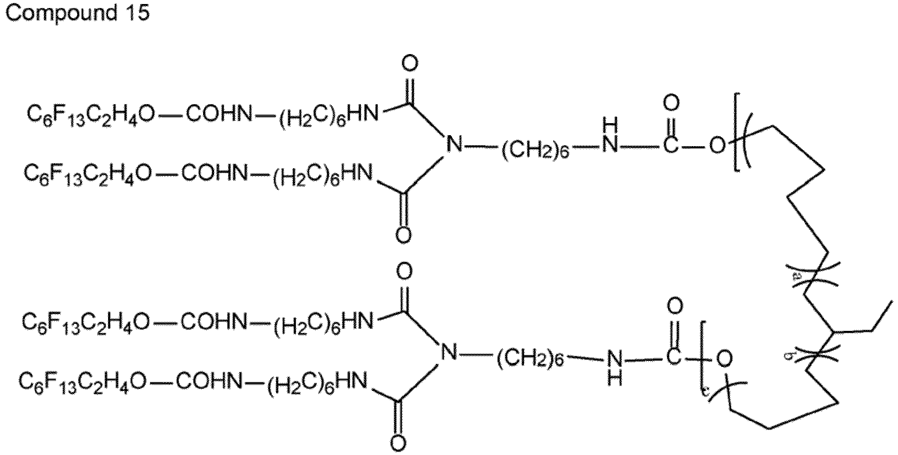

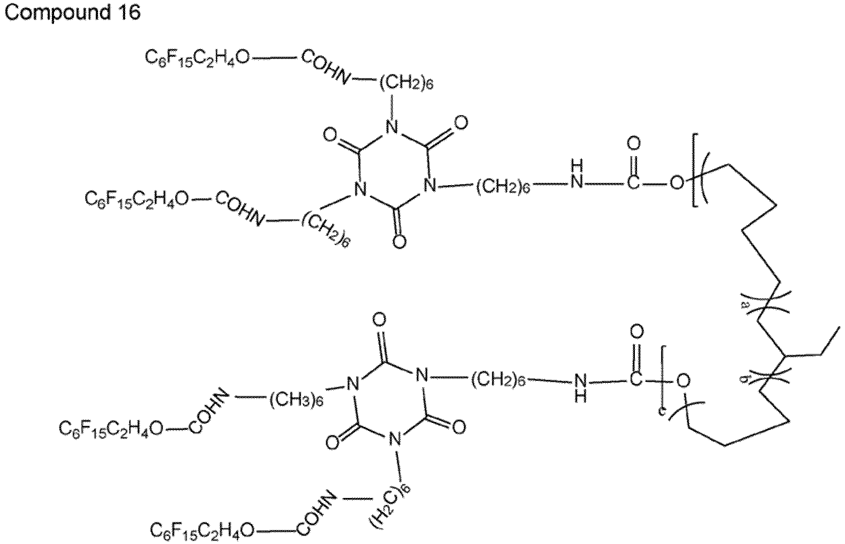

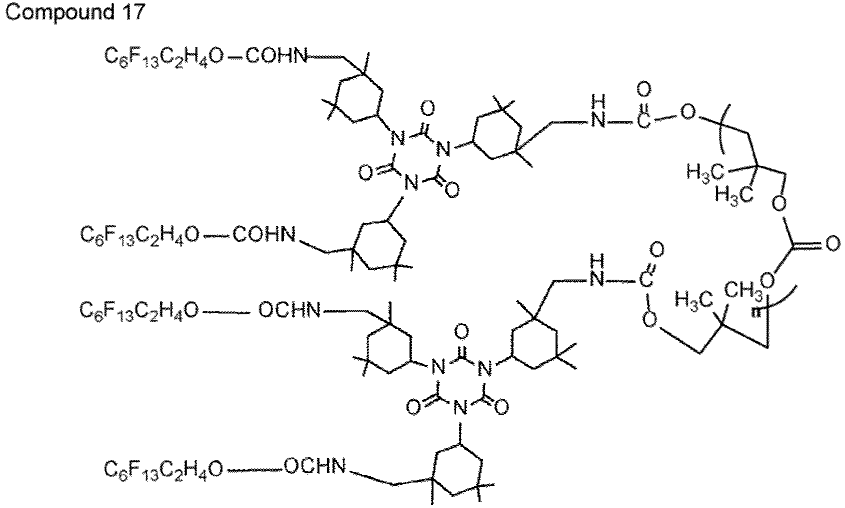

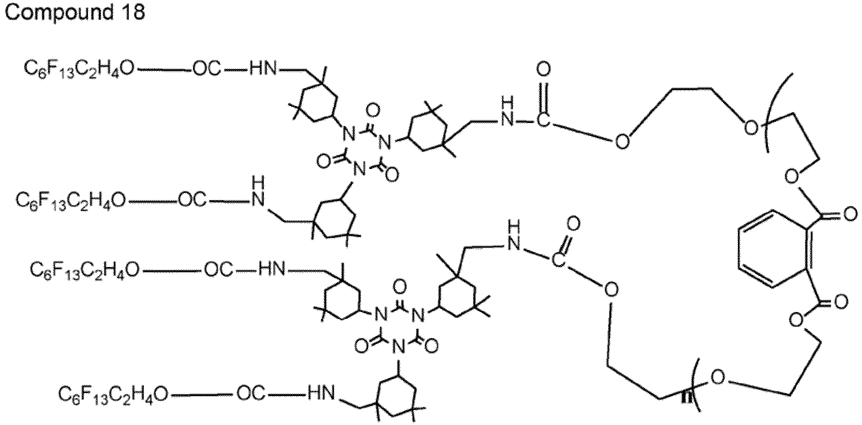

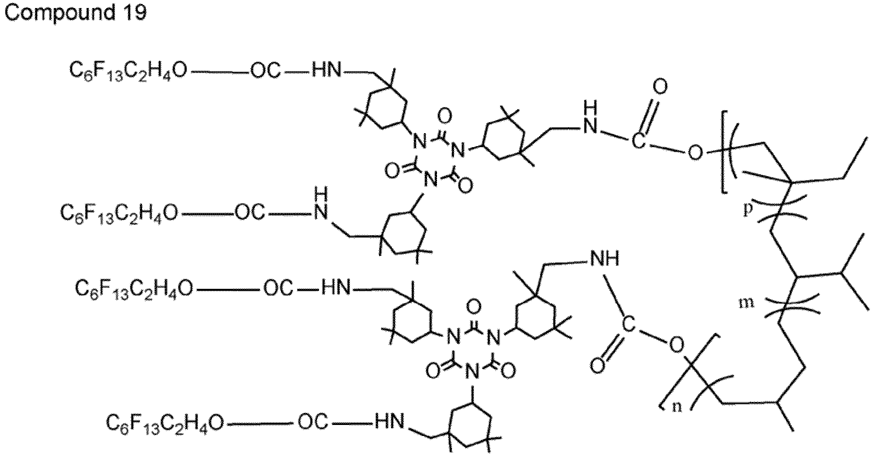

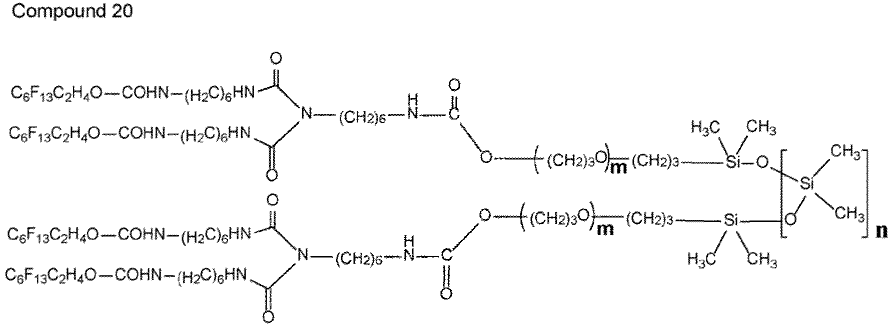

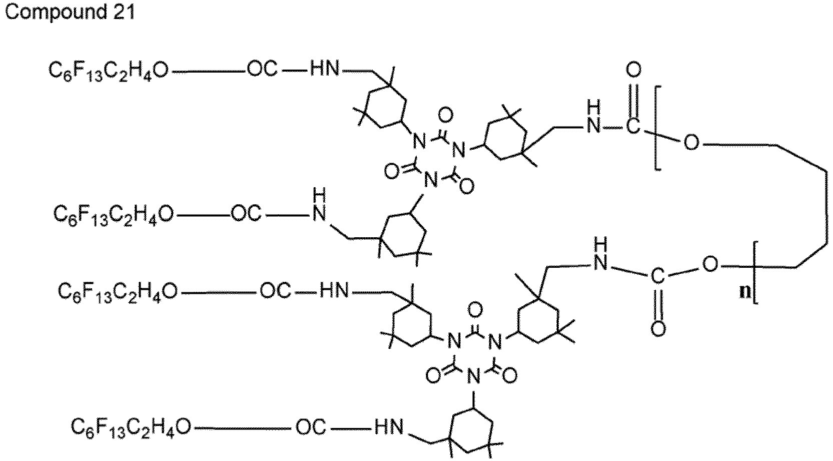

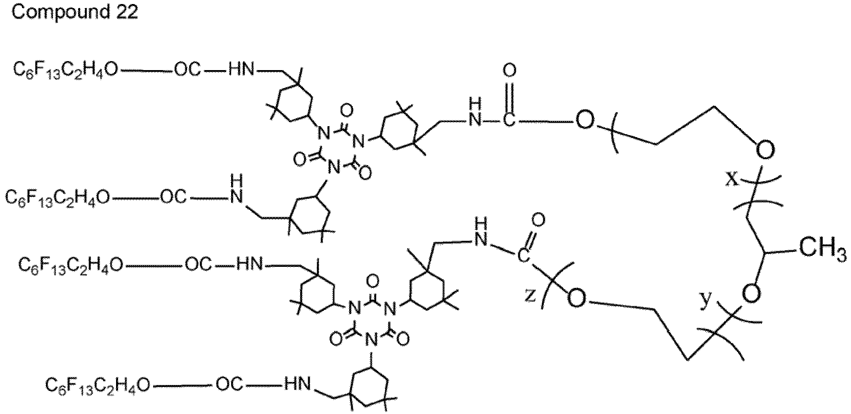

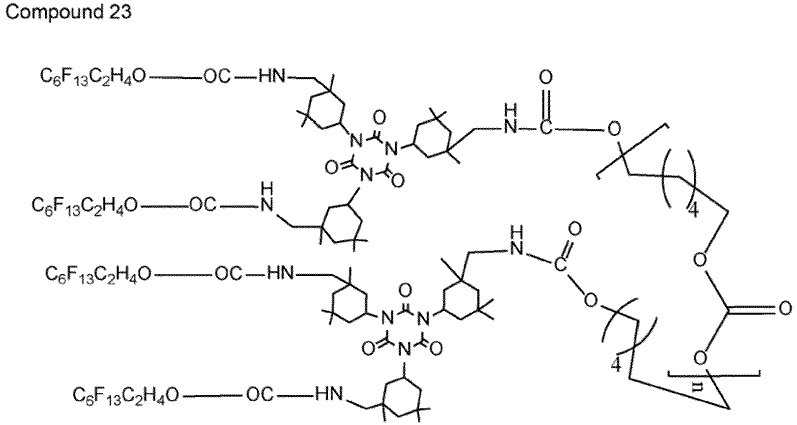

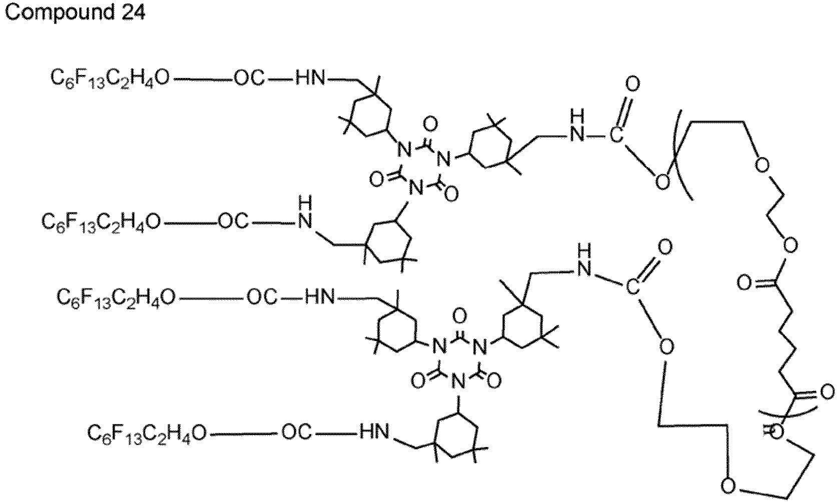

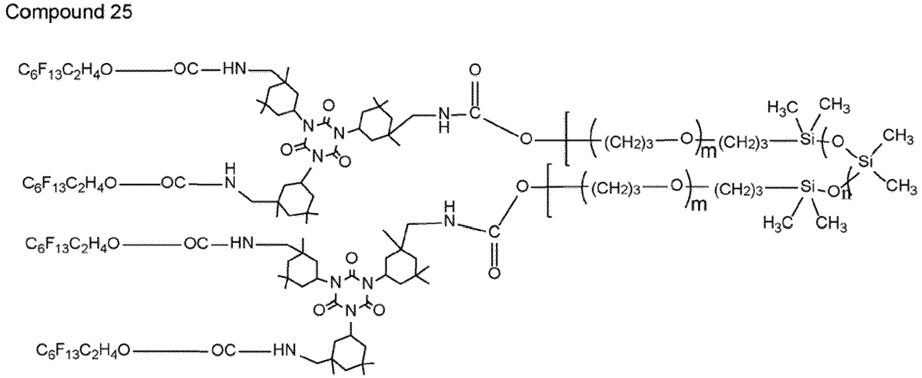

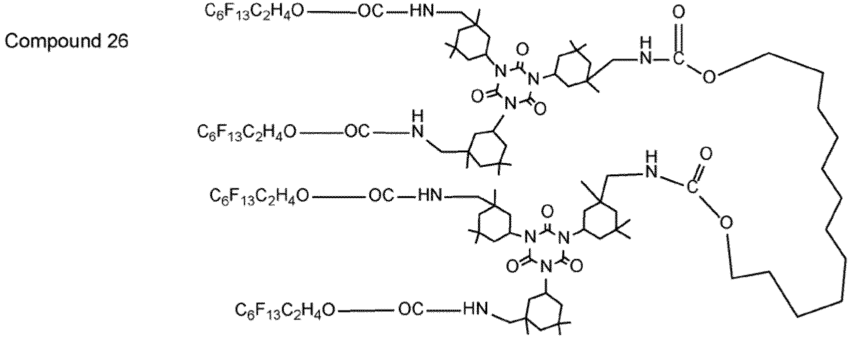

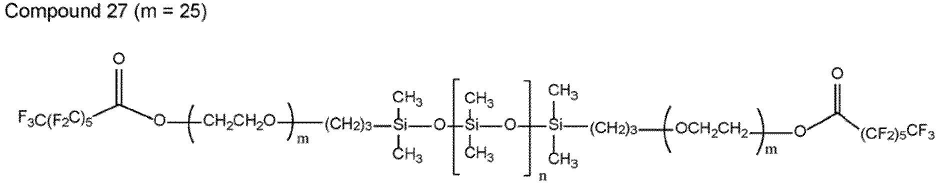

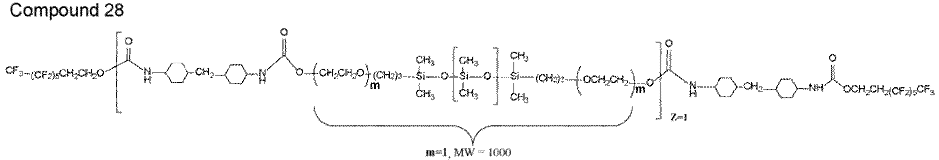

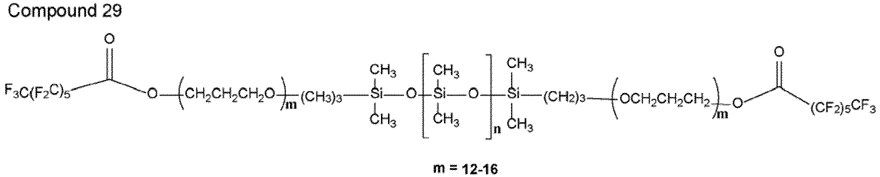

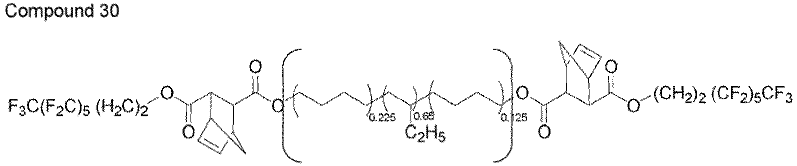

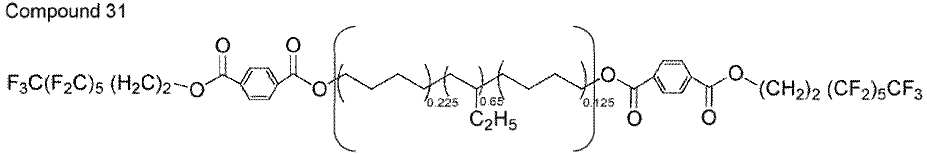

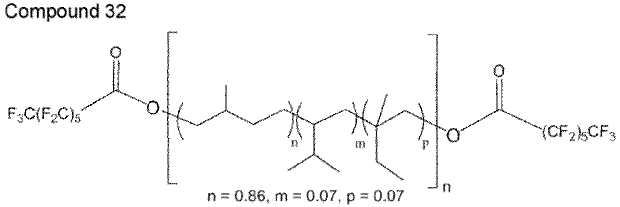

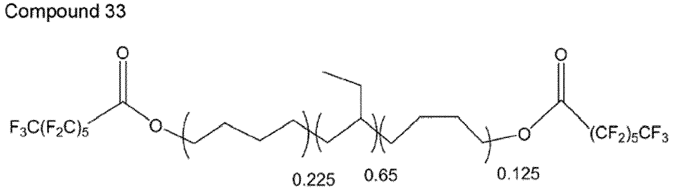

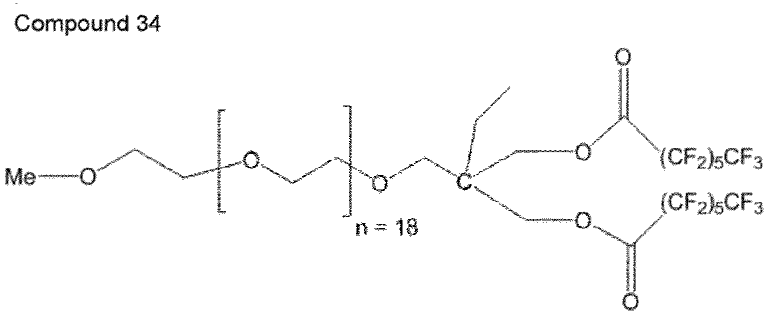

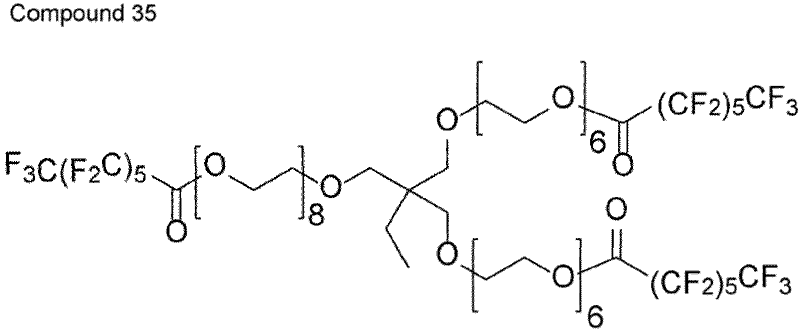

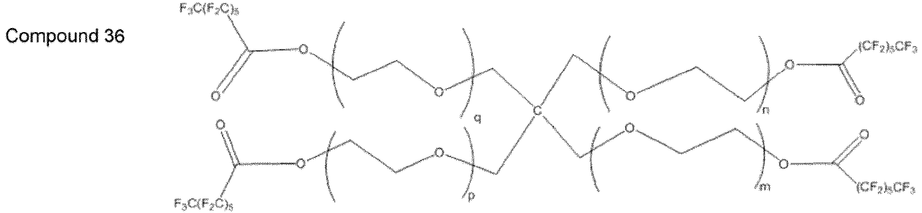

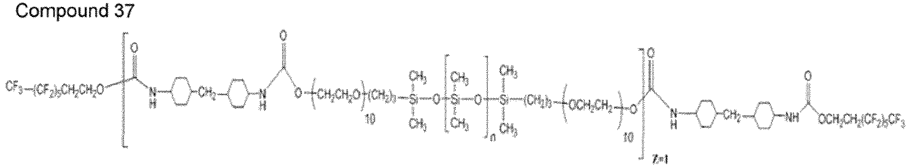

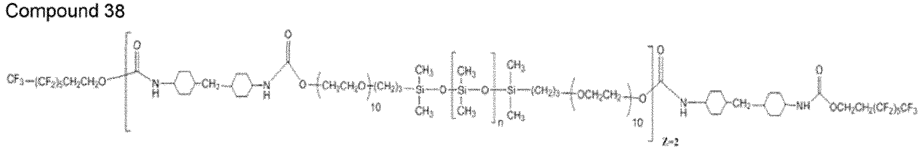

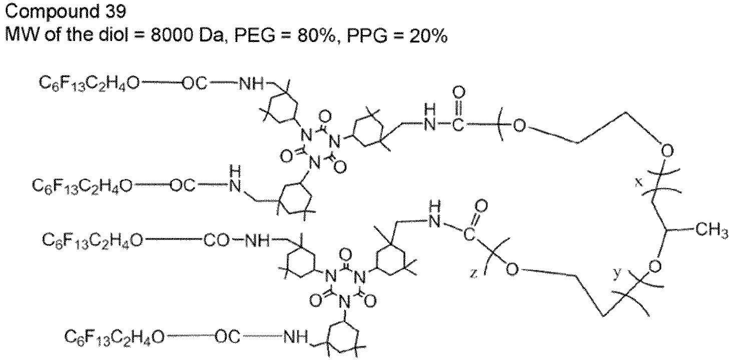

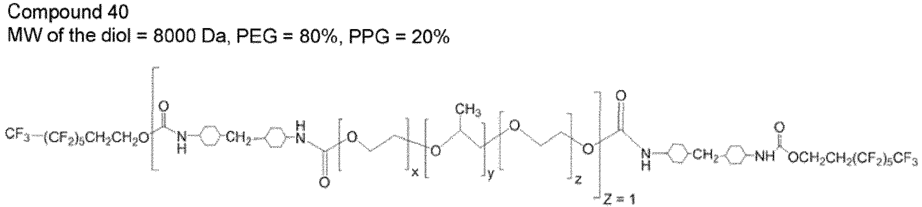

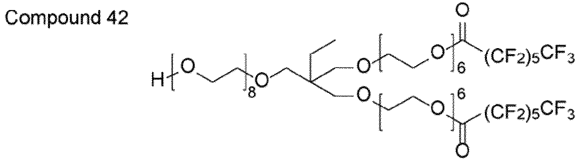

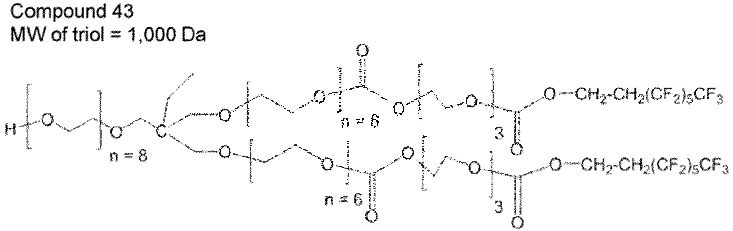

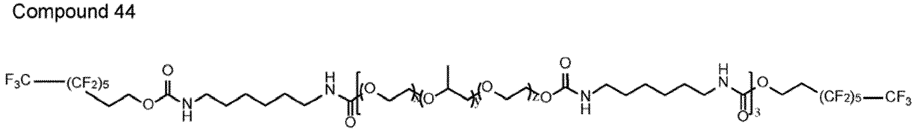

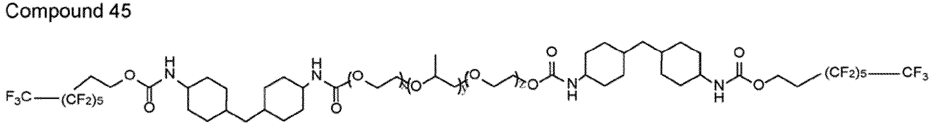

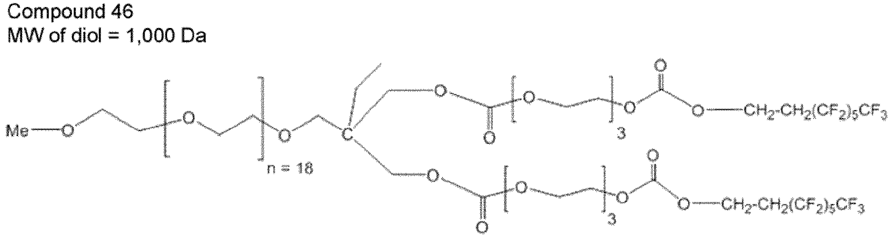

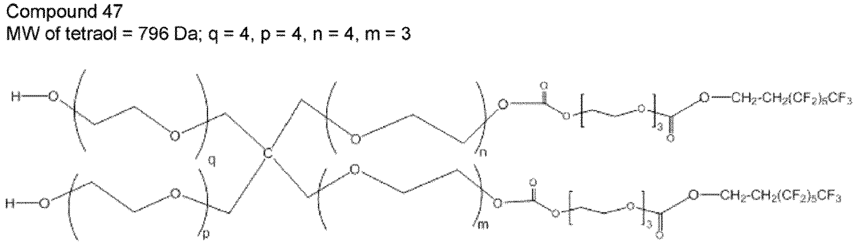

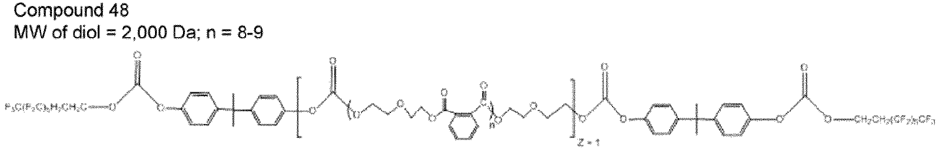

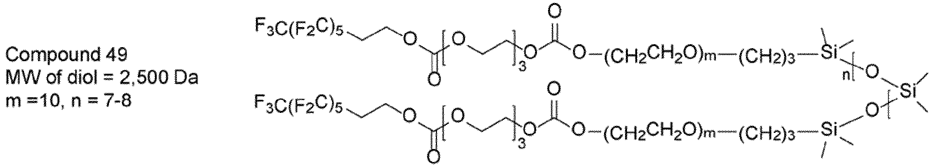

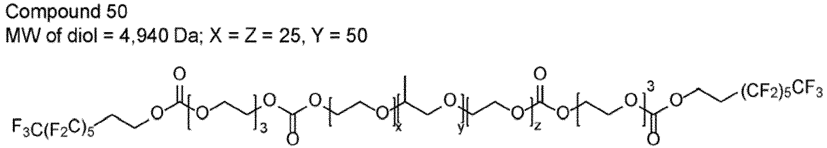

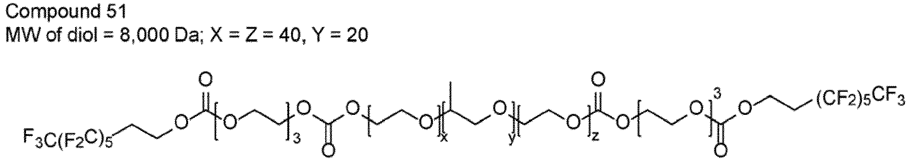

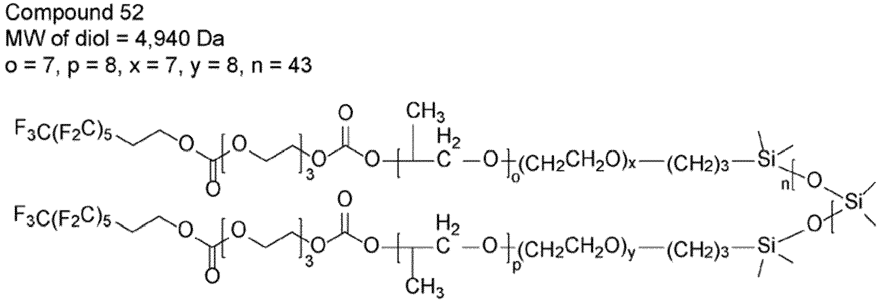

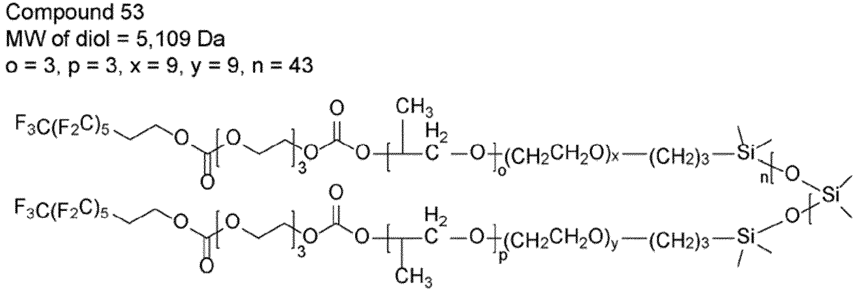

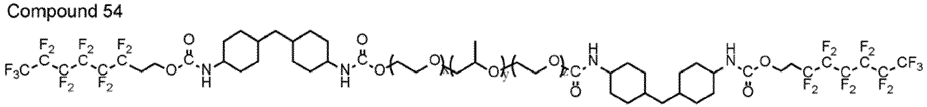

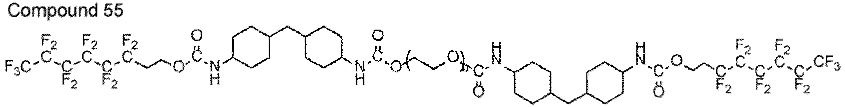

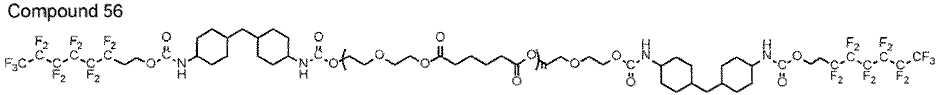

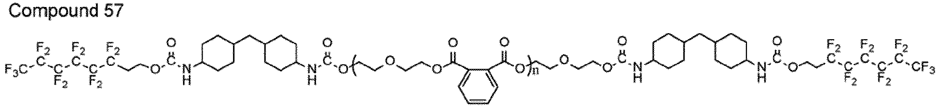

7. The use of any one of claims 1-6, wherein the base polymer
i) is selected from the group comprising of silicone, polyolefin, polyester, polycarbonate, polysulfone, polyamide, polyether, polyurea, polyurethane (PU), polyetherimide, polystyrene, cellulosic polymer, polypropylene (PP), polyethylene (PE), polyvinylchloride (PVC), polyvinyl alcohol (PVA), polyvinylpyrrolidone (PVP), polyacrylamide (PAAM), polyethylene oxide (PEO), polyethylene oxide)-b-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyether-b-polyamide, a cyclic olefin polymer (COP), a cyclic olefin copolymer (COC), methylmethacrylate acrylonitrile butadiene styrene (MABS), styrene acrylonitrile (SAN), styrene methyl methacrylate (SMMA), methacrylate butadiene styrene (MBS), styrene butadiene (SB), poly(styrene-*block*-isobutylene-*block*-styrene) (SIBS), and ethylene-vinyl acetate (EVA); or
ii) is polyurethane (PU); or
iii) is silicone (SI).

8. The use of any of claims 1 to 7, wherein the composition is comprised in an article selected from:
(a) a medical device selected from medical instruments, dental devices, dental implants, drug delivery devices, grafts, stents, pacemakers, implantable cardioverter-defibrillators, cardiac resynchronization therapy devices, cardiovascular device leads, ventricular assist devices and drivelines, heart valves, vena cava filters, endovascular coils, catheters, catheter connectors, catheter valves, intravenous delivery lines, intravenous delivery manifolds, shunts, wound drains, drainage catheters, infusion ports, cochlear implants, endotracheal tubes, tracheostomy tubes, ventilator breathing tubes and circuits, implantable sensors, ophthalmic devices, orthopedic devices, dental implants, periodontal implants, breast implants, penile implants, maxillofacial implants, cosmetic implants, valves, appliances, scaffolding, suturing material, needles, hernia repair meshes, tension-free vaginal tape and vaginal slings, prosthetic neurological devices, and ear tubes, or
(b) a wound dressing, a bandage, a gauze, a tape, a pad, a sponge, a blood oxygenator, a ventilator, a pump, tubing, wiring, an electrode, a contraceptive device, a feminine hygiene product, an endoscope, a dialysis membrane, a guide wire, a fluid collection bag, a drug delivery bag and tubing, a feeding tube, a blood bag, or a tissue regeneration or cell culture devices; or
(c) a catheter, a drainage catheter, a stent, a shunt, an infusion port, an intravenous delivery line, a dental device, a blood bag, a breast implant, a penile implant, a wound drain, a feeding tube, an endotracheal tube, a breathing tube, an ear tube, or an endoscope.

## Patentansprüche

1. Verwendung einer Zusammensetzung, umfassend ein Basispolymer in Abmischung mit 0,05 % (w/w) bis 15 % (w/w) einer Verbindung einer der Formeln (I)-(XXI)
Formel (I):
**F_{T}-[B-A]ₙ-B-F_{T}** **(I)**
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist;
Formel (II):
**F_{T}-[B-A]ₙ-B-F_{T}** **(II)**
wobei
(i) B ein Urethan einschließt;
(ii) A Polypropylenoxid, Polyethylenoxid oder Polytetramethylenoxid einschließt;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 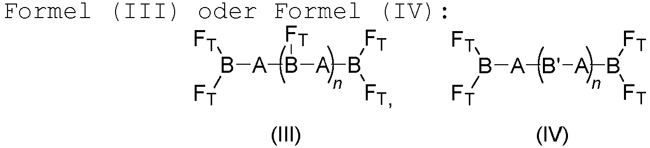
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3500 g/mol aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl zwischen 0 bis 10 ist;
Formel (V):
**F_{T}-[B-A]ₙ-B-F_{T}** **(V)**
wobei
(i) A ein oligomeres Segment ist, das Polypropylenoxid, Polyethylenoxid oder Polytetramethylenoxid einschließt und ein theoretisches Molekulargewicht von 500 bis 3000 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 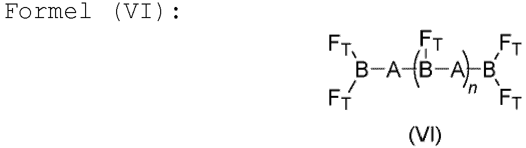
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (VII):
**F_{T}-[B-A]ₙ-B-F_{T}** **(VII)**
wobei
(i) A ein Polycarbonatpolyol mit einem theoretischen Molekulargewicht von 500 bis 3000 g/mol ist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 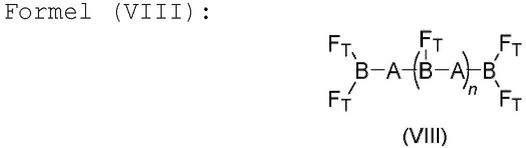
wobei
(i) A ein oligomeres Segment ist, das ein Polycarbonatpolyol mit einem theoretischen Molekulargewicht von 500 bis 3000 g/mol einschließt;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 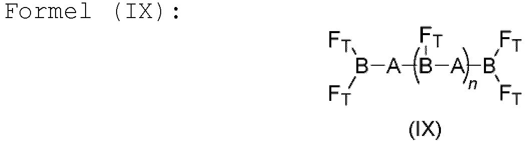
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1000 bis 5000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (X):
**F_{T}-[B-A]ₙ-B-F_{T}** **(X)**
wobei
(i) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3500 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 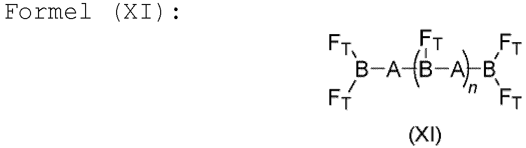
wobei
(i) A hydriertes Polybutadien, Polybutadien, hydriertes Polyisopren oder Polystyrol ist und ein theoretisches Molekulargewicht von 750 bis 3500 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 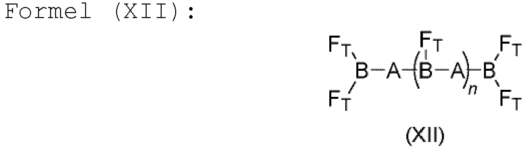
wobei
(i) A ein Polyester mit einem theoretischen Molekulargewicht von 500 bis 3500 g/mol ist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (XIII):
**F_{T}-A-F_{T}** **(XIII)**
wobei F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, und A ein verzweigtes oder unverzweigtes oligomeres Segment aus weniger als 20 Wiederholungseinheiten ist und aus Polyurethan-, Polyharnstoff-, Polyamid-, Polyalkylenoxid-, Polycarbonat-, Polyester-, Polylacton-, Polysilikon-, Polyethersulfon-, Polyolefin-, Polyvinylderivat-, Polypeptid-, Polysaccharid-, Polysiloxan-, Polydimethylsiloxan-, Polyethylen-butylen-, Polyisobutylen-, Polybutadien-, Polypropylenoxid-, Polyethylenoxid-, Polytetramethylenoxid- oder Polyethylenbutylen-Segmenten ausgewählt ist; 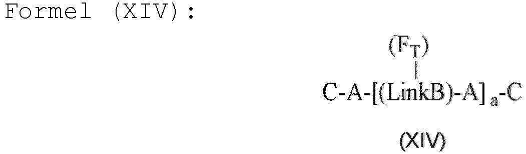 wobei
(i) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält, die kovalent an LinkB gebunden ist; und
(ii) C eine Kettenendgruppe mit einer Amin-, Alkohol- oder Carbonsäurefunktionalität ist;
(iii) A ein verzweigtes oder unverzweigtes oligomeres Segment aus weniger als 20 Wiederholungseinheiten, ausgewählt aus Polyurethan, Polyharnstoff, Polyamid, Polyalkylenoxid, Polycarbonat, Polyester, Polylacton, Polysilikon, Polyethersulfon, Polyolefin, Polyvinylderivat, Polypeptid, Polysaccharid, Polysiloxan, Polydimethylsiloxan, Polyethylen-butylen, Polyisobutylen, Polybutadien, Polypropylenoxid, Polyethylenoxid oder Polytetramethylenoxid, ist;
(iv) es sich bei LinkB um ein Kupplungssegment mit einem Molekulargewicht im Bereich von 40 bis 700 handelt, das aus der Gruppe von funktionalisierten Diaminen, Diisocyanaten, Disulfonsäuren, Dicarbonsäuren, Disäurechloriden und Dialdehyden ausgewählt ist, wobei die funktionalisierte Komponente eine sekundäre funktionelle Gruppe aufweist; und
(v) a eine ganze Zahl größer als 0 ist; 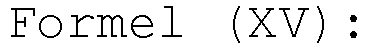 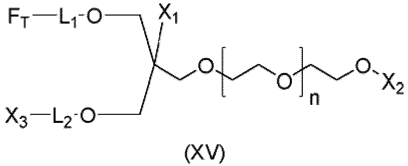
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; 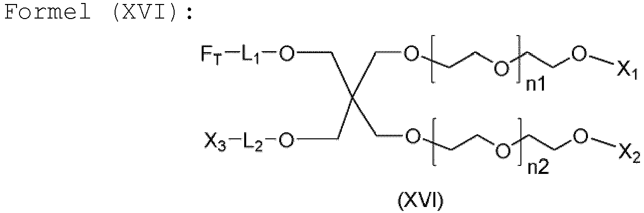
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁, X₂ und X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind;
Formel (XVII):
**G - Aₘ - [B - A]ₙ - B - G** **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat umfasst;
(ii) B jeweils unabhängig eine Bindung, ein oligomerer Linker oder ein Linker mit zwei endständigen Carbonylgruppen ist;
(iii) G jeweils H oder eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, mit der Maßgabe, dass mindestens ein G eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iv) n eine ganze Zahl von 1 bis 10 ist und
(v) m 0 oder 1 ist;
Formel (XVIII):
**F_{T}-OC(O)O-B-OC(O)O-[A-OC(O)O-B]ₙ-OC(O)O-F_{T}** **(XVIII)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist, und über eine Carbonatverknüpfung kovalent an B gebunden ist;
(ii) B ein Polyalkylenoxid oder eine durch die Formel: 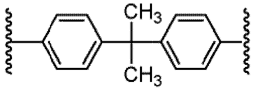 beschriebene Gruppierung umfasst und über eine Carbonatverknüpfung kovalent an A gebunden ist und
(iii) F_{T} eine oberflächenaktive Gruppe ist, die eine Polyfluororganogruppe umfasst, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, wobei F_{T} über eine Carbonatverknüpfung kovalent an B gebunden ist; und
(iv) n eine ganze Zahl von 1 bis 10 ist;
Formel (XIX):
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist;
(ii) F_{T} eine oberflächenaktive Gruppe ist, die eine Polyfluororganogruppe umfasst, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iii) m eine ganze Zahl von 2 bis 4 ist und
(iv) n eine ganze Zahl von 1 bis 10 ist; 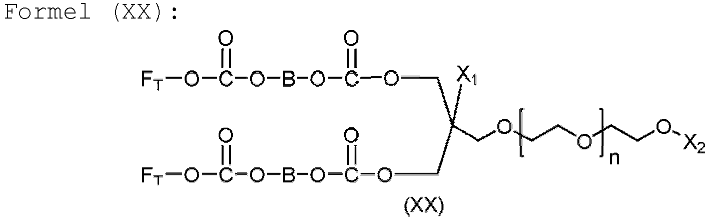
wobei
(i) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n eine ganze Zahl von 5 bis 100 ist; 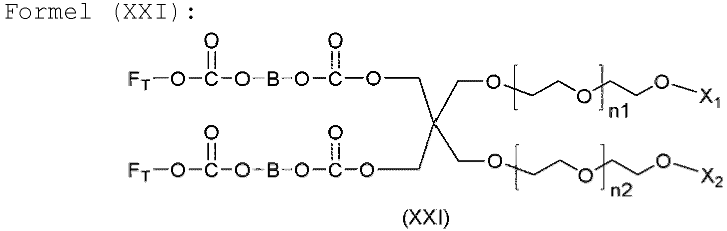
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n1 und n2 jeweils unabhängig eine ganze Zahl von 5 bis 50 sind;
zur Verringerung der Bakterienadhäsion an einer aus einem Basispolymer gebildeten Polymeroberfläche, wobei die Bakterienadhäsion gegenüber der aus dem Basispolymer in Abwesenheit der Verbindung gebildeten Polymeroberfläche um mindestens 50 % verringert ist und wobei die Bakterienadhäsion gemessen wird, indem man die Testoberflächen Bakterieninokulum in wässrigem Medium über einen angegebenen Zeitraum aussetzt, danach spült und die Oberflächen in Pufferlösung beschallt, um die anhaftenden Bakterien abzulösen, und dann die entfernten Bakterien durch Ausplattieren der Lösungen auf Bakterienwachstumssubstrat, Inkubieren über Nacht bei 37 °C und Durchführen von Koloniezählungen quantifiziert, wie in Beispiel 4 offenbart.

2. Verwendung einer Zusammensetzung, umfassend ein Basispolymer in Abmischung mit 0,05 % (w/w) bis 15 % (w/w) einer Verbindung einer der Formeln (I)-(XXI)
Formel (I):
**F_{T}-[B-A]ₙ-B-F_{T}** **(I)**
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist;
Formel (II):
**F_{T}-[B-A]ₙ-B-F_{T}** **(II)**
wobei
(i) B ein Urethan einschließt;
(ii) A Polypropylenoxid, Polyethylenoxid oder Polytetramethylenoxid einschließt;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 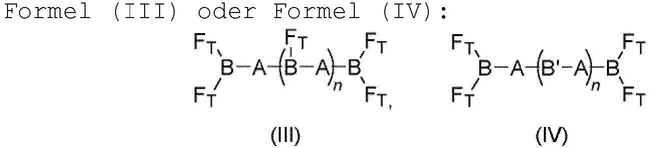
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3500 g/mol aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl zwischen 0 bis 10 ist;
Formel (V):
**F_{T}-[B-A]ₙ-B-F_{T}** **(V)**
wobei
(i) A ein oligomeres Segment ist, das Polypropylenoxid, Polyethylenoxid oder Polytetramethylenoxid einschließt und ein theoretisches Molekulargewicht von 500 bis 3000 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 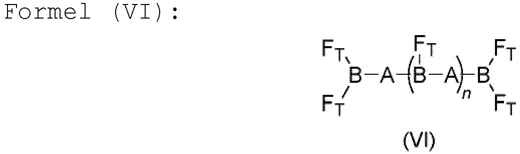
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (VII):
**F_{T}-[B-A]ₙ-B-F_{T}** **(VII)**
wobei
(i) A ein Polycarbonatpolyol mit einem theoretischen Molekulargewicht von 500 bis 3000 g/mol ist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 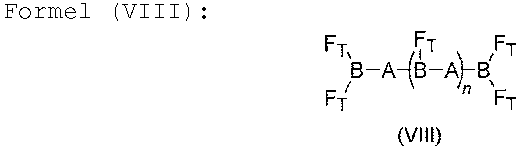
wobei
(i) A ein oligomeres Segment ist, das ein Polycarbonatpolyol mit einem theoretischen Molekulargewicht von 500 bis 3000 g/mol einschließt;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 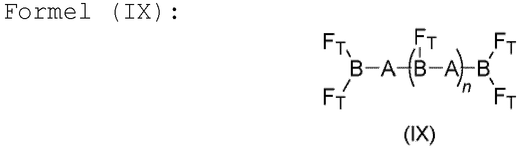
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1000 bis 5000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (X):
**F_{T}-[B-A]ₙ-B-F_{T}** **(X)**
wobei
(i) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3500 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 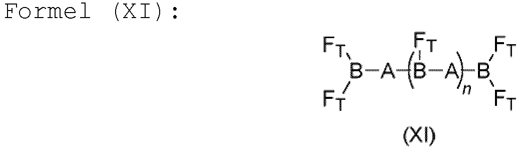
wobei
(i) A hydriertes Polybutadien, Polybutadien, hydriertes Polyisopren oder Polystyrol ist und ein theoretisches Molekulargewicht von 750 bis 3500 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 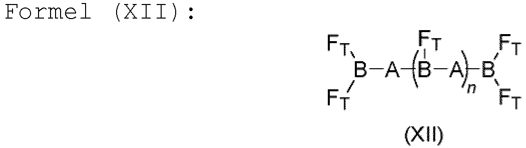
wobei
(i) A ein Polyester mit einem theoretischen Molekulargewicht von 500 bis 3500 g/mol ist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (XIII):
**F_{T}-A-F_{T}** **(XIII)**
wobei F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, und A ein verzweigtes oder unverzweigtes oligomeres Segment aus weniger als 20 Wiederholungseinheiten ist und aus Polyurethan-, Polyharnstoff-, Polyamid-, Polyalkylenoxid-, Polycarbonat-, Polyester-, Polylacton-, Polysilikon-, Polyethersulfon-, Polyolefin-, Polyvinylderivat-, Polypeptid-, Polysaccharid-, Polysiloxan-, Polydimethylsiloxan-, Polyethylen-butylen-, Polyisobutylen-, Polybutadien-, Polypropylenoxid-, Polyethylenoxid-, Polytetramethylenoxid- oder Polyethylenbutylen-Segmenten ausgewählt ist; 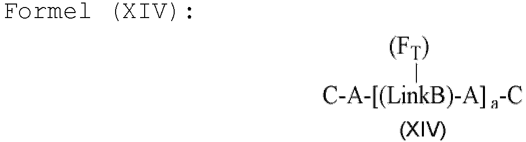 wobei
(i) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält, die kovalent an LinkB gebunden ist; und
(ii) C eine Kettenendgruppe mit einer Amin-, Alkohol- oder Carbonsäurefunktionalität ist;
(vi) A ein verzweigtes oder unverzweigtes oligomeres Segment aus weniger als 20 Wiederholungseinheiten, ausgewählt aus Polyurethan, Polyharnstoff, Polyamid, Polyalkylenoxid, Polycarbonat, Polyester, Polylacton, Polysilikon, Polyethersulfon, Polyolefin, Polyvinylderivat, Polypeptid, Polysaccharid, Polysiloxan, Polydimethylsiloxan, Polyethylen-butylen, Polyisobutylen, Polybutadien, Polypropylenoxid, Polyethylenoxid oder Polytetramethylenoxid, ist;
(vii) es sich bei LinkB um ein Kupplungssegment mit einem Molekulargewicht im Bereich von 40 bis 700 handelt, das aus der Gruppe von funktionalisierten Diaminen, Diisocyanaten, Disulfonsäuren, Dicarbonsäuren, Disäurechloriden und Dialdehyden ausgewählt ist, wobei die funktionalisierte Komponente eine sekundäre funktionelle Gruppe aufweist; und
(viii) a eine ganze Zahl größer als 0 ist; 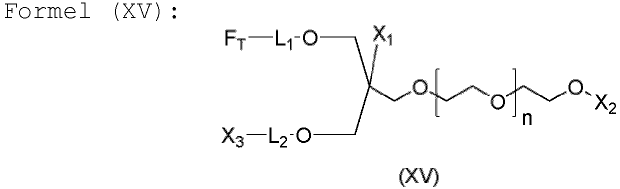
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; 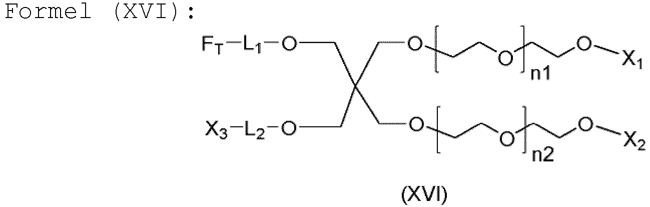
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁, X₂ und X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind;
Formel (XVII):
**G-Aₘ-[B-A]ₙ-B-G** **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat umfasst;
(ii) B jeweils unabhängig eine Bindung, ein oligomerer Linker oder ein Linker mit zwei endständigen Carbonylgruppen ist;
(iii) G jeweils H oder eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, mit der Maßgabe, dass mindestens ein G eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iv) n eine ganze Zahl von 1 bis 10 ist und
(v) m 0 oder 1 ist;
Formel (XVIII):
**F_{T}-OC(O)O-B-OC(O)O-[A-OC(O)O-B]ₙ-OC(O)O-F_{T}** **(XVIII)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist, und über eine Carbonatverknüpfung kovalent an B gebunden ist;
(ii) B ein Polyalkylenoxid oder eine durch die Formel: 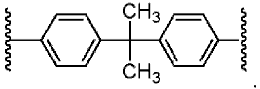 beschriebene Gruppierung umfasst und über eine Carbonatverknüpfung kovalent an A gebunden ist und
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, wobei F_{T} über eine Carbonatverknüpfung kovalent an B gebunden ist; und
(iv) n eine ganze Zahl von 1 bis 10 ist;
Formel (XIX):
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist;
(ii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iii) m eine ganze Zahl von 2 bis 4 ist und
(iv) n eine ganze Zahl von 1 bis 10 ist; 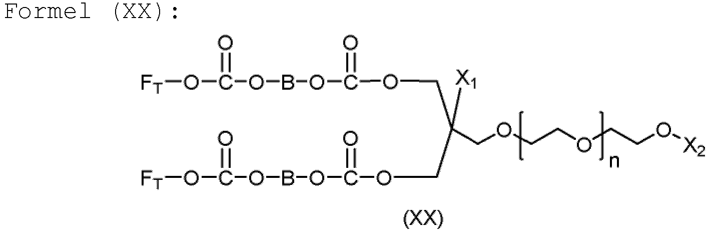
wobei
(i) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n eine ganze Zahl von 5 bis 100 ist; 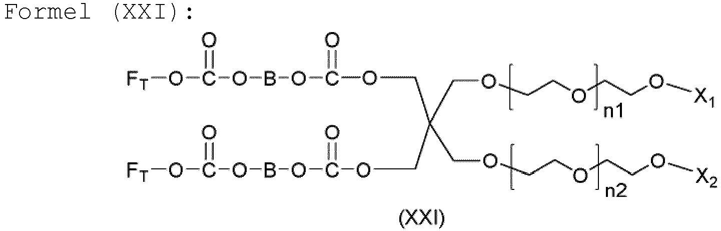
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n1 und n2 jeweils unabhängig eine ganze Zahl von 5 bis 50 sind;
zur Verringerung der durch Bakterien vermittelten Salzbildung auf einer Polymeroberfläche einer Urinverweilvorrichtung durch Bereitstellen der aus der Zusammensetzung gebildeten Polymeroberfläche und (ii) Inkontaktbringen der Polymeroberfläche mit Urin, wobei die Salzabscheidung unter Urinverweilbedingungen gegenüber der aus dem Basispolymer in Abwesenheit der Verbindung gebildeten Polymeroberfläche mindestens 20 % verringert ist, wobei die Salzabscheidung gemessen wird, indem man Testoberflächen in mit *P*. *mirabilis*-Bakterien inokuliertem künstlichem Urin 14 Tage inkubiert, anschließend die erhaltenen Salzabscheidungen in verdünnter Salpetersäure löst und den Calzium- und Magnesiumgehalt mittels Atomabsorptionsspektroskopie oder Massenspektrometrie mit induktiv gekoppeltem Plasma quantifiziert, wie in Beispiel 22 offenbart.

3. Verwendung einer Zusammensetzung, umfassend ein Basispolymer in Abmischung mit 0,05 % (w/w) bis 15 % (w/w) einer Verbindung einer der Formeln (I)-(XXI)
Formel (I):
**F_{T}-[B-A]ₙ-B-F_{T}** **(I)**
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist;
Formel (II):
**F_{T}-[B-A]ₙ-B-F_{T}** **(II)**
wobei
(i) B ein Urethan einschließt;
(ii) A Polypropylenoxid, Polyethylenoxid oder Polytetramethylenoxid einschließt;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 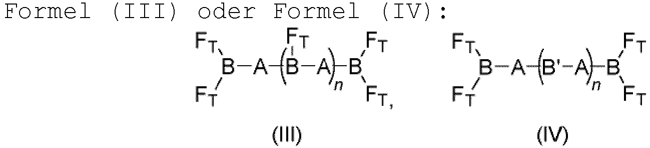
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3500 g/mol aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl zwischen 0 bis 10 ist;
Formel (V):
**F_{T}-[B-A]ₙ-B-F_{T}** **(V)**
wobei
(i) A ein oligomeres Segment ist, das Polypropylenoxid, Polyethylenoxid oder Polytetramethylenoxid einschließt und ein theoretisches Molekulargewicht von 500 bis 3000 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 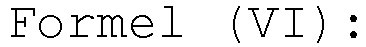 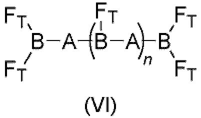
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (VII):
**F_{T}-[B-A]ₙ-B-F_{T}** **(VII)**
wobei
(i) A ein Polycarbonatpolyol mit einem theoretischen Molekulargewicht von 500 bis 3000 g/mol ist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 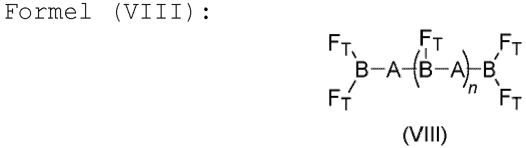
wobei
(i) A ein oligomeres Segment ist, das ein Polycarbonatpolyol mit einem theoretischen Molekulargewicht von 500 bis 3000 g/mol einschließt;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 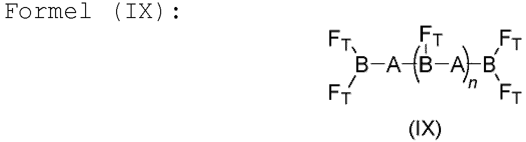
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1000 bis 5000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (X):
**F_{T}-[B-A]ₙ-B-F_{T}** **(X)**
wobei
(i) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3500 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 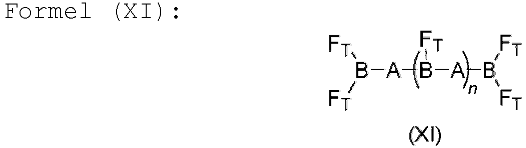
wobei
(i) A hydriertes Polybutadien, Polybutadien, hydriertes Polyisopren oder Polystyrol ist und ein theoretisches Molekulargewicht von 750 bis 3500 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 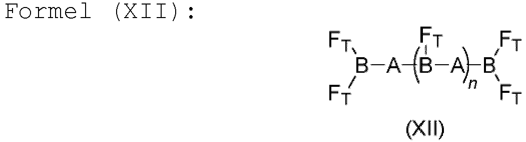
wobei
(i) A ein Polyester mit einem theoretischen Molekulargewicht von 500 bis 3500 g/mol ist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (XIII):
**F_{T}-A-F_{T}** **(XIII)**
wobei F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, und A ein verzweigtes oder unverzweigtes oligomeres Segment aus weniger als 20 Wiederholungseinheiten ist und aus Polyurethan-, Polyharnstoff-, Polyamid-, Polyalkylenoxid-, Polycarbonat-, Polyester-, Polylacton-, Polysilikon-, Polyethersulfon-, Polyolefin-, Polyvinylderivat-, Polypeptid-, Polysaccharid-, Polysiloxan-, Polydimethylsiloxan-, Polyethylen-butylen-, Polyisobutylen-, Polybutadien-, Polypropylenoxid-, Polyethylenoxid-, Polytetramethylenoxid- oder Polyethylenbutylen-Segmenten ausgewählt ist; 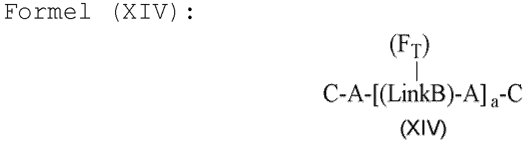 wobei
(i) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält, die kovalent an LinkB gebunden ist; und
(ii) C eine Kettenendgruppe mit einer Amin-, Alkohol- oder Carbonsäurefunktionalität ist;
(iii) A ein verzweigtes oder unverzweigtes oligomeres Segment aus weniger als 20 Wiederholungseinheiten, ausgewählt aus Polyurethan, Polyharnstoff, Polyamid, Polyalkylenoxid, Polycarbonat, Polyester, Polylacton, Polysilikon, Polyethersulfon, Polyolefin, Polyvinylderivat, Polypeptid, Polysaccharid, Polysiloxan, Polydimethylsiloxan, Polyethylen-butylen, Polyisobutylen, Polybutadien, Polypropylenoxid, Polyethylenoxid oder Polytetramethylenoxid, ist;
(iv) es sich bei LinkB um ein Kupplungssegment mit einem Molekulargewicht im Bereich von 40 bis 700 handelt, das aus der Gruppe von funktionalisierten Diaminen, Diisocyanaten, Disulfonsäuren, Dicarbonsäuren, Disäurechloriden und Dialdehyden ausgewählt ist, wobei die funktionalisierte Komponente eine sekundäre funktionelle Gruppe aufweist; und
(v) a eine ganze Zahl größer als 0 ist; 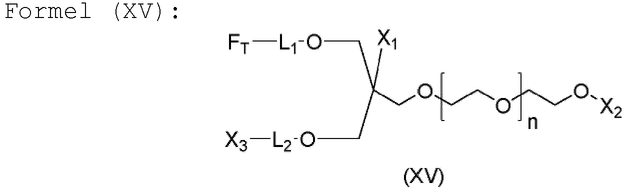
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; 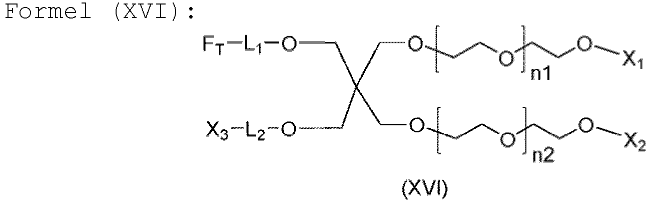
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind;
Formel (XVII):
**G-Aₘ-[B-A]ₙ-B-G** **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat umfasst;
(ii) B jeweils unabhängig eine Bindung, ein oligomerer Linker oder ein Linker mit zwei endständigen Carbonylgruppen ist;
(iii) G jeweils H oder eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, mit der Maßgabe, dass mindestens ein G eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iv) n eine ganze Zahl von 1 bis 10 ist und
(v) m 0 oder 1 ist;
Formel (XVIII):
**F_{T}-OC(O)O-B-OC(O)O-[A-OC(O)O-B]ₙ-OC(O)O-F_{T}** **(XVIII)**
wobei
A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist, und über eine Carbonatverknüpfung kovalent an B gebunden ist;
(ii) B ein Polyalkylenoxid oder eine durch die Formel: 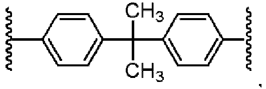 beschriebene Gruppierung umfasst und über eine Carbonatverknüpfung kovalent an A gebunden ist und
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, wobei F_{T} über eine Carbonatverknüpfung kovalent an B gebunden ist; und
(iv) n eine ganze Zahl von 1 bis 10 ist;
Formel (XIX):
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist;
(ii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iii) m eine ganze Zahl von 2 bis 4 ist und
(iv) n eine ganze Zahl von 1 bis 10 ist; 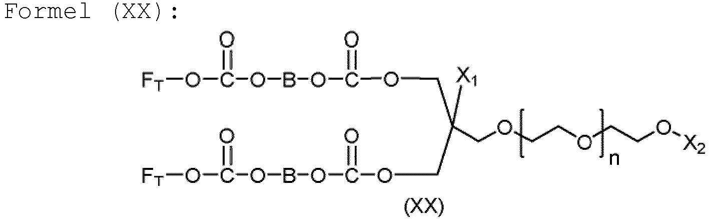
wobei
(i) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n eine ganze Zahl von 5 bis 100 ist; 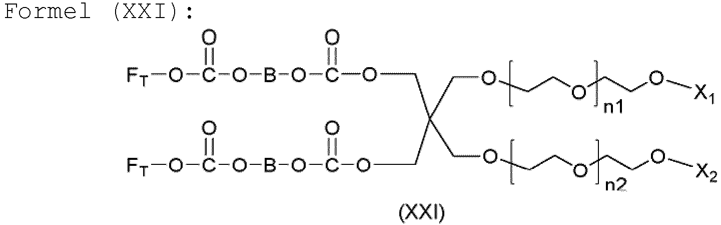
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n1 und n2 jeweils unabhängig eine ganze Zahl von 5 bis 50 sind;
zur Verringerung der Bakterienbiofilmbildung auf einer Polymeroberfläche durch Bereitstellen der aus der Zusammensetzung gebildeten Polymeroberfläche, wobei die Bakterienbiofilmbildung gegenüber der aus dem Basispolymer in Abwesenheit der Verbindung gebildeten Polymeroberfläche um mindestens 20 % verringert ist und wobei die Biofilmbildung gemessen wird, indem man die Testoberflächen Bakterieninokulum in wässrigem Medium unter Bedingungen, die die Biofilmbildung erlauben, aussetzt, danach die Oberflächen spült und dann die Biofilm-Oberflächenbedeckung mittels Lichtmikroskopie oder Rasterelektronenmikroskopie misst oder den Biofilm anfärbt und das UV-Absorptionsmaß der extrahierten Anfärbung misst, wie in Beispiel 18 offenbart.

4. Verwendung einer Zusammensetzung, umfassend ein Basispolymer in Abmischung mit 0,05 % (w/w) bis 15 % (w/w) einer Verbindung einer der Formeln (I)-(XXI)
Formel (I):
**F_{T}-[B-A]ₙ-B-F_{T}** **(I)**
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist;
Formel (II):
**F_{T}-[B-A]ₙ-B-F_{T}** **(II)**
wobei
(i) B ein Urethan einschließt;
(ii) A Polypropylenoxid, Polyethylenoxid oder Polytetramethylenoxid einschließt;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 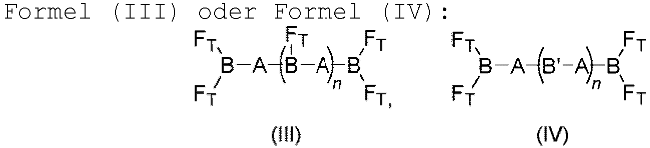
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3500 g/mol aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl zwischen 0 bis 10 ist;
Formel (V):
**F_{T}-[B-A]ₙ-B-F_{T}** **(V)**
wobei
(i) A ein oligomeres Segment ist, das Polypropylenoxid, Polyethylenoxid oder Polytetramethylenoxid einschließt und ein theoretisches Molekulargewicht von 500 bis 3000 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 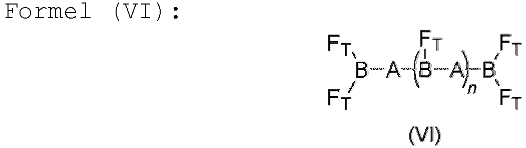
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (VII):
**F_{T}-[B-A]ₙ-B-F_{T}** **(VII)**
wobei
(i) A ein Polycarbonatpolyol mit einem theoretischen Molekulargewicht von 500 bis 3000 g/mol ist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 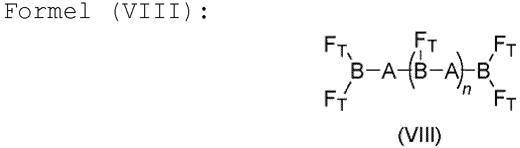
wobei
(i) A ein oligomeres Segment ist, das ein Polycarbonatpolyol mit einem theoretischen Molekulargewicht von 500 bis 3000 g/mol einschließt;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 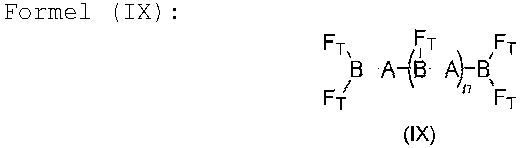
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1000 bis 5000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (X):
**F_{T}-[B-A]ₙ-B-F_{T}** **(X)**
wobei
(i) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3500 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 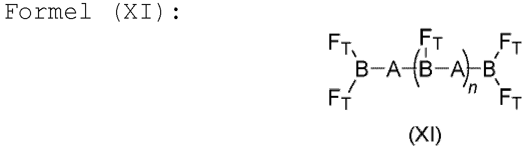
wobei
(i) A hydriertes Polybutadien, Polybutadien, hydriertes Polyisopren oder Polystyrol ist und ein theoretisches Molekulargewicht von 750 bis 3500 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 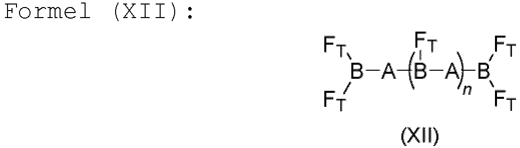
wobei
(i) A ein Polyester mit einem theoretischen Molekulargewicht von 500 bis 3500 g/mol ist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (XIII):
**F_{T}-A-F_{T}** **(XIII)**
wobei F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, und A ein verzweigtes oder unverzweigtes oligomeres Segment aus weniger als 20 Wiederholungseinheiten ist, das aus Polyurethan-, Polyharnstoff-, Polyamid-, Polyalkylenoxid-, Polycarbonat-, Polyester-, Polylacton-, Polysilikon-, Polyethersulfon-, Polyolefin-, Polyvinylderivat-, Polypeptid-, Polysaccharid-, Polysiloxan-, Polydimethylsiloxan-, Polyethylen-butylen-, Polyisobutylen-, Polybutadien-, Polypropylenoxid-, Polyethylenoxid-, Polytetramethylenoxid- oder Polyethylenbutylen-Segmenten ausgewählt ist; 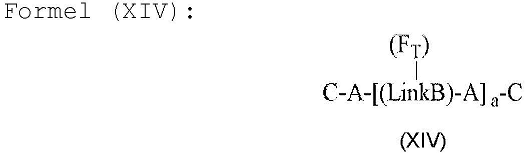 wobei
(i) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält, die kovalent an LinkB gebunden ist; und
(ii) C eine Kettenendgruppe mit einer Amin-, Alkohol- oder Carbonsäurefunktionalität ist;
(ix) A ein verzweigtes oder unverzweigtes oligomeres Segment aus weniger als 20 Wiederholungseinheiten, ausgewählt aus Polyurethan, Polyharnstoff, Polyamid, Polyalkylenoxid, Polycarbonat, Polyester, Polylacton, Polysilikon, Polyethersulfon, Polyolefin, Polyvinylderivat, Polypeptid, Polysaccharid, Polysiloxan, Polydimethylsiloxan, Polyethylen-butylen, Polyisobutylen, Polybutadien, Polypropylenoxid, Polyethylenoxid oder Polytetramethylenoxid, ist;
(x) es sich bei LinkB um ein Kupplungssegment mit einem Molekulargewicht im Bereich von 40 bis 700 handelt, das aus der Gruppe von funktionalisierten Diaminen, Diisocyanaten, Disulfonsäuren, Dicarbonsäuren, Disäurechloriden und Dialdehyden ausgewählt ist, wobei die funktionalisierte Komponente eine sekundäre funktionelle Gruppe aufweist; und
(xi) a eine ganze Zahl größer als 0 ist; 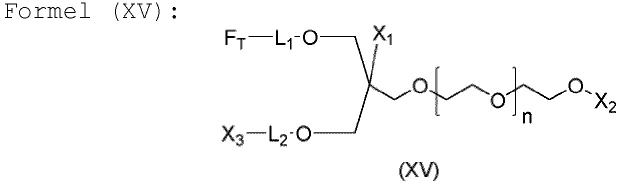
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; 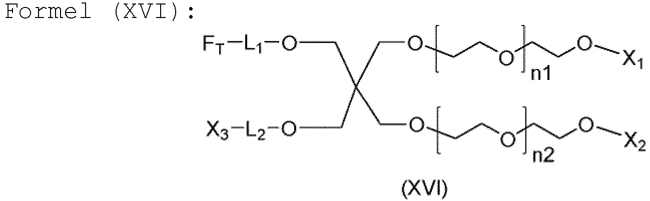
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind;
Formel (XVII):
**G - Am - [B - A]n - B - G** **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat umfasst;
(ii) B jeweils unabhängig eine Bindung, ein oligomerer Linker oder ein Linker mit zwei endständigen Carbonylgruppen ist;
(iii) G jeweils H oder eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, mit der Maßgabe, dass mindestens ein G eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iv) n eine ganze Zahl von 1 bis 10 ist und
(v) m 0 oder 1 ist;
Formel (XVIII):
**F_{T}-OC(O)O-B-OC(O)O-[A-OC(O)O-B]ₙ-OC(O)O-F_{T}** **(XVIII)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist, und über eine Carbonatverknüpfung kovalent an B gebunden ist;
(ii) B ein Polyalkylenoxid oder eine durch die Formel: 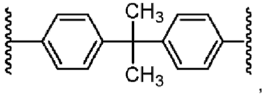 beschriebene Gruppierung umfasst und über eine Carbonatverknüpfung kovalent an A gebunden ist und
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, wobei F_{T} über eine Carbonatverknüpfung kovalent an B gebunden ist; und
(iv) n eine ganze Zahl von 1 bis 10 ist;
Formel (XIX):
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist;
(ii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iii) m eine ganze Zahl von 2 bis 4 ist und
(iv) n eine ganze Zahl von 1 bis 10 ist; 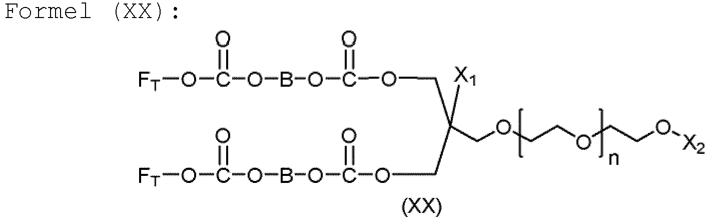
wobei
(i) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n eine ganze Zahl von 5 bis 100 ist; 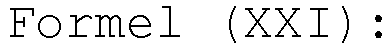 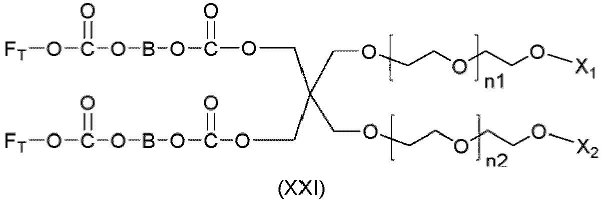
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n1 und n2 jeweils unabhängig eine ganze Zahl von 5 bis 50 sind;
zur Verringerung der Bakterienbiobelastung auf einer aus der Zusammensetzung gebildeten Polymeroberfläche, wobei die Biobelastung gegenüber der aus dem Basispolymer in Abwesenheit der Verbindung gebildeten Polymeroberfläche um mindestens 20 % verringert ist und wobei die Bakterienbiobelastung gemessen wird, indem man die Testoberflächen Bakterieninokulum in wässrigem Medium über einen angegebenen Zeitraum aussetzt, danach spült und die Oberflächen in Pufferlösung beschallt, um die anhaftenden Bakterien abzulösen, und dann die entfernten Bakterien durch Ausplattieren der Lösungen auf Bakterienwachstumssubstrat, Inkubieren über Nacht bei 37 °C und Durchführen von Koloniezählungen quantifiziert, wie in Beispiel 4 offenbart.

5. Verwendung einer Zusammensetzung, umfassend ein Basispolymer in Abmischung mit 0,05 % (w/w) bis 15 % (w/w) einer Verbindung einer der Formeln (I)-(XXI)
Formel (I):
**F_{T}-[B-A]ₙ-B-F_{T}** **(I)**
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist;
Formel (II):
**F_{T}-[B-A]ₙ-B-F_{T}** **(II)**
wobei
(i) B ein Urethan einschließt;
(ii) A Polypropylenoxid, Polyethylenoxid oder Polytetramethylenoxid einschließt;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 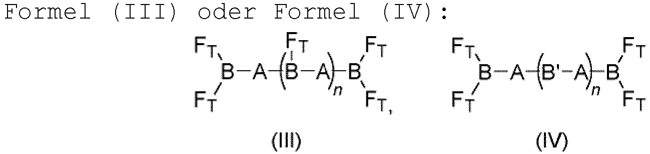
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3500 g/mol aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl zwischen 0 bis 10 ist;
Formel (V):
**F_{T}-[B-A]ₙ-B-F_{T}** **(V)**
wobei
(i) A ein oligomeres Segment ist, das Polypropylenoxid, Polyethylenoxid oder Polytetramethylenoxid einschließt und ein theoretisches Molekulargewicht von 500 bis 3000 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 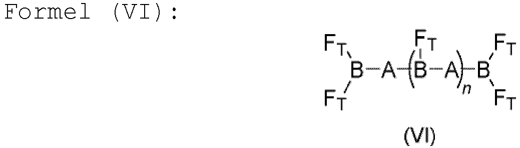
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (VII):
**F_{T}-[B-A]ₙ-B-F_{T}** **(VII)**
wobei
(i) A ein Polycarbonatpolyol mit einem theoretischen Molekulargewicht von 500 bis 3000 g/mol ist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 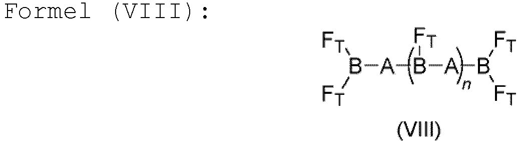
wobei
(i) A ein oligomeres Segment ist, das ein Polycarbonatpolyol mit einem theoretischen Molekulargewicht von 500 bis 3000 g/mol einschließt;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 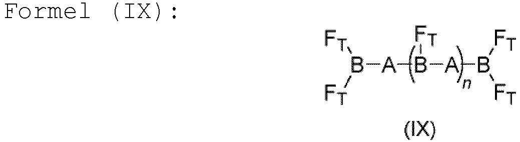
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1000 bis 5000 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (X):
**F_{T}-[B-A]ₙ-B-F_{T}** **(X)**
wobei
(i) A ein Segment ist, das aus der Gruppe bestehend aus hydriertem Polybutadien, hydriertem Polyisopren, Polysiloxan-Polyethylenglykol-Blockcopolymer und Polystyrol ausgewählt ist, und ein theoretisches Molekulargewicht von 750 bis 3500 g/mol aufweist;
(ii) B ein Segment ist, das aus einem Diisocyanat gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 1 bis 10 ist; 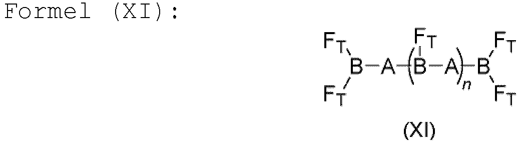
wobei
(i) A hydriertes Polybutadien, Polybutadien, hydriertes Polyisopren oder Polystyrol ist und ein theoretisches Molekulargewicht von 750 bis 3500 g/mol aufweist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist; 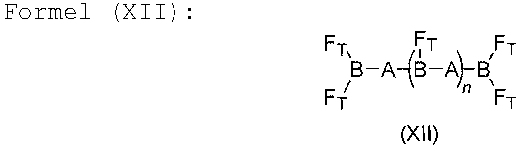
wobei
(i) A ein Polyester mit einem theoretischen Molekulargewicht von 500 bis 3500 g/mol ist;
(ii) B ein Segment ist, das aus einem Isocyanattrimer oder Biurettrimer gebildet ist;
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält; und
(iv) n eine ganze Zahl von 0 bis 10 ist;
Formel (XIII):
**F_{T}-A-F_{T}** **(XIII)**
wobei F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, und A ein verzweigtes oder unverzweigtes oligomeres Segment aus weniger als 20 Wiederholungseinheiten ist und aus Polyurethan-, Polyharnstoff-, Polyamid-, Polyalkylenoxid-, Polycarbonat-, Polyester-, Polylacton-, Polysilikon-, Polyethersulfon-, Polyolefin-, Polyvinylderivat-, Polypeptid-, Polysaccharid-, Polysiloxan-, Polydimethylsiloxan-, Polyethylen-butylen-, Polyisobutylen-, Polybutadien-, Polypropylenoxid-, Polyethylenoxid-, Polytetramethylenoxid- oder Polyethylenbutylen-Segmenten ausgewählt ist; 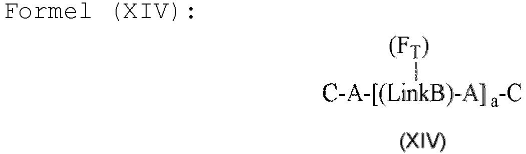 wobei
(i) F_{T} eine Polyfluororganogruppe ist, bei der es sich um eine gegebenenfalls durch ein, zwei oder drei nicht benachbarte Sauerstoffatome unterbrochene Kohlenwasserstoffgruppe handelt, in der zwei bis neunundfünzig Wasserstoffatome durch Fluoratome ersetzt sind und die eins bis dreißig Kohlenstoffatome enthält, die kovalent an LinkB gebunden ist; und
(ii) C eine Kettenendgruppe mit einer Amin-, Alkohol- oder Carbonsäurefunktionalität ist;
(iii) A ein verzweigtes oder unverzweigtes oligomeres Segment aus weniger als 20 Wiederholungseinheiten, ausgewählt aus Polyurethan, Polyharnstoff, Polyamid, Polyalkylenoxid, Polycarbonat, Polyester, Polylacton, Polysilikon, Polyethersulfon, Polyolefin, Polyvinylderivat, Polypeptid, Polysaccharid, Polysiloxan, Polydimethylsiloxan, Polyethylen-butylen, Polyisobutylen, Polybutadien, Polypropylenoxid, Polyethylenoxid oder Polytetramethylenoxid, ist;
(iv) es sich bei LinkB um ein Kupplungssegment mit einem Molekulargewicht im Bereich von 40 bis 700 handelt, das aus der Gruppe von funktionalisierten Diaminen, Diisocyanaten, Disulfonsäuren, Dicarbonsäuren, Disäurechloriden und Dialdehyden ausgewählt ist, wobei die funktionalisierte Komponente eine sekundäre funktionelle Gruppe aufweist; und
(v) a eine ganze Zahl größer als 0 ist; 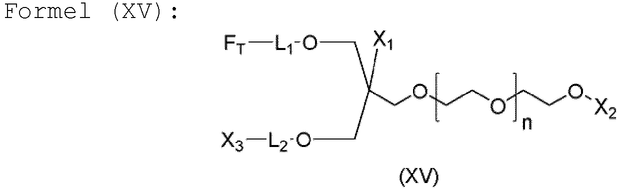
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; 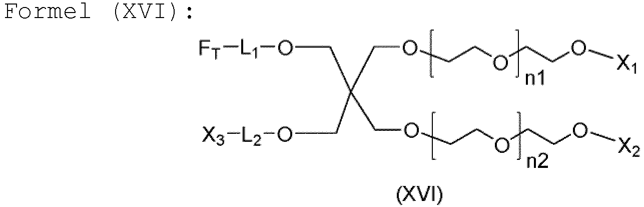
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind;
Formel (XVII):
**G-Aₘ-[B-A]ₙ-B-G** **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat umfasst;
(ii) B jeweils unabhängig eine Bindung, ein oligomerer Linker oder ein Linker mit zwei endständigen Carbonylgruppen ist;
(iii) G jeweils H oder eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, mit der Maßgabe, dass mindestens ein G eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iv) n eine ganze Zahl von 1 bis 10 ist und
(v) m 0 oder 1 ist;
Formel (XVIII):
**F_{T}-OC(O)O-B-OC(O)O-[A-OC(O)O-B]ₙ-OC(O)O-F_{T}** **(XVIII)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist, und über eine Carbonatverknüpfung kovalent an B gebunden ist;
(ii) B ein Polyalkylenoxid oder eine durch die Formel: 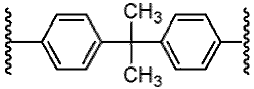 beschriebene Gruppierung umfasst und über eine Carbonatverknüpfung kovalent an A gebunden ist und
(iii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält, wobei F_{T} über eine Carbonatverknüpfung kovalent an B gebunden ist; und
(iv) n eine ganze Zahl von 1 bis 10 ist;
Formel (XIX):
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
wobei
(i) A ein Weichsegment umfasst, das aus hydriertem Polybutadien, hydriertem Polyisopren, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, einem Polysiloxan, Bisphenol-A-ethoxylat, Poly(ethylenoxid)-b-poly(propylenoxid)-b-poly(ethylenoxid), Polyethylenoxid, Polypropylenoxid oder Polytetramethylenoxid ausgewählt ist;
(ii) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(iii) m eine ganze Zahl von 2 bis 4 ist und
(iv) n eine ganze Zahl von 1 bis 10 ist; 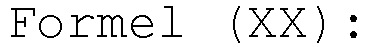 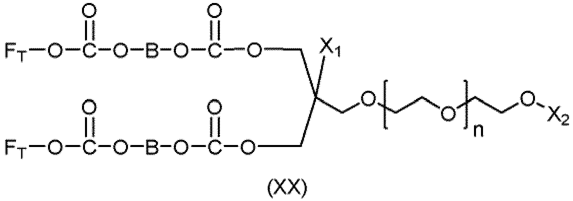
wobei
(i) F_{T} eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n eine ganze Zahl von 5 bis 100 ist; 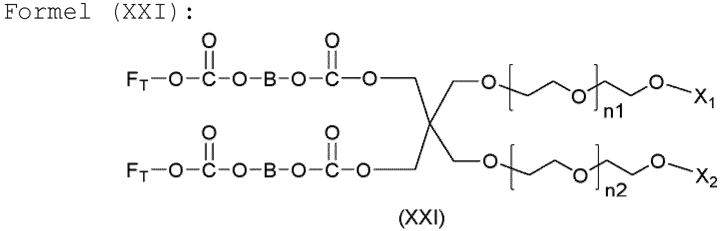
wobei
(i) F_{T} jeweils eine Polyfluororganogruppe ist, bei der es sich um eine Kohlenwasserstoffgruppe handelt, die gegebenenfalls durch ein, zwei oder drei nicht benachbarte Sauerstoffatome unterbrochen sein kann, in der zwei bis neunundfünzig Wasserstoffatome durch Fluoratome ersetzt sind und die eins bis dreißig Kohlenstoffatome enthält;
(ii) X₁ und X₂ jeweils unabhängig H, CH₃ oder CH₂CH₃ sind;
(iii) B ein Polyalkylenoxid umfasst und
(iv) n1 und n2 jeweils unabhängig eine ganze Zahl von 5 bis 50 sind;
zur Verringerung der Bakterienwachstumsrate auf einer Polymeroberfläche durch Bereitstellen einer Polymeroberfläche, die die Zusammensetzung umfasst, und (ii) Inkontaktbringen der Polymeroberfläche mit einem antimikrobiellen Mittel, antiseptischen Mittel oder Desinfektionsmittel, wobei die Bakterienwachstumsrate gegenüber der aus dem Basispolymer in Abwesenheit der Verbindung gebildeten Polymeroberfläche um mindestens 50 % verringert ist, wobei die Bakterienwachstumsrate gemessen wird, indem man zu zwei verschiedenen Zeitpunkten Proben der Oberfläche nimmt, spült und die Oberflächenproben in Pufferlösung beschallt, um anhaftende Bakterien abzulösen, dann die entfernten Bakterien durch Ausplattieren der Lösungen auf Bakterienwachstumssubstrat, Inkubieren über Nacht bei 37 °C und Durchführen von Koloniezählungen quantifiziert und dann die Änderung der Bakterienzahl im Lauf der Zeit berechnet, wie in Beispiel 14 offenbart.

6. Verwendung nach einem der Ansprüche 1 bis 5, wobei es sich bei der Verbindung um eine der Verbindungen 1-57 handelt:

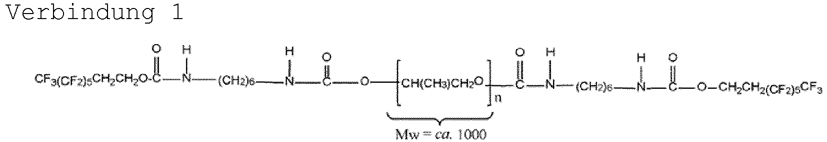

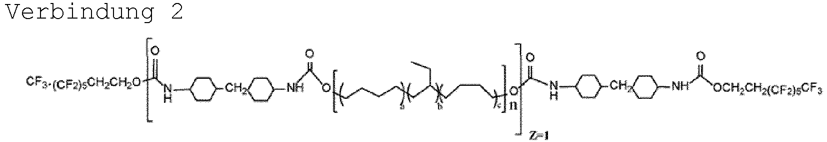

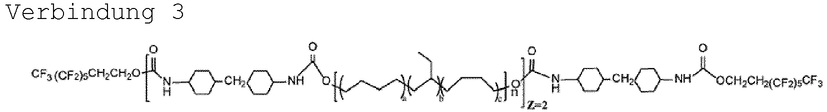

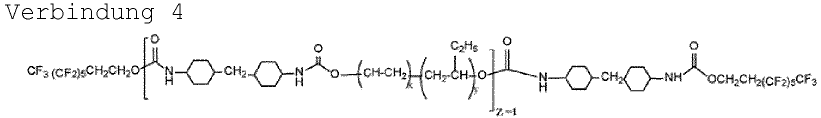

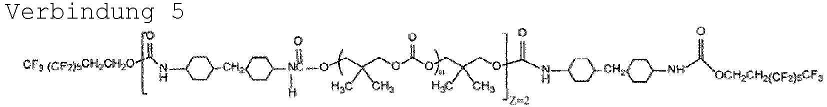

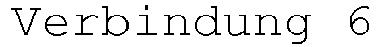

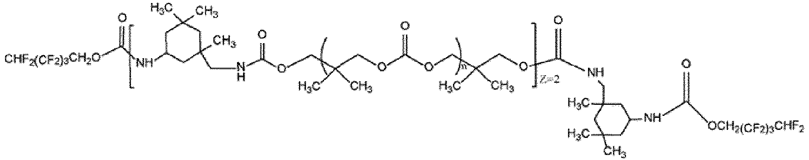

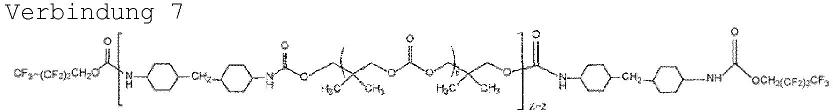

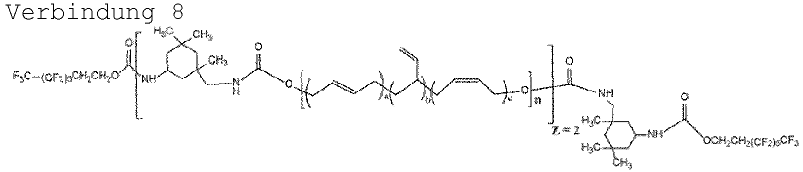

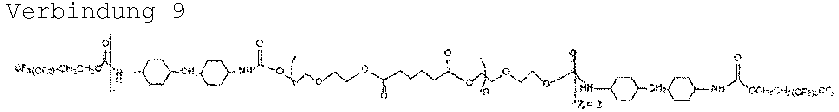

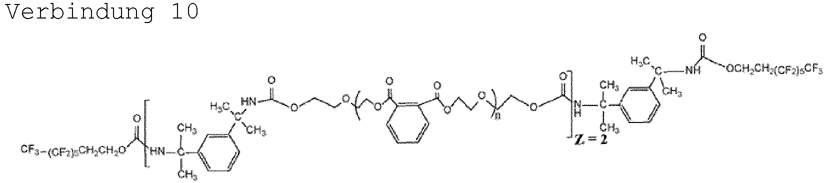

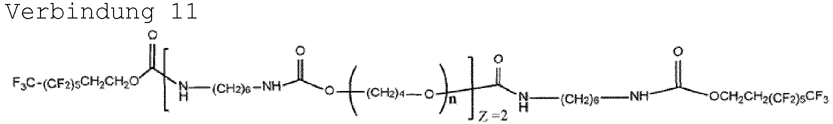

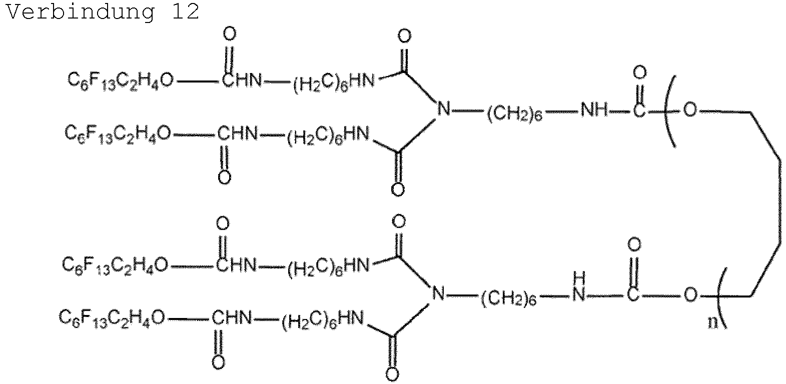

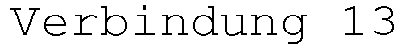

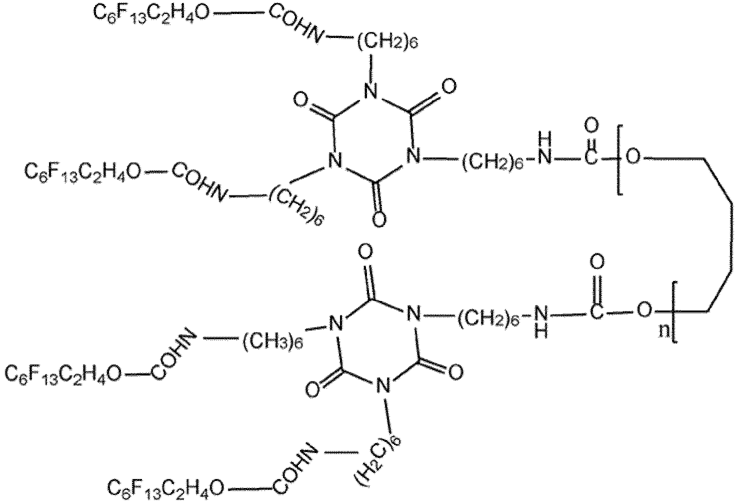

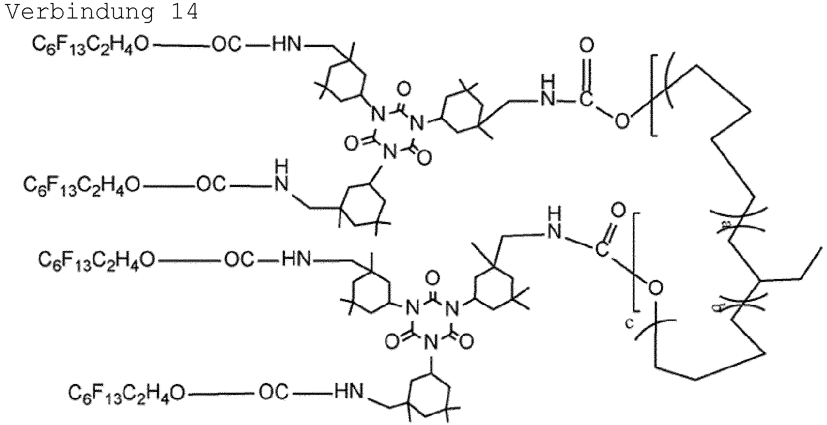

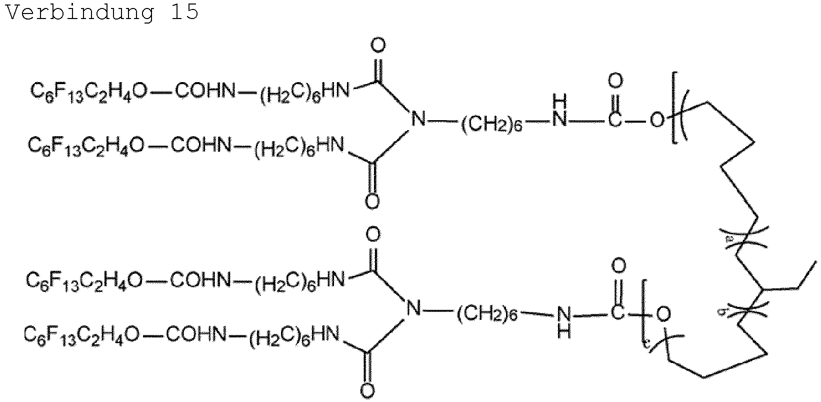

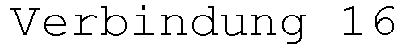

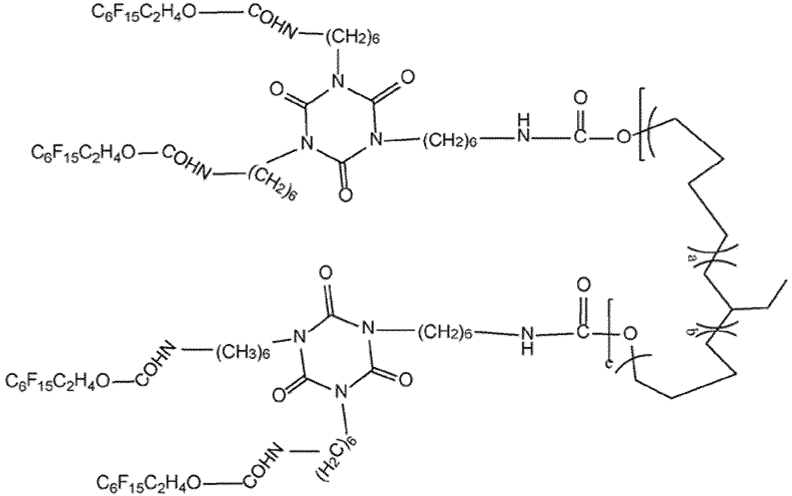

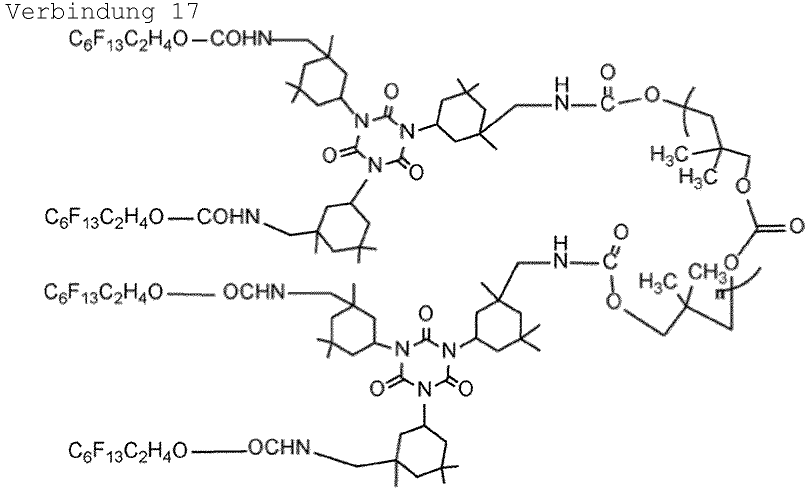

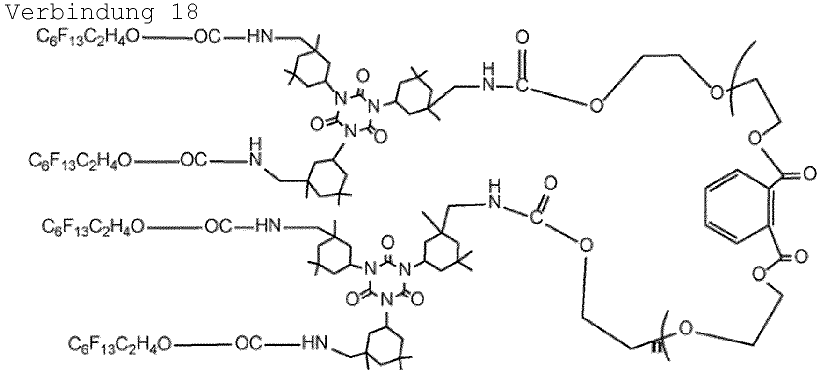

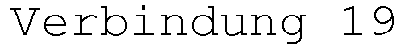

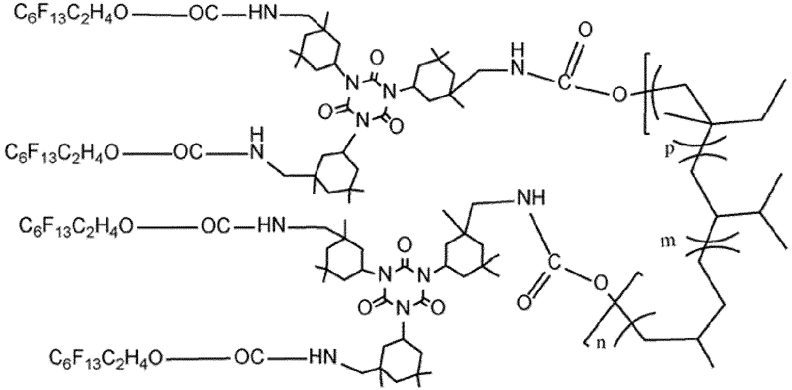

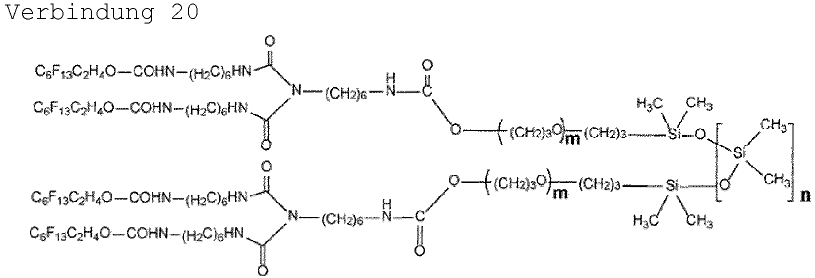

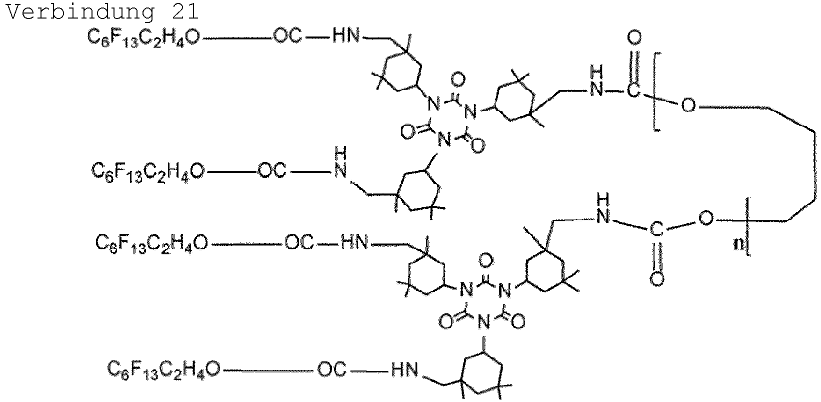

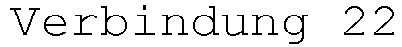

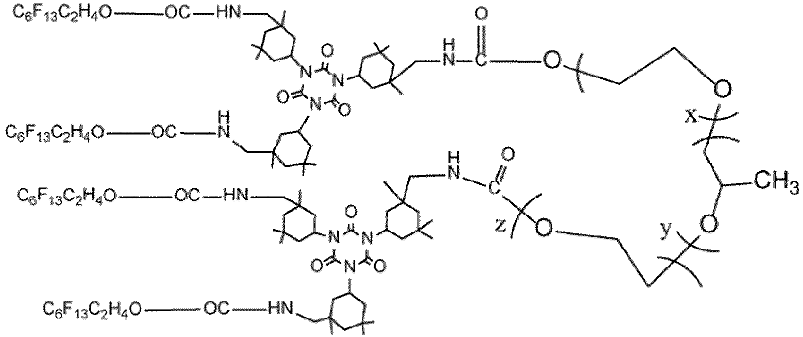

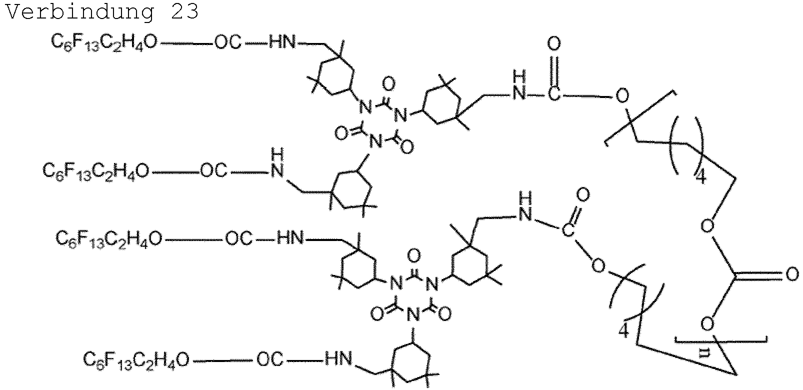

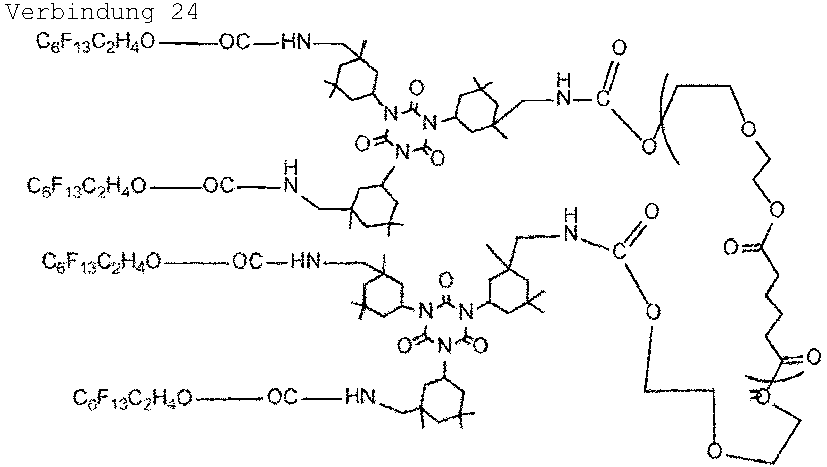

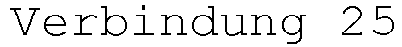

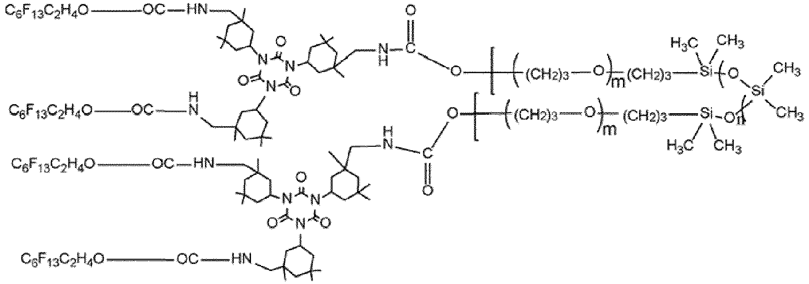

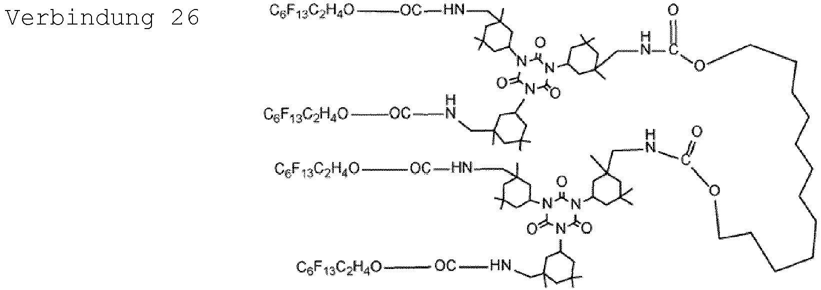

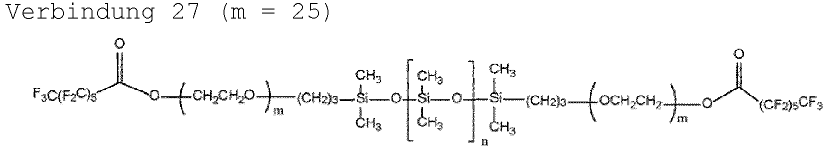

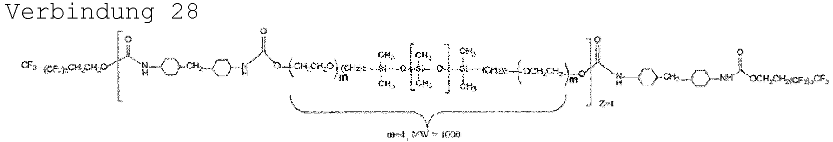

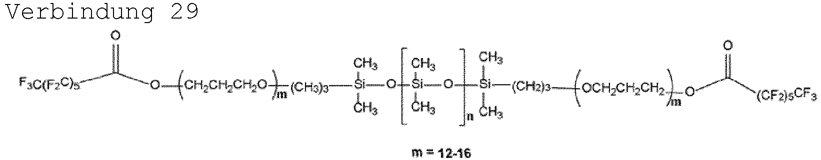

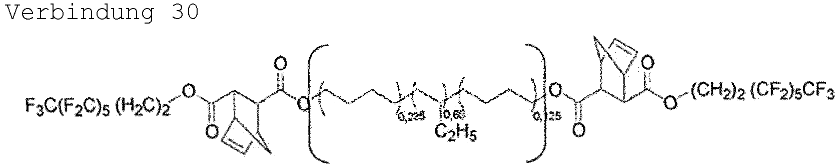

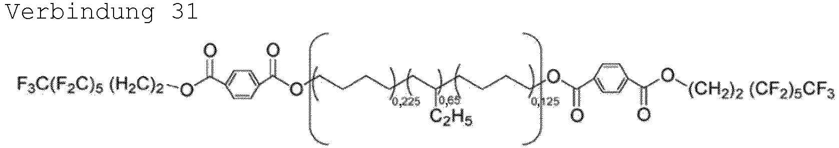

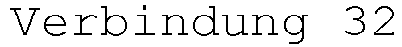

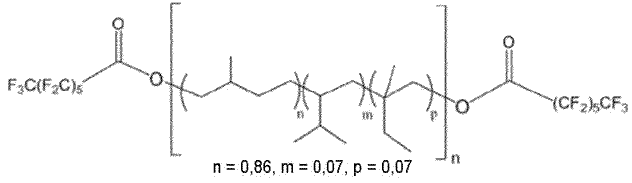

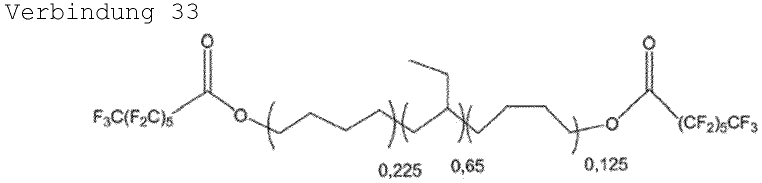

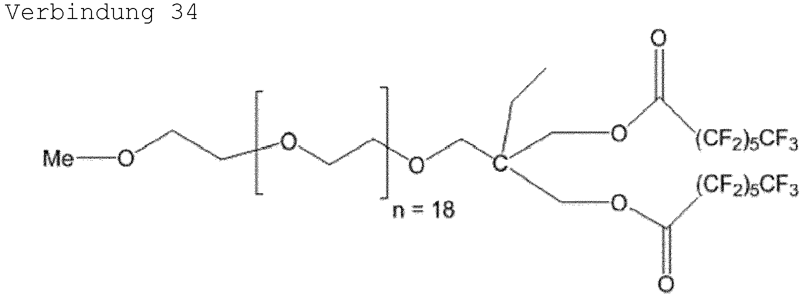

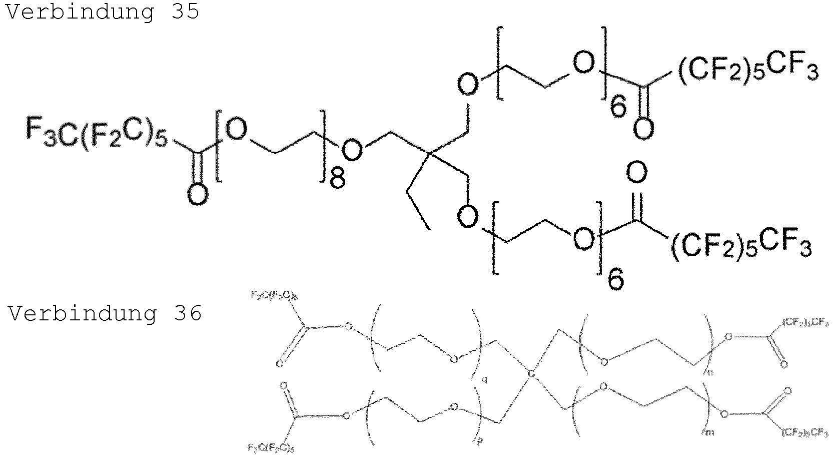

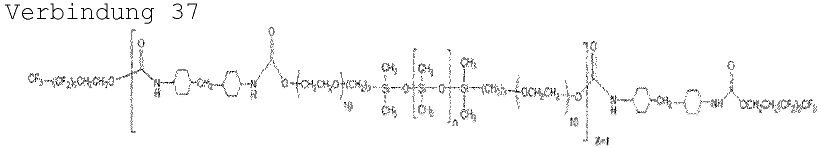

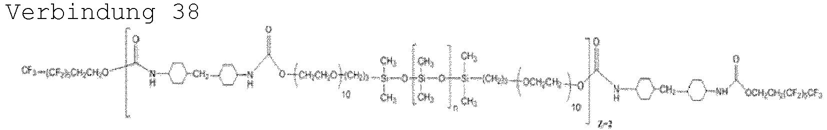

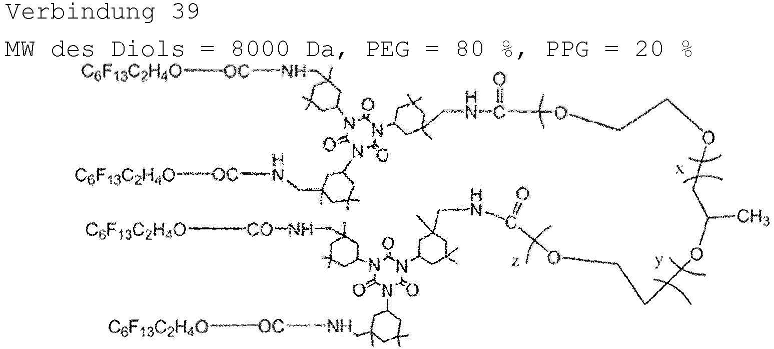

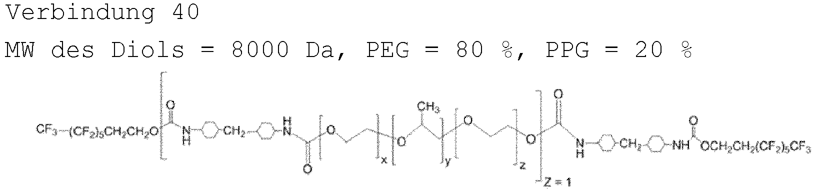

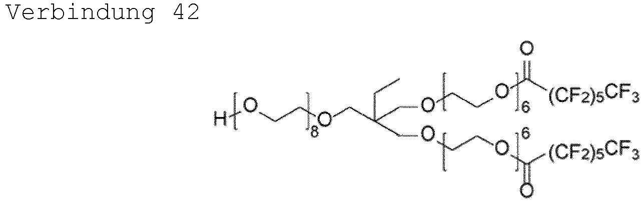

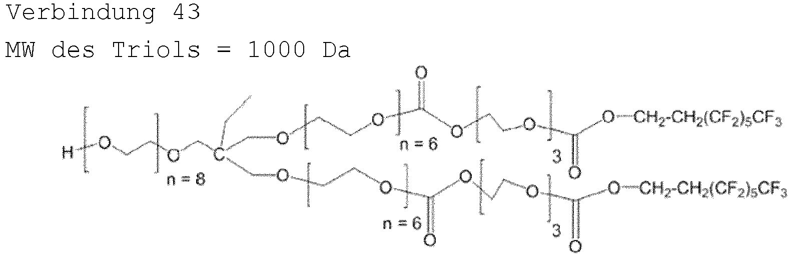

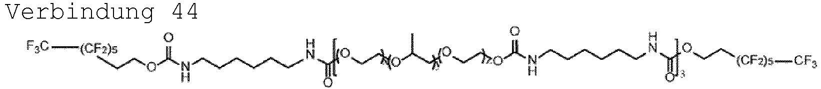

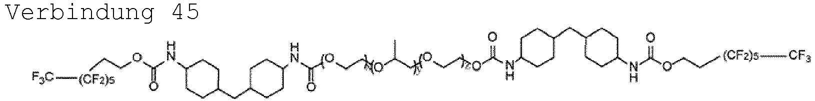

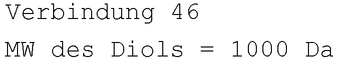

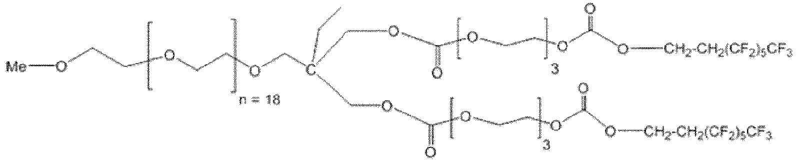

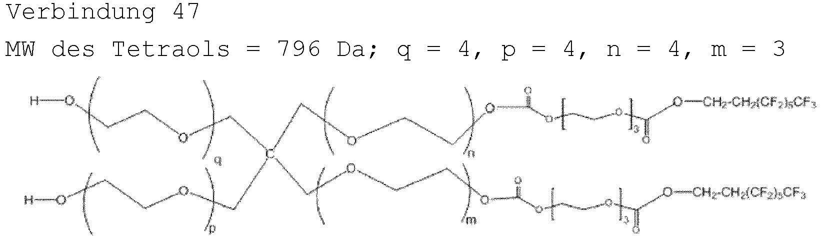

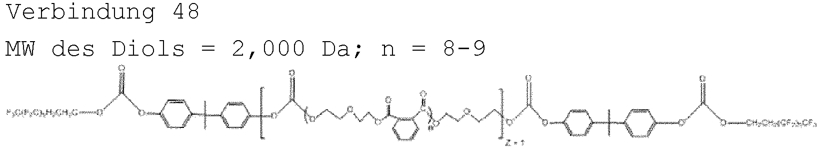

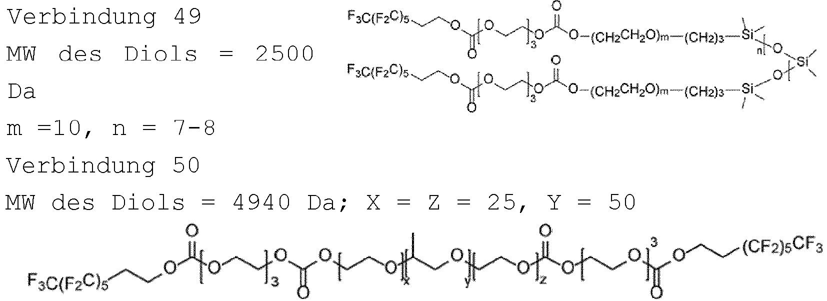

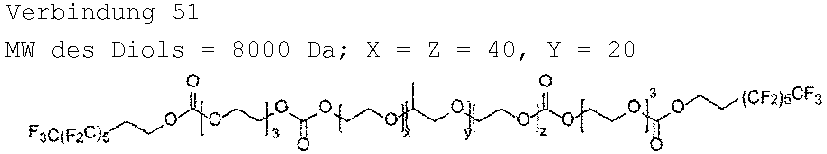

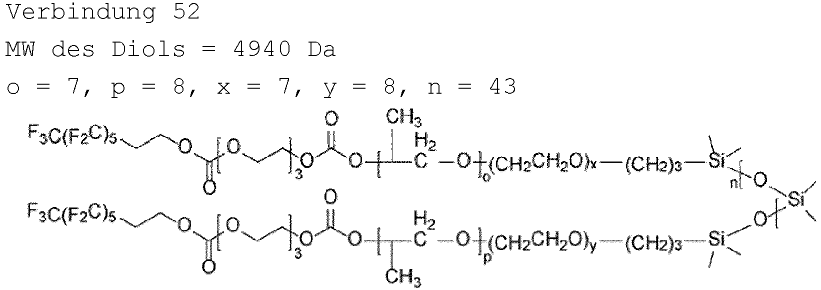

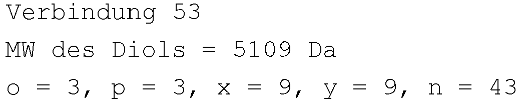

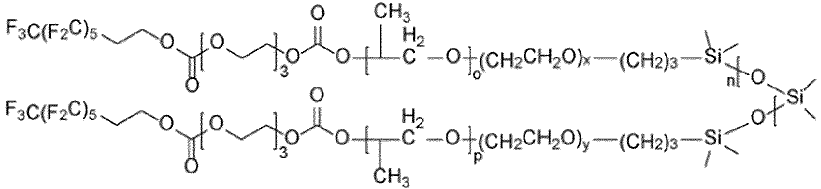

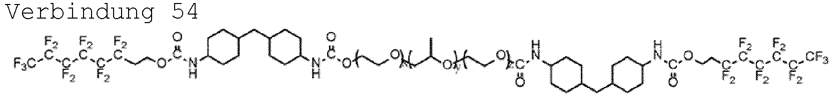

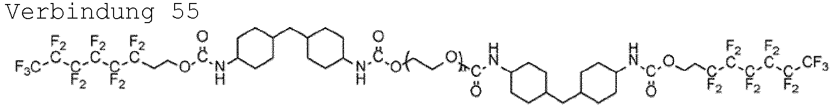

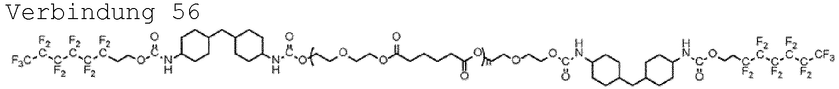

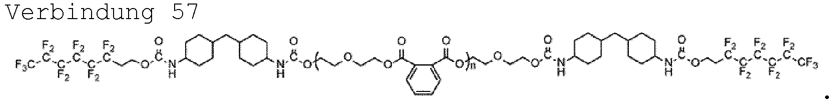

7. Verwendung nach einem der Ansprüche 1-6, wobei das Basispolymer
i) aus der Gruppe umfassend Silikon, Polyolefin, Polyester, Polycarbonat, Polysulfon, Polyamid, Polyether, Polyharnstoff, Polyurethan (PU), Polyetherimid, Polystyrol, Cellulosepolymer, Polypropylen (PP), Polyethylen (PE), Polyvinylchlorid (PVC), Polyvinylalkohol (PVA), Polyvinylpyrrolidon (PVP), Polyacrylamid (PAAM), Polyethylenoxid (PEO), Poly(ethylenoxid)-b-poly(propylenoxid)-b-poly(ethylenoxid), Poly(hydroxyethylmethacrylat) (PolyHEMA), Polyethylenterephthalat (PET), Polybutylenterephthalat (PBT), Polymethylmethacrylat (PMMA), Polyetheretherketon (PEEK), Polyether-b-Polyamid, einem Cycloolefinpolymer (COP), einem Cycloolefincopolymer (COC), Methylmethacrylat-Acrylnitril-Butadienyl-Styrol (MABS), Styrol-Acrylnitril (SAN), Styrol-Methylmethacrylat (SMMA), Methacrylat-Butadien-Styrol (MBS), Styrol-Butadien (SB), Poly(styrol-*block*-isobutylen-*block*-styrol) (SIBS) und Ethylen-Vinylacetat (EVA) ausgewählt ist oder
ii) Polyurethan (PU) ist oder
iii) Silikon (SI) ist.

8. Verwendung nach einem der Ansprüche 1 bis 7, wobei die Zusammensetzung in einem Gegenstand enthalten ist, der ausgewählt ist aus:
(a) einer medizinischen Vorrichtung, ausgewählt aus medizinischen Instrumenten, zahnmedizinischen Vorrichtungen, Zahnimplantaten, Arzneistoffzuführungsvorrichtungen, Transplantaten, Stents, Herzschrittmachern, implantierbaren Kardioverter-Defibrillatoren, Vorrichtungen für die kardiale Resynchronisationstherapie, Leitungen für kardiovaskuläre Vorrichtungen, Ventrikelunterstützungsvorrichtungen undantriebsleitungen, Herzklappen, Vena-cava-Filtern, endovaskulären Spulen, Kathetern, Katheteranschlüssen, Katheterventilen, Leitungen für die intravenöse Zufuhr, Verteiler für die intravenöse Zufuhr, Shunts, Wunddränagen, Dränagekathetern, Infusionsanschlüssen, Cochlea-Implantaten, Endotrachealtuben, Tracheostomietuben, Beatmungsschläuchen undkreisläufen, implantierbaren Sensoren, ophthalmischen Vorrichtungen, orthopädischen Vorrichtungen, Zahnimplantaten, Periodontalimplantaten, Brustimplantaten, Penisimplantaten, Kieferimplantaten, kosmetischen Implantaten, Ventilen, Geräten, Gerüsten, Nahtmaterial, Nadeln, Hernienreparaturnetzen, spannungsfreien Vaginalbändern und Vaginalschlingen, prostethischen neurologischen Vorrichtungen und Ohrschläuchen, oder
(b) einem Wundverband, einer Bandage, einer Gaze, einem Band, einem Kissen, einem Schwamm, einem Blutoxygenator, einem Beatmungsgerät, einer Pumpe, einem Schlauch, einem Draht, einer Elektrode, einem Verhütungsmittel, einem Damenhygieneprodukt, einem Endoskop, einer Dialysemembran, einem Führungsdraht, einem Flüssigkeitssammelbeutel, einem Arzneistoffzuführungsbeutel und -schlauch, einem Ernährungsschlauch, einen Blutbeutel oder Geräten für die Geweberegeneration oder Zellkultur oder
(c) einem Katheter, einem Dränagekatheter, einem Stent, einem Shunt, einem Infusionsanschluss, einer Leitung für die intravenöse Zufuhr, einer zahnmedizinischen Vorrichtung, einem Blutbeutel, einem Brustimplantat, einem Penisimplantat, einer Wunddränage, einem Ernährungsschlauch, einem Endotrachealtubus, einem Atemschlauch, einem Ohrschlauch oder einem Endoskop.

## Revendications

1. Utilisation d'une composition comprenant un polymère de base mélangé avec 0,05 % (p/p) à 15 % (p/p) d'un composé selon l'une quelconque des formules (I)-(XXI)
formule (I) :
**F_{T}-[B-A]ₙ-B-F_{T}** **(I)**
où
(i) A comprend un polybutadiène hydrogéné, un poly(carbonate de (2,2-diméthyl)-1,3-propylène), un polybutadiène, un poly(adipate de diéthylèneglycol), un poly(carbonate d'hexaméthylène), un poly(éthylène-co-butylène), un polyester (néopentylglycol-anhydride orthophtalique), un polyester (diéthylèneglycol-anhydride orthophtalique), un polyester (1,6-hexanediol-anhydride orthophtalique), ou un éthoxylate de bisphénol A ;
(ii) B est un segment comprenant un uréthane ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, et
(iv) n est un entier de 1 à 10 ;
formule (II) :
**F_{T}-[B-A]ₙ-B-F_{T}** **(II)**
où
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 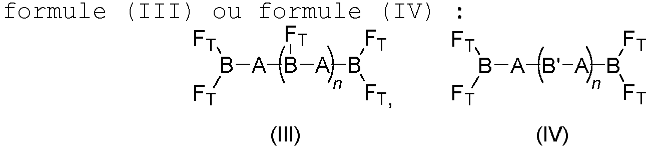
où
(i) A est un segment oligomérique contenant une liaison éther, une liaison ester, une liaison carbonate, ou un polyalkylène et ayant un poids moléculaire théorique allant de 500 à 3 500 g/mole ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier compris entre 0 et 10 ;
formule (V) :
**F_{T}-[B-A]ₙ-B-F_{T}** **(V)**
où
(i) A est un segment oligomérique comprenant poly(oxyde de propylène), poly(oxyde d'éthylène), ou poly(oxyde de tétraméthylène) et ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 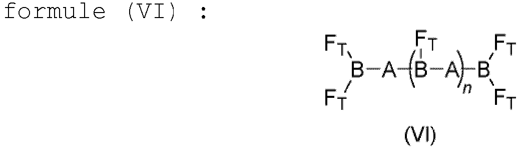
où
(i) A est un segment oligomérique comprenant poly(oxyde d'éthylène), poly(oxyde de propylène), poly(oxyde de tétraméthylène), ou un mélange correspondant, et ayant un poids moléculaire théorique allant de 500 à 3 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (VII) :
**F_{T}-[B-A]ₙ-B-F_{T}** **(VII)**
où
(i) A est un polycarbonate polyol ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 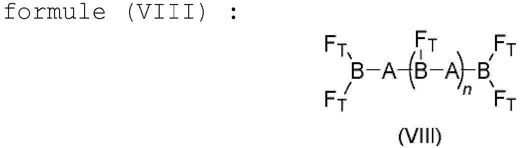
où
(i) A est un segment oligomérique comprenant un polycarbonate polyol ayant un poids moléculaire théorique allant de 500 à 3 000 g/mole ; ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 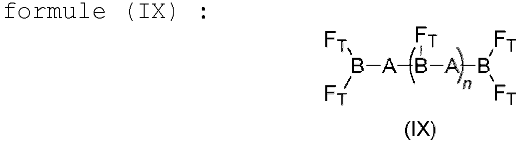
où
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (X) :
**F_{T}-[B-A]ₙ-B-F_{T}** **(X)**
où
(i) A est un segment choisi dans le groupe constitué par polybutadiène hydrogéné, polybutadiène, polyisoprène hydrogéné, copolymère à blocs de polysiloxane-polyéthylène glycol, et polystyrène et a un poids moléculaire théorique allant de 750 à 3 500 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 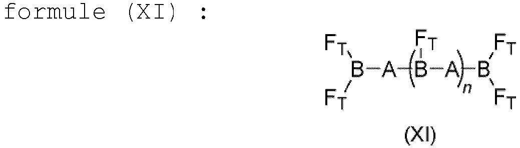
où
(i) A est polybutadiène hydrogéné, polybutadiène, polyisoprène hydrogéné, ou polystyrène et a un poids moléculaire théorique allant de 750 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 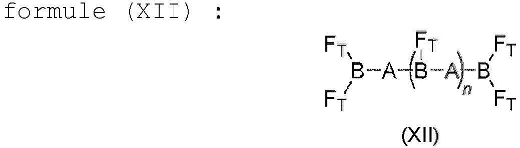
où
(i) A est un polyester ayant un poids moléculaire théorique allant de 500 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XIII) :
**F_{T}-A-F_{T}** **(XIII)**
où F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone et A est un segment oligomérique ramifié ou non ramifié de moins de 20 motifs répétitifs et est choisi parmi des segments de polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), poly(oxyde de tétraméthylène), ou polyéthylènebutylène ; 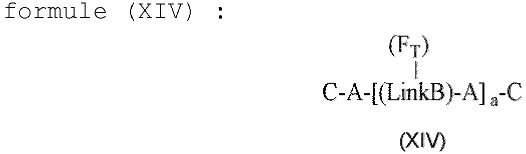 où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone fixés de manière covalente à LinkB ;
(ii) C est un groupe de terminaison de chaîne contenant une fonctionnalité amine, alcool ou acide carboxylique ;
(iii) A est un segment oligomérique ramifié ou non ramifié de moins de 20 motifs répétitifs choisis parmi polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), ou poly(oxyde de tétraméthylène) ;
(iv) LinkB est un segment de couplage ayant un poids moléculaire dans la plage de 40 à 700 choisi dans le groupe de diamines, diisocyanates, acides disulfoniques, acides dicarboxyliques, chlorures de diacides et dialdéhydes fonctionnalisés, où le composant fonctionnalisé possède un groupe fonctionnel secondaire ; et
(v) a est un entier supérieur à 0 ; 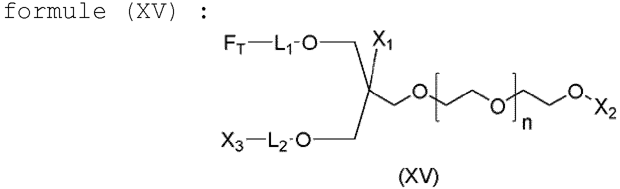
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; 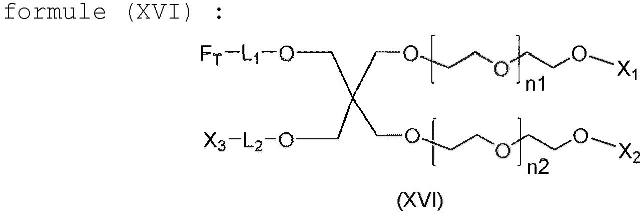
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ;
formule (XVII) :
**G-Aₘ-[B-A]ₙ-B-G** **(XVII)**
où
(i) chaque A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, à condition qu'au moins un G soit un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iv) n est un entier de 1 à 10 ; et
(v) m est 0 ou 1 ;
formule (XVIII) :
**F_{T}-OC(O)O-B-OC(O)O-[A-OC(O)O-B]ₙ-OC(O)O-F_{T}** **(XVIII)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) et est lié de manière covalente à B via une liaison carbonate ;
(ii) B comprend un poly(oxyde d'alkylène) ou un groupement décrit par la formule : 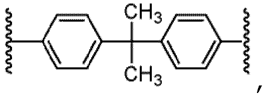 et est lié de manière covalente à A via une liaison carbonate ; et
(iii) F_{T} est un groupe actif en surface comprenant un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, où F_{T} est lié de manière covalente à B via une liaison carbonate ; et
(iv) n est un entier de 1 à 10 ;
formule (XIX) :
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) ;
(ii) F_{T} est un groupe actif en surface comprenant un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iii) m est un entier de 2 à 4 ; et
(iv) n est un entier de 1 à 10 ; 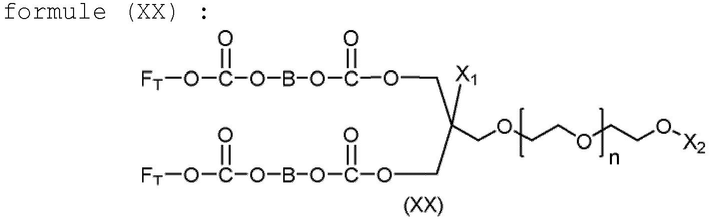
où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) n est un entier de 5 à 100 ; 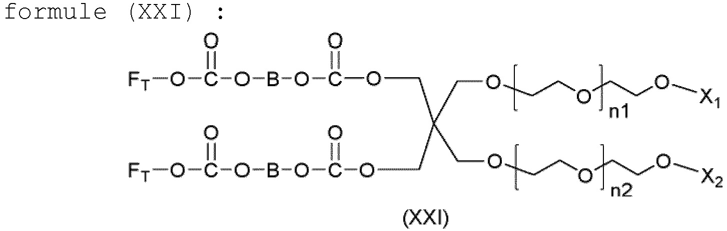
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) chacun parmi n1 et n2 est indépendamment un entier de 5 à 50 ;
pour la réduction de l'adhérence bactérienne à une surface polymérique formée à partir d'un polymère de base, l'adhérence bactérienne étant réduite d'au moins 50 % par rapport à la surface polymérique formée à partir du polymère de base en l'absence du composé, et l'adhérence bactérienne étant mesurée en exposant les surfaces de test à un inoculum bactérien dans un milieu aqueux pendant une quantité de temps spécifiée, puis le rinçage et la sonication des surfaces dans une solution tampon pour détacher des bactéries qui ont adhéré, et ensuite la quantification des bactéries retirées par placage des solutions sur un substrat de croissance bactérienne, l'incubation pendant la nuit à 37 °C, et la réalisation d'un comptage de colonie comme divulgué dans l'exemple 4.

2. Utilisation d'une composition comprenant un polymère de base mélangé avec 0,05 % (p/p) à 15 % (p/p) d'un composé selon l'une quelconque des formules (I)-(XXI)
formule (I) :
**F_{T}-[B-A]ₙ-B-F_{T}** **(I)**
où
(i) A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, et
(iv) n est un entier de 1 à 10 ;
formule (II) :
**F_{T}-[B-A]ₙ-B-F_{T}** **(II)**
où
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 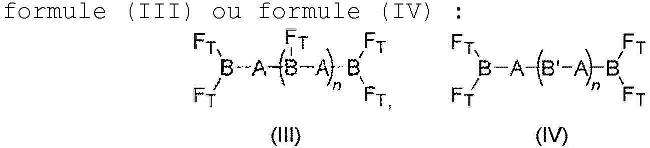
où
(i) A est un segment oligomérique contenant une liaison éther, une liaison ester, une liaison carbonate, ou un polyalkylène et ayant un poids moléculaire théorique allant de 500 à 3 500 g/mole ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier compris entre 0 et 10 ;
formule (V) :
**F_{T}-[B-A]ₙ-B-F_{T}** **(V)**
où
(i) A est un segment oligomérique comprenant poly(oxyde de propylène), poly(oxyde d'éthylène), ou poly(oxyde de tétraméthylène) et ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 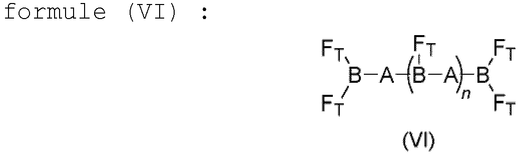
où
(i) A est un segment oligomérique comprenant poly(oxyde d'éthylène), poly(oxyde de propylène), poly(oxyde de tétraméthylène), ou un mélange correspondant, et ayant un poids moléculaire théorique allant de 500 à 3 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (VII) :
**F_{T}-[B-A]ₙ-B-F_{T}** **(VII)**
où
(i) A est un polycarbonate polyol ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 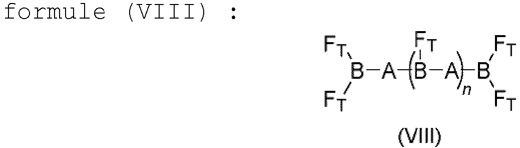
où
(i) A est un segment oligomérique comprenant un polycarbonate polyol ayant un poids moléculaire théorique allant de 500 à 3 000 g/mole ; ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 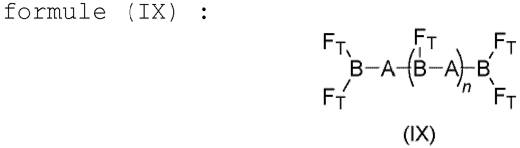
où
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (X) :
**F_{T}-[B-A]ₙ-B-F_{T}** **(X)**
où
(i) A est un segment choisi dans le groupe constitué par polybutadiène hydrogéné, polybutadiène, polyisoprène hydrogéné, copolymère à blocs de polysiloxane-polyéthylène glycol, et polystyrène et a un poids moléculaire théorique allant de 750 à 3 500 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 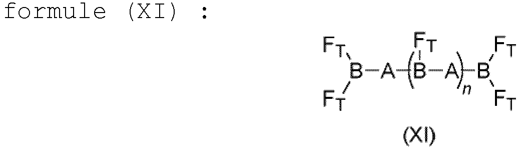
où
(i) A est polybutadiène hydrogéné, polybutadiène, polyisoprène hydrogéné, ou polystyrène et a un poids moléculaire théorique allant de 750 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XII) : 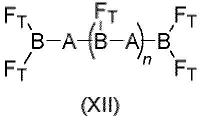 où
(i) A est un polyester ayant un poids moléculaire théorique allant de 500 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XIII) :
**F_{T}-A-F_{T}** **(XIII)**
où F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone et A est un segment oligomérique ramifié ou non ramifié de moins de 20 motifs répétitifs et choisi parmi des segments polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), poly(oxyde de tétraméthylène), ou polyéthylènebutylène ; 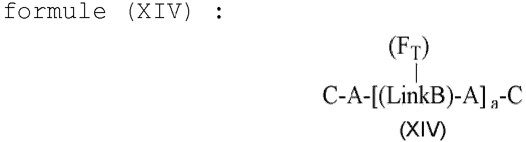 où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone fixés de manière covalente à LinkB ;
(ii) C est un groupe de terminaison de chaîne contenant une fonctionnalité amine, alcool ou acide carboxylique ;
(vi) A est un segment oligomérique ramifié ou non ramifié de moins de 20 motifs répétitifs choisis parmi polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), ou poly(oxyde de tétraméthylène) ;
(vii) LinkB est un segment de couplage ayant un poids moléculaire dans la plage de 40 à 700 choisi dans le groupe de diamines, diisocyanates, acides disulfoniques, acides dicarboxyliques, chlorures de diacides et dialdéhydes fonctionnalisés, où le composant fonctionnalisé possède un groupe fonctionnel secondaire ; et
(viii) a est un entier supérieur à 0 ; 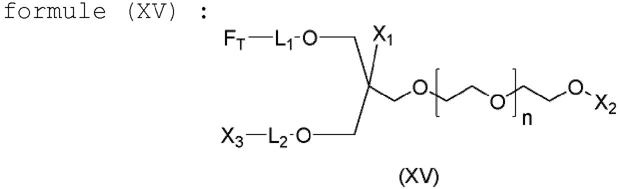
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; 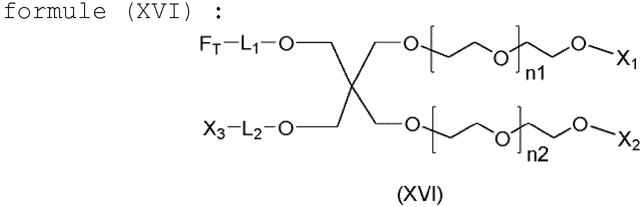
où
(i) chaque F_{T} est un groupe polyfluoroorganique, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ;
formule (XVII) :
**G - Aₘ - [B - A]ₙ - B - G** **(XVII)**
où
(i) chaque A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, à condition qu'au moins un G soit un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iv) n est un entier de 1 à 10 ; et
(v) m est 0 ou 1 ;
formule (XVIII) :
**F_{T}-OC(O)O-B-OC(O)O-[A-OC(O)O-B]ₙ-OC(O)O-F_{T}** **(XVIII)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) et est lié de manière covalente à B via une liaison carbonate ;
(ii) B comprend un poly(oxyde d'alkylène) ou un groupement décrit par la formule : 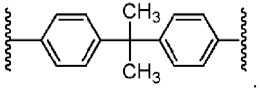 et est lié de manière covalente à A via une liaison carbonate ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, où F_{T} est lié de manière covalente à B via une liaison carbonate ; et
(iv) n est un entier de 1 à 10 ;
formule (XIX) :
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) ;
(ii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iii) m est un entier de 2 à 4 ; et
(iv) n est un entier de 1 à 10 ; 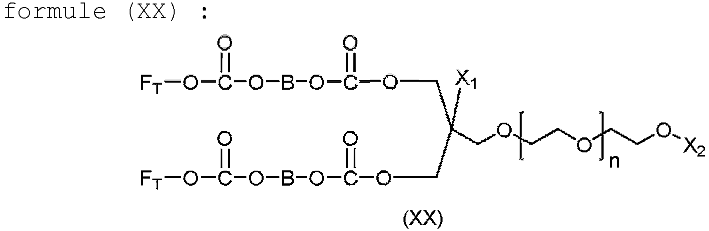
où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) n est un entier de 5 à 100 ; 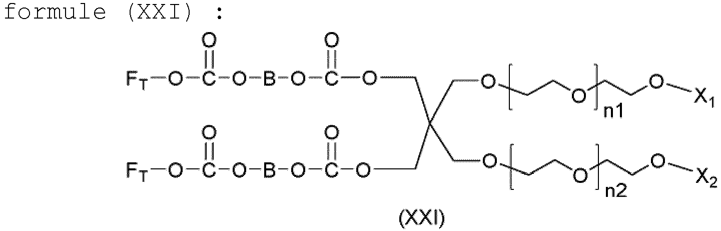
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) chacun parmi n1 et n2 est indépendamment un entier de 5 à 50 ;
pour la réduction d'une formation de sel médiée par des bactéries sur une surface polymérique d'un dispositif de logement d'urine en fournissant une surface polymérique formée à partir de la composition et (ii) la mise en contact de ladite surface polymérique avec de l'urine, un dépôt de sel étant réduit d'au moins 20 % dans des conditions de logement d'urine par rapport à la surface polymérique formée à partir du polymère de base en l'absence du composé, un dépôt de sel étant mesuré par une incubation de surfaces de tests dans une urine artificielle dans laquelle on a inoculé des bactéries de *P. mirabilis* pendant 14 jours, suivie par une dissolution des dépôts de sel résultants dans de l'acide nitrique dilué et une quantification de la teneur en calcium et en magnésium en utilisant une spectroscopie d'absorption atomique ou une spectroscopie de masse par plasma couplé de manière inductive comme divulgué dans l'exemple 22.

3. Utilisation d'une composition comprenant un polymère de base mélangé avec 0,05 % (p/p) à 15 % (p/p) d'un composé selon l'une quelconque des formules (I)-(XXI)
formule (I) :
F_{T}-[B-A]ₙ-B-F_{T} (I)
où
(i) A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, et
(iv) n est un entier de 1 à 10 ;
formule (II) :
**F_{T}-[B-A]ₙ-B-F_{T}** **(II)**
où
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 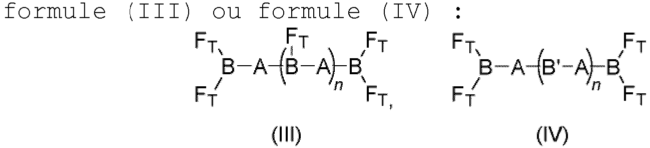
où
(i) A est un segment oligomérique contenant une liaison éther, une liaison ester, une liaison carbonate, ou un polyalkylène et ayant un poids moléculaire théorique allant de 500 à 3 500 g/mole ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier compris entre 0 et 10 ;
formule (V) :
**F_{T}-[B-A]ₙ-B-F_{T}** **(V)**
où
(i) A est un segment oligomérique comprenant poly(oxyde de propylène), poly(oxyde d'éthylène), ou poly(oxyde de tétraméthylène) et ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 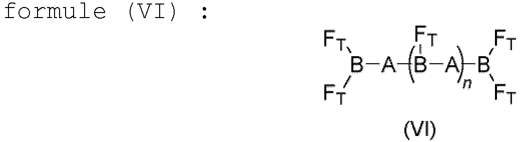
où
(i) A est un segment oligomérique comprenant poly(oxyde d'éthylène), poly(oxyde de propylène), poly(oxyde de tétraméthylène), ou un mélange correspondant, et ayant un poids moléculaire théorique allant de 500 à 3 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (VII) :
**F_{T}-[B-A]ₙ-B-F_{T}** **(VII)**
où
(i) A est un polycarbonate polyol ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 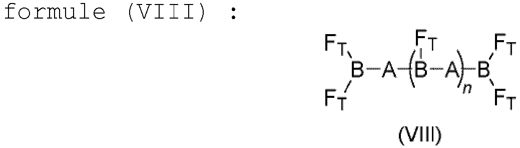
où
(i) A est un segment oligomérique comprenant un polycarbonate polyol ayant un poids moléculaire théorique allant de 500 à 3 000 g/mole ; ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 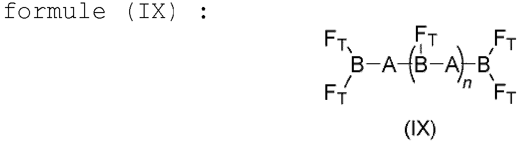
où
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (X) :
**F_{T}-[B-A]ₙ-B-F_{T}** **(X)**
où
(i) A est un segment choisi dans le groupe constitué par polybutadiène hydrogéné, polybutadiène, polyisoprène hydrogéné, copolymère à blocs de polysiloxane-polyéthylène glycol, et polystyrène et a un poids moléculaire théorique allant de 750 à 3 500 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 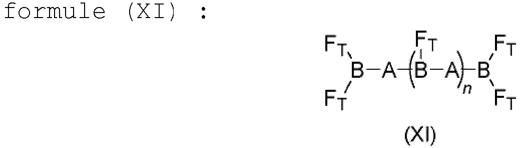
où
(i) A est polybutadiène hydrogéné, polybutadiène, polyisoprène hydrogéné, ou polystyrène et a un poids moléculaire théorique allant de 750 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 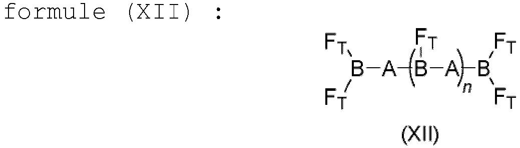
où
(i) A est un polyester ayant un poids moléculaire théorique allant de 500 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XIII) :
**F_{T}-A-F_{T}** **(XIII)**
où F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone et A est un segment oligomérique ramifié ou non ramifié de moins de 20 motifs répétitifs et choisi parmi des segments polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), poly(oxyde de tétraméthylène), ou polyéthylènebutylène ; 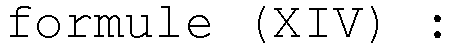 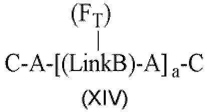 où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone fixés de manière covalente à LinkB ;
(ii) C est un groupe de terminaison de chaîne contenant une fonctionnalité amine, alcool ou acide carboxylique ;
(iii) A est un segment oligomérique ramifié ou non ramifié de moins de 20 motifs répétitifs choisis parmi polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), ou poly(oxyde de tétraméthylène) ;
(iv) LinkB est un segment de couplage ayant un poids moléculaire dans la plage de 40 à 700 choisi dans le groupe de diamines, diisocyanates, acides disulfoniques, acides dicarboxyliques, chlorures de diacides et dialdéhydes fonctionnalisés, où le composant fonctionnalisé possède un groupe fonctionnel secondaire ; et
(v) a est un entier supérieur à 0 ; 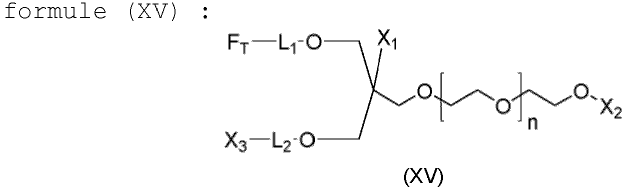
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; 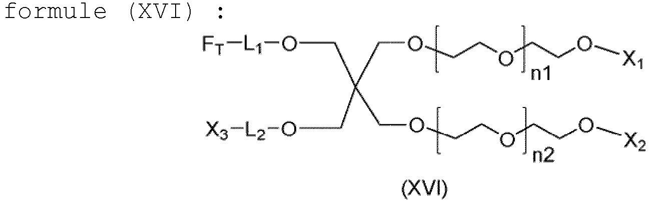
où
(i) chaque F_{T} est un groupe polyfluoroorganique, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ;
formule (XVII) :
**G - Aₘ - [B - A]ₙ - B - G** **(XVII)**
où
(i) chaque A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, à condition qu'au moins un G soit un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iv) n est un entier de 1 à 10 ; et
(v) m est 0 ou 1 ;
formule (XVIII) :
**F_{T}-OC(O)O-B-OC(O)O-[A-OC(O)O-B]ₙ-OC(O)O-F_{T}** **(XVIII)**
où
A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) et est lié de manière covalente à B via une liaison carbonate ;
(ii) B comprend un poly(oxyde d'alkylène) ou un groupement décrit par la formule : 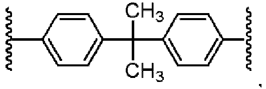 et est lié de manière covalente à A via une liaison carbonate ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, où F_{T} est lié de manière covalente à B via une liaison carbonate ; et
(iv) n est un entier de 1 à 10 ;
formule (XIX) :
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) ;
(ii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iii) m est un entier de 2 à 4 ; et
(iv) n est un entier de 1 à 10 ; 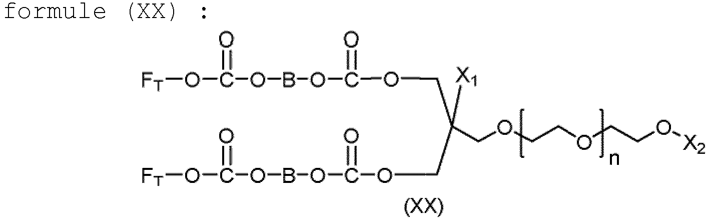
où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) n est un entier de 5 à 100 ; 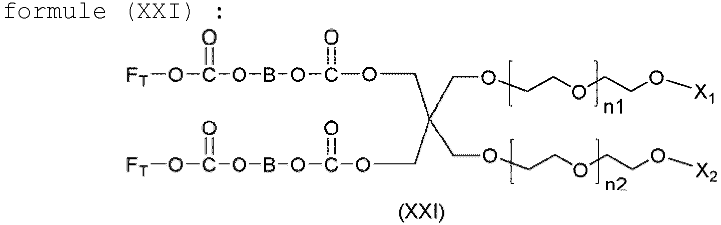
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) chacun parmi n1 et n2 est indépendamment un entier de 5 à 50 ;
pour la réduction de la formation d'un biofilm bactérien sur une surface polymérique en fournissant la surface polymérique formée à partir de la composition, la formation d'un biofilm bactérien étant réduite d'au moins 20 % par rapport à la surface polymérique formée à partir du polymère de base en l'absence du composé, et la formation d'un biofilm étant mesurée en exposant les surfaces de test à un inoculum bactérien dans un milieu aqueux dans des conditions qui permettent la formation d'un biofilm, puis le rinçage des surfaces, et ensuite la mesure de la couverture de surface de biofilm en utilisant une microscopie optique ou une microscopie électronique à balayage, ou la coloration du biofilm et la mesure de l'absorbance d'UV de la teinture extraite comme divulgué dans l'exemple 18.

4. Utilisation d'une composition comprenant un polymère de base mélangé avec 0,05 % (p/p) à 15 % (p/p) d'un composé selon l'une quelconque des formules (I)-(XXI)
formule (I) :
**F_{T}-[B-A]ₙ-B-F_{T}** **(I)**
où
(i) A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ;
formule (II) :
**F_{T}-[B-A]ₙ-B-F_{T}** **(II)**
où
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 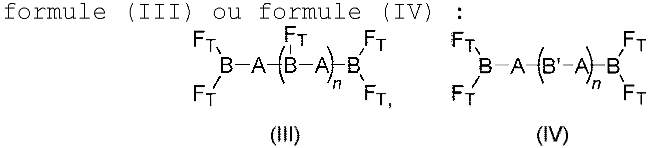
où
(i) A est un segment oligomérique contenant une liaison éther, une liaison ester, une liaison carbonate, ou un polyalkylène et ayant un poids moléculaire théorique allant de 500 à 3 500 g/mole ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier compris entre 0 et 10 ;
formule (V) :
**F_{T}-[B-A]ₙ-B-F_{T}** **(V)**
où
(i) A est un segment oligomérique comprenant poly(oxyde de propylène), poly(oxyde d'éthylène), ou poly(oxyde de tétraméthylène) et ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 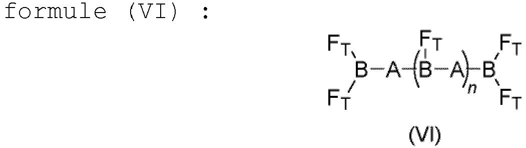
où
(i) A est un segment oligomérique comprenant poly(oxyde d'éthylène), poly(oxyde de propylène), poly(oxyde de tétraméthylène), ou un mélange correspondant, et ayant un poids moléculaire théorique allant de 500 à 3 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (VII) :
**F_{T}-[B-A]ₙ-B-F_{T}** **(VII)**
où
(i) A est un polycarbonate polyol ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 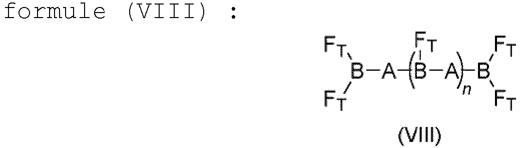
où
(i) A est un segment oligomérique comprenant un polycarbonate polyol ayant un poids moléculaire théorique allant de 500 à 3 000 g/mole ; ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 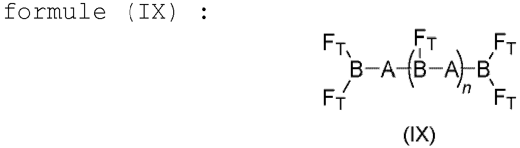
où
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (X) :
**F_{T}-[B-A]ₙ-B-F_{T}** **(X)**
où
(i) A est un segment choisi dans le groupe constitué par polybutadiène hydrogéné, polybutadiène, polyisoprène hydrogéné, copolymère à blocs de polysiloxane-polyéthylène glycol, et polystyrène et a un poids moléculaire théorique allant de 750 à 3 500 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 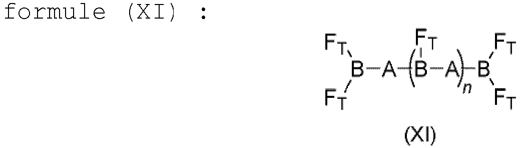
où
(i) A est polybutadiène hydrogéné, polybutadiène, polyisoprène hydrogéné, ou polystyrène et a un poids moléculaire théorique allant de 750 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 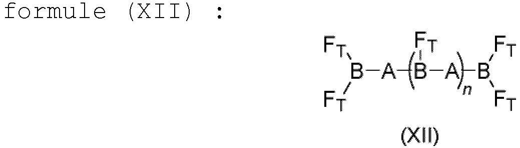
où
(i) A est un polyester ayant un poids moléculaire théorique allant de 500 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XIII) :
**F_{T}-A-F_{T}** **(XIII)**
où F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone et A est un segment oligomérique ramifié ou non ramifié de moins de 20 motifs répétitifs choisis parmi des segments polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), poly(oxyde de tétraméthylène), ou polyéthylènebutylène ; 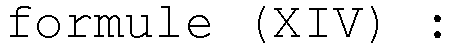 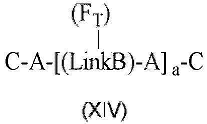 où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone fixés de manière covalente à LinkB ;
(ii) C est un groupe de terminaison de chaîne contenant une fonctionnalité amine, alcool ou acide carboxylique ;
(ix) A est un segment oligomérique ramifié ou non ramifié de moins de 20 motifs répétitifs choisis parmi polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), ou poly(oxyde de tétraméthylène) ;
(x) LinkB est un segment de couplage ayant un poids moléculaire dans la plage de 40 à 700 choisi dans le groupe de diamines, diisocyanates, acides disulfoniques, acides dicarboxyliques, chlorures de diacides et dialdéhydes fonctionnalisés, où le composant fonctionnalisé possède un groupe fonctionnel secondaire ; et
(xi) a est un entier supérieur à 0 ; 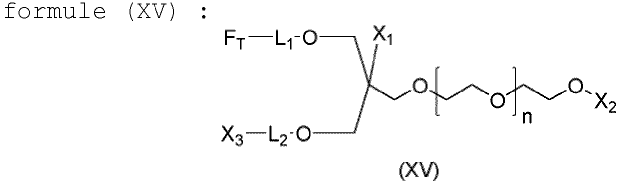
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; 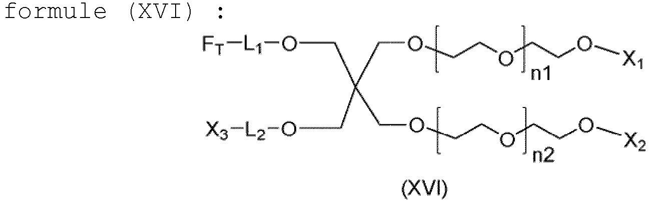
où
(i) chaque F_{T} est un groupe polyfluoroorganique, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ;
formule (XVII) :
**G - Aₘ - [B - A]ₙ - B - G** **(XVII)**
où
(i) chaque A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, à condition qu'au moins un G soit un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iv) n est un entier de 1 à 10 ; et
(v) m est 0 ou 1 ;
formule (XVIII) :
F_{T}-OC(O)O-B-OC(O)O-[A-OC(O)O-B]ₙ-OC(O)O-F_{T} (XVIII)
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylène glycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) et est lié de manière covalente à B via une liaison carbonate ;
(ii) B comprend un poly(oxyde d'alkylène) ou un groupement décrit par la formule : 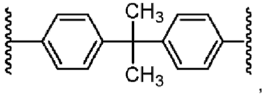 et est lié de manière covalente à A via une liaison carbonate ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, où F_{T} est lié de manière covalente à B via une liaison carbonate ; et
(iv) n est un entier de 1 à 10 ;
formule (XIX) :
**F_{T}-OC(O)O-(CH₂CH₂O)ₘ-OC(O)O-[A-OC(O)O-(CH₂CH₂O)ₘ]ₙ-OC(O)O-F_{T}** **(XIX)**
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylène glycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) ;
(ii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iii) m est un entier de 2 à 4 ; et
(iv) n est un entier de 1 à 10 ; 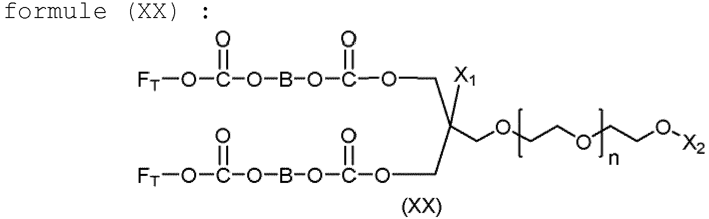
où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) n est un entier de 5 à 100 ; 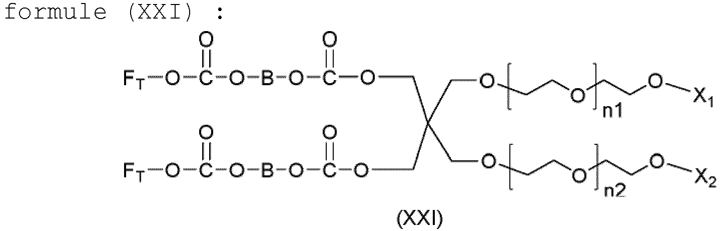
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) chacun parmi n1 et n2 est indépendamment un entier de 5 à 50 ;
pour la réduction de la biocharge bactérienne sur une surface polymérique formant la surface polymérique à partir de la composition, la biocharge étant réduite d'au moins 20 % par rapport à la surface polymérique formée à partir du polymère de base en l'absence du composé, et la biocharge bactérienne étant mesurée en exposant les surfaces de test à un inoculum bactérien dans un milieu aqueux pendant une quantité de temps spécifiée, puis le rinçage et la sonication des surfaces dans une solution tampon pour détacher des bactéries ayant adhéré, et ensuite la quantification des bactéries retirées par placage des solutions sur un substrat de croissance bactérienne, l'incubation pendant la nuit à 37 °C, et la réalisation d'un comptage de colonie comme divulgué dans l'exemple 4.

5. Utilisation d'une composition comprenant un polymère de base mélangé avec 0,05 % (p/p) à 15 % (p/p) d'un composé selon l'une quelconque des formules (I)-(XXI)
formule (I) :
**F_{T}-[B-A]ₙ-B-F_{T}** **(I)**
où
(i) A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ;
formule (II) :
**F_{T}-[B-A]ₙ-B-F_{T}** **(II)**
où
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 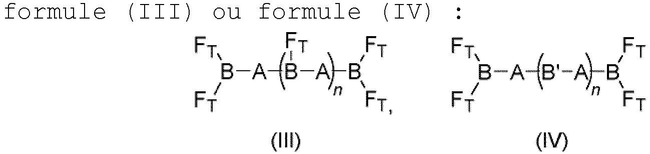
où
(i) A est un segment oligomérique contenant une liaison éther, une liaison ester, une liaison carbonate, ou un polyalkylène et ayant un poids moléculaire théorique allant de 500 à 3 500 g/mole ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier compris entre 0 et 10 ;
formule (V) :
**F_{T}-[B-A]ₙ-B-F_{T}** **(V)**
où
(i) A est un segment oligomérique comprenant poly(oxyde de propylène), poly(oxyde d'éthylène), ou poly(oxyde de tétraméthylène) et ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 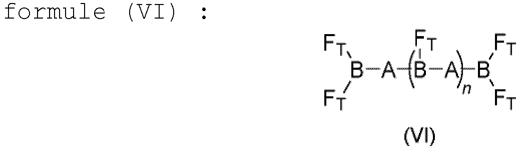
où
(i) A est un segment oligomérique comprenant poly(oxyde d'éthylène), poly(oxyde de propylène), poly(oxyde de tétraméthylène), ou un mélange correspondant, et ayant un poids moléculaire théorique allant de 500 à 3 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (VII) :
**F_{T}-[B-A]ₙ-B-F_{T}** **(VII)**
où
(i) A est un polycarbonate polyol ayant un poids moléculaire théorique allant de 500 à 3 000 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 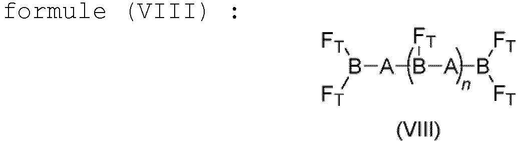
où
(i) A est un segment oligomérique comprenant un polycarbonate polyol ayant un poids moléculaire théorique allant de 500 à 3 000 g/mole ; ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 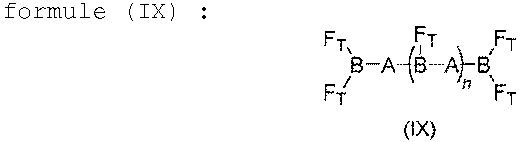
où
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (X) :
**F_{T}-[B-A]ₙ-B-F_{T}** **(X)**
où
(i) A est un segment choisi dans le groupe constitué par polybutadiène hydrogéné, polyisoprène hydrogéné, copolymère à blocs de polysiloxane-polyéthylène glycol, et polystyrène et a un poids moléculaire théorique allant de 750 à 3 500 g/mole ;
(ii) B est un segment formé à partir d'un diisocyanate ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 1 à 10 ; 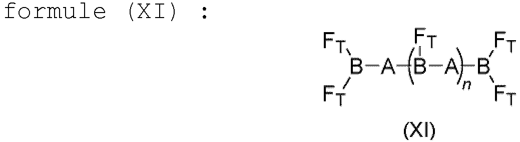
où
(i) A est polybutadiène hydrogéné, polybutadiène, polyisoprène hydrogéné, ou polystyrène et a un poids moléculaire théorique allant de 750 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ; 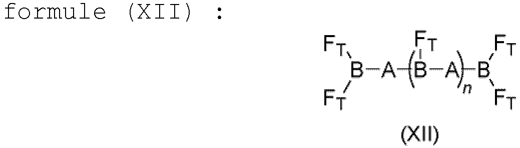
où
(i) A est un polyester ayant un poids moléculaire théorique allant de 500 à 3 500 g/mole ;
(ii) B est un segment comprenant un trimère d'isocyanurate ou un trimère de biuret ;
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ; et
(iv) n est un entier de 0 à 10 ;
formule (XIII) :
**F_{T}-A-F_{T}** **(XIII)**
où F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone et A est un segment oligomérique ramifié ou non ramifié de moins de 20 motifs répétitifs et choisi parmi des segments polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), poly(oxyde de tétraméthylène), ou polyéthylènebutylène ; 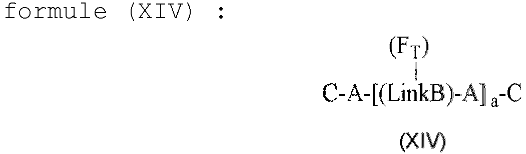 où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone fixés de manière covalente à LinkB ;
(ii) C est un groupe de terminaison de chaîne contenant une fonctionnalité amine, alcool ou acide carboxylique ;
(iii) A est un segment oligomérique ramifié ou non ramifié de moins de 20 motifs répétitifs choisis parmi polyuréthane, polyurée, polyamide, poly(oxyde d'alkylène), polycarbonate, polyester, polylactone, polysilicone, polyéthersulfone, polyoléfine, dérivé de polyvinyle, polypeptide, polysaccharide, polysiloxane, polydiméthylsiloxane, polyéthylène-butylène, polyisobutylène, polybutadiène, poly(oxyde de propylène), poly(oxyde d'éthylène), ou poly(oxyde de tétraméthylène) ;
(iv) LinkB est un segment de couplage ayant un poids moléculaire dans la plage de 40 à 700 choisi dans le groupe de diamines, diisocyanates, acides disulfoniques, acides dicarboxyliques, chlorures de diacides et dialdéhydes fonctionnalisés, où le composant fonctionnalisé possède un groupe fonctionnel secondaire ; et
(v) a est un entier supérieur à 0 ; 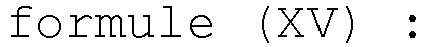 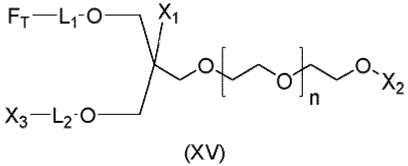
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; 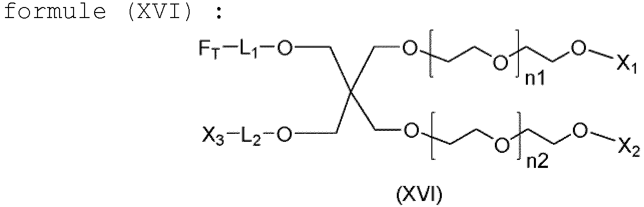
où
(i) chaque F_{T} est un groupe polyfluoroorganique, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ;
formule (XVII) :
G - Aₘ - [B - A]ₙ - B - G (XVII)
où
(i) chaque A comprend polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, à condition qu'au moins un G soit un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iv) n est un entier de 1 à 10 ; et
(v) m est 0 ou 1 ;
formule (XVIII) :
F_{T}-OC(O)O-B-OC(O)O-[A-OC(O)O-B]ₙ-OC(O)O-F_{T} (XVIII)
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) et est lié de manière covalente à B via une liaison carbonate ;
(ii) B comprend un poly(oxyde d'alkylène) ou un groupement décrit par la formule : 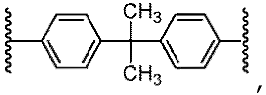 et est lié de manière covalente à A via une liaison carbonate ; et
(iii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone, où F_{T} est lié de manière covalente à B via une liaison carbonate ; et
(iv) n est un entier de 1 à 10 ;
formule (XIX) :
F_{T}-OC(O)O-(CH₂CH₂O)ₘ-OC(O)O-[A-OC(O)O-(CH₂CH₂O)ₘ]ₙ-OC(O)O-F_{T} (XIX)
où
(i) A comprend un segment souple choisi parmi polybutadiène hydrogéné, polyisoprène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, éthoxylate de bisphénol A, poly(oxyde d'éthylène)-b-poly(oxyde de propylène)-b-poly(oxyde d'éthylène), poly(oxyde d'éthylène), poly(oxyde de propylène), ou poly(oxyde de tétraméthylène) ;
(ii) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(iii) m est un entier de 2 à 4 ; et
(iv) n est un entier de 1 à 10 ; 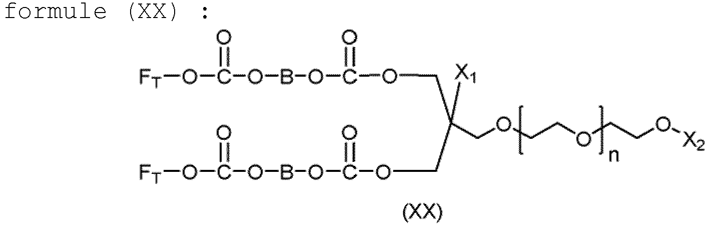
où
(i) F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) n est un entier de 5 à 100 ; 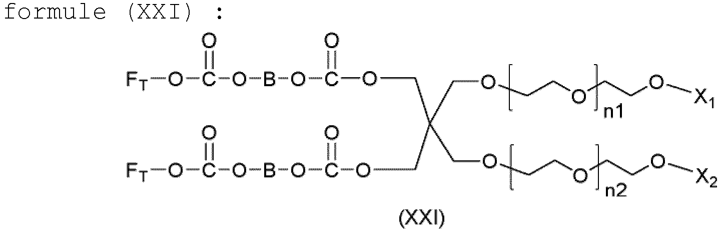
où
(i) chaque F_{T} est un groupe polyfluoroorgano, qui est un groupe hydrocarboné qui peut éventuellement être interrompu par un, deux ou trois atomes d'oxygène non contigus, dans lequel de deux à cinquante-neuf atomes d'hydrogène sont remplacés par des atomes de fluor et qui contient un à trente atomes de carbone ;
(ii) chacun parmi X₁ et X₂ est indépendamment H, CH₃, ou CH₂CH₃ ;
(iii) B comprend un poly(oxyde d'alkylène) ; et
(iv) chacun parmi n1 et n2 est indépendamment un entier de 5 à 50 ;
pour la réduction de la vitesse d'une croissance bactérienne sur une surface polymérique en fournissant une surface polymérique comprenant la composition, et (ii) la mise en contact de la surface polymérique avec un agent antimicrobien, un agent antiseptique ou un désinfectant, la vitesse d'une croissance bactérienne étant réduite d'au moins 50 % par rapport à la surface polymérique formée à partir du polymère de base en l'absence du composé, la vitesse d'une croissance bactérienne étant mesurée en échantillonnant la surface en deux points dans le temps, le rinçage et la sonication des échantillons de surface dans une solution tampon pour détacher des bactéries qui ont adhéré, ensuite la quantification des bactéries retirées par placage des solutions sur un substrat de croissance bactérienne, l'incubation pendant la nuit à 37 °C, et la réalisation d'un comptage de colonie, et ensuite le calcul du changement du nombre de bactéries dans le temps comme divulgué dans l'exemple 14.

6. Utilisation selon l'une quelconque des revendications 1-5, le composé étant l'un quelconque parmi les composés 1-57

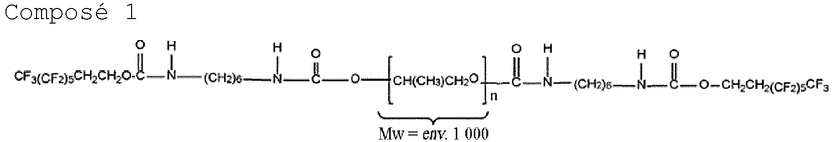

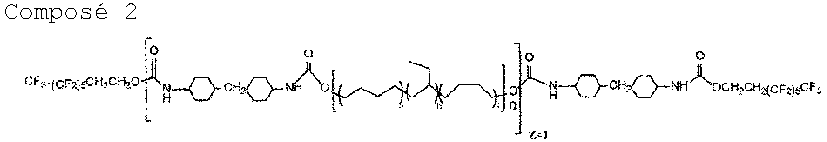

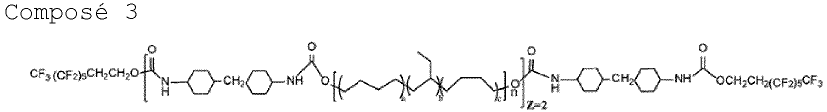

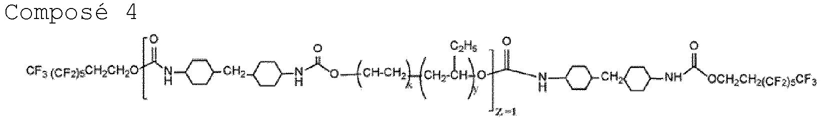

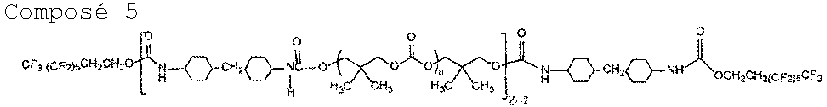

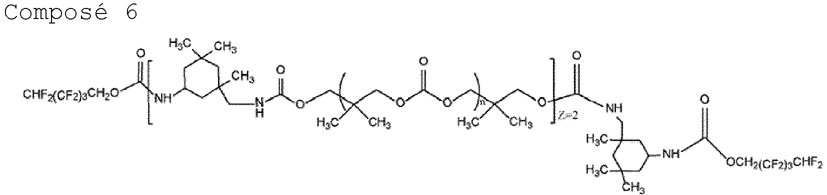

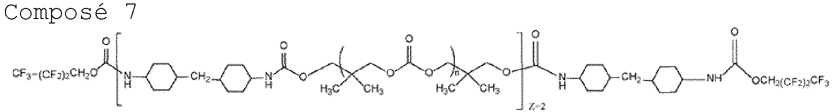

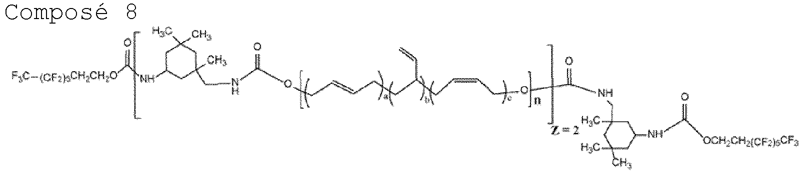

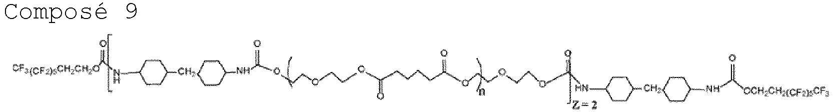

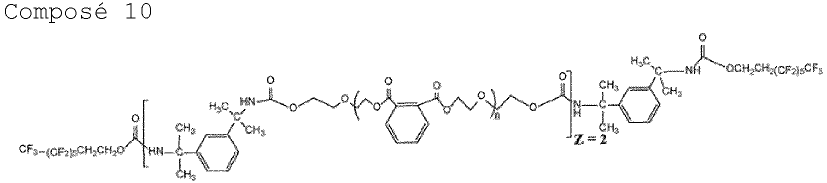

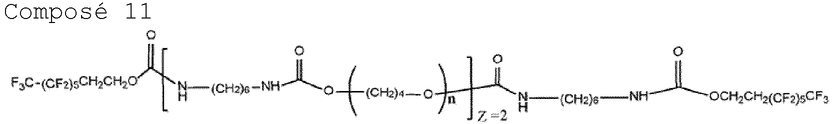

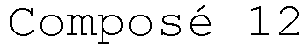

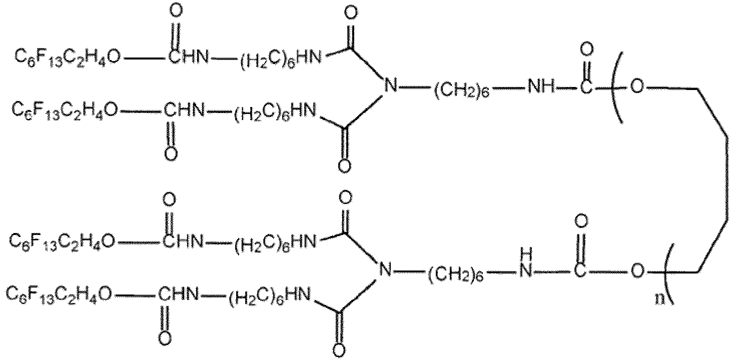

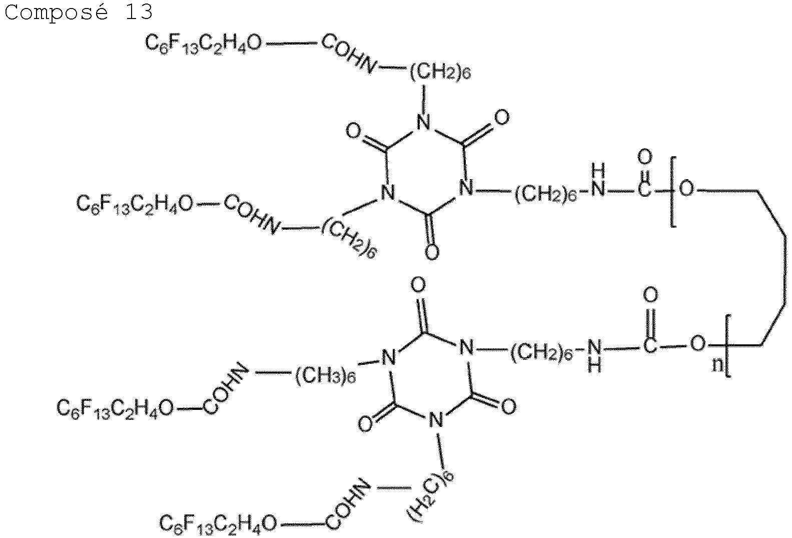

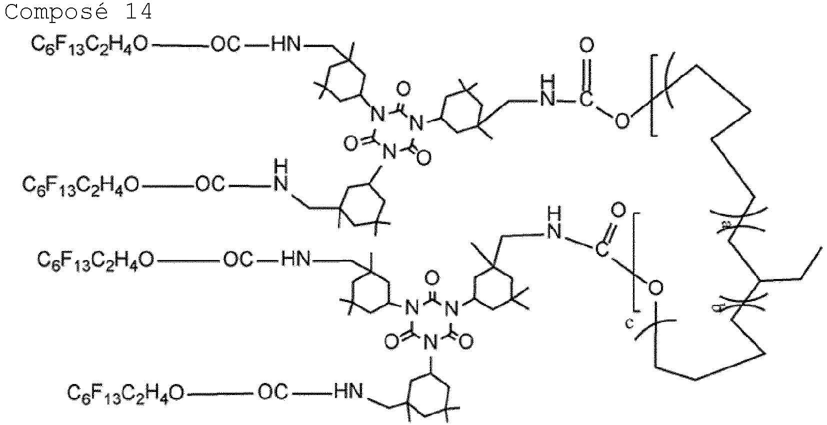

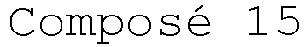

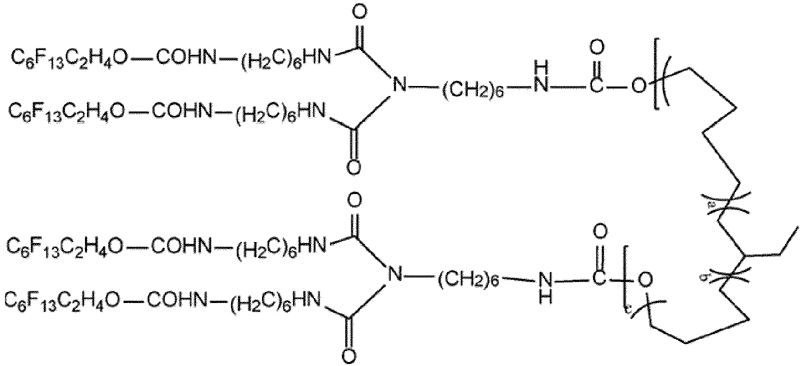

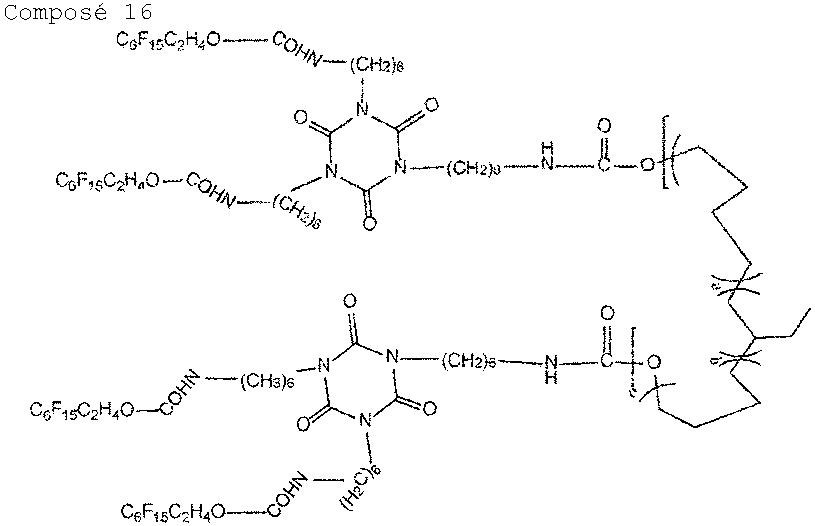

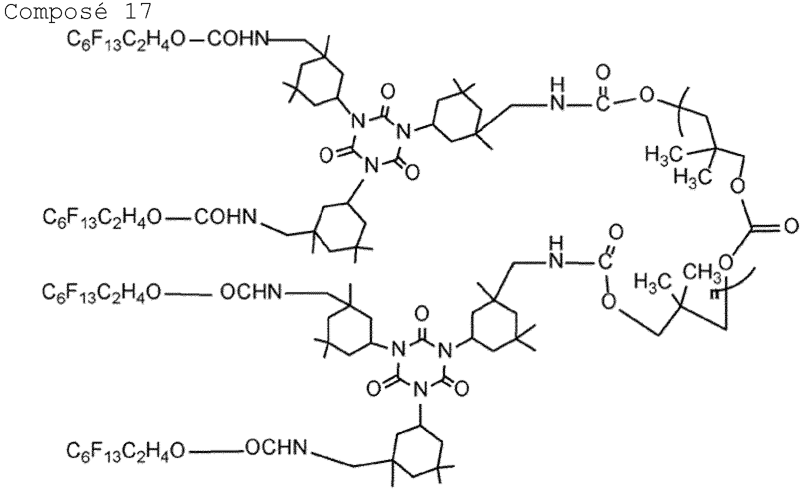

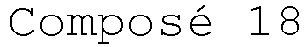

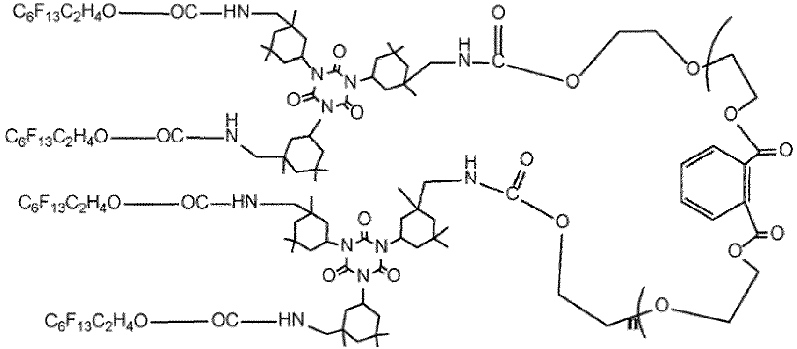

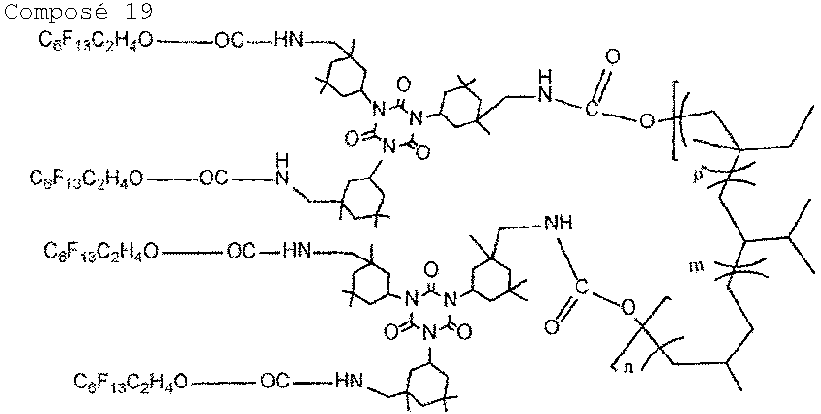

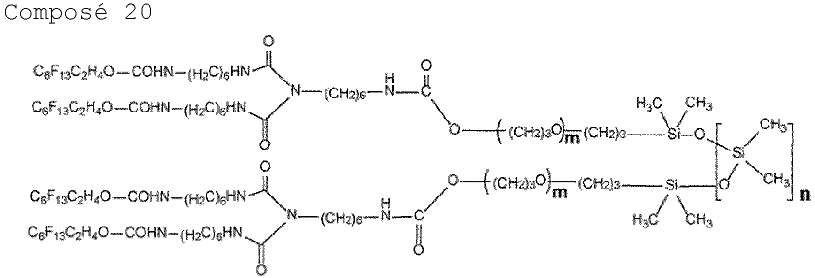

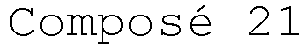

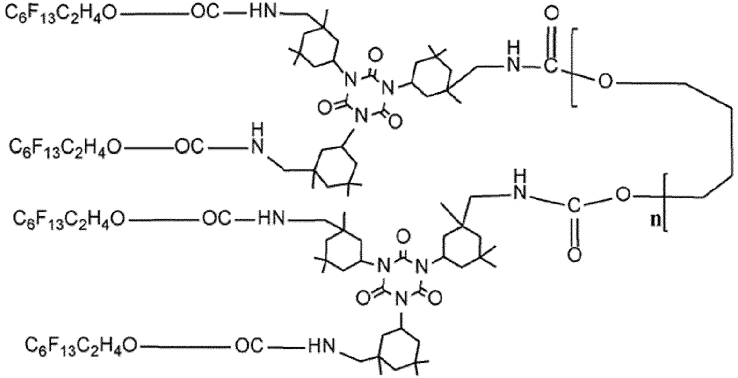

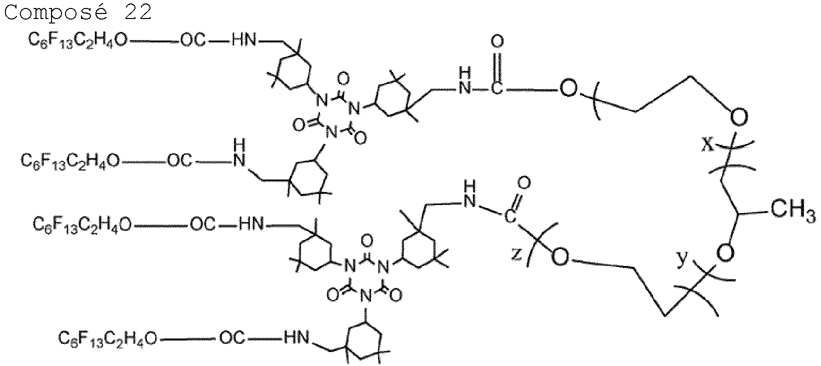

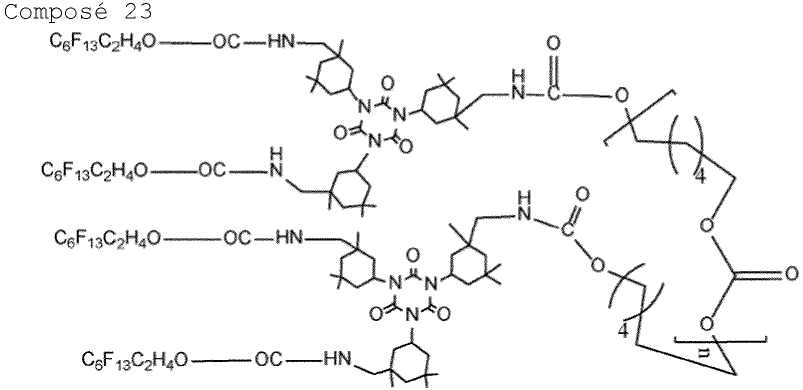

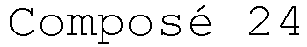

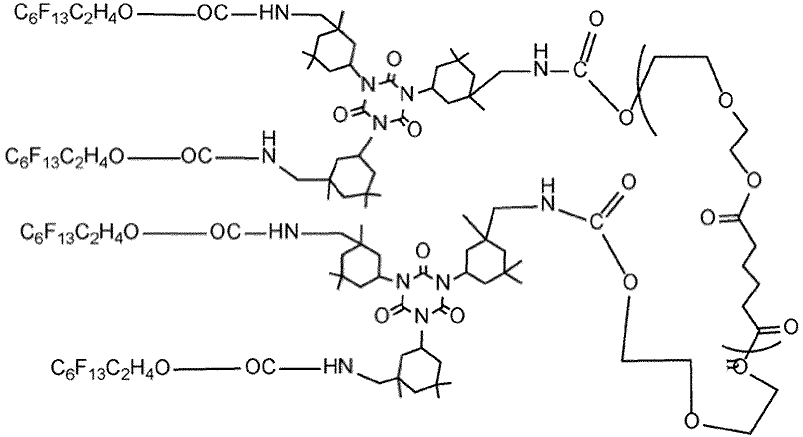

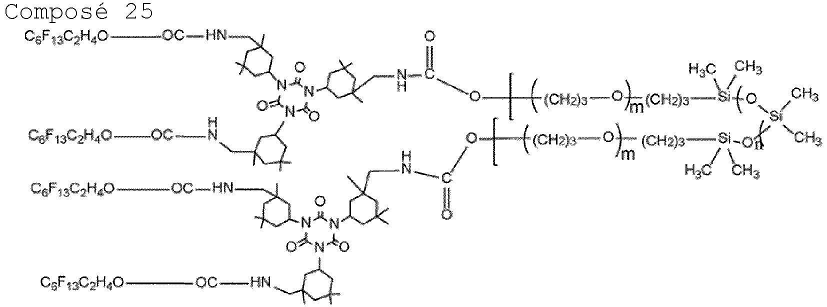

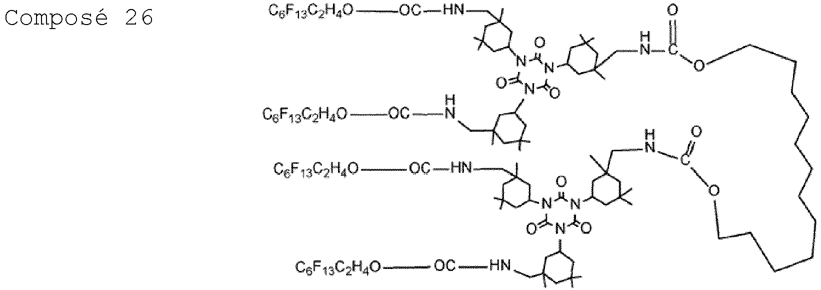

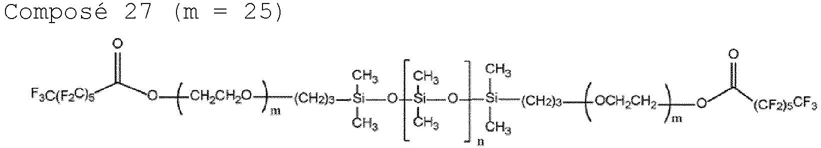

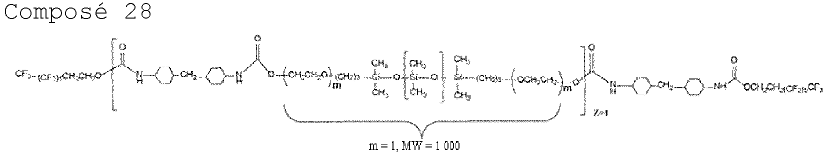

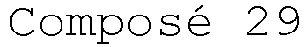

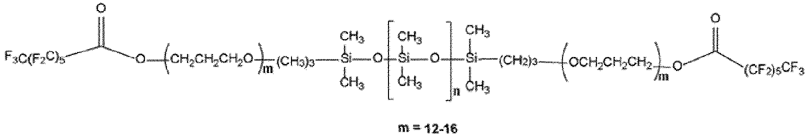

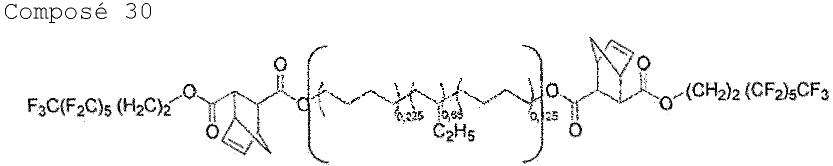

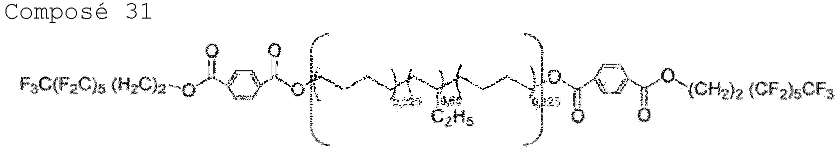

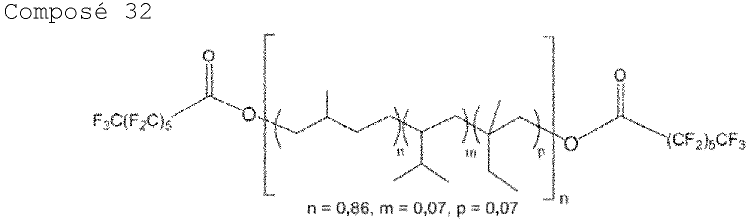

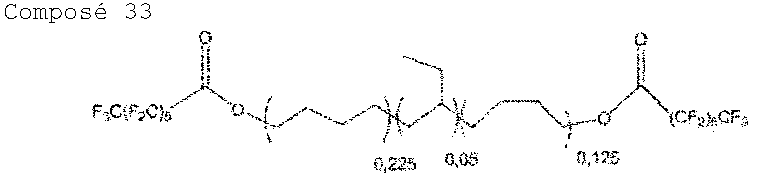

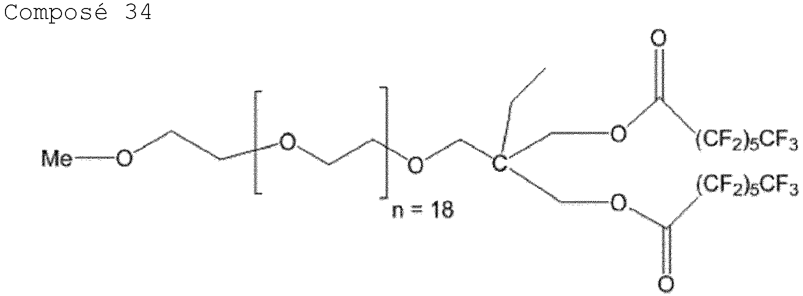

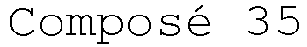

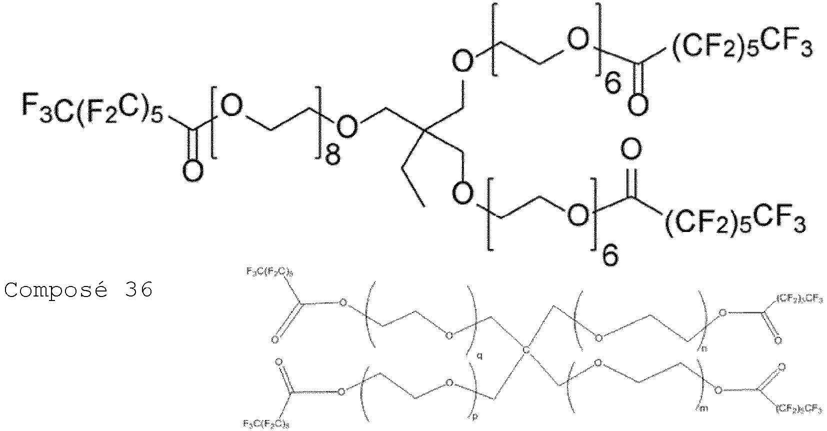

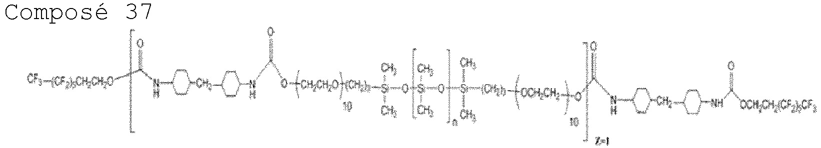

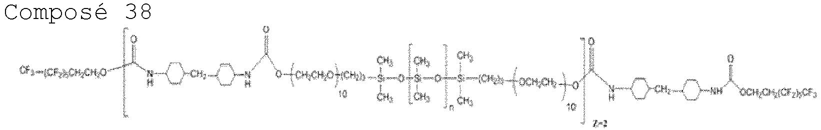

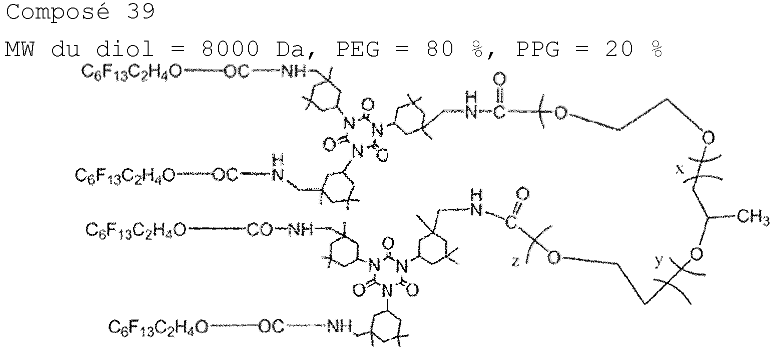

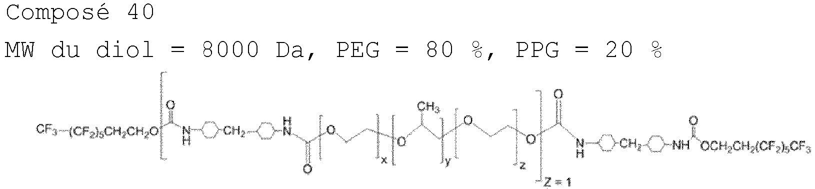

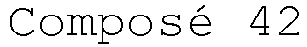

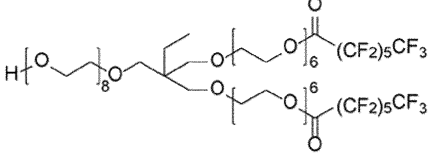

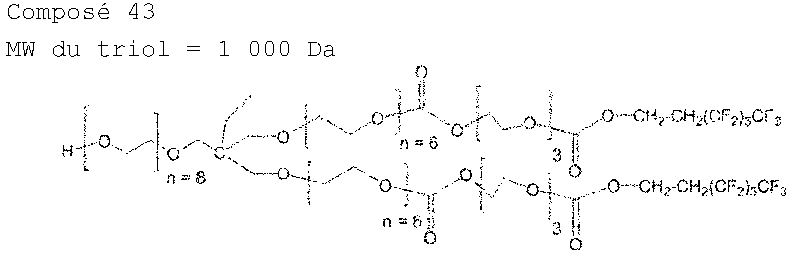

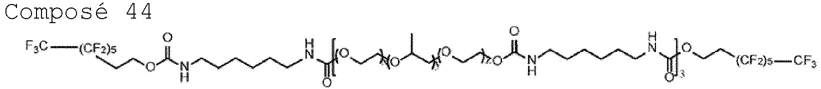

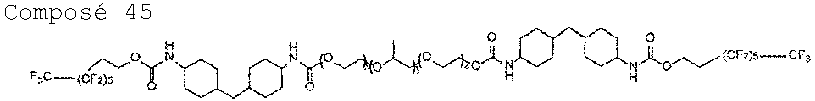

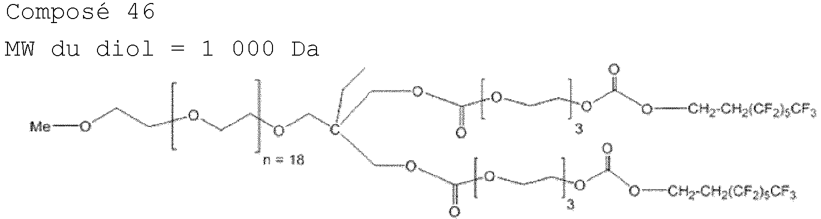

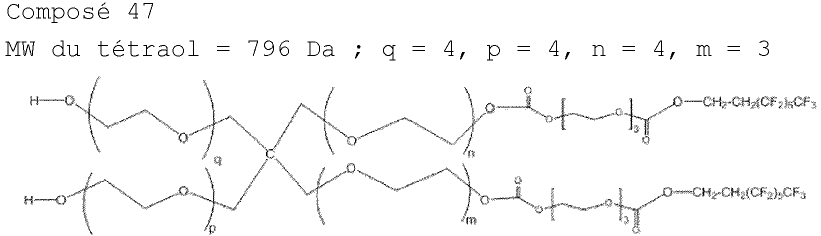

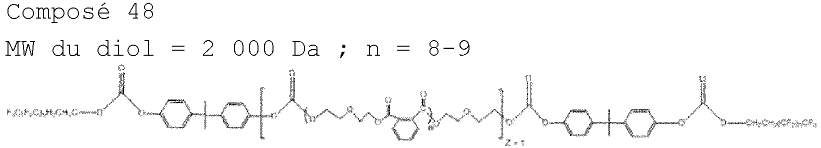

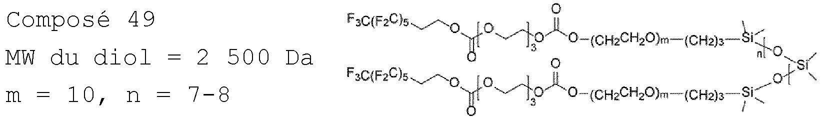

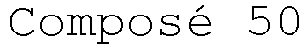

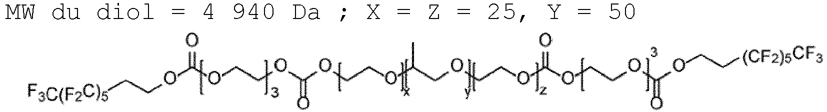

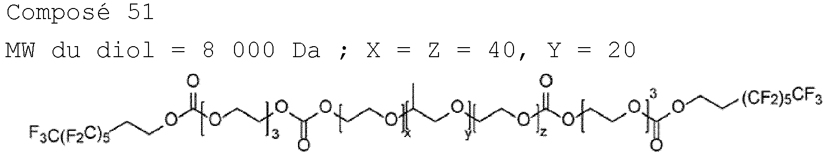

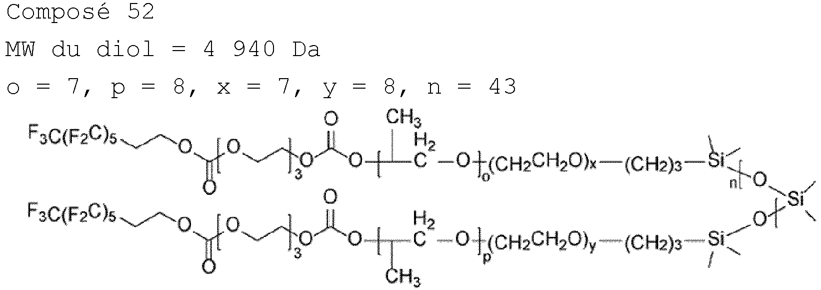

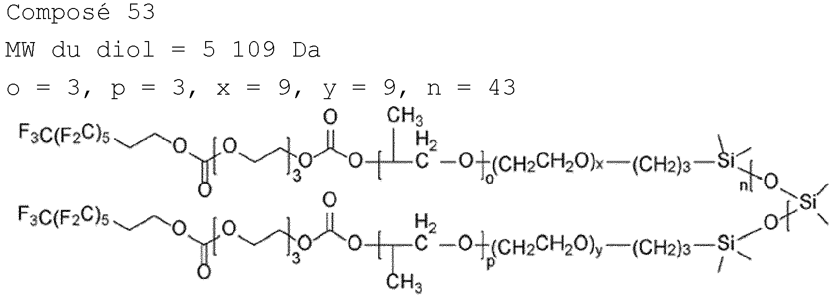

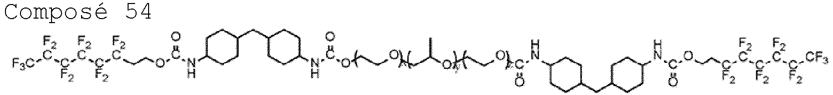

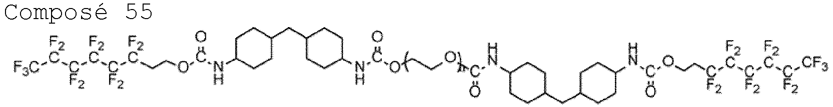

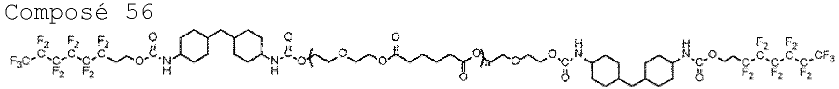

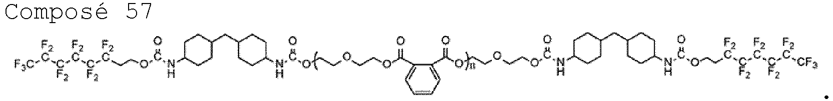

7. Utilisation selon l'une quelconque des revendications 1-6, le polymère de base
i) étant choisi dans le groupe composé de silicone, polyoléfine, polyester, polycarbonate, polysulfone, polyamide, polyéther, polyurée, polyuréthane (PU), polyétherimide, polystyrène, polymère cellulosique, polypropylène (PP), polyéthylène (PE), poly(chlorure de vinyle) (PVC), poly(alcool vinylique) (PVA), polyvinylpyrrolidone (PVP), polyacrylamide (PAAM), poly(oxyde d'éthylène) (PEO), poly(oxyde d'éthylène)-b-poly(oxyde de propylène)-b-poly(oxyde d'éthylène), poly(méthacrylate d'hydroxyéthyle) (polyHEMA), poly(téréphtalate d'éthylène) (PET), poly(téréphtalate de butylène) (PBT), poly(méthacrylate de méthyle) (PMMA), polyéther éther cétone (PEEK), polyéther-b-polyamide, un polymère d'oléfine cyclique (COP), un copolymère d'oléfine cyclique (COC), méthacrylate de méthyle acrylonitrile butadiène styrène (MABS), styrène acrylonitrile (SAN), styrène méthacrylate de méthyle (SMMA), méthacrylate butadiène styrène (MBS), styrène butadiène (SB), poly(styrène-*bloc*-isobutylène-*bloc-*styrène) (SIBS), et éthylène-acétate de vinyle (EVA) ; ou
ii) étant un polyuréthane (PU) ; ou
iii) étant une silicone (SI).

8. Utilisation selon l'une quelconque des revendications 1 à 7, la composition étant comprise dans un article choisi parmi :
(a) un dispositif médical choisi parmi des instruments médicaux, des dispositifs dentaires, des implants dentaires, des dispositifs d'administration de médicaments, des greffons, des stents, des stimulateurs cardiaques, des cardioverters-défibrillateurs implantables, des dispositifs de thérapie de resynchronisation cardiaque, des dérivations de dispositifs cardiovasculaires, des dispositifs d'assistance ventriculaire et des transmissions, des valves cardiaques, des filtres de veine cave, des antennes endovasculaires, des cathéters, des connecteurs de cathéter, des valves de cathéter, des lignes de distribution intraveineuse, des collecteurs de distribution intraveineuse, des shunts, des drains de plaie, des cathéters de drainage, des orifices de perfusion, des implants cochléaires, des tubes endotrachéaux, des tubes de trachéotomie, des tubes et circuits respiratoires de ventilateur, des capteurs implantables, des dispositifs ophtalmiques, des dispositifs orthopédiques, des implants dentaires, des implants parodontaux, des implants mammaires, des implants péniens, des implants maxillofaciaux, des implants cosmétiques, des valves, des appareils, des échafaudages, du matériel de suture, des aiguilles, des filets de réparation de hernie, du ruban vaginal sans tension et des élingues vaginales, des dispositifs neurologiques prothétiques et des tubes d'oreille, ou
(b) un pansement, un bandage, une gaze, un ruban adhésif, un tampon, une éponge, un oxygénateur de sang, un ventilateur, une pompe, un tube, un câblage, une électrode, un contraceptif, un produit d'hygiène féminine, un endoscope, une membrane de dialyse, un fil-guide, un sac de collecte de fluides, un sac et un tube d'administration de médicaments, un tube d'alimentation, un sac de sang ou un dispositif de régénération des tissus ou de culture cellulaire ; ou
(c) un cathéter, un cathéter de drainage, un stent, un shunt, un orifice de perfusion, une ligne d'administration intraveineuse, un appareil dentaire, un sac de sang, un implant mammaire, un implant pénien, un drain de plaie, un tube d'alimentation, un tube endotrachéal, un tube respiratoire, un tube auriculaire ou un endoscope.
